# EUROPEAN PATENT SPECIFICATION

(11) **EP 2 373 690 B1**
(45) Date of publication and mention of the grant of the patent: **11.02.2015**
(21) Application number: 09799402.4
(22) Date of filing: 08.12.2009
(51) Int. Cl.: C07K 16/28, C07K 14/705

(54) **ANTIBODIES SPECIFIC FOR TMEM154**
TMEM154-SPEZIFISCHE ANTIKÖRPER
ANTICORPS SPÉCIFIQUES DU TMEM154

(30) Priority: 08.12.2008 US 120540 P
(43) Date of publication of application: 12.10.2011
(60) Divisional application: 14195219.2
(73) Proprietor: Compugen Ltd., 69512 Tel Aviv (IL)
(72) Inventor: LEVINE, Zurit, Herzliya (IL); ROSENBERG, Avi Yeshah, 44622 Kfar-Saba (IL); ROTMAN, Galit, Herzeliya (IL); TOPORIK, Amir, Holon 58847 (IL); KINAR, Yaron, Tel-Aviv (IL); NEMZAR, Sergey, 43352 Raanana (IL); COHEN-DAYAG, Anat, Rehovot 76303 (IL); WALACH, Shira, Hod Ha Sharon (IL); SAMEACH-GREENWALD, Shirley, Kfar-Saba (IL); DASSA, Liat, 56478 Yahud (IL); BEIMAN, Merav, Nes Tsiyona (IL); MONTIA, Eve, Rehovot 76328 (IL); LEVY, Ofer, 99770 D.N. Shimshon (IL); NOVIK, Amit, 30500 Binyamina (IL)
(74) Representative: Fuchs Patentanwälte Partnerschaft mbB
(86) International application number: PCT/IB2009/055585
(87) International publication number: WO 2010/067308

(56) References cited:
- WO-A2-03/090694
- WO-A2-2004/110369

### SEQUENCE LISTING

<110> Compugen Ltd
<120> POLYPEPTIDES AND POLYNUCLEOTIDES, AND USES THEREOF AS A DRUG
   TARGET FOR PRODUCING DRUGS AND BIOLOGICS
<130> 0000
<160> 201
<210> 1
   <211> 916
   <212> DNA
   <213> Homo sapiens
<400> 1
   gggacggagg cggggcccag gttggggcgg ggccgggccc aggtacagcg gccctgcggc 60
   tggcgcggcg gacgggatga ggcgctgcag tctctgcgct ttcgacgccg cccgggggcc 120
   caggcggctg atgcgtgtgg gcctcgcgct gatcttggtg ggccacgtga acctgctgct 180
   gggggccgtg ctgcatggca ccgtcctgcg gcacgtggcc aatccccgcg gcgctgtcac 240
   gccggagtac accgtagcca atgtcatctc tgtcggctcg gggctgctga gcgtttccgt 300
   gggacttgtg gccctcctgg cgtccaggaa ccttcttcgc cctccactgc actgggtcct 360
   gctggcacta gctctggtga acctgctctt gtccgttgcc tgctccctgg gcctccttct 420
   tgctgtgtca ctcactgtgg ccaacggtgg ccgccgcctt attgctgact gccacccagg 480
   actgctggat cctctggtac cactggatga ggggccggga catactgact gcccctttga 540
   ccccacaaga atctatgata cagccttggc tctctggatc ccttctttgc tcatgtctgc 600
   aggggaggct gctctatctg gttactgctg tgtggctgca ctcactctac gtggagttgg 660
   gccctgcagg aaggacggac ttcaggggca gctagaggaa atgacagagc ttgaatctcc 720
   taaatgtaaa aggcaggaaa atgagcagct actggatcaa aatcaagaaa tccgggcatc 780
   acagagaagt tgggtttagg acagcaggtg ctgttccgag actcagtcct aaagggtttt 840
   ttttcccact aagcaagggg ccctgacctc gggatgagat aacaaattgt aataaagtaa 900
   cttctctttt cttcta 916
<210> 2
   <211> 78
   <212> DNA
   <213> Artificial
<220>
   <223> Synthetic polynucleotide
<400> 2
   gtgagcgcgg caggcgaccc gggcgggggc cgggctcccg gagagcccag caggccaaag 60
   gctttgtgtc ttccacag 78
<210> 3
   <211> 994
   <212> DNA
   <213> Homo sapiens
<400> 3
   gggacggagg cggggcccag gttggggcgg ggccgggccc aggtacagcg gccctgcggc     60
   tggcgcggcg gacgggatga ggcgctgcag tctctgcgct ttcgacgccg cccgggggcc    120
   caggcggctg atgcgtgtgg gcctcgcgct gatcttggtg ggccacgtga acctgctgct    180
   gggggccgtg ctgcatggca ccgtcctgcg gcacgtggcc aatccccgcg gcgctgtcac    240
   gccggagtac accgtagcca atgtcatctc tgtcggctcg gggctgctgg tgagcgcggc     300
   aggcgacccg ggcgggggcc gggctcccgg agagcccagc aggccaaagg ctttgtgtct    360
   tccacagagc gtttccgtgg gacttgtggc cctcctggcg tccaggaacc ttcttcgccc    420
   tccactgcac tgggtcctgc tggcactagc tctggtgaac ctgctcttgt ccgttgcctg    480
   ctccctgggc ctccttcttg ctgtgtcact cactgtggcc aacggtggcc gccgccttat    540
   tgctgactgc cacccaggac tgctggatcc tctggtacca ctggatgagg ggccgggaca    600
   tactgactgc ccctttgacc ccacaagaat ctatgataca gccttggctc tctggatccc    660
   ttctttgctc atgtctgcag gggaggctgc tctatctggt tactgctgtg tggctgcact     720
   cactctacgt ggagttgggc cctgcaggaa ggacggactt caggggcagc tagaggaaat    780
   gacagagctt gaatctccta aatgtaaaag gcaggaaaat gagcagctac tggatcaaaa    840
   tcaagaaatc cgggcatcac agagaagttg ggtttaggac agcaggtgct gttccgagac    900
   tcagtcctaa agggtttttt ttcccactaa gcaaggggcc ctgacctcgg gatgagataa    960
   caaattgtaa taaagtaact tctcttttct tcta                                994
<210> 4
   <211> 1332
   <212> DNA
   <213> Homo sapiens
<400> 4
   gggacggagg cggggcccag gttggggcgg ggccgggccc aggtacagcg gccctgcggc     60
   tggcgcggcg gacgggatga ggcgctgcag tctctgcgct ttcgacgccg cccgggggcc    120
   caggcggctg atgcgtgtgg gcctcgcgct gatcttggtg ggccacgtga acctgctgct    180
   gggggccgtg ctgcatggca ccgtcctgcg gcacgtggcc aatccccgcg gcgctgtcac    240
   gccggagtac accgtagcca atgtcatctc tgtcggctcg gggctgctga gcgtttccgt     300
   gggacttgtg gccctcctgg cgtccaggaa ccttcttcgc cctccactgc actgggtcct    360
   gctggcacta gctctggtga acctgctctt gtccgttgcc tgctccctgg gcctccttct    420
   tgctgtgtca ctcactgtgg ccaacggtgg ccgccgcctt attgctgact gccacccagg    480
   actgctggat cctctggtac cactggatga ggggccggga catactgact gcccctttga    540
   ccccacaaga atctatgata cagccttggc tctctggatc ccttctttgc tcatgtctgc    600
   aggggaggct gctctatctg gttactgctg tgtggctgca ctcactctac gtggagttgg    660
   gccctgcagg aaggacggac ttcaggggca ggtaaggaag gcaaacagga agggttcatt 720
   ccacagagac tggctgtgtt gaaaagatgc tgaccggccg ggtgcggagg ctcacacctg 780
   taatcccagc actttgggag gctgaggcag gcggatcact tgaggtcagg agtttgagac 840
   cagcttggac aacatggtga aacctcgtct ctactaaaaa tacaaaaaat taggtggggt 900
   ggtgggcgcc tgtaatccca gctacttgga aggctgaggc aggagaatcg cttgaacccg 960
   gaaggcggag gttgcagtga accgagatca cgccactgca ctccagcctg ggcaacagag 1020
   tgaggctccg tcacaaaaaa caaaacaaaa aaaagatgct gacctatcat tcagggcgag 1080
   ggtgtctaac tctatcttct tctgcagcta gaggaaatga cagagcttga atctcctaaa 1140
   tgtaaaaggc aggaaaatga gcagctactg gatcaaaatc aagaaatccg ggcatcacag 1200
   agaagttggg tttaggacag caggtgctgt tccgagactc agtcctaaag ggtttttttt 1260
   cccactaagc aaggggccct gacctcggga tgagataaca aattgtaata aagtaacttc 1320
   tcttttcttc ta 1332
<210> 5
   <211> 871
   <212> DNA
   <213> Homo sapiens
<400> 5
   gggacggagg cggggcccag gttggggcgg ggccgggccc aggtacagcg gccctgcggc 60
   tggcgcggcg gacgggatga ggcgctgcag tctctgcgct ttcgacgccg cccgggggcc 120
   caggcggctg atgcgtgtgg gcctcgcgct gatcttggtg ggccacgtga acctgctgct 180
   gggggccgtg ctgcatggca ccgtcctgcg gcacgtggcc aatccccgcg gcgctgtcac 240
   gccggagtac accgtagcca atgtcatctc tgtcggctcg gggctgctga gcgtttccgt 300
   gggacttgtg gccctcctgg cgtccaggaa ccttcttcgc cctccactgc actgggtcct 360
   gctggcacta gctctggtga acctgctctt gtccgttgcc tgctccctgg gcctccttct 420
   tgctgtgtca ctcactgtgg ccaacggtgg ccgccgcctt attgctgact gccacccagg 480
   actgctggat cctctggtac cactggatga ggggccggga catactgact gcccctttga 540
   ccccacaaga atctatgata cagccttggc tctctggatc ccttctttgc tcatgtctgc 600
   aggggaggct gctctatctg gttactgctg tgtggctgca ctcactctac gtggagttgg 660
   gccctgcagg aaggacggac ttcaggggca ggtagtagct gggtgtgacg caagagtgaa 720
   acagaaagcc tggcagccac ggtttcctgg gattaaagtc aaagcattat gaatatggca 780
   ctaaagtgac tgagctacca gaccaatgat cctgtaaggc agccacagaa ctaaaaaaca 840
   acaattatta ttaaactgct ctggattctc a 871
<210> 6
   <211> 709
   <212> DNA
   <213> Homo sapiens
<400> 6
   gggacggagg cggggcccag gttggggcgg ggccgggccc aggtacagcg gccctgcggc      60
   tggcgcggcg gacgggatga ggcgctgcag tctctgcgct ttcgacgccg cccgggggcc     120
   caggcggctg atgcgtgtgg gcctcgcgct gatcttggtg ggccacgtga acctgctgct     180
   gggggccgtg ctgcatggca ccgtcctgcg gcacgtggcc aatccccgcg gcgctgtcac     240
   gccggagtac accgtagcca atgtcatctc tgtcggctcg gggctgctga gcgtttccgt     300
   gggacttgtg gccctcctgg cgtccaggaa ccttcttcgc cctccactgg atacagcctt     360
   ggctctctgg atcccttctt tgctcatgtc tgcaggggag gctgctctat ctggttactg     420
   ctgtgtggct gcactcactc tacgtggagt tgggccctgc aggaaggacg gacttcaggg     480
   gcagctagag gaaatgacag agcttgaatc tcctaaatgt aaaaggcagg aaaatgagca     540
   gctactggat caaaatcaag aaatccgggc atcacagaga agttgggttt aggacagcag     600
   gtgctgttcc gagactcagt cctaaagggt tttttttccc actaagcaag gggccctgac      660
   ctcgggatga gataacaaat tgtaataaag taacttctct tttcttcta                 709
<210> 7
   <211> 240
   <212> PRT
   <213> Homo sapiens
<400> 7
   Met Arg Arg Cys Ser Leu Cys Ala Phe Asp Ala Ala Arg Gly Pro Arg
   1               5                   10                  15
   Arg Leu Met Arg Val Gly Leu Ala Leu Ile Leu Val Gly His Val Asn
               20                  25                  30
   Leu Leu Leu Gly Ala Val Leu His Gly Thr Val Leu Arg His Val Ala
           35                  40                  45
   Asn Pro Arg Gly Ala Val Thr Pro Glu Tyr Thr Val Ala Asn Val Ile
       50                  55                  60
   Ser Val Gly Ser Gly Leu Leu Ser Val Ser Val Gly Leu Val Ala Leu
   65                  70                  75                  80
   Leu Ala Ser Arg Asn Leu Leu Arg Pro Pro Leu His Trp Val Leu Leu
                   85                  90                  95
   Ala Leu Ala Leu Val Asn Leu Leu Leu Ser Val Ala Cys Ser Leu Gly
               100                 105                 110
   Leu Leu Leu Ala Val Ser Leu Thr Val Ala Asn Gly Gly Arg Arg Leu
           115                 120                 125
   Ile Ala Asp Cys His Pro Gly Leu Leu Asp Pro Leu Val Pro Leu Asp
       130                 135                 140
   Glu Gly Pro Gly His Thr Asp Cys Pro Phe Asp Pro Thr Arg Ile Tyr
   145                 150                 155                 160
   Asp Thr Ala Leu Ala Leu Trp Ile Pro Ser Leu Leu Met Ser Ala Gly
                   165                 170                 175
   Glu Ala Ala Leu Ser Gly Tyr Cys Cys Val Ala Ala Leu Thr Leu Arg
               180                 185                 190
   Gly Val Gly Pro Cys Arg Lys Asp Gly Leu Gln Gly Gln Leu Glu Glu
           195                 200                 205
   Met Thr Glu Leu Glu Ser Pro Lys Cys Lys Arg Gln Glu Asn Glu Gln
       210                 215                 220
   Leu Leu Asp Gln Asn Gln Glu Ile Arg Ala Ser Gln Arg Ser Trp Val
   225                 230                 235                 240
<210> 8
   <211> 240
   <212> PRT
   <213> Homo sapiens
<400> 8
   Met Arg Arg Cys Ser Leu Cys Ala Phe Asp Ala Ala Arg Gly Pro Arg
   1               5                   10                  15
   Arg Leu Met Arg Val Gly Leu Ala Leu Ile Leu Val Gly His Val Asn
               20                  25                  30
   Leu Leu Leu Gly Ala Val Leu His Gly Thr Val Leu Arg His Val Ala
           35                  40                  45
   Asn Pro Arg Gly Ala Val Thr Pro Glu Tyr Thr Val Ala Asn Val Ile
       50                  55                  60
   Ser Val Gly Ser Gly Leu Leu Ser Val Ser Val Gly Leu Val Thr Leu
   65                  70                  75                  80
   Leu Ala Ser Arg Asn Leu Leu Arg Pro Pro Leu His Trp Val Leu Leu
                   85                  90                  95
   Ala Leu Ala Leu Val Asn Leu Leu Leu Ser Val Ala Cys Ser Leu Gly
               100                 105                 110
   Leu Leu Leu Ala Val Ser Leu Thr Val Ala Asn Gly Gly Arg Arg Leu
           115                 120                 125
   Ile Ala Asp Cys His Pro Gly Leu Leu Asp Pro Leu Val Pro Leu Asp
       130                 135                 140
   Glu Gly Pro Gly His Thr Asp Cys Pro Phe Asp Pro Thr Arg Ile Tyr
   145                 150                 155                 160
   Asp Thr Ala Leu Ala Leu Trp Ile Pro Ser Leu Leu Met Ser Ala Gly
                   165                 170                 175
   Glu Ala Ala Leu Ser Gly Tyr Cys Cys Val Ala Ala Leu Thr Leu Arg
               180                 185                 190
   Gly Val Gly Pro Cys Arg Lys Asp Gly Leu Gln Gly Gln Leu Glu Glu
           195                 200                 205
   Met Thr Glu Leu Glu Ser Pro Lys Cys Lys Arg Gln Glu Asn Glu Gln
       210                 215                 220
   Leu Leu Asp Gln Asn Gln Glu Ile Arg Ala Ser Gln Arg Ser Trp Val
   225                 230                 235                 240
<210> 9
   <211> 48
   <212> DNA
   <213> Artificial
<220>
   <223> Synthetic polynucleotide
<400> 9
   gtaaggaagg caaacaggaa gggttcattc cacagagact ggctgtgt 48
<210> 10
   <211> 266
   <212> PRT
   <213> Homo sapiens
<400> 10
   Met Arg Arg Cys Ser Leu Cys Ala Phe Asp Ala Ala Arg Gly Pro Arg
   1 5 10 15
   Arg Leu Met Arg Val Gly Leu Ala Leu Ile Leu Val Gly His Val Asn
   20 25 30
   Leu Leu Leu Gly Ala Val Leu His Gly Thr Val Leu Arg His Val Ala
   35 40 45
   Asn Pro Arg Gly Ala Val Thr Pro Glu Tyr Thr Val Ala Asn Val Ile
   50 55 60
   Ser Val Gly Ser Gly Leu Leu Val Ser Ala Ala Gly Asp Pro Gly Gly
   65 70 75 80
   Gly Arg Ala Pro Gly Glu Pro Ser Arg Pro Lys Ala Leu Cys Leu Pro
   85 90 95
   Gln Ser Val Ser Val Gly Leu Val Ala Leu Leu Ala Ser Arg Asn Leu
   100 105 110
   Leu Arg Pro Pro Leu His Trp Val Leu Leu Ala Leu Ala Leu Val Asn
   115 120 125
   Leu Leu Leu Ser Val Ala Cys Ser Leu Gly Leu Leu Leu Ala Val Ser
   130 135 140
   Leu Thr Val Ala Asn Gly Gly Arg Arg Leu Ile Ala Asp Cys His Pro
   145 150 155 160
   Gly Leu Leu Asp Pro Leu Val Pro Leu Asp Glu Gly Pro Gly His Thr
   165 170 175
   Asp Cys Pro Phe Asp Pro Thr Arg Ile Tyr Asp Thr Ala Leu Ala Leu
   180 185 190
   Trp Ile Pro Ser Leu Leu Met Ser Ala Gly Glu Ala Ala Leu Ser Gly
   195 200 205
   Tyr Cys Cys Val Ala Ala Leu Thr Leu Arg Gly Val Gly Pro Cys Arg
   210 215 220
   Lys Asp Gly Leu Gln Gly Gln Leu Glu Glu Met Thr Glu Leu Glu Ser
   225 230 235 240
   Pro Lys Cys Lys Arg Gln Glu Asn Glu Gln Leu Leu Asp Gln Asn Gln
   245 250 255
   Glu Ile Arg Ala Ser Gln Arg Ser Trp Val
   260 265
<210> 11
   <211> 221
   <212> PRT
   <213> Homo sapiens
<400> 11
   Met Arg Arg Cys Ser Leu Cys Ala Phe Asp Ala Ala Arg Gly Pro Arg
   1 5 10 15
   Arg Leu Met Arg Val Gly Leu Ala Leu Ile Leu Val Gly His Val Asn
   20 25 30
   Leu Leu Leu Gly Ala Val Leu His Gly Thr Val Leu Arg His Val Ala
   35 40 45
   Asn Pro Arg Gly Ala Val Thr Pro Glu Tyr Thr Val Ala Asn Val Ile
   50 55 60
   Ser Val Gly Ser Gly Leu Leu Ser Val Ser Val Gly Leu Val Ala Leu
   65 70 75 80
   Leu Ala Ser Arg Asn Leu Leu Arg Pro Pro Leu His Trp Val Leu Leu
   85 90 95
   Ala Leu Ala Leu Val Asn Leu Leu Leu Ser Val Ala Cys Ser Leu Gly
   100 105 110
   Leu Leu Leu Ala Val Ser Leu Thr Val Ala Asn Gly Gly Arg Arg Leu
   115 120 125
   Ile Ala Asp Cys His Pro Gly Leu Leu Asp Pro Leu Val Pro Leu Asp
   130 135 140
   Glu Gly Pro Gly His Thr Asp Cys Pro Phe Asp Pro Thr Arg Ile Tyr
   145 150 155 160
   Asp Thr Ala Leu Ala Leu Trp Ile Pro Ser Leu Leu Met Ser Ala Gly
   165 170 175
   Glu Ala Ala Leu Ser Gly Tyr Cys Cys Val Ala Ala Leu Thr Leu Arg
   180 185 190
   Gly Val Gly Pro Cys Arg Lys Asp Gly Leu Gln Gly Gln Val Arg Lys
   195 200 205
   Ala Asn Arg Lys Gly Ser Phe His Arg Asp Trp Leu Cys
   210 215 220
<210> 12
   <211> 231
   <212> PRT
   <213> Homo sapiens
<400> 12
   Met Arg Arg Cys Ser Leu Cys Ala Phe Asp Ala Ala Arg Gly Pro Arg
   1 5 10 15
   Arg Leu Met Arg Val Gly Leu Ala Leu Ile Leu Val Gly His Val Asn
   20 25 30
   Leu Leu Leu Gly Ala Val Leu His Gly Thr Val Leu Arg His Val Ala
   35 40 45
   Asn Pro Arg Gly Ala Val Thr Pro Glu Tyr Thr Val Ala Asn Val Ile
   50 55 60
   Ser Val Gly Ser Gly Leu Leu Ser Val Ser Val Gly Leu Val Ala Leu
   65 70 75 80
   Leu Ala Ser Arg Asn Leu Leu Arg Pro Pro Leu His Trp Val Leu Leu
                   85                  90                  95
   Ala Leu Ala Leu Val Asn Leu Leu Leu Ser Val Ala Cys Ser Leu Gly
               100                 105                 110
   Leu Leu Leu Ala Val Ser Leu Thr Val Ala Asn Gly Gly Arg Arg Leu
           115                 120                 125
   Ile Ala Asp Cys His Pro Gly Leu Leu Asp Pro Leu Val Pro Leu Asp
       130                 135                 140
   Glu Gly Pro Gly His Thr Asp Cys Pro Phe Asp Pro Thr Arg Ile Tyr
   145                 150                 155                 160
   Asp Thr Ala Leu Ala Leu Trp Ile Pro Ser Leu Leu Met Ser Ala Gly
                   165                 170                 175
   Glu Ala Ala Leu Ser Gly Tyr Cys Cys Val Ala Ala Leu Thr Leu Arg
               180                 185                 190
   Gly Val Gly Pro Cys Arg Lys Asp Gly Leu Gln Gly Gln Val Val Ala
           195                 200                 205
   Gly Cys Asp Ala Arg Val Lys Gln Lys Ala Trp Gln Pro Arg Phe Pro
       210                 215                 220
   Gly Ile Lys Val Lys Ala Leu
   225                 230
<210> 13
   <211> 171
   <212> PRT
   <213> Homo sapiens
<400> 13
   Met Arg Arg Cys Ser Leu Cys Ala Phe Asp Ala Ala Arg Gly Pro Arg
   1 5 10 15
   Arg Leu Met Arg Val Gly Leu Ala Leu Ile Leu Val Gly His Val Asn
   20 25 30
   Leu Leu Leu Gly Ala Val Leu His Gly Thr Val Leu Arg His Val Ala
   35 40 45
   Asn Pro Arg Gly Ala Val Thr Pro Glu Tyr Thr Val Ala Asn Val Ile
   50 55 60
   Ser Val Gly Ser Gly Leu Leu Ser Val Ser Val Gly Leu Val Ala Leu
   65 70 75 80
   Leu Ala Ser Arg Asn Leu Leu Arg Pro Pro Leu Asp Thr Ala Leu Ala
   85 90 95
   Leu Trp Ile Pro Ser Leu Leu Met Ser Ala Gly Glu Ala Ala Leu Ser
   100 105 110
   Gly Tyr Cys Cys Val Ala Ala Leu Thr Leu Arg Gly Val Gly Pro Cys
   115 120 125
   Arg Lys Asp Gly Leu Gln Gly Gln Leu Glu Glu Met Thr Glu Leu Glu
   130 135 140
   Ser Pro Lys Cys Lys Arg Gln Glu Asn Glu Gln Leu Leu Asp Gln Asn
   145 150 155 160
   Gln Glu Ile Arg Ala Ser Gln Arg Ser Trp Val
   165 170
<210> 14
   <211> 1252
   <212> DNA
   <213> Homo sapiens
<400> 14
   gaaggagatc aggcagagga gagaagagtc agtgtgaact aggctggtgg ctctgtccgg      60
   tagggagacg gaaagggtgc cgcccatcat agaagtacca gaacttgagc tggactttgc     120
   tgatttagct tatggaagag gaaccagaaa tttgtccttg aataatgttt cccgacaacg     180
   aagaggcaga aggatctggg cctgtgcgcg acgccccggg ggacgaggct catggagaag     240
   tttcgggcgg tgctggacct gcacgtcaag caccacagcg ccttgggcta cggcctggtg     300
   accctgctga cggcgggcgg ggagcgcatc ttctccgccg tggcattcca gtgcccgtgc     360
   agcgccgcct ggaacctgcc ctacggcctg gtcttcttgc tggtgccggc gctcgcgctc     420
   ttcctcctgg gctacgtgct gagcgcacgc acgtggcgcc tgctcaccgg atgctgctcc     480
   agcgcccgcg cgagttgcgg atcggcgctg cgcggctccc tggtgtgcgc gcaaatcagc     540
   gcggccgccg cgctcgcgcc cctcacctgg gtggccgtgg cgctgctcgg gggcgccttt     600
   tacgagtgcg cggccaccgg gagcgcggcc ttcgcgcagc gcctgtgcct cggccgcaac     660
   cgcagctgcg ccgcggagct gccgctggtg ccgtgcaacc aggccaaggc gtcggacgtg     720
   caggacctcc tgaaggatct gaaggctcag tcgcaggtgt tgggctggat cttgatagca     780
   gttgttatca tcattcttct gatttttaca tctgtcaccc gatgcctatc tccagttagt     840
   tttctgcagc tgaaattctg gaaaatctat ttggaacagg agcagcagat ccttaaaagt     900
   aaagccacag agcatgcaac tgaattggca aaagagaata ttaaatgttt ctttgagggc     960
   tcgcatccaa aagaatataa cactccaagc atgaaagagt ggcagcaaat ttcatcactg    1020
   tatactttca atccgaaggg ccagtactac agcatgttgc acaaatatgt caacagaaaa    1080
   gagaagactc acagtatcag gtctactgaa ggagatacgg tgattcctgt tcttggcttt    1140
   gtagattcat ctggtataaa cagcactcct gagttatgac cttttgaatg agtagaaaaa    1200
   aaaattgttt tgaattattg ctttattaaa aaataaacat tggtattttt tg            1252
<210> 15
   <211> 315
   <212> PRT
   <213> Homo sapiens
<400> 15
   Met Glu Lys Phe Arg Ala Val Leu Asp Leu His Val Lys His His Ser
   1               5                   10                  15
   Ala Leu Gly Tyr Gly Leu Val Thr Leu Leu Thr Ala Gly Gly Glu Arg
               20                  25                  30
   Ile Phe Ser Ala Val Ala Phe Gln Cys Pro Cys Ser Ala Ala Trp Asn
           35                  40                  45
   Leu Pro Tyr Gly Leu Val Phe Leu Leu Val Pro Ala Leu Ala Leu Phe
       50                  55                  60
   Leu Leu Gly Tyr Val Leu Ser Ala Arg Thr Trp Arg Leu Leu Thr Gly
   65                  70                  75                  80
   Cys Cys Ser Ser Ala Arg Ala Ser Cys Gly Ser Ala Leu Arg Gly Ser
                   85                  90                  95
   Leu Val Cys Thr Gln Ile Ser Ala Ala Ala Ala Leu Ala Pro Leu Thr
               100                 105                 110
   Trp Val Ala Val Ala Leu Leu Gly Gly Ala Phe Tyr Glu Cys Ala Ala
           115                 120                 125
   Thr Gly Ser Ala Ala Phe Ala Gln Arg Leu Cys Leu Gly Arg Asn Arg
       130                 135                 140
   Ser Cys Ala Ala Glu Leu Pro Leu Val Pro Cys Asn Gln Ala Lys Ala
   145                 150                 155                 160
   Ser Asp Val Gln Asp Leu Leu Lys Asp Leu Lys Ala Gln Ser Gln Val
                   165                 170                 175
   Leu Gly Trp Ile Leu Ile Ala Val Val Ile Ile Ile Leu Leu Ile Phe
               180                 185                 190
   Thr Ser Val Thr Arg Cys Leu Ser Pro Val Ser Phe Leu Gln Leu Lys
           195                 200                 205
   Phe Trp Lys Ile Tyr Leu Glu Gln Glu Gln Gln Ile Leu Lys Ser Lys
       210                 215                 220
   Ala Thr Glu His Ala Thr Glu Leu Ala Lys Glu Asn Ile Lys Cys Phe
   225                 230                 235                 240
   Phe Glu Gly Ser His Pro Lys Glu Tyr Asn Thr Pro Ser Met Lys Glu
                   245                 250                 255
   Trp Gln Gln Ile Ser Ser Leu Tyr Thr Phe Asn Pro Lys Gly Gln Tyr
               260                 265                 270
   Tyr Ser Met Leu His Lys Tyr Val Asn Arg Lys Glu Lys Thr His Ser
           275                 280                 285
   Ile Arg Ser Thr Glu Gly Asp Thr Val Ile Pro Val Leu Gly Phe Val
       290                 295                 300
   Asp Ser Ser Gly Ile Asn Ser Thr Pro Glu Leu
   305                 310                 315
<210> 16
   <211> 143
   <212> PRT
   <213> Homo sapiens
<400> 16
   Met Phe Pro Val Leu Gly Trp Ile Leu Ile Ala Val Val Ile Ile Ile
   1 5 10 15
   Leu Leu Ile Phe Thr Ser Val Thr Arg Cys Leu Ser Pro Val Ser Phe
   20 25 30
   Leu Gln Leu Lys Phe Trp Lys Ile Tyr Leu Glu Gln Glu Gln Gln Ile
   35 40 45
   Leu Lys Ser Lys Ala Thr Glu His Ala Thr Glu Leu Ala Lys Glu Asn
   50 55 60
   Ile Lys Cys Phe Phe Glu Gly Ser His Pro Lys Glu Tyr Asn Thr Pro
   65 70 75 80
   Ser Met Lys Glu Trp Gln Gln Ile Ser Ser Leu Tyr Thr Phe Asn Pro
   85 90 95
   Lys Gly Gln Tyr Tyr Ser Met Leu His Lys Tyr Val Asn Arg Lys Glu
   100 105 110
   Lys Thr His Ser Ile Arg Ser Thr Glu Gly Asp Thr Val Ile Pro Val
   115 120 125
   Leu Gly Phe Val Asp Ser Ser Gly Ile Asn Ser Thr Pro Glu Leu
   130 135 140
<210> 17
   <211> 158
   <212> PRT
   <213> Homo sapiens
<400> 17
   Arg Trp Gly Arg Leu Gly Gly Ala Glu Arg Pro Ser Phe Leu Arg Ala
   1 5 10 15
   Ala Gly Val Leu Gly Trp Ile Leu Ile Ala Val Val Ile Ile Ile Leu
   20 25 30
   Leu Ile Phe Thr Ser Val Thr Arg Cys Leu Ser Pro Val Ser Phe Leu
   35 40 45
   Gln Leu Lys Phe Trp Lys Ile Tyr Leu Glu Gln Glu Gln Gln Ile Leu
   50 55 60
   Lys Ser Lys Ala Thr Glu His Ala Thr Glu Leu Ala Lys Glu Asn Ile
   65 70 75 80
   Lys Cys Phe Phe Glu Gly Ser His Pro Lys Glu Tyr Asn Thr Pro Ser
   85 90 95
   Met Lys Glu Trp Gln Gln Ile Ser Ser Leu Tyr Thr Phe Asn Pro Lys
   100 105 110
   Gly Gln Tyr Tyr Ser Met Leu His Lys Tyr Val Asn Arg Lys Glu Lys
   115 120 125
   Thr His Ser Ile Arg Ser Thr Glu Gly Asp Thr Val Ile Pro Val Leu
   130 135 140
   Gly Phe Val Asp Ser Ser Gly Ile Asn Ser Thr Pro Glu Leu
   145 150 155
<210> 18
   <211> 315
   <212> PRT
   <213> Homo sapiens
<400> 18
<210> 19
   <211> 194
   <212> PRT
   <213> Homo sapiens
<400> 19
   Met Ala Ser Leu Trp Pro Ser Ala Leu Thr Phe Asn Thr Asp Ala Asn
   1               5                   10                  15
   Ile Pro Gly Pro Leu Gly Phe Cys Gly Gly Trp Val Arg Leu Cys Ser
               20                  25                  30
   Leu Ser Ser Leu Thr Pro Pro Cys Gly Arg Arg Leu Val Pro Cys Leu
           35                  40                  45
   Ser Ala Pro Ala Pro Asn Ala Pro Arg Leu Pro Ala Pro Ala Arg Cys
       50                  55                  60
   Ser Ile Gly Ala Leu Ile Gly Leu Ser Val Ala Ala Val Val Leu Leu
   65                  70                  75                  80
   Ala Phe Ile Val Thr Ala Cys Val Leu Cys Tyr Leu Phe Ile Ser Ser
                   85                  90                  95
   Lys Pro His Thr Lys Leu Asp Leu Gly Leu Ser Leu Gln Thr Ala Gly
               100                 105                 110
   Pro Glu Glu Val Ser Pro Asp Cys Gln Gly Val Asn Thr Gly Met Ala
           115                 120                 125
   Ala Glu Val Pro Lys Val Ser Pro Leu Gln Gln Ser Tyr Ser Cys Leu
       130                 135                 140
   Asn Pro Gln Leu Glu Ser Asn Glu Gly Gln Ala Val Asn Ser Lys Arg
   145                 150                 155                 160
   Leu Leu His His Cys Phe Met Ala Thr Val Thr Thr Ser Asp Ile Pro
                   165                 170                 175
   Gly Ser Pro Glu Glu Ala Ser Val Pro Asn Pro Asp Leu Cys Gly Pro
               180                 185                 190
   Val Pro
<210> 20
   <211> 614
   <212> DNA
   <213> Homo sapiens
<400> 20
   atggcttctc tctggccctc agctctcacc tttaacacgg atgcgaacat ccccgggccc 60
   ctgggttttt gcggaggttg ggtgaggttg tgttcactct cctcactgac cccgccgtgt 120
   ggtcgccggc tggtgccttg tctgtctgcc ccagccccga atgccccgag actcccggcg 180
   ccggctcggt gcagcattgg cgctctcata ggcctgtccg tagcagcagt ggttcttctc 240
   gccttcattg ttaccgcctg tgtgctctgc tacctgttca tcagctctaa gccccacaca 300
   aagttggacc tgggcttgag cttacagaca gcaggccctg aggaggtttc tcctgactgc 360
   caaggtgtga acacaggcat ggcggcagaa gtgccaaaag tgagccctct ccagcagagt 420
   tactcctgct tgaacccgca gctggagagc aatgaggggc aggctgtgaa ctccaaacgc 480
   ctcctccatc attgcttcat ggccacagtg accaccagtg acattccagg cagccctgag 540
   gaagcctctg tacccaaccc tgacctatgt ggaccagtcc cataaacatt caataaatgt 600
   ctccatacca tccc 614
<210> 21
   <211> 3517
   <212> DNA
   <213> Homo sapiens
<400> 21
   acgcggctgc acggaccgcc aatgctgctg cctcccagag cagagggagg cagaagagga 60
   gggagggaga agagtggaag ggagggggag gcggcacttg ggctgcagct cgcaaccaga 120
   ggcagtgtct ctcagctctc ggaaggaccg agagaggcaa ccgcggagta ggatgctctg 180
   cggggcgccc agagccgggg gcgcttgaag cagcttcggg gactggggca accgggcagt 240
   tttcaccatg catcagtccc tgactcagca gcggtccagc gacatgtccc tgcccgattc 300
   catgggagca ttcaatcgga ggaaacgaaa ctccatctat gtcaccgtga ctttgcttat 360
   tgtgtccgtg ttaattctca cagtgggcct tgctgcaacc accaggaccc agaatgtgac 420
   tgtaggaggt tattaccccg gagttattct cggctttgga tcgttccttg gaatcattgg 480
   atcaaacctt attgagaaca aaaggcagat gctggtggct tctatcgtgt ttatcagctt 540
   tggtgtgatt gcggcttttt gttgtgccat agttgacggg gtctttgctg ccagacacat 600
   tgatctgaaa ccactctacg ctaaccggtg ccattatgtt cccaagacat cacagaagga 660
   agctgaggag gtcataagtt cctcaaccaa aaattctcct tccacgaggg ttatgaggaa 720
   ccttacccag gcagctagag aggttaactg ccctcacctc agccgtgaat tctgcacacc 780
   tcgcatccgg ggcaacacct gcttctgctg tgacctctac aactgtggca accgggtgga 840
   gatcactggt gggtactacg aatacatcga tgtcagcagt tgccaagata tcatccacct 900
   ctaccacctg ctctggtctg ccaccatcct caacattgtt ggcctgttcc tgggcatcat 960
   cactgccgct gtccttggag gctttaagga catgaaccca actctcccag cactgaactg 1020
   ttctgttgaa aatacccatc caacagtttc ttactatgct catccccaag tggcatccta 1080
   caatacctac taccatagcc ctcctcacct gccaccatat tctgcttatg actttcagca 1140
   ttccggtgtc tttccatcct cccctccctc tggactttct gatgagcccc agtctgcctc 1200
   tccctcaccc agctacatgt ggtcctcaag tgcaccgccc cgttactctc caccctacta 1260
   tccacctttt gaaaagccac caccttacag tccctaaaga ggaatgcctg ctggctattg 1320
   agattattgt ggcttttgta tttctgcttc agtggaagtg tgtagggtac aaaatttaaa 1380
   gtgtgactct tatgcataaa gttttacaat ggcctgccag gctagggaaa gatagggacg 1440
   aagcttattc attattagtg cagagcaggg gtggtcaggc tgaacgcagc acagaagggc 1500
   agctcacatt ctctaagcaa gactggggag ccagcccagc aagaagcttg tttggacttg 1560
   cattacccta tgctccacct ctgtattcag cagaagtgtg gttgccatct ttttcacttt 1620
   atgtaaagga gtgttgccct cgggcccttg gcagattgcc accccagcac ctaggttgaa 1680
   gcacctggtt tataggccct atctttccct acccctaaag tcagtcccta aggacaattt 1740
   cccagctgat ggggctacac agtagttcca atacagagag ttctggctaa gattttgttt 1800
   gcttgtgtct ggatgttgaa aaagactgcc cgtatctctt actccttcct tctctgtgag 1860
   tattgtaaaa atggctgttg tgatcactca gctcagcttt tgttattggt acctcctaaa 1920
   gggaaaagtg caatattctt gcatcttcag tagtggggaa caggatgtat tgttccggaa 1980
   acactgaaat acacagcaac atgtgagatg ttttaagtag atcacttagg agacagtggt 2040
   tctactacat gttgcattat tacaaaatac atttgctaca ggagatataa atcttatggt 2100
   tgtaattcag agtttaaaaa tgttataaat taggttcttg ggtcgtgata tgaattgtta 2160
   ctaatctttg tgactattta atcttcaaat attgtgctta accccagcaa tccgcacgta 2220
   tcctgcaccc caccccaaaa gagtcatctg tattttaatg ccactggtct tatcggtcct 2280
   tttgtctgtt gagaccagtc atgacagcat tcaagattat gaaagtgtta caatgccgct 2340
   tcaagtctgc aaaacctcaa acgtagccaa cttgacaaat atttaagtgt tacggcagat 2400
   ttaaaatcca tctggcacac cgtggtaggt atttgtacag ttcttttaat tacacatagc 2460
   tttaaaccat caacctgatg agtttaaagc ttttgcaccc atgccttcac ttcagaatga 2520
   acaccttcat tgtgatctta tgttaacctg agaattgatt taaaggaaga ttgataatcc 2580
   tatactttat aacgtaaaaa tacaggggct acaggagggt acctaattag acagttctcc 2640
   aaacacagaa cacacactgg aaaattttcc ggccaatttt gctacctccc aacttgatgg 2700
   attagaggta gcgcaaatgc tggtgctccc atctaccttg tagacactta gccatcaaga 2760
   atcaaggcac aagaagtgca ctctctcatt aacagtaaat gtttgcaaga tattcagttt 2820
   aactttcagc atcatgaatg ttcttatcca gattttgaat ccgaaaaact ataatccttt 2880
   tatgttatac aaaattacta tgatttttta cagttctgag catattaaaa ttctactgga 2940
   tttcaaaaag agactaatac ccaactgact aactaaacaa atatcaactt gtaatactca 3000
   atgaattttt ttgccattta catttgaccg ttggctttag tgaatgtcca tatttaattt 3060
   tttaaggcac cattacacag tttatcctac atttatcaca tttcttaaag tgttaagatt 3120
   ctatggctca tttctatgta tttttcttac tttacaaaat aacctgaaac agtatagatt 3180
   ttgtaacact taatttgagc agctttttta ttacattgaa ttatataaag tgcatgttac 3240
   cttagaaaaa ttaatatttg ctgctttact cttttgcaaa acatttgctg taatgaatgg 3300
   atttgtattt ccaatatgta tcttgactgc attttgtaat atttactgct ttattcctaa 3360
   ttctgcttta aagtactgaa ctgggcatga aacattaaaa tattaatcca gaaactgtat 3420
   aaactggatg ttgcttaaaa tctgtatcac tgccatgttg aaaactcaga ctgcttttgt 3480
   gatgtttcaa atgaataaaa ctatcctccc ctcgtta 3517
<210> 22
   <211> 3445
   <212> DNA
   <213> Homo sapiens
<400> 22
   acgcggctgc acggaccgcc aatgctgctg cctcccagag cagagggagg cagaagagga     60
   gggagggaga agagtggaag ggagggggag gcggcacttg ggctgcagct cgcaaccaga    120
   ggcagtgtct ctcagctctc ggaaggaccg agagaggcaa ccgcggagta ggatgctctg    180
   cggggcgccc agagccgggg gcgcttgaag cagcttcggg gactggggca accgggcagt    240
   tttcaccatg catcagtccc tgactcagca gcggtccagc gacatgtccc tgcccgattc    300
   catgggagca ttcaatcgga ggaaacgaaa ctccatctat gtcaccgtga ctttgcttat    360
   tgtgtccgtg ttaattctca cagtgggcct tgctgcaacc accaggaccc agaatgtgac    420
   tgtaggaggt tattaccccg gagttattct cggctttgga tcgttccttg gaatcattgg 480
   atcaaacctt attgagaaca aaaggcagat gctggtggct tctatcgtgt ttatcagctt 540
   tggtgtgatt gcggcttttt gttgtgccat agttgacggg gtctttgctg ccagacacat 600
   tgatctgaaa ccactctacg ctaaccggtg ccattatgtt cccaagacat cacagaagga 660
   agctgaggag gttaactgcc ctcacctcag ccgtgaattc tgcacacctc gcatccgggg 720
   caacacctgc ttctgctgtg acctctacaa ctgtggcaac cgggtggaga tcactggtgg 780
   gtactacgaa tacatcgatg tcagcagttg ccaagatatc atccacctct accacctgct 840
   ctggtctgcc accatcctca acattgttgg cctgttcctg ggcatcatca ctgccgctgt 900
   ccttggaggc tttaaggaca tgaacccaac tctcccagca ctgaactgtt ctgttgaaaa 960
   tacccatcca acagtttctt actatgctca tccccaagtg gcatcctaca atacctacta 1020
   ccatagccct cctcacctgc caccatattc tgcttatgac tttcagcatt ccggtgtctt 1080
   tccatcctcc cctccctctg gactttctga tgagccccag tctgcctctc cctcacccag 1140
   ctacatgtgg tcctcaagtg caccgccccg ttactctcca ccctactatc caccttttga 1200
   aaagccacca ccttacagtc cctaaagagg aatgcctgct ggctattgag attattgtgg 1260
   cttttgtatt tctgcttcag tggaagtgtg tagggtacaa aatttaaagt gtgactctta 1320
   tgcataaagt tttacaatgg cctgccaggc tagggaaaga tagggacgaa gcttattcat 1380
   tattagtgca gagcaggggt ggtcaggctg aacgcagcac agaagggcag ctcacattct 1440
   ctaagcaaga ctggggagcc agcccagcaa gaagcttgtt tggacttgca ttaccctatg 1500
   ctccacctct gtattcagca gaagtgtggt tgccatcttt ttcactttat gtaaaggagt 1560
   gttgccctcg ggcccttggc agattgccac cccagcacct aggttgaagc acctggttta 1620
   taggccctat ctttccctac ccctaaagtc agtccctaag gacaatttcc cagctgatgg 1680
   ggctacacag tagttccaat acagagagtt ctggctaaga ttttgtttgc ttgtgtctgg 1740
   atgttgaaaa agactgcccg tatctcttac tccttccttc tctgtgagta ttgtaaaaat 1800
   ggctgttgtg atcactcagc tcagcttttg ttattggtac ctcctaaagg gaaaagtgca 1860
   atattcttgc atcttcagta gtggggaaca ggatgtattg ttccggaaac actgaaatac 1920
   acagcaacat gtgagatgtt ttaagtagat cacttaggag acagtggttc tactacatgt 1980
   tgcattatta caaaatacat ttgctacagg agatataaat cttatggttg taattcagag 2040
   tttaaaaatg ttataaatta ggttcttggg tcgtgatatg aattgttact aatctttgtg 2100
   actatttaat cttcaaatat tgtgcttaac cccagcaatc cgcacgtatc ctgcacccca 2160
   ccccaaaaga gtcatctgta ttttaatgcc actggtctta tcggtccttt tgtctgttga 2220
   gaccagtcat gacagcattc aagattatga aagtgttaca atgccgcttc aagtctgcaa 2280
   aacctcaaac gtagccaact tgacaaatat ttaagtgtta cggcagattt aaaatccatc 2340
   tggcacaccg tggtaggtat ttgtacagtt cttttaatta cacatagctt taaaccatca 2400
   acctgatgag tttaaagctt ttgcacccat gccttcactt cagaatgaac accttcattg 2460
   tgatcttatg ttaacctgag aattgattta aaggaagatt gataatccta tactttataa 2520
   cgtaaaaata caggggctac aggagggtac ctaattagac agttctccaa acacagaaca 2580
   cacactggaa aattttccgg ccaattttgc tacctcccaa cttgatggat tagaggtagc 2640
   gcaaatgctg gtgctcccat ctaccttgta gacacttagc catcaagaat caaggcacaa 2700
   gaagtgcact ctctcattaa cagtaaatgt ttgcaagata ttcagtttaa ctttcagcat 2760
   catgaatgtt cttatccaga ttttgaatcc gaaaaactat aatcctttta tgttatacaa 2820
   aattactatg attttttaca gttctgagca tattaaaatt ctactggatt tcaaaaagag 2880
   actaataccc aactgactaa ctaaacaaat atcaacttgt aatactcaat gaattttttt 2940
   gccatttaca tttgaccgtt ggctttagtg aatgtccata tttaattttt taaggcacca 3000
   ttacacagtt tatcctacat ttatcacatt tcttaaagtg ttaagattct atggctcatt 3060
   tctatgtatt tttcttactt tacaaaataa cctgaaacag tatagatttt gtaacactta 3120
   atttgagcag cttttttatt acattgaatt atataaagtg catgttacct tagaaaaatt 3180
   aatatttgct gctttactct tttgcaaaac atttgctgta atgaatggat ttgtatttcc 3240
   aatatgtatc ttgactgcat tttgtaatat ttactgcttt attcctaatt ctgctttaaa 3300
   gtactgaact gggcatgaaa cattaaaata ttaatccaga aactgtataa actggatgtt 3360
   gcttaaaatc tgtatcactg ccatgttgaa aactcagact gcttttgtga tgtttcaaat 3420
   gaataaaact atcctcccct cgtta 3445
<210> 23
   <211> 49
   <212> DNA
   <213> Artificial
<220>
   <223> Synthetic polynucleotide
<400> 23
   gtaagccgcc tccacttcct actcctggga aggaggatcg caaaagctg 49
<210> 24
   <211> 3193
   <212> DNA
   <213> Homo sapiens
<400> 24
   acgcggctgc acggaccgcc aatgctgctg cctcccagag cagagggagg cagaagagga     60
   gggagggaga agagtggaag ggagggggag gcggcacttg ggctgcagct cgcaaccaga    120
   ggcagtgtct ctcagctctc ggaaggaccg agagaggcaa ccgcggagta ggatgctctg    180
   cggggcgccc agagccgggg gcgcttgaag cagcttcggg gactggggca accgggcagt    240
   tttcaccatg catcagtccc tgactcagca gcggtccagc gacatgtccc tgcccgattc    300
   catgggagca ttcaatcgga ggaaacgaaa ctccatctat gtcaccgtga ctttgcttat    360
   tgtgtccgtg ttaattctca cagtgggcct tgctgcaacc accaggaccc agaatgtgac    420
   tgtaggaggt tattaccccg gagttattct cggctttgga tcgttccttg gaatcattgg 480
   atcaaacctt attgagaaca aaaggcagat gctggtggct tctatcgtgt ttatcagctt 540
   tggtgtgatt gcggcttttt gttgtgccat agttgacggg gtctttgctg ccagacacat 600
   tgatctgaaa ccactctacg ctaaccggtg ccattatgtt cccaagacat cacagaagga 660
   agctgaggag aacccaactc tcccagcact gaactgttct gttgaaaata cccatccaac 720
   agtttcttac tatgctcatc cccaagtggc atcctacaat acctactacc atagccctcc 780
   tcacctgcca ccatattctg cttatgactt tcagcattcc ggtgtctttc catcctcccc 840
   tccctctgga ctttctgatg agccccagtc tgcctctccc tcacccagct acatgtggtc 900
   ctcaagtgca ccgccccgtt actctccacc ctactatcca ccttttgaaa agccaccacc 960
   ttacagtccc taaagaggaa tgcctgctgg ctattgagat tattgtggct tttgtatttc 1020
   tgcttcagtg gaagtgtgta gggtacaaaa tttaaagtgt gactcttatg cataaagttt 1080
   tacaatggcc tgccaggcta gggaaagata gggacgaagc ttattcatta ttagtgcaga 1140
   gcaggggtgg tcaggctgaa cgcagcacag aagggcagct cacattctct aagcaagact 1200
   ggggagccag cccagcaaga agcttgtttg gacttgcatt accctatgct ccacctctgt 1260
   attcagcaga agtgtggttg ccatcttttt cactttatgt aaaggagtgt tgccctcggg 1320
   cccttggcag attgccaccc cagcacctag gttgaagcac ctggtttata ggccctatct 1380
   ttccctaccc ctaaagtcag tccctaagga caatttccca gctgatgggg ctacacagta 1440
   gttccaatac agagagttct ggctaagatt ttgtttgctt gtgtctggat gttgaaaaag 1500
   actgcccgta tctcttactc cttccttctc tgtgagtatt gtaaaaatgg ctgttgtgat 1560
   cactcagctc agcttttgtt attggtacct cctaaaggga aaagtgcaat attcttgcat 1620
   cttcagtagt ggggaacagg atgtattgtt ccggaaacac tgaaatacac agcaacatgt 1680
   gagatgtttt aagtagatca cttaggagac agtggttcta ctacatgttg cattattaca 1740
   aaatacattt gctacaggag atataaatct tatggttgta attcagagtt taaaaatgtt 1800
   ataaattagg ttcttgggtc gtgatatgaa ttgttactaa tctttgtgac tatttaatct 1860
   tcaaatattg tgcttaaccc cagcaatccg cacgtatcct gcaccccacc ccaaaagagt 1920
   catctgtatt ttaatgccac tggtcttatc ggtccttttg tctgttgaga ccagtcatga 1980
   cagcattcaa gattatgaaa gtgttacaat gccgcttcaa gtctgcaaaa cctcaaacgt 2040
   agccaacttg acaaatattt aagtgttacg gcagatttaa aatccatctg gcacaccgtg 2100
   gtaggtattt gtacagttct tttaattaca catagcttta aaccatcaac ctgatgagtt 2160
   taaagctttt gcacccatgc cttcacttca gaatgaacac cttcattgtg atcttatgtt 2220
   aacctgagaa ttgatttaaa ggaagattga taatcctata ctttataacg taaaaataca 2280
   ggggctacag gagggtacct aattagacag ttctccaaac acagaacaca cactggaaaa 2340
   ttttccggcc aattttgcta cctcccaact tgatggatta gaggtagcgc aaatgctggt 2400
   gctcccatct accttgtaga cacttagcca tcaagaatca aggcacaaga agtgcactct 2460
   ctcattaaca gtaaatgttt gcaagatatt cagtttaact ttcagcatca tgaatgttct 2520
   tatccagatt ttgaatccga aaaactataa tccttttatg ttatacaaaa ttactatgat   2580
   tttttacagt tctgagcata ttaaaattct actggatttc aaaaagagac taatacccaa   2640
   ctgactaact aaacaaatat caacttgtaa tactcaatga atttttttgc catttacatt   2700
   tgaccgttgg ctttagtgaa tgtccatatt taatttttta aggcaccatt acacagttta   2760
   tcctacattt atcacatttc ttaaagtgtt aagattctat ggctcatttc tatgtatttt   2820
   tcttacttta caaaataacc tgaaacagta tagattttgt aacacttaat ttgagcagct   2880
   tttttattac attgaattat ataaagtgca tgttacctta gaaaaattaa tatttgctgc   2940
   tttactcttt tgcaaaacat ttgctgtaat gaatggattt gtatttccaa tatgtatctt   3000
   gactgcattt tgtaatattt actgctttat tcctaattct gctttaaagt actgaactgg   3060
   gcatgaaaca ttaaaatatt aatccagaaa ctgtataaac tggatgttgc ttaaaatctg   3120
   tatcactgcc atgttgaaaa ctcagactgc ttttgtgatg tttcaaatga ataaaactat   3180
   cctcccctcg tta                                                      3193
<210> 25
   <211> 165
   <212> PRT
   <213> Homo sapiens
<400> 25
   Leu Cys Ser Leu Ser Ser Leu Thr Pro Pro Cys Gly Arg Arg Leu Val
   1               5                   10                  15
   Pro Cys Leu Ser Ala Pro Ala Pro Asn Ala Pro Arg Leu Pro Ala Pro
               20                  25                  30
   Ala Arg Cys Ser Ile Gly Ala Leu Ile Gly Leu Ser Val Ala Ala Val
           35                  40                  45
   Val Leu Leu Ala Phe Ile Val Thr Ala Cys Val Leu Cys Tyr Leu Phe
       50                  55                  60
   Ile Ser Ser Lys Pro His Thr Lys Leu Asp Leu Gly Leu Ser Leu Gln
   65                  70                  75                  80
   Thr Ala Gly Pro Glu Glu Val Ser Pro Asp Cys Gln Gly Val Asn Thr
                   85                  90                  95
   Gly Met Ala Ala Glu Val Pro Lys Val Ser Pro Leu Gln Gln Ser Tyr
               100                 105                 110
   Ser Cys Leu Asn Pro Gln Leu Glu Ser Asn Glu Gly Gln Ala Val Asn
           115                 120                 125
   Ser Lys Arg Leu Leu His His Cys Phe Met Ala Thr Val Thr Thr Ser
       130                 135                 140
   Asp Ile Pro Gly Ser Pro Glu Glu Ala Ser Val Pro Asn Pro Asp Leu
   145                 150                 155                 160
   Cys Gly Pro Val Pro
                   165
<210> 26
   <211> 3454
   <212> DNA
   <213> Homo sapiens
<400> 26
   acgcggctgc acggaccgcc aatgctgctg cctcccagag cagagggagg cagaagagga 60
   gggagggaga agagtggaag ggagggggag gcggcacttg ggctgcagct cgcaaccaga 120
   ggcagtgtct ctcagctctc ggaaggaccg agagaggcaa ccgcggagta ggatgctctg 180
   cggggcgccc agagccgggg gcgcttgaag cagcttcggg gactggggca accgggcagt 240
   tttcaccatg catcagtccc tgactcagca gcggtccagc gacatgtccc tgcccgattc 300
   catgggagca ttcaatcgga ggaaacgaaa ctccatctat gtcaccgtga ctttgcttat 360
   tgtgtccgtg ttaattctca cagtgggcct tgctgcaacc accaggaccc agaatgtgac 420
   tgtaggaggt tattaccccg gagttattct ggtggcttct atcgtgttta tcagctttgg 480
   tgtgattgcg gctttttgtt gtgccatagt tgacggggtc tttgctgcca gacacattga 540
   tctgaaacca ctctacgcta accggtgcca ttatgttccc aagacatcac agaaggaagc 600
   tgaggaggtc ataagttcct caaccaaaaa ttctccttcc acgagggtta tgaggaacct 660
   tacccaggca gctagagagg ttaactgccc tcacctcagc cgtgaattct gcacacctcg 720
   catccggggc aacacctgct tctgctgtga cctctacaac tgtggcaacc gggtggagat 780
   cactggtggg tactacgaat acatcgatgt cagcagttgc caagatatca tccacctcta 840
   ccacctgctc tggtctgcca ccatcctcaa cattgttggc ctgttcctgg gcatcatcac 900
   tgccgctgtc cttggaggct ttaaggacat gaacccaact ctcccagcac tgaactgttc 960
   tgttgaaaat acccatccaa cagtttctta ctatgctcat ccccaagtgg catcctacaa 1020
   tacctactac catagccctc ctcacctgcc accatattct gcttatgact ttcagcattc 1080
   cggtgtcttt ccatcctccc ctccctctgg actttctgat gagccccagt ctgcctctcc 1140
   ctcacccagc tacatgtggt cctcaagtgc accgccccgt tactctccac cctactatcc 1200
   accttttgaa aagccaccac cttacagtcc ctaaagagga atgcctgctg gctattgaga 1260
   ttattgtggc ttttgtattt ctgcttcagt ggaagtgtgt agggtacaaa atttaaagtg 1320
   tgactcttat gcataaagtt ttacaatggc ctgccaggct agggaaagat agggacgaag 1380
   cttattcatt attagtgcag agcaggggtg gtcaggctga acgcagcaca gaagggcagc 1440
   tcacattctc taagcaagac tggggagcca gcccagcaag aagcttgttt ggacttgcat 1500
   taccctatgc tccacctctg tattcagcag aagtgtggtt gccatctttt tcactttatg 1560
   taaaggagtg ttgccctcgg gcccttggca gattgccacc ccagcaccta ggttgaagca 1620
   cctggtttat aggccctatc tttccctacc cctaaagtca gtccctaagg acaatttccc 1680
   agctgatggg gctacacagt agttccaata cagagagttc tggctaagat tttgtttgct 1740
   tgtgtctgga tgttgaaaaa gactgcccgt atctcttact ccttccttct ctgtgagtat 1800
   tgtaaaaatg gctgttgtga tcactcagct cagcttttgt tattggtacc tcctaaaggg 1860
   aaaagtgcaa tattcttgca tcttcagtag tggggaacag gatgtattgt tccggaaaca 1920
   ctgaaataca cagcaacatg tgagatgttt taagtagatc acttaggaga cagtggttct   1980
   actacatgtt gcattattac aaaatacatt tgctacagga gatataaatc ttatggttgt   2040
   aattcagagt ttaaaaatgt tataaattag gttcttgggt cgtgatatga attgttacta   2100
   atctttgtga ctatttaatc ttcaaatatt gtgcttaacc ccagcaatcc gcacgtatcc   2160
   tgcaccccac cccaaaagag tcatctgtat tttaatgcca ctggtcttat cggtcctttt   2220
   gtctgttgag accagtcatg acagcattca agattatgaa agtgttacaa tgccgcttca   2280
   agtctgcaaa acctcaaacg tagccaactt gacaaatatt taagtgttac ggcagattta   2340
   aaatccatct ggcacaccgt ggtaggtatt tgtacagttc ttttaattac acatagcttt   2400
   aaaccatcaa cctgatgagt ttaaagcttt tgcacccatg ccttcacttc agaatgaaca   2460
   ccttcattgt gatcttatgt taacctgaga attgatttaa aggaagattg ataatcctat   2520
   actttataac gtaaaaatac aggggctaca ggagggtacc taattagaca gttctccaaa   2580
   cacagaacac acactggaaa attttccggc caattttgct acctcccaac ttgatggatt   2640
   agaggtagcg caaatgctgg tgctcccatc taccttgtag acacttagcc atcaagaatc   2700
   aaggcacaag aagtgcactc tctcattaac agtaaatgtt tgcaagatat tcagtttaac   2760
   tttcagcatc atgaatgttc ttatccagat tttgaatccg aaaaactata atccttttat   2820
   gttatacaaa attactatga ttttttacag ttctgagcat attaaaattc tactggattt   2880
   caaaaagaga ctaataccca actgactaac taaacaaata tcaacttgta atactcaatg   2940
   aatttttttg ccatttacat ttgaccgttg gctttagtga atgtccatat ttaatttttt   3000
   aaggcaccat tacacagttt atcctacatt tatcacattt cttaaagtgt taagattcta   3060
   tggctcattt ctatgtattt ttcttacttt acaaaataac ctgaaacagt atagattttg   3120
   taacacttaa tttgagcagc ttttttatta cattgaatta tataaagtgc atgttacctt   3180
   agaaaaatta atatttgctg ctttactctt ttgcaaaaca tttgctgtaa tgaatggatt   3240
   tgtatttcca atatgtatct tgactgcatt ttgtaatatt tactgcttta ttcctaattc   3300
   tgctttaaag tactgaactg ggcatgaaac attaaaatat taatccagaa actgtataaa   3360
   ctggatgttg cttaaaatct gtatcactgc catgttgaaa actcagactg cttttgtgat   3420
   gtttcaaatg aataaaacta tcctcccctc gtta                               3454
<210> 27
   <211> 108
   <212> DNA
   <213> Artificial
<220>
   <223> Synthetic polynucleotide
<400> 27
   gttgtgttca ctctcctcac tgaccccgcc gtgtggtcgc cggctggtgc cttgtctgtc   60
   tgccccagcc ccgaatgccc cgagactccc ggcgccggct cggtgcag                108
<210> 28
   <211> 2880
   <212> DNA
   <213> Homo sapiens
<400> 28
   catttgctat gaatattctc tataacaaag caagacaaat ttagcagcac ttcattgcat 60
   ctggatgggg gagagagctg gacaatttct tgctaacaag agatggttaa ctgccctcac 120
   ctcagccgtg aattctgcac acctcgcatc cggggcaaca cctgcttctg ctgtgacctc 180
   tacaactgtg gcaaccgggt ggagatcact ggtgggtact acgaatacat cgatgtcagc 240
   agttgccaag atatcatcca cctctaccac ctgctctggt ctgccaccat cctcaacatt 300
   gttggcctgt tcctgggcat catcactgcc gctgtccttg gaggctttaa ggacatgaac 360
   ccaactctcc cagcactgaa ctgttctgtt gaaaataccc atccaacagt ttcttactat 420
   gctcatcccc aagtggcatc ctacaatacc tactaccata gccctcctca cctgccacca 480
   tattctgctt atgactttca gcattccggt gtctttccat cctcccctcc ctctggactt 540
   tctgatgagc cccagtctgc ctctccctca cccagctaca tgtggtcctc aagtgcaccg 600
   ccccgttact ctccacccta ctatccacct tttgaaaagc caccacctta cagtccctaa 660
   agaggaatgc ctgctggcta ttgagattat tgtggctttt gtatttctgc ttcagtggaa 720
   gtgtgtaggg tacaaaattt aaagtgtgac tcttatgcat aaagttttac aatggcctgc 780
   caggctaggg aaagataggg acgaagctta ttcattatta gtgcagagca ggggtggtca 840
   ggctgaacgc agcacagaag ggcagctcac attctctaag caagactggg gagccagccc 900
   agcaagaagc ttgtttggac ttgcattacc ctatgctcca cctctgtatt cagcagaagt 960
   gtggttgcca tctttttcac tttatgtaaa ggagtgttgc cctcgggccc ttggcagatt 1020
   gccaccccag cacctaggtt gaagcacctg gtttataggc cctatctttc cctaccccta 1080
   aagtcagtcc ctaaggacaa tttcccagct gatggggcta cacagtagtt ccaatacaga 1140
   gagttctggc taagattttg tttgcttgtg tctggatgtt gaaaaagact gcccgtatct 1200
   cttactcctt ccttctctgt gagtattgta aaaatggctg ttgtgatcac tcagctcagc 1260
   ttttgttatt ggtacctcct aaagggaaaa gtgcaatatt cttgcatctt cagtagtggg 1320
   gaacaggatg tattgttccg gaaacactga aatacacagc aacatgtgag atgttttaag 1380
   tagatcactt aggagacagt ggttctacta catgttgcat tattacaaaa tacatttgct 1440
   acaggagata taaatcttat ggttgtaatt cagagtttaa aaatgttata aattaggttc 1500
   ttgggtcgtg atatgaattg ttactaatct ttgtgactat ttaatcttca aatattgtgc 1560
   ttaaccccag caatccgcac gtatcctgca ccccacccca aaagagtcat ctgtatttta 1620
   atgccactgg tcttatcggt ccttttgtct gttgagacca gtcatgacag cattcaagat 1680
   tatgaaagtg ttacaatgcc gcttcaagtc tgcaaaacct caaacgtagc caacttgaca 1740
   aatatttaag tgttacggca gatttaaaat ccatctggca caccgtggta ggtatttgta 1800
   cagttctttt aattacacat agctttaaac catcaacctg atgagtttaa agcttttgca 1860
   cccatgcctt cacttcagaa tgaacacctt cattgtgatc ttatgttaac ctgagaattg 1920
   atttaaagga agattgataa tcctatactt tataacgtaa aaatacaggg gctacaggag 1980
   ggtacctaat tagacagttc tccaaacaca gaacacacac tggaaaattt tccggccaat 2040
   tttgctacct cccaacttga tggattagag gtagcgcaaa tgctggtgct cccatctacc 2100
   ttgtagacac ttagccatca agaatcaagg cacaagaagt gcactctctc attaacagta 2160
   aatgtttgca agatattcag tttaactttc agcatcatga atgttcttat ccagattttg 2220
   aatccgaaaa actataatcc ttttatgtta tacaaaatta ctatgatttt ttacagttct 2280
   gagcatatta aaattctact ggatttcaaa aagagactaa tacccaactg actaactaaa 2340
   caaatatcaa cttgtaatac tcaatgaatt tttttgccat ttacatttga ccgttggctt 2400
   tagtgaatgt ccatatttaa ttttttaagg caccattaca cagtttatcc tacatttatc 2460
   acatttctta aagtgttaag attctatggc tcatttctat gtatttttct tactttacaa 2520
   aataacctga aacagtatag attttgtaac acttaatttg agcagctttt ttattacatt 2580
   gaattatata aagtgcatgt taccttagaa aaattaatat ttgctgcttt actcttttgc 2640
   aaaacatttg ctgtaatgaa tggatttgta tttccaatat gtatcttgac tgcattttgt 2700
   aatatttact gctttattcc taattctgct ttaaagtact gaactgggca tgaaacatta 2760
   aaatattaat ccagaaactg tataaactgg atgttgctta aaatctgtat cactgccatg 2820
   ttgaaaactc agactgcttt tgtgatgttt caaatgaata aaactatcct cccctcgtta 2880
<210> 29
   <211> 349
   <212> PRT
   <213> Homo sapiens
<400> 29
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Gly Phe Gly Ser Phe Leu Gly Ile Ile Gly Ser Asn
   65                  70                  75                  80
   Leu Ile Glu Asn Lys Arg Gln Met Leu Val Ala Ser Ile Val Phe Ile
                   85                  90                  95
   Ser Phe Gly Val Ile Ala Ala Phe Cys Cys Ala Ile Val Asp Gly Val
               100                 105                 110
   Phe Ala Ala Arg His Ile Asp Leu Lys Pro Leu Tyr Ala Asn Arg Cys
           115                 120                 125
   His Tyr Val Pro Lys Thr Ser Gln Lys Glu Ala Glu Glu Val Ile Ser
       130                 135                 140
   Ser Ser Thr Lys Asn Ser Pro Ser Thr Arg Val Met Arg Asn Leu Thr
   145                 150                 155                 160
   Gln Ala Ala Arg Glu Val Asn Cys Pro His Leu Ser Arg Glu Phe Cys
                   165                 170                 175
   Thr Pro Arg Ile Arg Gly Asn Thr Cys Phe Cys Cys Asp Leu Tyr Asn
               180                 185                 190
   Cys Gly Asn Arg Val Glu Ile Thr Gly Gly Tyr Tyr Glu Tyr Ile Asp
           195                 200                 205
   Val Ser Ser Cys Gln Asp Ile Ile His Leu Tyr His Leu Leu Trp Ser
       210                 215                 220
   Ala Thr Ile Leu Asn Ile Val Gly Leu Phe Leu Gly Ile Ile Thr Ala
   225                 230                 235                 240
   Ala Val Leu Gly Gly Phe Lys Asp Met Asn Pro Thr Leu Pro Ala Leu
                   245                 250                 255
   Asn Cys Ser Val Glu Asn Thr His Pro Thr Val Ser Tyr Tyr Ala His
               260                 265                 270
   Pro Gln Val Ala Ser Tyr Asn Thr Tyr Tyr His Ser Pro Pro His Leu
           275                 280                 285
   Pro Pro Tyr Ser Ala Tyr Asp Phe Gln His Ser Gly Val Phe Pro Ser
       290                 295                 300
   Ser Pro Pro Ser Gly Leu Ser Asp Glu Pro Gln Ser Ala Ser Pro Ser
   305                 310                 315                 320
   Pro Ser Tyr Met Trp Ser Ser Ser Ala Pro Pro Arg Tyr Ser Pro Pro
                   325                 330                 335
   Tyr Tyr Pro Pro Phe Glu Lys Pro Gln Pro Tyr Ser Pro
               340                 345
<210> 30
   <211> 349
   <212> PRT
   <213> Homo sapiens
<400> 30
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Gly Phe Gly Ser Phe Leu Gly Ile Ile Gly Ser Asn
   65                  70                  75                  80
   Leu Ile Glu Asn Lys Arg Gln Met Leu Val Ala Ser Ile Val Phe Ile
                   85                  90                  95
   Ser Phe Gly Val Ile Ala Ala Phe Cys Cys Ala Ile Val Asp Gly Val
               100                 105                 110
   Phe Ala Ala Arg His Ile Asp Leu Lys Pro Leu Tyr Ala Asn Arg Cys
           115                 120                 125
   His Tyr Val Pro Lys Thr Ser Gln Lys Glu Ala Glu Glu Val Ile Ser
   130 135 140
   Ser Ser Thr Lys Asn Ser Pro Ser Thr Arg Val Met Arg Asn Leu Thr
   145 150 155 160
   Gln Ala Ala Arg Glu Val Asn Cys Pro His Leu Ser Arg Glu Phe Cys
   165 170 175
   Thr Pro Arg Ile Arg Gly Asn Thr Cys Phe Cys Cys Asp Leu Tyr Asn
   180 185 190
   Cys Gly Asn Arg Val Glu Ile Thr Gly Gly Tyr Tyr Glu Tyr Ile Asp
   195 200 205
   Val Ser Ser Cys Gln Asp Ile Ile His Leu Tyr His Leu Leu Trp Ser
   210 215 220
   Ala Thr Ile Leu Asn Ile Val Gly Leu Phe Leu Gly Ile Ile Thr Ala
   225 230 235 240
   Ala Val Leu Gly Gly Phe Lys Asp Met Asn Pro Thr Leu Pro Ala Leu
   245 250 255
   Asn Cys Ser Val Glu Asn Thr His Pro Thr Val Ser Tyr Tyr Ala His
   260 265 270
   Pro Gln Val Ala Ser Tyr Asn Thr Tyr Tyr His Ser Pro Pro His Leu
   275 280 285
   Pro Pro Tyr Ser Ala Tyr Asp Phe Gln His Ser Gly Val Phe Pro Ser
   290 295 300
   Ser Pro Pro Ser Gly Leu Ser Asp Glu Pro Gln Ser Ala Ser Pro Ser
   305 310 315 320
   Pro Ser Tyr Met Trp Ser Ser Ser Ala Pro Pro Arg Tyr Ser Pro Pro
   325 330 335
   Tyr Tyr Pro Pro Phe Glu Lys Pro Pro Pro Tyr Ser Pro
   340 345
<210> 31
   <211> 349
   <212> PRT
   <213> Homo sapiens
<400> 31
<210> 32
   <211> 185
   <212> PRT
   <213> Homo sapiens
<400> 32
   Met Val Asn Cys Pro His Leu Ser Arg Glu Phe Cys Thr Pro Arg Ile
   1               5                   10                  15
   Arg Gly Asn Thr Cys Phe Cys Cys Asp Leu Tyr Asn Cys Gly Asn Arg
               20                  25                  30
   Val Glu Ile Thr Gly Gly Tyr Tyr Glu Tyr Ile Asp Val Ser Ser Cys
           35                  40                  45
   Gln Asp Ile Ile His Leu Tyr His Leu Leu Trp Ser Ala Thr Ile Leu
       50                  55                  60
   Asn Ile Val Gly Leu Phe Leu Gly Ile Ile Thr Ala Ala Val Leu Gly
   65                  70                  75                  80
   Gly Phe Lys Asp Met Asn Pro Thr Leu Pro Ala Leu Asn Cys Ser Val
                   85                  90                  95
   Glu Asn Thr His Pro Thr Val Ser Tyr Tyr Ala His Pro Gln Val Ala
               100                 105                 110
   Ser Tyr Asn Thr Tyr Tyr His Ser Pro Pro His Leu Pro Pro Tyr Ser
           115                 120                 125
   Ala Tyr Asp Phe Gln His Ser Gly Val Phe Pro Ser Ser Pro Pro Ser
       130                 135                 140
   Gly Leu Ser Asp Glu Pro Gln Ser Ala Ser Pro Ser Pro Ser Tyr Met
   145                 150                 155                 160
   Trp Ser Ser Ser Ala Pro Pro Arg Tyr Ser Pro Pro Tyr Tyr Pro Pro
                   165                 170                 175
   Phe Glu Lys Pro Pro Pro Tyr Ser Pro
               180                 185
<210> 33
   <211> 325
   <212> PRT
   <213> Homo sapiens
<400> 33
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Gly Phe Gly Ser Phe Leu Gly Ile Ile Gly Ser Asn
   65                  70                  75                  80
   Leu Ile Glu Asn Lys Arg Gln Met Leu Val Ala Ser Ile Val Phe Ile
                   85                  90                  95
   Ser Phe Gly Val Ile Ala Ala Phe Cys Cys Ala Ile Val Asp Gly Val
               100                 105                 110
   Phe Ala Ala Arg His Ile Asp Leu Lys Pro Leu Tyr Ala Asn Arg Cys
           115                 120                 125
   His Tyr Val Pro Lys Thr Ser Gln Lys Glu Ala Glu Glu Val Asn Cys
       130                 135                 140
   Pro His Leu Ser Arg Glu Phe Cys Thr Pro Arg Ile Arg Gly Asn Thr
   145                 150                 155                 160
   Cys Phe Cys Cys Asp Leu Tyr Asn Cys Gly Asn Arg Val Glu Ile Thr
                   165                 170                 175
   Gly Gly Tyr Tyr Glu Tyr Ile Asp Val Ser Ser Cys Gln Asp Ile Ile
               180                 185                 190
   His Leu Tyr His Leu Leu Trp Ser Ala Thr Ile Leu Asn Ile Val Gly
           195                 200                 205
   Leu Phe Leu Gly Ile Ile Thr Ala Ala Val Leu Gly Gly Phe Lys Asp
       210                 215                 220
   Met Asn Pro Thr Leu Pro Ala Leu Asn Cys Ser Val Glu Asn Thr His
   225                 230                 235                 240
   Pro Thr Val Ser Tyr Tyr Ala His Pro Gln Val Ala Ser Tyr Asn Thr
                   245                 250                 255
   Tyr Tyr His Ser Pro Pro His Leu Pro Pro Tyr Ser Ala Tyr Asp Phe
               260                 265                 270
   Gln His Ser Gly Val Phe Pro Ser Ser Pro Pro Ser Gly Leu Ser Asp
           275                 280                 285
   Glu Pro Gln Ser Ala Ser Pro Ser Pro Ser Tyr Met Trp Ser Ser Ser
       290                 295                 300
   Ala Pro Pro Arg Tyr Ser Pro Pro Tyr Tyr Pro Pro Phe Glu Lys Pro
   305                 310                 315                 320
   Pro Pro Tyr Ser Pro
                   325
<210> 34
   <211> 25
   <212> DNA
   <213> Artificial
<220>
   <223> synthetic polynucleotide
<400> 34
   aatcattatg ctgaggattt ggaaa 25
<210> 35
   <211> 241
   <212> PRT
   <213> Homo sapiens
<400> 35
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Gly Phe Gly Ser Phe Leu Gly Ile Ile Gly Ser Asn
   65                  70                  75                  80
   Leu Ile Glu Asn Lys Arg Gln Met Leu Val Ala Ser Ile Val Phe Ile
                   85                  90                  95
   Ser Phe Gly Val Ile Ala Ala Phe Cys Cys Ala Ile Val Asp Gly Val
               100                 105                 110
   Phe Ala Ala Arg His Ile Asp Leu Lys Pro Leu Tyr Ala Asn Arg Cys
           115                 120                 125
   His Tyr Val Pro Lys Thr Ser Gln Lys Glu Ala Glu Glu Asn Pro Thr
       130                 135                 140
   Leu Pro Ala Leu Asn Cys Ser Val Glu Asn Thr His Pro Thr Val Ser
   145                 150                 155                 160
   Tyr Tyr Ala His Pro Gln Val Ala Ser Tyr Asn Thr Tyr Tyr His Ser
                   165                 170                 175
   Pro Pro His Leu Pro Pro Tyr Ser Ala Tyr Asp Phe Gln His Ser Gly
               180                 185                 190
   Val Phe Pro Ser Ser Pro Pro Ser Gly Leu Ser Asp Glu Pro Gln Ser
           195                 200                 205
   Ala Ser Pro Ser Pro Ser Tyr Met Trp Ser Ser Ser Ala Pro Pro Arg
       210                 215                 220
   Tyr Ser Pro Pro Tyr Tyr Pro Pro Phe Glu Lys Pro Pro Pro Tyr Ser
   225                 230                 235                 240
   Pro
<210> 36
   <211> 328
   <212> PRT
   <213> Homo sapiens
<400> 36
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Val Ala Ser Ile Val Phe Ile Ser Phe Gly Val Ile
   65                  70                  75                  80
   Ala Ala Phe Cys Cys Ala Ile Val Asp Gly Val Phe Ala Ala Arg His
                   85                  90                  95
   Ile Asp Leu Lys Pro Leu Tyr Ala Asn Arg Cys His Tyr Val Pro Lys
               100                 105                 110
   Thr Ser Gln Lys Glu Ala Glu Glu Val Ile Ser Ser Ser Thr Lys Asn
           115                 120                 125
   Ser Pro Ser Thr Arg Val Met Arg Asn Leu Thr Gln Ala Ala Arg Glu
       130                 135                 140
   Val Asn Cys Pro His Leu Ser Arg Glu Phe Cys Thr Pro Arg Ile Arg
   145                 150                 155                 160
   Gly Asn Thr Cys Phe Cys Cys Asp Leu Tyr Asn Cys Gly Asn Arg Val
                   165                 170                 175
   Glu Ile Thr Gly Gly Tyr Tyr Glu Tyr Ile Asp Val Ser Ser Cys Gln
               180                 185                 190
   Asp Ile Ile His Leu Tyr His Leu Leu Trp Ser Ala Thr Ile Leu Asn
           195                 200                 205
   Ile Val Gly Leu Phe Leu Gly Ile Ile Thr Ala Ala Val Leu Gly Gly
       210                 215                 220
   Phe Lys Asp Met Asn Pro Thr Leu Pro Ala Leu Asn Cys Ser Val Glu
   225                 230                 235                 240
   Asn Thr His Pro Thr Val Ser Tyr Tyr Ala His Pro Gln Val Ala Ser
                   245                 250                 255
   Tyr Asn Thr Tyr Tyr His Ser Pro Pro His Leu Pro Pro Tyr Ser Ala
               260                 265                 270
   Tyr Asp Phe Gln His Ser Gly Val Phe Pro Ser Ser Pro Pro Ser Gly
           275                 280                 285
   Leu Ser Asp Glu Pro Gln Ser Ala Ser Pro Ser Pro Ser Tyr Met Trp
       290                 295                 300
   Ser Ser Ser Ala Pro Pro Arg Tyr Ser Pro Pro Tyr Tyr Pro Pro Phe
   305                 310                 315                 320
   Glu Lys Pro Pro Pro Tyr Ser Pro
                   325
<210> 37
   <211> 22
   <212> DNA
   <213> Artificial
<220>
   <223> Synthetic polynucleotide
<400> 37
   cctcccatct ccttcatcac at 22
<210> 38
   <211> 3349
   <212> DNA
   <213> Homo sapiens
<400> 38
   ggctggagag gcgtgataat agaaccagtg cttcaacagc aacttctctt ttgcaatcct     60
   tgctggtctg acaggctcct gcagaattac atcacttcct aaaaaacagg cacacacctc    120
   tccttcaagt cagcaccgac ccaaacaagc aggaaacagg aaatgttcac gtttcagaga    180
   ggctgcagcc cggcgcagca tcctgagcgc gcctctgccg aggcgagcgg acatgcaggc    240
   tccccgcgca gccctagtct tcgccctggt gatcgcgctc gttcccgtcg gccggggtaa    300
   ttatgaggaa ttagaaaact caggagatac aactgtggaa tctgaaagac caaataaagt    360
   gactattcca agcacatttg ctgcagtgac catcaaagaa acattaaatg caaatataaa    420
   ttctaccaac tttgctccgg atgaaaatca gttagagttt atactgatgg tgttaatccc    480
   attgatttta ttggtcctct tacttttatc cgtggtattc cttgcaacat actataaaag    540
   aaaaagaact aaacaagaac cttctagcca aggatctcag agtgctttac agacatatga    600
   actgggaagt gaaaacgtga aagtccctat ttttgaggaa gatacaccct ctgttatgga    660
   aattgaaatg gaagagcttg ataaatggat gaacagcatg aatagaaatg ccgactttga    720
   atgtttacct accttgaagg aagagaagga atcaaatcac aacccaagtg acagtgaatc    780
   ctaaacctga atggcgctca tgttttccaa gagaagcagc ccctgaggga gtctgctgag    840
   gctgccaaca gaggatgaag aggatacaaa tttaattaat ttcaaatcaa catagacaca    900
   agaacctttt gctgtttctt ccaacgccca ctcttcctaa tgatggcatc acttgcactt    960
   ggaagaatgt gcaattgaga agtactagga aaaggcctgg ctgccatcca tcgctgcctc   1020
   tgagggtgga gaaggaggcg ggtgatgtgc tcacttctga tcaacatgtg ttgcctcctc   1080
   tcagccaact tctagctcac tgcactcact ctggtcatga taaatgttcg tcacctttct   1140
   gcttcattcc ttagggccta aatcaggaag ctgttttatc gatggtttcc ttttgggtca   1200
   gtaaccagct ttggataatt tcctctgatt attcaagtcg tgggacaggt aaactacatt   1260
   cagcaggaac ttttctcgag gagtgttatg tcatggaaaa gacaccaaac acagcaagta   1320
   ttttaatgaa tacaccatcc cagggggtca gtaagctctg cctgccaaga agacacagtg   1380
   agaggggtcc acagtcctga tgaggtggcg tttggtaact tgtagaccct agcatggcca   1440
   ggtctggtca cccttaagaa cttctcagag aaactaggaa tcttcagtga aagaactaat   1500
   gttctcctca gctgaaattc ccttgcttgt cagcatttct gcaaagctca cacttgtttc   1560
   accatacctc ccttggatgt gacatgtagg taggaagtat gtgcaggtgg gagtcatctg   1620
   tcagccttct atgtttcaga gatcctgaag gtggtttgaa acaaacagaa gaggagcagg   1680
   aaatatccgt gcctgtggca gatctcactc atcatgctta gcattctctc ccgccaagct   1740
   gggataagcc tcatgtccta acacagcaca acaggaggtc tctgtcagtc catcagagat   1800
   gacattctat gtgatatttt tgacatcctt gtgctaaaag caatggcaca aaatggaaaa   1860
   gggcctattg accacaccta ctccagtaaa attgttcttc atttattcct taattttcta   1920
   aatctgaccc ctttaaagca atctagcaaa ttgagaatcc tcagctctcc ttggatacct   1980
   gatattttat ttcaagaaag agacaaagaa ggaaaatttt atttatttta ctacccacat   2040
   ataaaccgaa gggagatggg actacccaaa catttgctgc tcaattttgt gtcttgtgct   2100
   tgaaagtctg ccctaatgca taacaaaaac tacttgtctc ctaccttttg ggatcccttt   2160
   aacaagtatt tgccttctga actacgtgga taatttcaaa ggcagagttc cagaccagag   2220
   aggtctttcc atacaatgaa agctataact agctggttgt gtttaaccac tgctatcacg   2280
   ctatcctggg actgcataga actttgacaa aagacagact tcatggtaca caacttcaac   2340
   agattttctg tcatattctc accagcacat ctgaatgagg ctttgtgttt tccttgctct   2400
   cttgcatgtt ccttttcaac tcatggccca cagtgactct cagagattta tgccaaaatt   2460
   gcatacaatt gttttctgaa tcataacttg tctatttttc tgcctatgtg tgctactttc   2520
   agtttgtttc tcatcaacat tttgactctc agaagagcct ccatttgccc ctttctctct   2580
   ttaggtatct aagatctttg aacacctgga cctttacatt tgatccaacc ctatcaaata   2640
   atgaacttct cagagaggca tctggggtcc tggaacttca tgttgatgaa gtcatattat   2700
   ataatatgat aaaaatattc tcatgcagta ttttaaataa tttcaaattc tagaagaagc   2760
   aaatttcagc gacatgtcat tgagttttta tttggtaaag ctataattgt gcagtgtaca   2820
   aagcactttt taaaaagata gtttattctg tcagggtata tgaagttagt atacagccag   2880
   aacagccaag cctcaattct tgtaccttgt gtctttttat tactgtttaa tcaatagata   2940
   tcatatgttt atgacagttt caagaattgt ttttaaaccc aaacttaatt ttatgtttca   3000
   gactattgtt agaaaaacaa aacaaaaaac aaaaaaaccc tcactaattt gccctaattg   3060
   gatagggcaa tcagaacaaa atctgacttt cgaatattta aaagatatgt aagtttgatt   3120
   gcattttcgt acattttaag caaactaggt taacaacaac atagcctagt caaacttctc   3180
   aggaaacttg ttttaataaa tatgtaaaaa tacccattca tgactcttga cccaatggtg   3240
   tgactcctcc ctctgggaag ggctttgtta cccaatgcaa gaccacagcc tggatccacc   3300
   ttgatgtagg tggctcctgg ataacccaca tcaaacaaat gtgataaag                3349
<210> 39
   <211> 2767
   <212> DNA
   <213> Homo sapiens
<400> 39
   ggctggagag gcgtgataat agaaccagtg cttcaacagc aacttctctt ttgcaatcct 60
   tgctggtctg acaggctcct gcagaattac atcacttcct aaaaaacagg cacacacctc 120
   tccttcaagt cagcaccgac ccaaacaagc aggaaacagg aaatgttcac gtttcagaga 180
   ggctgcagcc cggcgcagca tcctgagcgc gcctctgccg aggcgagcgg acatgcaggc 240
   tccccgcgca gccctagtct tcgccctggt gatcgcgctc gttcccgtcg gccggggtaa 300
   ttatgaggaa ttagaaaact caggagatac aactgtggaa tctgaaagac caaataaagt 360
   gactattcca agcacatttg ctgcagtgac catcaaagaa acattaaatg caaatataaa 420
   ttctaccaac tttgctccgg atgaaaatca gttagagttt atactgatgg tgttaatccc 480
   attgatttta ttggtcctct tacttttatc cgtggtattc cttgcaacat actataaaag 540
   aaaaagaact aaacaagaac cttctagcca aggatctcag agtgctttac agacatatga 600
   actgggaagt gaaaacgtga aagtccctat ttttgaggaa gatacaccct ctgttatgga 660
   aattgaaatg gaagagcttg ataaatggat gaacagcatg aatagaaatg ccgactttga 720
   atgtttacct accttgaagg aagagaagga atcaaatcac aacccaagtg acagtgaatc 780
   ctaaacctga atggcgctca tgttttccaa gagaagcagc ccctgaggga gtctgctgag 840
   gctgccaaca gaggatgaag aggatacaaa tttaattaat ttcaaatcaa catagacaca 900
   agaacctttt gctgtttctt ccaacgccca ctcttcctaa tgatggcatc acttgcactt 960
   ggaagaatgt gcaattgaga agtactagga aaaggcctgg ctgccatcca tcgctgcctc 1020
   tgagggtgga gaaggaggcg ggtgatgtgc tcacttctga tcaacatgtg ttgcctcctc 1080
   tcagccaact tctagctcac tgcactcact ctggtcatga taaatgttcg tcacctttct 1140
   gcttcattcc ttagggccta aatcaggaag ctgttttatc gatggtttcc ttttgggtca 1200
   gtaaccagct ttggataatt tcctctgatt attcaagtcg tgggacaggt aaactacatt 1260
   cagcaggaac ttttctcgag gagtgttatg tcatggaaaa gacaccaaac acagcaagta    1320
   ttttaatgaa tacaccatcc cagggggtca gtaagctctg cctgccaaga agacacagtg   1380
   agaggggtcc acagtcctga tgaggtggcg tttggtaact tgtagaccct agcatggcca    1440
   ggtctggtca cccttaagaa cttctcagag aaactaggaa tcttcagtga aagaactaat    1500
   gttctcctca gctgaaattc ccttgcttgt cagcatttct gcaaagctca cacttgtttc    1560
   accatacctc ccttggatgt gacatgtagg taggaagtat gtgcaggtgg gagtcatctg    1620
   tcagccttct atgtttcaga gatcctgaag gtggtttgaa acaaacagaa gaggagcagg    1680
   aaatatccgt gcctgtggca gatctcactc atcatgctta gcattctctc ccgccaagct    1740
   gggataagcc tcatgtccta acacagcaca acaggaggtc tctgtcagtc catcagagat    1800
   gacattctat gtgatatttt tgacatcctt gtgctaaaag caatggcaca aaatggaaaa    1860
   gggcctattg accacaccta ctccagtaaa attgttcttc atttattcct taattttcta    1920
   aatctgaccc ctttaaagca atctagcaaa ttgagaatcc tcagctctcc ttggatacct    1980
   gatattttat ttcaagaaag agacaaagaa ggaaaatttt atttatttta ctacccacat    2040
   ataaaccgaa gggagatggg actacccaaa catttgctgc tcaattttgt gtcttgtgct    2100
   tgaaagtctg ccctaatgca taacaaaaac tacttgtctc ctaccttttg ggatcccttt    2160
   aacaagtatt tgccttctga actacgtgga taatttcaaa ggcagagttc cagaccagag    2220
   aggtctttcc atacaatgaa agctataact agctggttgt gtttaaccac tgctatcacg    2280
   ctatcctggg actgcataga actttgacaa aagacagact tcatggtaca caacttcaac    2340
   agattttctg tcatattctc accagcacat ctgaatgagg ctttgtgttt tccttgctct    2400
   cttgcatgtt ccttttcaac tcatggccca cagtgactct cagagattta tgccaaaatt    2460
   gcatacaatt gttttctgaa tcataacttg tctatttttc tgcctatgtg tgctactttc    2520
   agtttgtttc tcatcaacat tttgactctc agaagagcct ccatttgccc ctttctctct    2580
   ttaggtatct aagatctttg aacacctgga cctttacatt tgatccaacc ctatcaaata    2640
   atgaacttct cagagaggca tctggggtcc tggaacttca tgttgatgaa gtcatattat    2700
   ataatatgat aaaaatattc tcatgcagta ttttaaataa tttcaaattc tagaagaagc    2760
   aaatttc                                                              2767
<210> 40
   <211> 3398
   <212> DNA
   <213> Homo sapiens
<400> 40
   ggctggagag gcgtgataat agaaccagtg cttcaacagc aacttctctt ttgcaatcct     60
   tgctggtctg acaggctcct gcagaattac atcacttcct aaaaaacagg cacacacctc    120
   tccttcaagt cagcaccgac ccaaacaagc aggaaacagg aaatgttcac gtttcagaga    180
   ggctgcagcc cggcgcagca tcctgagcgc gcctctgccg aggcgagcgg acatgcaggc    240
   tccccgcgca gccctagtct tcgccctggt gatcgcgctc gttcccgtcg gccggggtaa 300
   gccgcctcca cttcctactc ctgggaagga ggatcgcaaa agctggtaat tatgaggaat 360
   tagaaaactc aggagataca actgtggaat ctgaaagacc aaataaagtg actattccaa 420
   gcacatttgc tgcagtgacc atcaaagaaa cattaaatgc aaatataaat tctaccaact 480
   ttgctccgga tgaaaatcag ttagagttta tactgatggt gttaatccca ttgattttat 540
   tggtcctctt acttttatcc gtggtattcc ttgcaacata ctataaaaga aaaagaacta 600
   aacaagaacc ttctagccaa ggatctcaga gtgctttaca gacatatgaa ctgggaagtg 660
   aaaacgtgaa agtccctatt tttgaggaag atacaccctc tgttatggaa attgaaatgg 720
   aagagcttga taaatggatg aacagcatga atagaaatgc cgactttgaa tgtttaccta 780
   ccttgaagga agagaaggaa tcaaatcaca acccaagtga cagtgaatcc taaacctgaa 840
   tggcgctcat gttttccaag agaagcagcc cctgagggag tctgctgagg ctgccaacag 900
   aggatgaaga ggatacaaat ttaattaatt tcaaatcaac atagacacaa gaaccttttg 960
   ctgtttcttc caacgcccac tcttcctaat gatggcatca cttgcacttg gaagaatgtg 1020
   caattgagaa gtactaggaa aaggcctggc tgccatccat cgctgcctct gagggtggag 1080
   aaggaggcgg gtgatgtgct cacttctgat caacatgtgt tgcctcctct cagccaactt 1140
   ctagctcact gcactcactc tggtcatgat aaatgttcgt cacctttctg cttcattcct 1200
   tagggcctaa atcaggaagc tgttttatcg atggtttcct tttgggtcag taaccagctt 1260
   tggataattt cctctgatta ttcaagtcgt gggacaggta aactacattc agcaggaact 1320
   tttctcgagg agtgttatgt catggaaaag acaccaaaca cagcaagtat tttaatgaat 1380
   acaccatccc agggggtcag taagctctgc ctgccaagaa gacacagtga gaggggtcca 1440
   cagtcctgat gaggtggcgt ttggtaactt gtagacccta gcatggccag gtctggtcac 1500
   ccttaagaac ttctcagaga aactaggaat cttcagtgaa agaactaatg ttctcctcag 1560
   ctgaaattcc cttgcttgtc agcatttctg caaagctcac acttgtttca ccatacctcc 1620
   cttggatgtg acatgtaggt aggaagtatg tgcaggtggg agtcatctgt cagccttcta 1680
   tgtttcagag atcctgaagg tggtttgaaa caaacagaag aggagcagga aatatccgtg 1740
   cctgtggcag atctcactca tcatgcttag cattctctcc cgccaagctg ggataagcct 1800
   catgtcctaa cacagcacaa caggaggtct ctgtcagtcc atcagagatg acattctatg 1860
   tgatattttt gacatccttg tgctaaaagc aatggcacaa aatggaaaag ggcctattga 1920
   ccacacctac tccagtaaaa ttgttcttca tttattcctt aattttctaa atctgacccc 1980
   tttaaagcaa tctagcaaat tgagaatcct cagctctcct tggatacctg atattttatt 2040
   tcaagaaaga gacaaagaag gaaaatttta tttattttac tacccacata taaaccgaag 2100
   ggagatggga ctacccaaac atttgctgct caattttgtg tcttgtgctt gaaagtctgc 2160
   cctaatgcat aacaaaaact acttgtctcc taccttttgg gatcccttta acaagtattt 2220
   gccttctgaa ctacgtggat aatttcaaag gcagagttcc agaccagaga ggtctttcca 2280
   tacaatgaaa gctataacta gctggttgtg tttaaccact gctatcacgc tatcctggga 2340
   ctgcatagaa ctttgacaaa agacagactt catggtacac aacttcaaca gattttctgt 2400
   catattctca ccagcacatc tgaatgaggc tttgtgtttt ccttgctctc ttgcatgttc 2460
   cttttcaact catggcccac agtgactctc agagatttat gccaaaattg catacaattg 2520
   ttttctgaat cataacttgt ctatttttct gcctatgtgt gctactttca gtttgtttct 2580
   catcaacatt ttgactctca gaagagcctc catttgcccc tttctctctt taggtatcta 2640
   agatctttga acacctggac ctttacattt gatccaaccc tatcaaataa tgaacttctc 2700
   agagaggcat ctggggtcct ggaacttcat gttgatgaag tcatattata taatatgata 2760
   aaaatattct catgcagtat tttaaataat ttcaaattct agaagaagca aatttcagcg 2820
   acatgtcatt gagtttttat ttggtaaagc tataattgtg cagtgtacaa agcacttttt 2880
   aaaaagatag tttattctgt cagggtatat gaagttagta tacagccaga acagccaagc 2940
   ctcaattctt gtaccttgtg tctttttatt actgtttaat caatagatat catatgttta 3000
   tgacagtttc aagaattgtt tttaaaccca aacttaattt tatgtttcag actattgtta 3060
   gaaaaacaaa acaaaaaaca aaaaaaccct cactaatttg ccctaattgg atagggcaat 3120
   cagaacaaaa tctgactttc gaatatttaa aagatatgta agtttgattg cattttcgta 3180
   cattttaagc aaactaggtt aacaacaaca tagcctagtc aaacttctca ggaaacttgt 3240
   tttaataaat atgtaaaaat acccattcat gactcttgac ccaatggtgt gactcctccc 3300
   tctgggaagg gctttgttac ccaatgcaag accacagcct ggatccacct tgatgtaggt 3360
   ggctcctgga taacccacat caaacaaatg tgataaag 3398
<210> 41
   <211> 776
   <212> DNA
   <213> Homo sapiens
<400> 41
   ggctggagag gcgtgataat agaaccagtg cttcaacagc aacttctctt ttgcaatcct 60
   tgctggtctg acaggctcct gcagaattac atcacttcct aaaaaacagg cacacacctc 120
   tccttcaagt cagcaccgac ccaaacaagc aggaaacagg aaatgttcac gtttcagaga 180
   ggctgcagcc cggcgcagca tcctgagcgc gcctctgccg aggcgagcgg acatgcaggc 240
   tccccgcgca gccctagtct tcgccctggt gatcgcgctc gttcccgtcg gccggggtaa 300
   ttatgaggaa ttagaaaact caggagatac aactgtggaa tctgaaagac caaataaagt 360
   gactattcca agcacatttg ctgcagtgac catcaaagaa acattaaatg caaatataaa 420
   ttctaccaac tttgctccgg atgaaaatca gttagagttt atactgatgg tgttaatccc 480
   attgatttta ttggtcctct tacttttatc cgtggtattc cttgcaacat actataaaag 540
   aaaaagaact aaacaagaac cttctagcca aggatctcag agtgctttac agacatgtaa 600
   aatacaacta tcatggaagg tcataccagc gttttgcttg gaaagctccc acagaaatgc 660
   tttataggag agcatggaca cctgcacact atgtcccctg ggaagttaga agcagacata 720
   ctgtttctta ctgcacttgg gtagagcctg gtctgttata ctatactagg aaataa 776
<210> 42
   <211> 183
   <212> PRT
   <213> Homo sapiens
<400> 42
   Met Gln Ala Pro Arg Ala Ala Leu Val Phe Ala Leu Val Ile Ala Leu
   1               5                   10                  15
   Val Pro Val Gly Arg Gly Asn Tyr Glu Glu Leu Glu Asn Ser Gly Asp
               20                  25                  30
   Thr Thr Val Glu Ser Glu Arg Pro Asn Lys Val Thr Ile Pro Ser Thr
           35                  40                  45
   Phe Ala Ala Val Thr Ile Lys Glu Thr Leu Asn Ala Asn Ile Asn Ser
       50                  55                  60
   Thr Asn Phe Ala Pro Asp Glu Asn Gln Leu Glu Phe Ile Leu Met Val
   65                  70                  75                  80
   Leu Ile Pro Leu Ile Leu Leu Val Leu Leu Leu Leu Ser Val Val Phe
                   85                  90                  95
   Leu Ala Thr Tyr Tyr Lys Arg Lys Arg Thr Lys Gln Glu Pro Ser Ser
               100                 105                 110
   Gln Gly Ser Gln Ser Ala Leu Gln Thr Tyr Glu Leu Gly Ser Glu Asn
           115                 120                 125
   Val Lys Val Pro Ile Phe Glu Glu Asp Thr Pro Ser Val Met Glu Ile
       130                 135                 140
   Glu Met Glu Glu Leu Asp Lys Trp Met Asn Ser Met Asn Arg Asn Ala
   145                 150                 155                 160
   Asp Phe Glu Cys Leu Pro Thr Leu Lys Glu Glu Lys Glu Ser Asn His
                   165                 170                 175
   Asn Pro Ser Asp Ser Glu Ser
               180
<210> 43
   <211> 128
   <212> PRT
   <213> Homo sapiens
<400> 43
   Glu Thr Leu Asn Ala Asn Ile Asn Ser Thr Asn Phe Ala Pro Asp Glu
   1               5                   10                  15
   Asn Gln Leu Glu Phe Ile Leu Met Val Leu Ile Pro Leu Ile Leu Leu
               20                  25                  30
   Val Leu Leu Leu Leu Ser Val Val Phe Leu Ala Thr Tyr Tyr Lys Arg
           35                  40                  45
   Lys Arg Thr Lys Gln Glu Pro Ser Ser Gln Gly Ser Gln Ser Ala Leu
       50                  55                  60
   Gln Thr Tyr Glu Leu Gly Ser Glu Asn Val Lys Val Pro Ile Phe Glu
   65                  70                  75                  80
   Glu Asp Thr Pro Ser Val Met Glu Ile Glu Met Glu Glu Leu Asp Lys
                   85                  90                  95
   Trp Met Asn Ser Met Asn Arg Asn Ala Asp Phe Glu Cys Leu Pro Thr
               100                 105                 110
   Leu Lys Glu Glu Lys Glu Ser Asn His Asn Pro Ser Asp Ser Glu Ser
           115                 120                 125
<210> 44
   <211> 183
   <212> PRT
   <213> Homo sapiens
<400> 44
   Met Gln Ala Pro Arg Ala Ala Leu Val Phe Ala Leu Val Ile Ala Leu
   1 5 10 15
   Val Pro Val Gly Arg Gly Asn Tyr Glu Glu Leu Glu Asn Ser Gly Asp
   20 25 30
   Thr Thr Val Glu Ser Glu Arg Pro Asn Lys Val Thr Ile Pro Ser Thr
   35 40 45
   Phe Ala Ala Val Thr Ile Lys Glu Thr Leu Asn Ala Asn Ile Asn Ser
   50 55 60
   Thr Asn Phe Ala Pro Asp Glu Asn Gln Leu Glu Phe Ile Leu Met Val
   65 70 75 80
   Leu Ile Pro Leu Ile Leu Leu Val Leu Leu Leu Leu Phe Val Val Phe
   85 90 95
   Leu Ala Thr Tyr Tyr Lys Arg Lys Arg Thr Lys Gln Glu Pro Ser Ser
   100 105 110
   Gln Gly Ser Gln Ser Ala Leu Gln Thr Tyr Glu Leu Gly Ser Glu Asn
   115 120 125
   Val Lys Val Pro Ile Phe Glu Glu Asp Thr Pro Ser Val Met Glu Ile
   130 135 140
   Glu Met Glu Glu Leu Asp Lys Trp Met Asn Ser Met Asn Arg Asn Ala
   145 150 155 160
   Asp Phe Glu Cys Leu Pro Thr Leu Lys Glu Glu Lys Glu Ser Asn His
   165 170 175
   Asn Pro Ser Asp Ser Glu Ser
   180
<210> 45
   <211> 105
   <212> PRT
   <213> Homo sapiens
<400> 45
<210> 46
   <211> 144
   <212> PRT
   <213> Homo sapiens
<400> 46
   Met Gln Ala Pro Arg Ala Ala Leu Val Phe Ala Leu Val Ile Ala Leu
   1               5                   10                  15
   Val Pro Val Gly Arg Gly Asn Tyr Glu Glu Leu Glu Asn Ser Gly Asp
               20                  25                  30
   Thr Thr Val Glu Ser Glu Arg Pro Asn Lys Val Thr Ile Pro Ser Thr
           35                  40                  45
   Phe Ala Ala Val Thr Ile Lys Glu Thr Leu Asn Ala Asn Ile Asn Ser
       50                  55                  60
   Thr Asn Phe Ala Pro Asp Glu Asn Gln Leu Glu Phe Ile Leu Met Val
   65                  70                  75                  80
   Leu Ile Pro Leu Ile Leu Leu Val Leu Leu Leu Leu Ser Val Val Phe
                   85                  90                  95
   Leu Ala Thr Tyr Tyr Lys Arg Lys Arg Thr Lys Gln Glu Pro Ser Ser
               100                 105                 110
   Gln Gly Ser Gln Ser Ala Leu Gln Thr Cys Lys Ile Gln Leu Ser Trp
           115                 120                 125
   Lys Val Ile Pro Ala Phe Cys Leu Glu Ser Ser His Arg Asn Ala Leu
       130                 135                 140
<210> 47
   <211> 21
   <212> PRT
   <213>
<213> Artificial
<220>
   <223> Synthetic polypeptide
<400> 47
   Thr Val Leu Arg His Val Ala Asn Pro Arg Gly Ala Val Thr Pro Glu
   1               5                   10                  15
   Tyr Thr Val Ala Asn
               20
<210> 48
   <211> 48
   <212> PRT
   <213> Artificial
<220>
   <223> Synthetic polypeptide
<400> 48
   Leu Ala Val Ser Leu Thr Val Ala Asn Gly Gly Arg Arg Leu Ile Ala
   1 5 10 15
   Asp Cys His Pro Gly Leu Leu Asp Pro Leu Val Pro Leu Asp Glu Gly
   20 25 30
   Pro Gly His Thr Asp Cys Pro Phe Asp Pro Thr Arg Ile Tyr Asp Thr
   35 40 45
<210> 49
   <211> 20
   <212> PRT
   <213> Artificial
<220>
   <223> Synthetic polypeptide
<400> 49
   Met Arg Arg Cys Ser Leu Cys Ala Phe Asp Ala Ala Arg Gly Pro Arg
   1 5 10 15
   Arg Leu Met Arg
   20
<210> 50
   <211> 15
   <212> PRT
   <213> Artificial
<220>
   <223> Synthetic polypeptide
<400> 50
   Asp Pro Gly Gly Gly Arg Ala Pro Gly Glu Pro Ser Arg Pro Lys
   1 5 10 15
<210> 51
   <211> 57
   <212> PRT
   <213> Artificial
<220>
   <223> synthetic polypeptide
<400> 51
   Cys Cys Val Ala Ala Leu Thr Leu Arg Gly Val Gly Pro Cys Arg Lys
   1 5 10 15
   Asp Gly Leu Gln Gly Gln Leu Glu Glu Met Thr Glu Leu Glu Ser Pro
   20 25 30
   Lys Cys Lys Arg Gln Glu Asn Glu Gln Leu Leu Asp Gln Asn Gln Glu
   35 40 45
   Ile Arg Ala Ser Gln Arg Ser Trp Val
   50 55
<210> 52
   <211> 9
   <212> PRT
   <213> Artificial
<220>
   <223> Synthetic polypeptide
<400> 52
   Gln Cys Pro Cys Ser Ala Ala Trp Asn
   1 5
<210> 53
   <211> 51
   <212> PRT
   <213> Artificial
<220>
   <223> Synthetic polypeptide
<400> 53
   Glu Cys Ala Ala Thr Gly Ser Ala Ala Phe Ala Gln Arg Leu Cys Leu
   1 5 10 15
   Gly Arg Asn Arg Ser Cys Ala Ala Glu Leu Pro Leu Val Pro Cys Asn
   20 25 30
   Gln Ala Lys Ala Ser Asp Val Gln Asp Leu Leu Lys Asp Leu Lys Ala
   35 40 45
   Gln Ser Gln
   50
<210> 54
   <211> 9
   <212> PRT
   <213> Artificial
<220>
   <223> synthetic polypeptide
<400> 54
   Thr Thr Arg Thr Gln Asn Val Thr Val
   1 5
<210> 55
   <211> 116
   <212> PRT
   <213> Homo sapiens
<400> 55
   Asp Gly Val Phe Ala Ala Arg His Ile Asp Leu Lys Pro Leu Tyr Ala
   1               5                   10                  15
   Asn Arg Cys His Tyr Val Pro Lys Thr Ser Gln Lys Glu Ala Glu Glu
               20                  25                  30
   Val Ile Ser Ser Ser Thr Lys Asn Ser Pro Ser Thr Arg Val Met Arg
           35                  40                  45
   Asn Leu Thr Gln Ala Ala Arg Glu Val Asn Cys Pro His Leu Ser Arg
       50                  55                  60
   Glu Phe Cys Thr Pro Arg Ile Arg Gly Asn Thr Cys Phe Cys Cys Asp
   65                  70                  75                  80
   Leu Tyr Asn Cys Gly Asn Arg Val Glu Ile Thr Gly Gly Tyr Tyr Glu
                   85                  90                  95
   Tyr Ile Asp Val Ser Ser Cys Gln Asp Ile Ile His Leu Tyr His Leu
               100                 105                 110
   Leu Trp Ser Ala
           115
<210> 56
   <211> 92
   <212> PRT
   <213> Homo sapiens
<400> 56
   Asp Gly Val Phe Ala Ala Arg His Ile Asp Leu Lys Pro Leu Tyr Ala
   1               5                   10                  15
   Asn Arg Cys His Tyr Val Pro Lys Thr Ser Gln Lys Glu Ala Glu Glu
               20                  25                  30
   Val Asn Cys Pro His Leu Ser Arg Glu Phe Cys Thr Pro Arg Ile Arg
           35                  40                  45
   Gly Asn Thr Cys Phe Cys Cys Asp Leu Tyr Asn Cys Gly Asn Arg Val
       50                  55                  60
   Glu Ile Thr Gly Gly Tyr Tyr Glu Tyr Ile Asp Val Ser Ser Cys Gln
   65                  70                  75                  80
   Asp Ile Ile His Leu Tyr His Leu Leu Trp Ser Ala
                   85                  90
<210> 57
   <211> 106
   <212> PRT
   <213> Homo sapiens
<400> 57
   Gly Gly Phe Lys Asp Met Asn Pro Thr Leu Pro Ala Leu Asn Cys Ser
   1               5                   10                  15
   Val Glu Asn Thr His Pro Thr Val Ser Tyr Tyr Ala His Pro Gln Val
               20                  25                  30
   Ala Ser Tyr Asn Thr Tyr Tyr His Ser Pro Pro His Leu Pro Pro Tyr
           35                  40                  45
   Ser Ala Tyr Asp Phe Gln His Ser Gly Val Phe Pro Ser Ser Pro Pro
       50                  55                  60
   Ser Gly Leu Ser Asp Glu Pro Gln Ser Ala Ser Pro Ser Pro Ser Tyr
   65                  70                  75                  80
   Met Trp Ser Ser Ser Ala Pro Pro Arg Tyr Ser Pro Pro Tyr Tyr Pro
                   85                  90                  95
   Pro Phe Glu Lys Pro Pro Pro Tyr Ser Pro
               100                 105
<210> 58
   <211> 132
   <212> PRT
   <213> Homo sapiens
<400> 58
   Asp Gly Val Phe Ala Ala Arg His Ile Asp Leu Lys Pro Leu Tyr Ala
   1               5                   10                  15
   Asn Arg Cys His Tyr Val Pro Lys Thr Ser Gln Lys Glu Ala Glu Glu
               20                  25                  30
   Asn Pro Thr Leu Pro Ala Leu Asn Cys Ser Val Glu Asn Thr His Pro
           35                  40                  45
   Thr Val Ser Tyr Tyr Ala His Pro Gln Val Ala Ser Tyr Asn Thr Tyr
       50                  55                  60
   Tyr His Ser Pro Pro His Leu Pro Pro Tyr Ser Ala Tyr Asp Phe Gln
   65                  70                  75                  80
   His Ser Gly Val Phe Pro Ser Ser Pro Pro Ser Gly Leu Ser Asp Glu
                   85                  90                  95
   Pro Gln Ser Ala Ser Pro Ser Pro Ser Tyr Met Trp Ser Ser Ser Ala
               100                 105                 110
   Pro Pro Arg Tyr Ser Pro Pro Tyr Tyr Pro Pro Phe Glu Lys Pro Pro
           115                 120                 125
   Pro Tyr Ser Pro
       130
<210> 59
   <211> 15
   <212> PRT
   <213> Artificial
<220>
   <223> Synthetic polypeptide
<400> 59
   Thr Thr Arg Thr Gln Asn Val Thr Val Gly Gly Tyr Tyr Pro Gly
   1               5                   10                  15
<210> 60
   <211> 55
   <212> PRT
   <213> Homo sapiens
<400> 60
   Met Ser Gly Ala Cys Thr Ser Tyr Val Ser Ala Glu Gln Glu Val Val
   1               5                   10                  15
   Arg Gly Phe Ser Cys Pro Arg Pro Gly Gly Glu Ala Ala Ala Val Phe
               20                  25                  30
   Cys Cys Gly Phe Arg Asp His Lys Tyr Cys Cys Asp Asp Pro His Ser
           35                  40                  45
   Phe Phe Pro Tyr Glu His Ser
       50                  55
<210> 61
   <211> 100
   <212> PRT
   <213> Homo sapiens
<400> 61
   Ser Ser Lys Pro His Thr Lys Leu Asp Leu Gly Leu Ser Leu Gln Thr
   1               5                   10                  15
   Ala Gly Pro Glu Glu Val Ser Pro Asp Cys Gln Gly Val Asn Thr Gly
               20                  25                  30
   Met Ala Ala Glu Val Pro Lys Val Ser Pro Leu Gln Gln Ser Tyr Ser
           35                  40                  45
   Cys Leu Asn Pro Gln Leu Glu Ser Asn Glu Gly Gln Ala Val Asn Ser
       50                  55                  60
   Lys Arg Leu Leu His His Cys Phe Met Ala Thr Val Thr Thr Ser Asp
   65                  70                  75                  80
   Ile Pro Gly Ser Pro Glu Glu Ala Ser Val Pro Asn Pro Asp Leu Cys
                   85                  90                  95
   Gly Pro Val Pro
               100
<210> 62
   <211> 71
   <212> PRT
   <213> Homo sapiens
<400> 62
   Met Ala Ser Leu Trp Pro Ser Ala Leu Thr Phe Asn Thr Asp Ala Asn
   1 5 10 15
   Ile Pro Gly Pro Leu Gly Phe Cys Gly Gly Trp Val Arg Leu Cys Ser
   20 25 30
   Leu Ser Ser Leu Thr Pro Pro Cys Gly Arg Arg Leu Val Pro Cys Leu
   35 40 45
   Ser Ala Pro Ala Pro Asn Ala Pro Arg Leu Pro Ala Pro Ala Arg Cys
   50 55 60
   Ser Ile Gly Ala Leu Ile Gly
   65 70
<210> 63
   <211> 51
   <212> PRT
   <213> Homo sapiens
<400> 63
   Glu Glu Leu Glu Asn Ser Gly Asp Thr Thr Val Glu Ser Glu Arg Pro
   1 5 10 15
   Asn Lys Val Thr Ile Pro Ser Thr Phe Ala Ala Val Thr Ile Lys Glu
   20 25 30
   Thr Leu Asn Ala Asn Ile Asn Ser Thr Asn Phe Ala Pro Asp Glu Asn
   35 40 45
   Gln Leu Glu
   50
<210> 64
   <211> 86
   <212> PRT
   <213> Homo sapiens
<400> 64
   Ala Thr Tyr Tyr Lys Arg Lys Arg Thr Lys Gln Glu Pro Ser Ser Gln
   1 5 10 15
   Gly Ser Gln Ser Ala Leu Gln Thr Tyr Glu Leu Gly Ser Glu Asn Val
   20 25 30
   Lys Val Pro Ile Phe Glu Glu Asp Thr Pro Ser Val Met Glu Ile Glu
   35 40 45
   Met Glu Glu Leu Asp Lys Trp Met Asn Ser Met Asn Arg Asn Ala Asp
   50 55 60
   Phe Glu Cys Leu Pro Thr Leu Lys Glu Glu Lys Glu Ser Asn His Asn
   65 70 75 80
   Pro Ser Asp Ser Glu Ser
   85
<210> 65
   <211> 21
   <212> DNA
   <213> Artificial
<220>
   <223> Synthetic polynucleotide
<400> 65
   aatttgtcaa gtcggtgcag c 21
<210> 66
   <211> 20
   <212> DNA
   <213> Artificial
<220>
   <223> Synthetic polynucleotide
<400> 66
   tcaccccttc atttttgcgt 20
<210> 67
   <211> 106
   <212> DNA
   <213> Artificial
<220>
   <223> Synthetic polynucleotide
<400> 67
   aatttgtcaa gtcggtgcag ctggcaagac ctaaaggatt atatgcgtca ggcaggagaa 60
   gtgacttatg cagatgctca caagggacgc aaaaatgaag gggtga 106
<210> 68
   <211> 21
   <212> DNA
   <213> Artificial
<220>
   <223> Synthetic polynucleotide
<400> 68
   gctccaggcc ataaggactt c 21
<210> 69
   <211> 21
   <212> DNA
   <213> Artificial
<220>
   <223> Synthetic polynucleotide
<400> 69
   cagcttcaaa ctctcccctg c 21
<210> 70
   <211> 103
   <212> DNA
   <213> Artificial
<220>
   <223> Synthetic polynucleotide
<400> 70
   gctccaggcc ataaggactt cattccaaat atgattacag gagcagccca ggcggatgta 60
   gctgttttag ttgtagatgc cagcagggga gagtttgaag ctg 103
<210> 71
   <211> 20
   <212> DNA
   <213> Artificial
<220>
   <223> Synthetic polynucleotide
<400> 71
   ttccttgcca ggacctagag 20
<210> 72
   <211> 20
   <212> DNA
   <213> Artificial
<220>
   <223> Synthetic polynucleotide
<400> 72
   cataaacctt tcgccttgac 20
<210> 73
   <211> 128
   <212> DNA
   <213> Artificial
<220>
   <223> Synthetic polynucleotide
<400> 73
   ttccttgcca ggacctagag tttgttcagt tccaccccac aggcatatat ggtgctggtt 60
   gtctcattac ggaaggatgt cgtggagagg gaggcattct cattaacagt caaggcgaaa 120
   ggtttatg 128
<210> 74
   <211> 20
   <212> DNA
   <213> Artificial
<220>
   <223> Synthetic polynucleotide
<400> 74
   cagccaggtt atgccaacac 20
<210> 75
   <211> 21
   <212> DNA
   <213> Artificial
<220>
   <223> Synthetic polynucleotide
<400> 75
   tcaaagcagg cgaacttcat c 20
<210> 76
   <211> 140
   <212> DNA
   <213> Artificial
<220>
   <223> Synthetic polynucleotide
<400> 76
   cagccaggtt atgccaacac tttgagggat gcagctccca aaatgtataa ggaagaaggc 60
   ctaaaagcat tctacaaggg ggttgctcct ctctggatga gacagatacc atacaccatg 120
   atgaagttcg cctgctttga 140
<210> 77
   <211> 20
   <212> DNA
   <213> Artificial
<220>
   <223> Synthetic polynucleotide
<400> 77
   cggtttgctg cggtaatcat 20
<210> 78
   <211> 21
   <212> DNA
   <213> Artificial
<220>
   <223> Synthetic polynucleotide
<400> 78
   tttcttgctg ccagtctgga c 21
<210> 79
   <211> 122
   <212> DNA
   <213> Artificial
<220>
   <223> Synthetic polynucleotide
<400> 79
   cggtttgctg cggtaatcat gaggataaga gagccacgaa ccacggcact gattttcagt 60
   tctgggaaaa tggtgtgcac aggagccaag agtgaagaac agtccagact ggcagcaaga 120
   aa 122
<210> 80
   <211> 19
   <212> DNA
   <213> Artificial
<220>
   <223> synthetic polynucleotide
<400> 80
   atttgggtcg cggttcttg 19
<210> 81
   <211> 21
   <212> DNA
   <213> Artificial
<220>
   <223> Synthetic polynucleotide
<400> 81
   tgccttgaca ttctcgatgg t 21
<210> 82
   <211> 133
   <212> DNA
   <213> Artificial
<220>
   <223> synthetic polynucleotide
<400> 82
   atttgggtcg cggttcttgt ttgtggatcg ctgtgatcgt cacttgacaa tgcagatctt 60
   cgtgaagact ctgactggta agaccatcac cctcgaggtt gagcccagtg acaccatcga 120
   gaatgtcaag gca 133
<210> 83
   <211> 20
   <212> DNA
   <213> Artificial
<220>
   <223> synthetic polynucleotide
<400> 83
   tgggaacaag agggcatctg 20
<210> 84
   <211> 22
   <212> DNA
   <213> Artificial
<220>
   <223> Synthetic polynucleotide
<400> 84
   ccaccactgc atcaaattca tg 22
<210> 85
   <211> 86
   <212> DNA
   <213> Artificial
<220>
   <223> Synthetic polynucleotide
<400> 85
   tgggaacaag agggcatctg ctaaagtttc agattccatt tctgctcagt atccagtagt 60
   ggatcatgaa tttgatgcag tggtgg 86
<210> 86
   <211> 21
   <212> DNA
   <213> Artificial
<220>
   <223> Synthetic polynucleotide
<400> 86
   tgacactggc aaaacaatgc a 21
<210> 87
   <211> 21
   <212> DNA
   <213> Artificial
<220>
   <223> synthetic polynucleotide
<400> 87
   ggtccttttc accagcaagc t 21
<210> 88
   <211> 94
   <212> DNA
   <213> Artificial
<220>
   <223> Synthetic polynucleotide
<400> 88
   tgacactggc aaaacaatgc agactttgct ttccttggtc aggcagtata atccaaagat 60
   ggtcaaggtc gcaagcttgc tggtgaaaag gacc 94
<210> 89
   <211> 20
   <212> DNA
   <213> Artificial
<220>
   <223> Synthetic polynucleotide
<400> 89
   gaggccgtca ccaagaacat 20
<210> 90
   <211> 19
   <212> DNA
   <213> Artificial
<220>
   <223> Synthetic polynucleotide
<400> 90
   ggacagccgg tcagagctc 19
<210> 91
   <211> 111
   <212> DNA
   <213> Artificial
<220>
   <223> Synthetic polynucleotide
<400> 91
   gaggccgtca ccaagaacat tcacgagtcc tgcatgagcc agataggctg gaaccgcatc 60
   atcgtggaga agcccttcgg gagggacctg cagagctctg accggctgtc c 111
<210> 92
   <211> 19
   <212> DNA
   <213> Artificial
<220>
   <223> synthetic polynucleotide
<400> 92
   cccctttgac cccacaaga 19
<210> 93
   <211> 21
   <212> DNA
   <213> Artificial
<220>
   <223> synthetic polynucleotide
<400> 93
   cagccacaca gcagtaacca g 21
<210> 94
   <211> 107
   <212> DNA
   <213> Artificial
<220>
   <223> synthetic polynucleotide
<400> 94
   cccctttgac cccacaagaa tctatgatac agccttggct ctctggatcc cttctttgct 60
   catgtctgca ggggaggctg ctctatctgg ttactgctgt gtggctg 107
<210> 95
   <211> 16
   <212> DNA
   <213> Artificial
<220>
   <223> Synthetic polynucleotide
<400> 95
   ggccaaggcg tcggac 16
<210> 96
   <211> 25
   <212> DNA
   <213> Artificial
<220>
   <223> synthetic polynucleotide
<400> 96
   gaaaactaac tggagatagg catcg 25
<210> 97
   <211> 143
   <212> DNA
   <213> Artificial
<220>
   <223> Synthetic polynucleotide
<400> 97
   ggccaaggcg tcggacgtgc aggacctcct gaaggatctg aaggctcagt cgcaggtgtt 60
   gggctggatc ttgatagcag ttgttatcat cattcttctg atttttacat ctgtcacccg 120
   atgcctatct ccagttagtt ttc 143
<210> 98
   <211> 18
   <212> DNA
   <213> Artificial
<220>
   <223> Synthetic polynucleotide
<400> 98
   tcggacgtgc aggacctc 18
<210> 99
   <211> 21
   <212> DNA
   <213> Artificial
<220>
   <223> Synthetic polynucleotide
<400> 99
   gagataggca tcgggtgaca g 21
<210> 100
   <211> 121
   <212> DNA
   <213> Artificial
<220>
   <223> Synthetic polynucleotide
<400> 100
   tcggacgtgc aggacctcct gaaggatctg aaggctcagt cgcaggtgtt gggctggatc 60
   ttgatagcag ttgttatcat cattcttctg atttttacat ctgtcacccg atgcctatct 120
   c 121
<210> 101
   <211> 26
   <212> DNA
   <213> Artificial
<220>
   <223> Synthetic polynucleotide
<400> 101
   ctggtggctt ctatcgtgtt tatcag 26
<210> 102
   <211> 23
   <212> DNA
   <213> Artificial
<220>
   <223> Synthetic polynucleotide
<400> 102
   cggttagcgt agagtggttt cag 23
<210> 103
   <211> 116
   <212> DNA
   <213> Artificial
<220>
   <223> Synthetic polynucleotide
<400> 103
   ctggtggctt ctatcgtgtt tatcagcttt ggtgtgattg cggctttttg ttgtgccata 60
   gttgacgggg tctttgctgc cagacacatt gatctgaaac cactctacgc taaccg 116
<210> 104
   <211> 25
   <212> DNA
   <213> Artificial
<220>
   <223> Synthetic polynucleotide
<400> 104
   ccttctagcc aaggatctca gagtg 25
<210> 105
   <211> 26
   <212> DNA
   <213> Artificial
<220>
   <223> Synthetic polynucleotide
<400> 105
   cttgggttgt gatttgattc cttctc 26
<210> 106
   <211> 209
   <212> DNA
   <213> Artificial
<220>
   <223> Synthetic polynucleotide
<400> 106
   ccttctagcc aaggatctca gagtgcttta cagacatatg aactgggaag tgaaaacgtg 60
   aaagtcccta tttttgagga agatacaccc tctgttatgg aaattgaaat ggaagagctt 120
   gataaatgga tgaacagcat gaatagaaat gccgactttg aatgtttacc taccttgaag 180
   gaagagaagg aatcaaatca caacccaag 209
<210> 107
   <211> 21
   <212> DNA
   <213> Artificial
<220>
   <223> Synthetic polynucleotide
<400> 107
   cagcattggc gctctcatag g 21
<210> 108
   <211> 22
   <212> DNA
   <213> Artificial
<220>
   <223> Synthetic polynucleotide
<400> 108
   gtgttcacac cttggcagtc ag 22
<210> 109
   <211> 183
   <212> DNA
   <213> Artificial
<220>
   <223> Synthetic polynucleotide
<400> 109
   cagcattggc gctctcatag gcctgtccgt agcagcagtg gttcttctcg ccttcattgt     60
   taccgcctgt gtgctctgct acctgttcat cagctctaag ccccacacaa agttggacct    120
   gggcttgagc ttacagacag caggccctga ggaggtttct cctgactgcc aaggtgtgaa    180
   cac                                                                  183
<210> 110
   <211> 1455
   <212> DNA
   <213> Homo sapiens
<400> 110
   atgaggcgct gcagtctctg cgctttcgac gccgcccggg ggcccaggcg gctgatgcgt     60
   gtgggcctcg cgctgatctt ggtgggccac gtgaacctgc tgctgggggc cgtgctgcat    120
   ggcaccgtcc tgcggcacgt ggccaatccc cgcggcgctg tcacgccgga gtacaccgta    180
   gccaatgtca tctctgtcgg ctcggggctg ctgagcgttt ccgtgggact tgtggccctc    240
   ctggcgtcca ggaaccttct tcgccctcca ctgcactggg tcctgctggc actagctctg    300
   gtgaacctgc tcttgtccgt tgcctgctcc ctgggcctcc ttcttgctgt gtcactcact    360
   gtggccaacg gtggccgccg ccttattgct gactgccacc caggactgct ggatcctctg    420
   gtaccactgg atgaggggcc gggacatact gactgcccct ttgaccccac aagaatctat    480
   gatacagcct tggctctctg gatcccttct ttgctcatgt ctgcagggga ggctgctcta 540
   tctggttact gctgtgtggc tgcactcact ctacgtggag ttgggccctg caggaaggac 600
   ggacttcagg ggcagctaga ggaaatgaca gagcttgaat ctcctaaatg taaaaggcag 660
   gaaaatgagc agctactgga tcaaaatcaa gaaatccggg catcacagag aagttgggtt 720
   ggaccggtcg ccaccatggt gagcaagggc gaggagctgt tcaccggggt ggtgcccatc 780
   ctggtcgagc tggacggcga cgtaaacggc cacaagttca gcgtgtccgg cgagggcgag 840
   ggcgatgcca cctacggcaa gctgaccctg aagttcatct gcaccaccgg caagctgccc 900
   gtgccctggc ccaccctcgt gaccaccctg acctacggcg tgcagtgctt cagccgctac 960
   cccgaccaca tgaagcagca cgacttcttc aagtccgcca tgcccgaagg ctacgtccag 1020
   gagcgcacca tcttcttcaa ggacgacggc aactacaaga cccgcgccga ggtgaagttc 1080
   gagggcgaca ccctggtgaa ccgcatcgag ctgaagggca tcgacttcaa ggaggacggc 1140
   aacatcctgg ggcacaagct ggagtacaac tacaacagcc acaacgtcta tatcatggcc 1200
   gacaagcaga agaacggcat caaggtgaac ttcaagatcc gccacaacat cgaggacggc 1260
   agcgtgcagc tcgccgacca ctaccagcag aacaccccca tcggcgacgg ccccgtgctg 1320
   ctgcccgaca accactacct gagcacccag tccgccctga gcaaagaccc caacgagaag 1380
   cgcgatcaca tggtcctgct ggagttcgtg accgccgccg ggatcactct cggcatggac 1440
   gagctgtaca agtaa 1455
<210> 111
   <211> 1437
   <212> DNA
   <213> Homo sapiens
<400> 111
   atggtgagca agggcgagga gctgttcacc ggggtggtgc ccatcctggt cgagctggac 60
   ggcgacgtaa acggccacaa gttcagcgtg tccggcgagg gcgagggcga tgccacctac 120
   ggcaagctga ccctgaagtt catctgcacc accggcaagc tgcccgtgcc ctggcccacc 180
   ctcgtgacca ccctgaccta cggcgtgcag tgcttcagcc gctaccccga ccacatgaag 240
   cagcacgact tcttcaagtc cgccatgccc gaaggctacg tccaggagcg caccatcttc 300
   ttcaaggacg acggcaacta caagacccgc gccgaggtga agttcgaggg cgacaccctg 360
   gtgaaccgca tcgagctgaa gggcatcgac ttcaaggagg acggcaacat cctggggcac 420
   aagctggagt acaactacaa cagccacaac gtctatatca tggccgacaa gcagaagaac 480
   ggcatcaagg tgaacttcaa gatccgccac aacatcgagg acggcagcgt gcagctcgcc 540
   gaccactacc agcagaacac ccccatcggc gacggccccg tgctgctgcc cgacaaccac 600
   tacctgagca cccagtccgc cctgagcaaa gaccccaacg agaagcgcga tcacatggtc 660
   ctgctggagt tcgtgaccgc cgccgggatc actctcggca tggacgagct gtacaagagg 720
   cgctgcagtc tctgcgcttt cgacgccgcc cgggggccca ggcggctgat gcgtgtgggc 780
   ctcgcgctga tcttggtggg ccacgtgaac ctgctgctgg gggccgtgct gcatggcacc 840
   gtcctgcggc acgtggccaa tccccgcggc gctgtcacgc cggagtacac cgtagccaat 900
   gtcatctctg tcggctcggg gctgctgagc gtttccgtgg gacttgtggc cctcctggcg 960
   tccaggaacc ttcttcgccc tccactgcac tgggtcctgc tggcactagc tctggtgaac 1020
   ctgctcttgt ccgttgcctg ctccctgggc ctccttcttg ctgtgtcact cactgtggcc 1080
   aacggtggcc gccgccttat tgctgactgc cacccaggac tgctggatcc tctggtacca 1140
   ctggatgagg ggccgggaca tactgactgc ccctttgacc ccacaagaat ctatgataca 1200
   gccttggctc tctggatccc ttctttgctc atgtctgcag gggaggctgc tctatctggt 1260
   tactgctgtg tggctgcact cactctacgt ggagttgggc cctgcaggaa ggacggactt 1320
   caggggcagc tagaggaaat gacagagctt gaatctccta aatgtaaaag gcaggaaaat 1380
   gagcagctac tggatcaaaa tcaagaaatc cgggcatcac agagaagttg ggtttag 1437
<210> 112
   <211> 723
   <212> DNA
   <213> Homo sapiens
<400> 112
   atgaggcgct gcagtctctg cgctttcgac gccgcccggg ggcccaggcg gctgatgcgt     60
   gtgggcctcg cgctgatctt ggtgggccac gtgaacctgc tgctgggggc cgtgctgcat    120
   ggcaccgtcc tgcggcacgt ggccaatccc cgcggcgctg tcacgccgga gtacaccgta    180
   gccaatgtca tctctgtcgg ctcggggctg ctgagcgttt ccgtgggact tgtggccctc    240
   ctggcgtcca ggaaccttct tcgccctcca ctgcactggg tcctgctggc actagctctg    300
   gtgaacctgc tcttgtccgt tgcctgctcc ctgggcctcc ttcttgctgt gtcactcact    360
   gtggccaacg gtggccgccg ccttattgct gactgccacc caggactgct ggatcctctg    420
   gtaccactgg atgaggggcc gggacatact gactgcccct ttgaccccac aagaatctat    480
   gatacagcct tggctctctg gatcccttct ttgctcatgt ctgcagggga ggctgctcta    540
   tctggttact gctgtgtggc tgcactcact ctacgtggag ttgggccctg caggaaggac    600
   ggacttcagg ggcagctaga ggaaatgaca gagcttgaat ctcctaaatg taaaaggcag    660
   gaaaatgagc agctactgga tcaaaatcaa gaaatccggg catcacagag aagttgggtt    720
   tag                                                                  723
<210> 113
   <211> 484
   <212> PRT
   <213> Homo sapiens
<400> 113
<210> 114
   <211> 478
   <212> PRT
   <213> Homo sapiens
<400> 114
<210> 115
   <211> 16
   <212> PRT
   <213> Artificial
<220>
   <223> Synthetic polypeptide
<400> 115
   Glu Gln Leu Leu Asp Gln Asn Gln Glu Ile Arg Ala Ser Gln Arg Ser
   1               5                   10                  15
<210> 116
   <211> 16
   <212> PRT
   <213> Artificial
<220>
   <223> Synthetic polypeptide
<400> 116
   Leu Asp Glu Gly Pro Gly His Thr Asp Cys Pro Phe Asp Pro Thr Arg
   1               5                   10                  15
<210> 117
   <211> 13
   <212> PRT
   <213> Artificial
<220>
   <223> Synthetic polypeptide
<400> 117
   Glu Lys Phe Arg Ala Val Leu Asp Leu His Val Lys His
   1               5                   10
<210> 118
   <211> 15
   <212> PRT
   <213> Artificial
<220>
   <223> synthetic polypeptide
<400> 118
   Cys Asn Gln Ala Lys Ala Ser Asp Val Gln Asp Leu Leu Lys Asp
   1               5                   10                  15
<210> 119
   <211> 1002
   <212> DNA
   <213> Homo sapiens
<400> 119
   atgcatcagt ccctgactca gcagcggtcc agcgacatgt ccctgcccga ttccatggga      60
   gcattcaatc ggaggaaacg aaactccatc tatgtcaccg tgactttgct tattgtgtcc     120
   gtgttaattc tcacagtggg ccttgctgca accaccagga cccagaatgt gactgtagga     180
   ggttattacc ccggagttat tctcggcttt ggatcgttcc ttggaatcat tggatcaaac     240
   cttattgaga acaaaaggca gatgctggtg gcttctatcg tgtttatcag ctttggtgtg     300
   attgcggctt tttgttgtgc catagttgac ggggtctttg ctgccagaca cattgatctg     360
   aaaccactct acgctaaccg gtgccattat gttcccaaga catcacagaa ggaagctgag     420
   gaggttaact gccctcacct cagccgtgaa ttctgcacac ctcgcatccg gggcaacacc     480
   tgcttctgct gtgacctcta caactgtggc aaccgggtgg agatcactgg tgggtactac     540
   gaatacatcg atgtcagcag ttgccaagat atcatccacc tctaccacct gctctggtct     600
   gccaccatcc tcaacattgt tggcctgttc ctgggcatca tcactgccgc tgtccttgga     660
   ggctttaagg acatgaaccc aactctccca gcactgaact gttctgttga aaatacccat     720
   ccaacagttt cttactatgc tcatccccaa gtggcatcct acaataccta ctaccatagc     780
   cctcctcacc tgccaccata ttctgcttat gactttcagc attccggtgt ctttccatcc     840
   tcccctccct ctggactttc tgatgagccc cagtctgcct ctccctcacc cagctacatg     900
   tggtcctcaa gtgcaccgcc ccgttactct ccaccctact atccaccttt tgaaaagcca     960
   ccaccttaca gtcccgacta caaagacgat gacgacaagt aa                      1002
<210> 120
   <211> 333
   <212> PRT
   <213> Homo sapiens
<400> 120
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Gly Phe Gly Ser Phe Leu Gly Ile Ile Gly Ser Asn
   65                  70                  75                  80
   Leu Ile Glu Asn Lys Arg Gln Met Leu Val Ala Ser Ile Val Phe Ile
                   85                  90                  95
   Ser Phe Gly Val Ile Ala Ala Phe Cys Cys Ala Ile Val Asp Gly Val
               100                 105                 110
   Phe Ala Ala Arg His Ile Asp Leu Lys Pro Leu Tyr Ala Asn Arg Cys
           115                 120                 125
   His Tyr Val Pro Lys Thr Ser Gln Lys Glu Ala Glu Glu Val Asn Cys
       130                 135                 140
   Pro His Leu Ser Arg Glu Phe Cys Thr Pro Arg Ile Arg Gly Asn Thr
   145                 150                 155                 160
   Cys Phe Cys Cys Asp Leu Tyr Asn Cys Gly Asn Arg Val Glu Ile Thr
                   165                 170                 175
   Gly Gly Tyr Tyr Glu Tyr Ile Asp Val Ser Ser Cys Gln Asp Ile Ile
               180                 185                 190
   His Leu Tyr His Leu Leu Trp Ser Ala Thr Ile Leu Asn Ile Val Gly
           195                 200                 205
   Leu Phe Leu Gly Ile Ile Thr Ala Ala Val Leu Gly Gly Phe Lys Asp
       210                 215                 220
   Met Asn Pro Thr Leu Pro Ala Leu Asn Cys Ser Val Glu Asn Thr His
   225                 230                 235                 240
   Pro Thr Val Ser Tyr Tyr Ala His Pro Gln Val Ala Ser Tyr Asn Thr
                   245                 250                 255
   Tyr Tyr His Ser Pro Pro His Leu Pro Pro Tyr Ser Ala Tyr Asp Phe
               260                 265                 270
   Gln His Ser Gly Val Phe Pro Ser Ser Pro Pro Ser Gly Leu Ser Asp
           275                 280                 285
   Glu Pro Gln Ser Ala Ser Pro Ser Pro Ser Tyr Met Trp Ser Ser Ser
       290                 295                 300
   Ala Pro Pro Arg Tyr Ser Pro Pro Tyr Tyr Pro Pro Phe Glu Lys Pro
   305                 310                 315                 320
   Pro Pro Tyr Ser Pro Asp Tyr Lys Asp Asp Asp Asp Lys
                   325                 330
<210> 121
   <211> 15
   <212> PRT
   <213> Artificial
<220>
   <223> Synthetic polypeptide
<400> 121
   Cys His Tyr Val Pro Lys Thr Ser Gln Lys Glu Ala Glu Glu Val
   1               5                   10                  15
<210> 122
   <211> 23
   <212> DNA
   <213> Artificial
<220>
   <223> Synthetic polynucleotide
<400> 122
   ctgaaggatc tgaaggctca gtc 23
<210> 123
   <211> 20
   <212> DNA
   <213> Artificial
<220>
   <223> Synthetic polynucleotide
<400> 123
   ggatctgctg ctcctgttcc 20
<210> 124
   <211> 163
   <212> DNA
   <213> Artificial
<220>
   <223> Synthetic polynucleotide
<400> 124
   ctgaaggatc tgaaggctca gtcgcaggtg ttgggctgga tcttgatagc agttgttatc 60
   atcattcttc tgatttttac atctgtcacc cgatgcctat ctccagttag ttttctgcag 120
   ctgaaattct ggaaaatcta tttggaacag gagcagcaga tcc 163
<210> 125
   <211> 669
   <212> DNA
   <213> Homo sapiens
<400> 125
   gaaggagatc aggcagagga gagaagagtc agtgtgaact aggctggtgg ctctgtccgg 60
   tagggagacg gaaagggtgc cgcccatcat agaagtacca gaacttgagc tggactttgc 120
   tgatttagct tatggaagag gaaccagaaa tttgtccttg aataatgttt cccgtgttgg 180
   gctggatctt gatagcagtt gttatcatca ttcttctgat ttttacatct gtcacccgat 240
   gcctatctcc agttagtttt ctgcagctga aattctggaa aatctatttg gaacaggagc 300
   agcagatcct taaaagtaaa gccacagagc atgcaactga attggcaaaa gagaatatta 360
   aatgtttctt tgagggctcg catccaaaag aatataacac tccaagcatg aaagagtggc 420
   agcaaatttc atcactgtat actttcaatc cgaagggcca gtactacagc atgttgcaca 480
   aatatgtcaa cagaaaagag aagactcaca gtatcaggtc tactgaagga gatacggtga 540
   ttcctgttct tggctttgta gattcatctg gtataaacag cactcctgag ttatgacctt 600
   ttgaatgagt agaaaaaaaa attgttttga attattgctt tattaaaaaa taaacattgg 660
   tattttttg 669
<210> 126
   <211> 101
   <212> DNA
   <213> Artificial
<220>
   <223> Synthetic polynucleotide
<400> 126
   aatcattatg ctgaggattt ggaaagggtg tttattcctc atggactaat tatggacagg 60
   actgaacgtc ttgctcgaga tgtgatgaag gagatgggag g 101
<210> 127
   <211> 117
   <212> PRT
   <213> Homo sapiens
<400> 127
   Leu Ser Pro Val Ser Phe Leu Gln Leu Lys Phe Trp Lys Ile Tyr Leu
   1               5                   10                  15
   Glu Gln Glu Gln Gln Ile Leu Lys Ser Lys Ala Thr Glu His Ala Thr
               20                  25                  30
   Glu Leu Ala Lys Glu Asn Ile Lys Cys Phe Phe Glu Gly Ser His Pro
           35                  40                  45
   Lys Glu Tyr Asn Thr Pro Ser Met Lys Glu Trp Gln Gln Ile Ser Ser
       50                  55                  60
   Leu Tyr Thr Phe Asn Pro Lys Gly Gln Tyr Tyr Ser Met Leu His Lys
   65                  70                  75                  80
   Tyr Val Asn Arg Lys Glu Lys Thr His Ser Ile Arg Ser Thr Glu Gly
                   85                  90                  95
   Asp Thr Val Ile Pro Val Leu Gly Phe Val Asp Ser Ser Gly Ile Asn
   100 105 110
   Ser Thr Pro Glu Leu
   115
<210> 128
   <211> 24
   <212> DNA
   <213> Artificial
<220>
   <223> Synthetic polynucleotide
<400> 128
   aacatcatgg atcagaacaa cagc 24
<210> 129
   <211> 26
   <212> DNA
   <213> Artificial
<220>
   <223> Synthetic polynucleotide
<400> 129
   atcattggac taaagatagg gattcc 26
<210> 130
   <211> 101
   <212> DNA
   <213> Artificial
<220>
   <223> Synthetic polynucleotide
<400> 130
   aacatcatgg atcagaacaa cagcctgcca ccttacgctc agggcttggc ctcccctcag 60
   ggtgccatga ctcccggaat ccctatcttt agtccaatga t 101
<210> 131
   <211> 888
   <212> DNA
   <213> Homo sapiens
<400> 131
   gtccgctccg gctcctgccg cgttccagca gccgtcactc ccgccgccgg cccccgccgc     60
   ccgccccgcc tgcccctacc cctccgcgcg ggccgggcac ctggccgccg ctcggtcctc    120
   ggggccccgc gctgctgtct ctgtctcggc ttctctcggc ccctgggtct cttcgtctct    180
   gccgttctca ggctcagctc cgtctcgctc ggtccctcgc ttccccgccg ggctctagcc    240
   ggccgtctgg tggcccgagg tggcggcggg ctgggcgcgg ggcgcgatga gcggcgcctg    300
   cacgagctac gtgagcgcag agcaggaggt ggtgcgcggc ttcagctgcc cgcggccggg    360
   gggcgaggcg gccgctgtct tctgctgcgg cttccgcgac cacaagtact gctgcgacga    420
   cccgcacagc ttcttcccct acgagcacag ctacatgtgg tggctcagca ttggcgctct    480
   cataggcctg tccgtagcag cagtggttct tctcgccttc attgttaccg cctgtgtgct    540
   ctgctacctg ttcatcagct ctaagcccca cacaaagttg gacctgggct tgagcttaca    600
   gacagcaggc cctgaggagg tttctcctga ctgccaaggt gtgaacacag gcatggcggc    660
   agaagtgcca aaagtgagcc ctctccagca gagttactcc tgcttgaacc cgcagctgga    720
   gagcaatgag gggcaggctg tgaactccaa acgcctcctc catcattgct tcatggccac    780
   agtgaccacc agtgacattc caggcagccc tgaggaagcc tctgtaccca accctgacct    840
   atgtggacca gtcccataaa cattcaataa atgtctccat accatccc                 888
<210> 132
   <211> 72
   <212> PRT
   <213> Homo sapiens
<400> 132
   Met Ser Gly Ala Cys Thr Ser Tyr Val Ser Ala Glu Gln Glu Val Val
   1               5                   10                  15
   Arg Gly Phe Ser Cys Pro Arg Pro Gly Gly Glu Ala Ala Ala Val Phe
               20                  25                  30
   Cys Cys Gly Phe Arg Asp His Lys Tyr Cys Cys Asp Asp Pro His Ser
           35                  40                  45
   Phe Phe Pro Tyr Glu His Ser Tyr Met Trp Trp Leu Arg Asp Gly Val
       50                  55                  60
   Ser Pro Cys Cys Pro Gly Trp Ser
   65                  70
<210> 133
   <211> 153
   <212> PRT
   <213> Homo sapiens
<400> 133
   Met Ser Gly Ala Cys Thr Ser Tyr Val Ser Ala Glu Gln Glu Val Val
   1               5                   10                  15
   Arg Gly Phe Ser Cys Pro Arg Pro Gly Gly Glu Ala Ala Ala Val Phe
               20                  25                  30
   Cys Cys Gly Phe Arg Asp His Lys Tyr Cys Cys Asp Asp Pro His Ser
           35                  40                  45
   Phe Phe Pro Tyr Glu His Ser Tyr Met Trp Trp Leu Ser Ile Gly Ala
       50                  55                  60
   Leu Ile Gly Leu Ser Val Ala Ala Val Val Leu Leu Ala Phe Ile Val
   65                  70                  75                  80
   Thr Ala Cys Val Leu Cys Tyr Leu Phe Ile Ser Ser Lys Pro His Thr
                   85                  90                  95
   Lys Leu Asp Leu Gly Leu Ser Leu Gln Thr Ala Gly Pro Val Gln Val
               100                 105                 110
   Ala Thr Asn Lys Arg Asn Gln Thr Val Leu Thr Leu Lys Gly Leu Thr
           115                 120                 125
   Trp Arg Thr Arg Gln Ala His Lys Trp Pro Gly Gly Pro Val Val Ala
       130                 135                 140
   Leu His Gly Ala Leu Gly Arg Lys Lys
   145                 150
<210> 134
   <211> 134
   <212> PRT
   <213> Homo sapiens
<400> 134
   Met Trp Trp Leu Ser Ile Gly Ala Leu Ile Gly Leu Ser Val Ala Ala
   1               5                   10                  15
   Val Val Leu Leu Ala Phe Ile Val Thr Ala Cys Val Leu Cys Tyr Leu
               20                  25                  30
   Phe Ile Ser Ser Lys Pro His Thr Lys Leu Asp Leu Gly Leu Ser Leu
           35                  40                  45
   Gln Thr Ala Gly Pro Glu Glu Val Ser Pro Asp Cys Gln Gly Val Asn
       50                  55                  60
   Thr Gly Met Ala Ala Glu Val Pro Lys Val Ser Pro Leu Gln Gln Ser
   65                  70                  75                  80
   Tyr Ser Cys Leu Asn Pro Gln Leu Glu Ser Asn Glu Gly Gln Ala Val
                   85                  90                  95
   Asn Ser Lys Arg Leu Leu His His Cys Phe Met Ala Thr Val Thr Thr
               100                 105                 110
   Ser Asp Ile Pro Gly Ser Pro Glu Glu Ala Ser Val Pro Asn Pro Asp
           115                 120                 125
   Leu Cys Gly Pro Val Pro
       130
<210> 135
   <211> 190
   <212> PRT
   <213> Homo sapiens
<400> 135
   Met Ser Gly Ala Cys Thr Ser Tyr Val Ser Ala Glu Gln Glu Val Val
   1               5                   10                  15
   Arg Gly Phe Ser Cys Pro Arg Pro Gly Gly Glu Ala Ala Ala Val Phe
               20                  25                  30
   Cys Cys Gly Phe Arg Asp His Lys Tyr Cys Cys Asp Asp Pro His Ser
           35                  40                  45
   Phe Phe Pro Tyr Glu His Ser Tyr Met Trp Trp Leu Ser Ile Gly Ala
       50                  55                  60
   Leu Ile Gly Leu Ser Val Ala Ala Val Val Leu Leu Ala Phe Ile Val
   65                  70                  75                  80
   Thr Ala Cys Val Leu Cys Tyr Leu Phe Ile Ser Ser Lys Pro His Thr
                   85                  90                  95
   Lys Leu Asp Leu Gly Leu Ser Leu Gln Thr Ala Gly Pro Glu Glu Val
               100                 105                 110
   Ser Pro Asp Cys Gln Gly Val Asn Thr Gly Met Ala Ala Glu Val Pro
           115                 120                 125
   Lys Val Ser Pro Leu Gln Gln Ser Tyr Ser Cys Leu Asn Pro Gln Leu
       130                 135                 140
   Glu Ser Asn Glu Gly Gln Ala Val Asn Ser Lys Arg Leu Leu His His
   145                 150                 155                 160
   Cys Phe Met Ala Thr Val Thr Thr Ser Asp Ile Pro Gly Ser Pro Glu
                   165                 170                 175
   Glu Ala Ser Val Pro Asn Pro Asp Leu Cys Gly Pro Val Pro
               180                 185                 190
<210> 136
   <211> 2405
   <212> DNA
   <213> Homo sapiens
<400> 136
   tccggcgtgg tgcgcaggcg cggtatcccc cctcccccgc cagctcgacc ccggtgtggt    60
   gcgcaggcgc agtctgcgca gggactggcg ggactgcgcg gcggcaacag cagacatgtc   120
   gggggtccgg ggcctgtcgc ggctgctgag cgctcggcgc ctggcgctgg ccaaggcgtg   180
   gccaacagtg ttgcaaacag gaacccgagg ttttcacttc actgttgatg ggaacaagag   240
   ggcatctgct aaagtttcag attccatttc tgctcagtat ccagtagtgg atcatgaatt   300
   tgatgcagtg gtggtaggcg ctggaggggc aggcttgcga gctgcatttg gcctttctga   360
   ggcagggttt aatacagcat gtgttaccaa gctgtttcct accaggtcac acactgttgc   420
   agcacaggga ggaatcaatg ctgctctggg gaacatggag gaggacaact ggaggtggca   480
   tttctacgac accgtgaagg gctccgactg gctgggggac caggatgcca tccactacat   540
   gacggagcag gcccccgccg ccgtggtcga gctagaaaat tatggcatgc cgtttagcag   600
   aactgaagat gggaagattt atcagcgtgc atttggtgga cagagcctca agtttggaaa   660
   gggcgggcag gcccatcggt gctgctgtgt ggctgatcgg actggccact cgctattgca   720
   caccttatat ggaaggtctc tgcgatatga taccagctat tttgtggagt attttgcctt   780
   ggatctcctg atggagaatg gggagtgccg tggtgtcatc gcactgtgca tagaggacgg   840
   gtccatccat cgcataagag caaagaacac tgttgttgcc acaggaggct acgggcgcac   900
   ctacttcagc tgcacgtctg cccacaccag cactggcgac ggcacggcca tgatcaccag   960
   ggcaggcctt ccttgccagg acctagagtt tgttcagttc caccctacag gcatatatgg  1020
   tgctggttgt ctcattacgg aaggatgtcg tggagaggga ggcattctca ttaacagtca  1080
   aggcgaaagg tttatggagc gatacgcccc tgtcgcgaag gacctggcgt ctagagatgt  1140
   ggtgtctcgg tccatgactc tggagatccg agaaggaaga ggctgtggcc ctgagaaaga  1200
   tcacgtctac ctgcagctgc accacctacc tccagagcag ctggccacgc gcctgcctgg  1260
   catttcagag acagccatga tcttcgctgg cgtggacgtc acgaaggagc cgatccctgt  1320
   cctccccacc gtgcattata acatgggcgg cattcccacc aactacaagg ggcaggtcct 1380
   gaggcacgtg aatggccagg atcagattgt gcccggcctg tacgcctgtg gggaggccgc 1440
   ctgtgcctcg gtacatggtg ccaaccgcct cggggcaaac tcgctcttgg acctggttgt 1500
   ctttggtcgg gcatgtgccc tgagcatcga agagtcatgc aggcctggag ataaagtccc 1560
   tccaattaaa ccaaacgctg gggaagaatc tgtcatgaat cttgacaaat tgagatttgc 1620
   tgatggaagc ataagaacat cggaactgcg actcagcatg cagaagtcaa tgcaaaatca 1680
   tgctgccgtg ttccgtgtgg gaagcgtgtt gcaagaaggt tgtgggaaaa tcagcaagct 1740
   ctatggagac ctaaagcacc tgaagacgtt cgaccgggga atggtctgga acacggacct 1800
   ggtggagacc ctggagctgc agaacctgat gctgtgtgcg ctgcagacca tctacggagc 1860
   agaggcacgg aaggagtcac ggggcgcgca tgccagggaa gactacaagg tgcggattga 1920
   tgagtacgat tactccaagc ccatccaggg gcaacagaag aagccctttg aggagcactg 1980
   gaggaagcac accctgtcct atgtggacgt tggcactggg aaggtcactc tggaatatag 2040
   acccgtgatc gacaaaactt tgaacgaggc tgactgtgcc accgtcccgc cagccattcg 2100
   ctcctactga tgagacaaga tgtggtgatg acagaatcag cttttgtaat tatgtataat 2160
   agctcatgca tgtgtccatg tcataactgt cttcatacgc ttctgcactc tggggaagaa 2220
   ggagtacatt gaagggagat tggcacctag tggctgggag cttgccagga acccagtggc 2280
   cagggagcgt ggcacttacc tttgtccctt gcttcattct tgtgagatga taaaactggg 2340
   cacagctctt aaataaaata taaatgaaca aactttcttt tatttccaaa aaaaaaaaaa 2400
   aaaaa 2405
<210> 137
   <211> 1435
   <212> DNA
   <213> Homo sapiens
<400> 137
   ggcggggcct gcttctcctc agcttcaggc ggctgcgacg agccctcagg cgaacctctc      60
   ggctttcccg cgcggcgccg cctcttgctg cgcctccgcc tcctcctctg ctccgccacc     120
   ggcttcctcc tcctgagcag tcagcccgcg cgccggccgg ctccgttatg gcgacccgca     180
   gccctggcgt cgtgattagt gatgatgaac caggttatga ccttgattta ttttgcatac     240
   ctaatcatta tgctgaggat ttggaaaggg tgtttattcc tcatggacta attatggaca     300
   ggactgaacg tcttgctcga gatgtgatga aggagatggg aggccatcac attgtagccc     360
   tctgtgtgct caaggggggc tataaattct ttgctgacct gctggattac atcaaagcac      420
   tgaatagaaa tagtgataga tccattccta tgactgtaga ttttatcaga ctgaagagct     480
   attgtaatga ccagtcaaca ggggacataa aagtaattgg tggagatgat ctctcaactt     540
   taactggaaa gaatgtcttg attgtggaag atataattga cactggcaaa acaatgcaga     600
   ctttgctttc cttggtcagg cagtataatc caaagatggt caaggtcgca agcttgctgg     660
   tgaaaaggac cccacgaagt gttggatata agccagactt tgttggattt gaaattccag     720
   acaagtttgt tgtaggatat gcccttgact ataatgaata cttcagggat ttgaatcatg     780
   tttgtgtcat tagtgaaact ggaaaagcaa aatacaaagc ctaagatgag agttcaagtt     840
   gagtttggaa acatctggag tcctattgac atcgccagta aaattatcaa tgttctagtt     900
   ctgtggccat ctgcttagta gagctttttg catgtatctt ctaagaattt tatctgtttt     960
   gtactttaga aatgtcagtt gctgcattcc taaactgttt atttgcacta tgagcctata    1020
   gactatcagt tccctttggg cggattgttg tttaacttgt aaatgaaaaa attctcttaa    1080
   accacagcac tattgagtga aacattgaac tcatatctgt aagaaataaa gagaagatat    1140
   attagttttt taattggtat tttaattttt atatatgcag gaaagaatag aagtgattga    1200
   atattgttaa ttataccacc gtgtgttaga aaagtaagaa gcagtcaatt ttcacatcaa    1260
   agacagcatc taagaagttt tgttctgtcc tggaattatt ttagtagtgt ttcagtaatg    1320
   ttgactgtat tttccaactt gttcaaatta ttaccagtga atctttgtca gcagttccct    1380
   tttaaatgca aatcaataaa ttcccaaaaa tttaaaaaaa aaaaaaaaaa aaaaa         1435
<210> 138
   <211> 2395
   <212> DNA
   <213> Homo sapiens
<400> 138
   agaggcaggg gctggcctgg gatgcgcgcg cacctgccct cgccccgccc cgcccgcacg      60
   aggggtggtg gccgaggccc cgccccgcac gcctcgcctg aggcgggtcc gctcagccca     120
   ggcgcccgcc cccgcccccg ccgattaaat gggccggcgg ggctcagccc ccggaaacgg    180
   tcgtacactt cggggctgcg agcgcggagg gcgacgacga cgaagcgcag acagcgtcat     240
   ggcagagcag gtggccctga gccggaccca ggtgtgcggg atcctgcggg aagagctttt     300
   ccagggcgat gccttccatc agtcggatac acacatattc atcatcatgg gtgcatcggg     360
   tgacctggcc aagaagaaga tctaccccac catctggtgg ctgttccggg atggccttct     420
   gcccgaaaac accttcatcg tgggctatgc ccgttcccgc ctcacagtgg ctgacatccg     480
   caaacagagt gagcccttct tcaaggccac cccagaggag aagctcaagc tggaggactt     540
   ctttgcccgc aactcctatg tggctggcca gtacgatgat gcagcctcct accagcgcct     600
   caacagccac atgaatgccc tccacctggg gtcacaggcc aaccgcctct tctacctggc     660
   cttgcccccg accgtctacg aggccgtcac caagaacatt cacgagtcct gcatgagcca     720
   gataggctgg aaccgcatca tcgtggagaa gcccttcggg agggacctgc agagctctga     780
   ccggctgtcc aaccacatct cctccctgtt ccgtgaggac cagatctacc gcatcgacca     840
   ctacctgggc aaggagatgg tgcagaacct catggtgctg agatttgcca acaggatctt     900
   cggccccatc tggaaccggg acaacatcgc ctgcgttatc ctcaccttca aggagccctt     960
   tggcactgag ggtcgcgggg gctatttcga tgaatttggg atcatccggg acgtgatgca    1020
   gaaccaccta ctgcagatgc tgtgtctggt ggccatggag aagcccgcct ccaccaactc    1080
   agatgacgtc cgtgatgaga aggtcaaggt gttgaaatgc atctcagagg tgcaggccaa 1140
   caatgtggtc ctgggccagt acgtggggaa ccccgatgga gagggcgagg ccaccaaagg 1200
   gtacctggac gaccccacgg tgccccgcgg gtccaccacc gccacttttg cagccgtcgt 1260
   cctctatgtg gagaatgaga ggtgggatgg ggtgcccttc atcctgcgct gcggcaaggc 1320
   cctgaacgag cgcaaggccg aggtgaggct gcagttccat gatgtggccg gcgacatctt 1380
   ccaccagcag tgcaagcgca acgagctggt gatccgcgtg cagcccaacg aggccgtgta 1440
   caccaagatg atgaccaaga agccgggcat gttcttcaac cccgaggagt cggagctgga 1500
   cctgacctac ggcaacagat acaagaacgt gaagctccct gacgcctacg agcgcctcat 1560
   cctggacgtc ttctgcggga gccagatgca cttcgtgcgc agcgacgagc tccgtgaggc 1620
   ctggcgtatt ttcaccccac tgctgcacca gattgagctg gagaagccca agcccatccc 1680
   ctatatttat ggcagccgag gccccacgga ggcagacgag ctgatgaaga gagtgggttt 1740
   ccagtatgag ggcacctaca agtgggtgaa cccccacaag ctctgagccc tgggcaccca 1800
   cctccacccc cgccacggcc accctccttc ccgccgcccg accccgagtc gggaggactc 1860
   cgggaccatt gacctcagct gcacattcct ggccccgggc tctggccacc ctggcccgcc 1920
   cctcgctgct gctactaccc gagcccagct acattcctca gctgccaagc actcgagacc 1980
   atcctggccc ctccagaccc tgcctgagcc caggagctga gtcacctcct ccactcactc 2040
   cagcccaaca gaaggaagga ggagggcgcc cattcgtctg tcccagagct tattggccac 2100
   tgggtctcac tcctgagtgg ggccagggtg ggagggaggg acaaggggga ggaaaggggc 2160
   gagcacccac gtgagagaat ctgcctgtgg ccttgcccgc cagcctcagt gccacttgac 2220
   attccttgtc accagcaaca tctcgagccc cctggatgtc ccctgtccca ccaactctgc 2280
   actccatggc caccccgtgc cacccgtagg cagcctctct gctataagaa aagcagacgc 2340
   agcagctggg accccctcca acctcaatgc cctgccatta aatccgcaaa cagcc 2395
<210> 139
   <211> 2201
   <212> DNA
   <213> Homo sapiens
<400> 139
   cgggatttgg gtcgcggttc ttgtttgtgg atcgctgtga tcgtcacttg acaatgcaga 60
   tcttcgtgaa gactctgact ggtaagacca tcaccctcga ggttgagccc agtgacacca 120
   tcgagaatgt caaggcaaag atccaagata aggaaggcat ccctcctgac cagcagaggc 180
   tgatctttgc tggaaaacag ctggaagatg ggcgcaccct gtctgactac aacatccaga 240
   aagagtccac cctgcacctg gtgctccgtc tcagaggtgg gatgcaaatc ttcgtgaaga 300
   cactcactgg caagaccatc acccttgagg tggagcccag tgacaccatc gagaacgtca 360
   aagcaaagat ccaggacaag gaaggcattc ctcctgacca gcagaggttg atctttgccg 420
   gaaagcagct ggaagatggg cgcaccctgt ctgactacaa catccagaaa gagtctaccc 480
   tgcacctggt gctccgtctc agaggtggga tgcagatctt cgtgaagacc ctgactggta 540
   agaccatcac cctcgaggtg gagcccagtg acaccatcga gaatgtcaag gcaaagatcc 600
   aagataagga aggcattcct cctgatcagc agaggttgat ctttgccgga aaacagctgg 660
   aagatggtcg taccctgtct gactacaaca tccagaaaga gtccaccttg cacctggtac 720
   tccgtctcag aggtgggatg caaatcttcg tgaagacact cactggcaag accatcaccc 780
   ttgaggtcga gcccagtgac actatcgaga acgtcaaagc aaagatccaa gacaaggaag 840
   gcattcctcc tgaccagcag aggttgatct ttgccggaaa gcagctggaa gatgggcgca 900
   ccctgtctga ctacaacatc cagaaagagt ctaccctgca cctggtgctc cgtctcagag 960
   gtgggatgca gatcttcgtg aagaccctga ctggtaagac catcaccctc gaagtggagc 1020
   cgagtgacac cattgagaat gtcaaggcaa agatccaaga caaggaaggc atccctcctg 1080
   accagcagag gttgatcttt gccggaaaac agctggaaga tggtcgtacc ctgtctgact 1140
   acaacatcca gaaagagtcc accttgcacc tggtgctccg tctcagaggt gggatgcaga 1200
   tcttcgtgaa gaccctgact ggtaagacca tcactctcga ggtggagccg agtgacacca 1260
   ttgagaatgt caaggcaaag atccaagaca aggaaggcat ccctcctgat cagcagaggt 1320
   tgatctttgc tgggaaacag ctggaagatg gacgcaccct gtctgactac aacatccaga 1380
   aagagtccac cctgcacctg gtgctccgtc ttagaggtgg gatgcagatc ttcgtgaaga 1440
   ccctgactgg taagaccatc actctcgaag tggagccgag tgacaccatt gagaatgtca 1500
   aggcaaagat ccaagacaag gaaggcatcc ctcctgacca gcagaggttg atctttgctg 1560
   ggaaacagct ggaagatgga cgcaccctgt ctgactacaa catccagaaa gagtccaccc 1620
   tgcacctggt gctccgtctt agaggtggga tgcagatctt cgtgaagacc ctgactggta 1680
   agaccatcac tctcgaagtg gagccgagtg acaccattga gaatgtcaag gcaaagatcc 1740
   aagacaagga aggcatccct cctgaccagc agaggttgat ctttgctggg aaacagctgg 1800
   aagatggacg caccctgtct gactacaaca tccagaaaga gtccaccctg cacctggtgc 1860
   tccgtctcag aggtgggatg cagatcttcg tgaagaccct gactggtaag accatcaccc 1920
   tcgaggtgga gcccagtgac accatcgaga atgtcaaggc aaagatccaa gataaggaag 1980
   gcatccctcc tgatcagcag aggttgatct ttgctgggaa acagctggaa gatggacgca 2040
   ccctgtctga ctacaacatc cagaaagagt ccactctgca cttggtcctg cgcttgaggg 2100
   ggggtgtcta agtttcccct tttaaggttt caacaaattt cattgcactt tcctttcaat 2160
   aaagttgttg cattcccaaa aaaaaaaaaa aaaaaaaaaa a 2201
<210> 140
   <211> 1867
   <212> DNA
   <213> Homo sapiens
<400> 140
   ggttcgctgt ggcgggcgcc tgggccgccg gctgtttaac ttcgcttccg ctggcccata      60
   gtgatctttg cagtgaccca gcagcatcac tgtttcttgg cgtgtgaaga taacccaagg     120
   aattgaggaa gttgctgaga agagtgtgct ggagatgctc taggaaaaaa ttgaatagtg     180
   agacgagttc cagcgcaagg gtttctggtt tgccaagaag aaagtgaaca tcatggatca     240
   gaacaacagc ctgccacctt acgctcaggg cttggcctcc cctcagggtg ccatgactcc     300
   cggaatccct atctttagtc caatgatgcc ttatggcact ggactgaccc cacagcctat     360
   tcagaacacc aatagtctgt ctattttgga agagcaacaa aggcagcagc agcaacaaca     420
   acagcagcag cagcagcagc agcagcaaca gcaacagcag cagcagcagc agcagcagca     480
   gcagcagcag cagcagcagc agcagcagca gcaacaggca gtggcagctg cagccgttca     540
   gcagtcaacg tcccagcagg caacacaggg aacctcaggc caggcaccac agctcttcca     600
   ctcacagact ctcacaactg cacccttgcc gggcaccact ccactgtatc cctcccccat     660
   gactcccatg acccccatca ctcctgccac gccagcttcg gagagttctg ggattgtacc     720
   gcagctgcaa aatattgtat ccacagtgaa tcttggttgt aaacttgacc taaagaccat     780
   tgcacttcgt gcccgaaacg ccgaatataa tcccaagcgg tttgctgcgg taatcatgag     840
   gataagagag ccacgaacca cggcactgat tttcagttct gggaaaatgg tgtgcacagg     900
   agccaagagt gaagaacagt ccagactggc agcaagaaaa tatgctagag ttgtacagaa     960
   gttgggtttt ccagctaagt tcttggactt caagattcag aatatggtgg ggagctgtga    1020
   tgtgaagttt cctataaggt tagaaggcct tgtgctcacc caccaacaat ttagtagtta    1080
   tgagccagag ttatttcctg gtttaatcta cagaatgatc aaacccagaa ttgttctcct    1140
   tatttttgtt tctggaaaag ttgtattaac aggtgctaaa gtcagagcag aaatttatga    1200
   agcatttgaa aacatctacc ctattctaaa gggattcagg aagacgacgt aatggctctc    1260
   atgtaccctt gcctccccca cccccttctt tttttttttt taaacaaatc agtttgtttt    1320
   ggtaccttta aatggtggtg ttgtgagaag atggatgttg agttgcaggg tgtggcacca    1380
   ggtgatgccc ttctgtaagt gcccaccgcg ggatgccggg aaggggcatt atttgtgcac    1440
   tgagaacacc gcgcagcgtg actgtgagtt gctcataccg tgctgctatc tgggcagcgc    1500
   tgcccattta tttatatgta gattttaaac actgctgttg acaagttggt ttgagggaga    1560
   aaactttaag tgttaaagcc acctctataa ttgattggac tttttaattt taatgttttt    1620
   ccccatgaac cacagttttt atatttctac cagaaaagta aaaatctttt ttaaaagtgt    1680
   tgtttttcta atttataact cctaggggtt atttctgtgc cagacacatt ccacctctcc    1740
   agtattgcag gacagaatat atgtgttaat gaaaatgaat ggctgtacat atttttttct    1800
   ttcttcagag tactctgtac aataaatgca gtttataaaa gtgttaaaaa aaaaaaaaaa    1860
   aaaaaaa                                                              1867
<210> 141
   <211> 684
   <212> PRT
   <213> Homo sapiens
<400> 141
   Met Ala Arg His Arg Asn Val Arg Gly Tyr Asn Tyr Asp Glu Asp Phe
   1               5                   10                  15
   Glu Asp Asp Asp Leu Tyr Gly Gln Ser Val Glu Asp Asp Tyr Cys Ile
               20                  25                  30
   Ser Pro Ser Thr Ala Ala Gln Phe Ile Tyr Ser Arg Arg Asp Lys Pro
           35                  40                  45
   Ser Val Glu Pro Val Glu Glu Tyr Asp Tyr Glu Asp Leu Lys Glu Ser
       50                  55                  60
   Ser Asn Ser Val Ser Asn His Gln Leu Ser Gly Phe Asp Gln Ala Arg
   65                  70                  75                  80
   Leu Tyr Ser Cys Leu Asp His Met Arg Glu Val Leu Gly Asp Ala Val
                   85                  90                  95
   Pro Asp Glu Ile Leu Ile Glu Ala Val Leu Lys Asn Lys Phe Asp Val
               100                 105                 110
   Gln Lys Ala Leu Ser Gly Val Leu Glu Gln Asp Arg Val Gln Ser Leu
           115                 120                 125
   Lys Asp Lys Asn Glu Ala Thr Val Ser Thr Gly Lys Ile Ala Lys Gly
       130                 135                 140
   Lys Pro Val Asp Ser Gln Thr Ser Arg Ser Glu Ser Glu Ile Val Pro
   145                 150                 155                 160
   Lys Val Ala Lys Met Thr Val Ser Gly Lys Lys Gln Thr Met Gly Phe
                   165                 170                 175
   Glu Val Pro Gly Val Ser Ser Glu Glu Asn Gly His Ser Phe His Thr
               180                 185                 190
   Pro Gln Lys Gly Pro Pro Ile Glu Asp Ala Ile Ala Ser Ser Asp Val
           195                 200                 205
   Leu Glu Thr Ala Ser Lys Ser Ala Asn Pro Pro His Thr Ile Gln Ala
       210                 215                 220
   Ser Glu Glu Gln Ser Ser Thr Pro Ala Pro Val Lys Lys Ser Gly Lys
   225                 230                 235                 240
   Leu Arg Gln Gln Ile Asp Val Lys Ala Glu Leu Glu Lys Arg Gln Gly
                   245                 250                 255
   Gly Lys Gln Leu Leu Asn Leu Val Val Ile Gly His Val Asp Ala Gly
               260                 265                 270
   Lys Ser Thr Leu Met Gly His Met Leu Tyr Leu Leu Gly Asn Ile Asn
           275                 280                 285
   Lys Arg Thr Met His Lys Tyr Glu Gln Glu Ser Lys Lys Ala Gly Lys
       290                 295                 300
   Ala Ser Phe Ala Tyr Ala Trp Val Leu Asp Glu Thr Gly Glu Glu Arg
   305                 310                 315                 320
   Glu Arg Gly Val Thr Met Asp Val Gly Met Thr Lys Phe Glu Thr Thr
                   325                 330                 335
   Thr Lys Val Ile Thr Leu Met Asp Ala Pro Gly His Lys Asp Phe Ile
               340                 345                 350
   Pro Asn Met Ile Thr Gly Ala Ala Gln Ala Asp Val Ala Val Leu Val
           355                 360                 365
   Val Asp Ala Ser Arg Gly Glu Phe Glu Ala Gly Phe Glu Thr Gly Gly
       370                 375                 380
   Gln Thr Arg Glu His Gly Leu Leu Val Arg Ser Leu Gly Val Thr Gln
   385                 390                 395                 400
   Leu Ala Val Ala Val Asn Lys Met Asp Gln Val Asn Trp Gln Gln Glu
                   405                 410                 415
   Arg Phe Gln Glu Ile Thr Gly Lys Leu Gly His Phe Leu Lys Gln Ala
               420                 425                 430
   Gly Phe Lys Glu Ser Asp Val Gly Phe Ile Pro Thr Ser Gly Leu Ser
           435                 440                 445
   Gly Glu Asn Leu Ile Thr Arg Ser Gln Ser Ser Glu Leu Thr Lys Trp
       450                 455                 460
   Tyr Lys Gly Leu Cys Leu Leu Glu Gln Ile Asp Ser Phe Lys Pro Pro
   465                 470                 475                 480
   Gln Arg Ser Ile Asp Lys Pro Phe Arg Leu Cys Val Ser Asp Val Phe
                   485                 490                 495
   Lys Asp Gln Gly Ser Gly Phe Cys Ile Thr Gly Lys Ile Glu Ala Gly
               500                 505                 510
   Tyr Ile Gln Thr Gly Asp Arg Leu Leu Ala Met Pro Pro Asn Glu Thr
           515                 520                 525
   Cys Thr Val Lys Gly Ile Thr Leu His Asp Glu Pro Val Asp Trp Ala
       530                 535                 540
   Ala Ala Gly Asp His Val Ser Leu Thr Leu Val Gly Met Asp Ile Ile
   545                 550                 555                 560
   Lys Ile Asn Val Gly Cys Ile Phe Cys Gly Pro Lys Val Pro Ile Lys
                   565                 570                 575
   Ala Cys Thr Arg Phe Arg Ala Arg Ile Leu Ile Phe Asn Ile Glu Ile
               580                 585                 590
   Pro Ile Thr Lys Gly Phe Pro Val Leu Leu His Tyr Gln Thr Val Ser
           595                 600                 605
   Glu Pro Ala Val Ile Lys Arg Leu Ile Ser Val Leu Asn Lys Ser Thr
       610                 615                 620
   Gly Glu Val Thr Lys Lys Lys Pro Lys Phe Leu Thr Lys Gly Gln Asn
   625                 630                 635                 640
   Ala Leu Val Glu Leu Gln Thr Gln Arg Pro Ile Ala Leu Glu Leu Tyr
                   645                 650                 655
   Lys Asp Phe Lys Glu Leu Gly Arg Phe Met Leu Arg Tyr Gly Gly Ser
               660                 665                 670
   Thr Ile Ala Ala Gly Val Val Thr Glu Ile Lys Glu
           675                 680
<210> 142
   <211> 664
   <212> PRT
   <213> Homo sapiens
<400> 142
<210> 143
   <211> 494
   <212> PRT
   <213> Homo sapiens
<400> 143
   Met Pro Arg Val Tyr Ile Gly Arg Leu Ser Tyr Gln Ala Arg Glu Arg
   1               5                   10                  15
   Asp Val Glu Arg Phe Phe Lys Gly Tyr Gly Lys Ile Leu Glu Val Asp
               20                  25                  30
   Leu Lys Asn Gly Tyr Gly Phe Val Glu Phe Asp Asp Leu Arg Asp Ala
           35                  40                  45
   Asp Asp Ala Val Tyr Glu Leu Asn Gly Lys Asp Leu Cys Gly Glu Arg
       50                  55                  60
   Val Ile Val Glu His Ala Arg Gly Pro Arg Arg Asp Gly Ser Tyr Gly
   65                  70                  75                  80
   Ser Gly Arg Ser Gly Tyr Gly Tyr Arg Arg Ser Gly Arg Asp Lys Tyr
                   85                  90                  95
   Gly Pro Pro Thr Arg Thr Glu Tyr Arg Leu Ile Val Glu Asn Leu Ser
               100                 105                 110
   Ser Arg Cys Ser Trp Gln Asp Leu Lys Asp Tyr Met Arg Gln Ala Gly
           115                 120                 125
   Glu Val Thr Tyr Ala Asp Ala His Lys Gly Arg Lys Asn Glu Gly Val
       130                 135                 140
   Ile Glu Phe Val Ser Tyr Ser Asp Met Lys Arg Ala Leu Glu Lys Leu
   145                 150                 155                 160
   Asp Gly Thr Glu Val Asn Gly Arg Lys Ile Arg Leu Val Glu Asp Lys
                   165                 170                 175
   Pro Gly Ser Arg Arg Arg Arg Ser Tyr Ser Arg Ser Arg Ser His Ser
               180                 185                 190
   Arg Ser Arg Ser Arg Ser Arg His Ser Arg Lys Ser Arg Ser Arg Ser
           195                 200                 205
   Gly Ser Ser Lys Ser Ser His Ser Lys Ser Arg Ser Arg Ser Arg Ser
       210                 215                 220
   Gly Ser Arg Ser Arg Ser Lys Ser Arg Ser Arg Ser Gln Ser Arg Ser
   225                 230                 235                 240
   Arg Ser Lys Lys Glu Lys Ser Arg Ser Pro Ser Lys Glu Lys Ser Arg
                   245                 250                 255
   Ser Arg Ser His Ser Ala Gly Lys Ser Arg Ser Lys Ser Lys Asp Gln
               260                 265                 270
   Ala Glu Glu Lys Ile Gln Asn Asn Asp Asn Val Gly Lys Pro Lys Ser
           275                 280                 285
   Arg Ser Pro Ser Arg His Lys Ser Lys Ser Lys Ser Arg Ser Arg Ser
       290                 295                 300
   Gln Glu Arg Arg Val Glu Glu Glu Lys Arg Gly Ser Val Ser Arg Gly
   305                 310                 315                 320
   Arg Ser Gln Glu Lys Ser Leu Arg Gln Ser Arg Ser Arg Ser Arg Ser
                   325                 330                 335
   Lys Gly Gly Ser Arg Ser Arg Ser Arg Ser Arg Ser Lys Ser Lys Asp
               340                 345                 350
   Lys Arg Lys Gly Arg Lys Arg Ser Arg Glu Glu Ser Arg Ser Arg Ser
           355                 360                 365
   Arg Ser Arg Ser Lys Ser Glu Arg Ser Arg Lys Arg Gly Ser Lys Arg
       370                 375                 380
   Asp Ser Lys Ala Gly Ser Ser Lys Lys Lys Lys Lys Glu Asp Thr Asp
   385                 390                 395                 400
   Arg Ser Gln Ser Arg Ser Pro Ser Arg Ser Val Ser Lys Glu Arg Glu
   405 410 415
   His Ala Lys Ser Glu Ser Ser Gln Arg Glu Gly Arg Gly Glu Ser Glu
   420 425 430
   Asn Ala Gly Thr Asn Gln Glu Thr Arg Ser Arg Ser Arg Ser Asn Ser
   435 440 445
   Lys Ser Lys Pro Asn Leu Pro Ser Glu Ser Arg Ser Arg Ser Lys Ser
   450 455 460
   Ala Ser Lys Thr Arg Ser Arg Ser Lys Ser Arg Ser Arg Ser Ala Ser
   465 470 475 480
   Arg Ser Pro Ser Arg Ser Arg Ser Arg Ser His Ser Arg Ser
   485 490
<210> 144
   <211> 376
   <212> PRT
   <213> Homo sapiens
<400> 144
   Thr Phe Pro Arg Glu Trp Leu Cys Asp Arg His Leu Arg Glu Lys Met
   1 5 10 15
   Phe Ser Ser Val Ala His Leu Ala Arg Ala Asn Pro Phe Asn Thr Pro
   20 25 30
   His Leu Gln Leu Val His Asp Gly Leu Gly Asp Leu Arg Ser Ser Ser
   35 40 45
   Pro Gly Pro Thr Gly Gln Pro Arg Arg Pro Arg Asn Leu Ala Ala Ala
   50 55 60
   Ala Val Glu Glu Tyr Ser Cys Glu Phe Gly Ser Ala Lys Tyr Tyr Ala
   65 70 75 80
   Leu Cys Gly Phe Gly Gly Val Leu Ser Cys Gly Leu Thr His Thr Ala
   85 90 95
   Val Val Pro Leu Asp Leu Val Lys Cys Arg Met Gln Val Asp Pro Gln
   100 105 110
   Lys Tyr Lys Gly Ile Phe Asn Gly Phe Ser Val Thr Leu Lys Glu Asp
   115 120 125
   Gly Val Arg Gly Leu Ala Lys Gly Trp Ala Pro Thr Phe Leu Gly Tyr
   130 135 140
   Ser Met Gln Gly Leu Cys Lys Phe Gly Phe Tyr Glu Val Phe Lys Val
   145 150 155 160
   Leu Tyr Ser Asn Met Leu Gly Glu Glu Asn Thr Tyr Leu Trp Arg Thr
   165 170 175
   Ser Leu Tyr Leu Ala Ala Ser Ala Ser Ala Glu Phe Phe Ala Asp Ile
   180 185 190
   Ala Leu Ala Pro Met Glu Ala Ala Lys Val Arg Ile Gln Thr Gln Pro
   195 200 205
   Gly Tyr Ala Asn Thr Leu Arg Asp Ala Ala Pro Lys Met Tyr Lys Glu
   210 215 220
   Glu Gly Leu Lys Ala Phe Tyr Lys Gly Val Ala Pro Leu Trp Met Arg
   225 230 235 240
   Gln Ile Pro Tyr Thr Met Met Lys Phe Ala Cys Phe Glu Arg Thr Val
   245 250 255
   Glu Ala Leu Tyr Lys Phe Val Val Pro Lys Pro Arg Ser Glu Cys Ser
   260 265 270
   Lys Pro Glu Gln Leu Val Val Thr Phe Val Ala Gly Tyr Ile Ala Gly
   275 280 285
   Val Phe Cys Ala Ile Val Ser His Pro Ala Asp Ser Val Val Ser Val
   290 295 300
   Leu Asn Lys Glu Lys Gly Ser Ser Ala Ser Leu Val Leu Lys Arg Leu
   305 310 315 320
   Gly Phe Lys Gly Val Trp Lys Gly Leu Phe Ala Arg Ile Ile Met Ile
   325 330 335
   Gly Thr Leu Thr Ala Leu Gln Trp Phe Ile Tyr Asp Ser Val Lys Val
   340 345 350
   Tyr Phe Arg Leu Pro Arg Pro Pro Pro Pro Glu Met Pro Glu Ser Leu
   355 360 365
   Lys Lys Lys Leu Gly Leu Thr Gln
   370 375
<210> 145
   <211> 339
   <212> PRT
   <213> Homo sapiens
<400> 145
   Met Asp Gln Asn Asn Ser Leu Pro Pro Tyr Ala Gln Gly Leu Ala Ser
   1               5                   10                  15
   Pro Gln Gly Ala Met Thr Pro Gly Ile Pro Ile Phe Ser Pro Met Met
               20                  25                  30
   Pro Tyr Gly Thr Gly Leu Thr Pro Gln Pro Ile Gln Asn Thr Asn Ser
           35                  40                  45
   Leu Ser Ile Leu Glu Glu Gln Gln Arg Gln Gln Gln Gln Gln Gln Gln
       50                  55                  60
   Gln Gln Gln Gln Gln Gln Gln Gln Gln Gln Gln Gln Gln Gln Gln Gln
   65                  70                  75                  80
   Gln Gln Gln Gln Gln Gln Gln Gln Gln Gln Gln Gln Gln Gln Gln Ala
                   85                  90                  95
   Val Ala Ala Ala Ala Val Gln Gln Ser Thr Ser Gln Gln Ala Thr Gln
               100                 105                 110
   Gly Thr Ser Gly Gln Ala Pro Gln Leu Phe His Ser Gln Thr Leu Thr
           115                 120                 125
   Thr Ala Pro Leu Pro Gly Thr Thr Pro Leu Tyr Pro Ser Pro Met Thr
       130                 135                 140
   Pro Met Thr Pro Ile Thr Pro Ala Thr Pro Ala Ser Glu Ser Ser Gly
   145                 150                 155                 160
   Ile Val Pro Gln Leu Gln Asn Ile Val Ser Thr Val Asn Leu Gly Cys
                   165                 170                 175
   Lys Leu Asp Leu Lys Thr Ile Ala Leu Arg Ala Arg Asn Ala Glu Tyr
               180                 185                 190
   Asn Pro Lys Arg Phe Ala Ala Val Ile Met Arg Ile Arg Glu Pro Arg
           195                 200                 205
   Thr Thr Ala Leu Ile Phe Ser Ser Gly Lys Met Val Cys Thr Gly Ala
       210                 215                 220
   Lys Ser Glu Glu Gln Ser Arg Leu Ala Ala Arg Lys Tyr Ala Arg Val
   225                 230                 235                 240
   Val Gln Lys Leu Gly Phe Pro Ala Lys Phe Leu Asp Phe Lys Ile Gln
                   245                 250                 255
   Asn Met Val Gly Ser Cys Asp Val Lys Phe Pro Ile Arg Leu Glu Gly
               260                 265                 270
   Leu Val Leu Thr His Gln Gln Phe Ser Ser Tyr Glu Pro Glu Leu Phe
           275                 280                 285
   Pro Gly Leu Ile Tyr Arg Met Ile Lys Pro Arg Ile Val Leu Leu Ile
       290                 295                 300
   Phe Val Ser Gly Lys Val Val Leu Thr Gly Ala Lys Val Arg Ala Glu
   305                 310                 315                 320
   Ile Tyr Glu Ala Phe Glu Asn Ile Tyr Pro Ile Leu Lys Gly Phe Arg
                   325                 330                 335
   Lys Thr Thr
<210> 146
   <211> 26
   <212> PRT
   <213> Artificial
<220>
   <223> Synthetic polypeptide
<400> 146
   Val Ser Ala Ala Gly Asp Pro Gly Gly Gly Arg Ala Pro Gly Glu Pro
   1               5                   10                  15
   Ser Arg Pro Lys Ala Leu Cys Leu Pro Gln
               20                  25
<210> 147
   <211> 16
   <212> PRT
   <213> Artificial
<220>
   <223> Synthetic polypeptide
<400> 147
   Val Arg Lys Ala Asn Arg Lys Gly Ser Phe His Arg Asp Trp Leu Cys
   1               5                   10                  15
<210> 148
   <211> 26
   <212> PRT
   <213> Artificial
<220>
   <223> Synthetic polypeptide
<400> 148
   Val Val Ala Gly Cys Asp Ala Arg Val Lys Gln Lys Ala Trp Gln Pro
   1               5                   10                  15
   Arg Phe Pro Gly Ile Lys Val Lys Ala Leu
               20                  25
<210> 149
   <211> 18
   <212> PRT
   <213> Artificial
<220>
   <223> Synthetic polypeptide
<400> 149
   Arg Trp Gly Arg Leu Gly Gly Ala Glu Arg Pro Ser Phe Leu Arg Ala
   1               5                   10                  15
   Ala Gly
<210> 150
   <211> 56
   <212> PRT
   <213> Artificial
<220>
   <223> Synthetic polypeptide
<400> 150
   Met Ser Gly Ala Cys Thr Ser Tyr Val Ser Ala Glu Gln Glu Val Val
   1               5                   10                  15
   Arg Gly Phe Ser Cys Pro Arg Pro Gly Gly Glu Ala Ala Ala Val Phe
               20                  25                  30
   Cys Cys Gly Phe Arg Asp His Lys Tyr Cys Cys Asp Asp Pro His Ser
           35                  40                  45
   Phe Phe Pro Tyr Glu His Ser Tyr
       50                  55
<210> 151
   <211> 81
   <212> PRT
   <213> Artificial
<220>
   <223> Synthetic polypeptide
<400> 151
   Glu Glu Val Ser Pro Asp Cys Gln Gly Val Asn Thr Gly Met Ala Ala
   1               5                   10                  15
   Glu Val Pro Lys Val Ser Pro Leu Gln Gln Ser Tyr Ser Cys Leu Asn
               20                  25                  30
   Pro Gln Leu Glu Ser Asn Glu Gly Gln Ala Val Asn Ser Lys Arg Leu
           35                  40                  45
   Leu His His Cys Phe Met Ala Thr Val Thr Thr Ser Asp Ile Pro Gly
       50                  55                  60
   Ser Pro Glu Glu Ala Ser Val Pro Asn Pro Asp Leu Cys Gly Pro Val
   65                  70                  75                  80
   Pro
<210> 152
   <211> 130
   <212> PRT
   <213> Artificial
<220>
   <223> Synthetic polypeptide
<400> 152
   Ser Ile Gly Ala Leu Ile Gly Leu Ser Val Ala Ala Val Val Leu Leu
   1               5                   10                  15
   Ala Phe Ile Val Thr Ala Cys Val Leu Cys Tyr Leu Phe Ile Ser Ser
               20                  25                  30
   Lys Pro His Thr Lys Leu Asp Leu Gly Leu Ser Leu Gln Thr Ala Gly
           35                  40                  45
   Pro Glu Glu Val Ser Pro Asp Cys Gln Gly Val Asn Thr Gly Met Ala
       50                  55                  60
   Ala Glu Val Pro Lys Val Ser Pro Leu Gln Gln Ser Tyr Ser Cys Leu
   65                  70                  75                  80
   Asn Pro Gln Leu Glu Ser Asn Glu Gly Gln Ala Val Asn Ser Lys Arg
                   85                  90                  95
   Leu Leu His His Cys Phe Met Ala Thr Val Thr Thr Ser Asp Ile Pro
               100                 105                 110
   Gly Ser Pro Glu Glu Ala Ser Val Pro Asn Pro Asp Leu Cys Gly Pro
           115                 120                 125
   Val Pro
       130
<210> 153
   <211> 64
   <212> PRT
   <213> Artificial
<220>
   <223> Synthetic polypeptide
<400> 153
   Met Ala Ser Leu Trp Pro Ser Ala Leu Thr Phe Asn Thr Asp Ala Asn
   1               5                   10                  15
   Ile Pro Gly Pro Leu Gly Phe Cys Gly Gly Trp Val Arg Leu Cys Ser
               20                  25                  30
   Leu Ser Ser Leu Thr Pro Pro Cys Gly Arg Arg Leu Val Pro Cys Leu
           35                  40                  45
   Ser Ala Pro Ala Pro Asn Ala Pro Arg Leu Pro Ala Pro Ala Arg Cys
       50                  55                  60
<210> 154
   <211> 81
   <212> PRT
   <213> Artificial
<220>
   <223> Synthetic polypeptide
<400> 154
   Glu Glu Val Ser Pro Asp Cys Gln Gly Val Asn Thr Gly Met Ala Ala
   1               5                   10                  15
   Glu Val Pro Lys Val Ser Pro Leu Gln Gln Ser Tyr Ser Cys Leu Asn
               20                  25                  30
   Pro Gln Leu Glu Ser Asn Glu Gly Gln Ala Val Asn Ser Lys Arg Leu
           35                  40                  45
   Leu His His Cys Phe Met Ala Thr Val Thr Thr Ser Asp Ile Pro Gly
       50                  55                  60
   Ser Pro Glu Glu Ala Ser Val Pro Asn Pro Asp Leu Cys Gly Pro Val
   65                  70                  75                  80
   Pro
<210> 155
   <211> 165
   <212> PRT
   <213> Artificial
<220>
   <223> Synthetic polypeptide
<400> 155
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Gly Phe Gly Ser Phe Leu Gly Ile Ile Gly Ser Asn
   65                  70                  75                  80
   Leu Ile Glu Asn Lys Arg Gln Met Leu Val Ala Ser Ile Val Phe Ile
                   85                  90                  95
   Ser Phe Gly Val Ile Ala Ala Phe Cys Cys Ala Ile Val Asp Gly Val
               100                 105                 110
   Phe Ala Ala Arg His Ile Asp Leu Lys Pro Leu Tyr Ala Asn Arg Cys
           115                 120                 125
   His Tyr Val Pro Lys Thr Ser Gln Lys Glu Ala Glu Glu Val Ile Ser
       130                 135                 140
   Ser Ser Thr Lys Asn Ser Pro Ser Thr Arg Val Met Arg Asn Leu Thr
   145                 150                 155                 160
   Gln Ala Ala Arg Glu
                   165
<210> 156
   <211> 141
   <212> PRT
   <213> Artificial
<220>
   <223> Synthetic polypeptide
<400> 156
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Gly Phe Gly Ser Phe Leu Gly Ile Ile Gly Ser Asn
   65                  70                  75                  80
   Leu Ile Glu Asn Lys Arg Gln Met Leu Val Ala Ser Ile Val Phe Ile
                   85                  90                  95
   Ser Phe Gly Val Ile Ala Ala Phe Cys Cys Ala Ile Val Asp Gly Val
               100                 105                 110
   Phe Ala Ala Arg His Ile Asp Leu Lys Pro Leu Tyr Ala Asn Arg Cys
           115                 120                 125
   His Tyr Val Pro Lys Thr Ser Gln Lys Glu Ala Glu Glu
       130                 135                 140
<210> 157
   <211> 78
   <212> PRT
   <213> Artificial
<220>
   <223> Synthetic polypeptide
<400> 157
   Met Leu Val Ala Ser Ile Val Phe Ile Ser Phe Gly Val Ile Ala Ala
   1               5                   10                  15
   Phe Cys Cys Ala Ile Val Asp Gly Val Phe Ala Ala Arg His Ile Asp
               20                  25                  30
   Leu Lys Pro Leu Tyr Ala Asn Arg Cys His Tyr Val Pro Lys Thr Ser
           35                  40                  45
   Gln Lys Glu Ala Glu Glu Val Ile Ser Ser Ser Thr Lys Asn Ser Pro
       50                  55                  60
   Ser Thr Arg Val Met Arg Asn Leu Thr Gln Ala Ala Arg Glu
   65                  70                  75
<210> 158
   <211> 141
   <212> PRT
   <213> Artificial
<220>
   <223> Synthetic polypeptide
<400> 158
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Gly Phe Gly Ser Phe Leu Gly Ile Ile Gly Ser Asn
   65                  70                  75                  80
   Leu Ile Glu Asn Lys Arg Gln Met Leu Val Ala Ser Ile Val Phe Ile
                   85                  90                  95
   Ser Phe Gly Val Ile Ala Ala Phe Cys Cys Ala Ile Val Asp Gly Val
               100                 105                 110
   Phe Ala Ala Arg His Ile Asp Leu Lys Pro Leu Tyr Ala Asn Arg Cys
           115                 120                 125
   His Tyr Val Pro Lys Thr Ser Gln Lys Glu Ala Glu Glu
       130                 135                 140
<210> 159
   <211> 67
   <212> PRT
   <213> Artificial
<220>
   <223> Synthetic polypeptide
<400> 159
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile
   65
<210> 160
   <211> 120
   <212> PRT
   <213> Artificial
<220>
   <223> Synthetic polypeptide
<400> 160
   Met His Gln Ser Leu Thr Gln Gln Arg Ser Ser Asp Met Ser Leu Pro
   1               5                   10                  15
   Asp Ser Met Gly Ala Phe Asn Arg Arg Lys Arg Asn Ser Ile Tyr Val
               20                  25                  30
   Thr Val Thr Leu Leu Ile Val Ser Val Leu Ile Leu Thr Val Gly Leu
           35                  40                  45
   Ala Ala Thr Thr Arg Thr Gln Asn Val Thr Val Gly Gly Tyr Tyr Pro
       50                  55                  60
   Gly Val Ile Leu Val Ala Ser Ile Val Phe Ile Ser Phe Gly Val Ile
   65                  70                  75                  80
   Ala Ala Phe Cys Cys Ala Ile Val Asp Gly Val Phe Ala Ala Arg His
                   85                  90                  95
   Ile Asp Leu Lys Pro Leu Tyr Ala Asn Arg Cys His Tyr Val Pro Lys
               100                 105                 110
   Thr Ser Gln Lys Glu Ala Glu Glu
           115                 120
<210> 161
   <211> 55
   <212> PRT
   <213> Artificial
<220>
   <223> Synthetic polypeptide
<400> 161
   Met Gln Ala Pro Arg Ala Ala Leu Val Phe Ala Leu Val Ile Ala Leu
   1               5                   10                  15
   Val Pro Val Gly Arg Gly Asn Tyr Glu Glu Leu Glu Asn Ser Gly Asp
               20                  25                  30
   Thr Thr Val Glu Ser Glu Arg Pro Asn Lys Val Thr Ile Pro Ser Thr
           35                  40                  45
   Phe Ala Ala Val Thr Ile Lys
       50                  55
<210> 162
   <211> 23
   <212> PRT
   <213> Artificial
<220>
   <223> Synthetic polypeptide
<400> 162
   Cys Lys Ile Gln Leu Ser Trp Lys Val Ile Pro Ala Phe Cys Leu Glu
   1               5                   10                  15
   Ser Ser His Arg Asn Ala Leu
               20
<210> 163
   <211> 33
   <212> DNA
   <213> Artificial
<220>
   <223> Synthetic polynucleotide
<400> 163
   ctagctagcc accatgaggc gctgcagtct ctg 33
<210> 164
   <211> 32
   <212> DNA
   <213> Artificial
<220>
   <223> Synthetic polynucleotide
<400> 164
   cgcgaccggt ccaacccaac ttctctgtga tg 32
<210> 165
   <211> 34
   <212> DNA
   <213> Artificial
<220>
   <223> Synthetic polynucleotide
<400> 165
   cgattgtaca agaggcgctg cagtctctgc gctt 34
<210> 166
   <211> 31
   <212> DNA
   <213> Artificial
<220>
   <223> Synthetic polynucleotide
<400> 166
   gcgcgcggcc gcctaaaccc aacttctctg t 31
<210> 167
   <211> 32
   <212> DNA
   <213> Artificial
<220>
   <223> Synthetic polynucleotide
<400> 167
   ctagctagcc accatgcatc agtccctgac tc 32
<210> 168
   <211> 75
   <212> DNA
   <213> Artificial
<220>
   <223> Synthetic polynucleotide
<400> 168
   ataagaatgc ggccgctcat tacttgtcgt catcgtcttt gtagtcggga ctgtaaggtg 60
   gtggcttttc aaaag 75
<210> 169
   <211> 19
   <212> DNA
   <213> Artificial
<220>
   <223> Synthetic polynucleotide
<400> 169
   agagcccagc aggccaaag 19
<210> 170
   <211> 19
   <212> DNA
   <213> Artificial
<220>
   <223> Synthetic polynucleotide
<400> 170
   agcaggaccc agtgcagtg 19
<210> 171
   <211> 111
   <212> DNA
   <213> Artificial
<220>
   <223> Synthetic polynucleotide
<400> 171
   agagcccagc aggccaaagg ctttgtgtct tccacagagc gtttccgtgg gacttgtggc 60
   cctcctggcg tccaggaacc ttcttcgccc tccactgcac tgggtcctgc t 111
<210> 172
   <211> 30
   <212> DNA
   <213> Artificial
<220>
   <223> Synthetic polynucleotide
<400> 172
   ctagctagcc accatggaga agtttcgggc 30
<210> 173
   <211> 68
   <212> DNA
   <213> Artificial
<220>
   <223> Synthetic polynucleotide
<400> 173
   cgcaccggtt cattacttgt cgtcatcgtc tttgtagtct aactcaggag tgctgtttat 60
   accagatg 68
<210> 174
   <211> 972
   <212> DNA
   <213> Artificial
<220>
   <223> Synthetic polynucleotide
<400> 174
   atggagaagt ttcgggcggt gctggacctg cacgtcaagc accacagcgc cttgggctac      60
   ggcctggtga ccctgctgac ggcgggcggg gagcgcatct tctccgccgt ggcattccag     120
   tgcccgtgca gcgccgcctg gaacctgccc tacggcctgg tcttcttgct ggtgccggcg     180
   ctcgcgctct tcctcctggg ctacgtgctg agcgcacgca cgtggcgcct gctcaccgga     240
   tgctgctcca gcgcccgcgc gagttgcgga tcggcgctgc gcggctccct ggtgtgcgcg     300
   caaatcagcg cggccgccgc gctcgcgccc ctcacctggg tggccgtggc gctgctcggg     360
   ggcgcctttt acgagtgcgc ggccaccggg agcgcggcct tcgcgcagcg cctgtgcctc     420
   ggccgcaacc gcagctgcgc cgcggagctg ccgctggtgc cgtgcaacca ggccaaggcg     480
   tcggacgtgc aggacctcct gaaggatctg aaggctcagt cgcaggtgtt gggctggatc     540
   ttgatagcag ttgttatcat cattcttctg atttttacat ctgtcacccg atgcctatct     600
   ccagttagtt ttctgcagct gaaattctgg aaaatctatt tggaacagga gcagcagatc     660
   cttaaaagta aagccacaga gcatgcaact gaattggcaa aagagaatat taaatgtttc     720
   tttgagggct cgcatccaaa agaatataac actccaagca tgaaagagtg gcagcaaatt     780
   tcatcactgt atactttcaa tccgaagggc cagtactaca gcatgttgca caaatatgtc     840
   aacagaaaag agaagactca cagtatcagg tctactgaag gagatacggt gattcctgtt     900
   cttggctttg tagattcatc tggtataaac agcactcctg agttagacta caaagacgat     960
   gacgacaagt aa                                                         972
<210> 175
   <211> 323
   <212> PRT
   <213> Artificial
<220>
   <223> Synthetic polypeptide
<400> 175
   Met Glu Lys Phe Arg Ala Val Leu Asp Leu His Val Lys His His Ser
   1               5                   10                  15
   Ala Leu Gly Tyr Gly Leu Val Thr Leu Leu Thr Ala Gly Gly Glu Arg
                   20                  25                  30
   Ile Phe Ser Ala Val Ala Phe Gln Cys Pro Cys Ser Ala Ala Trp Asn
           35                  40                  45
   Leu Pro Tyr Gly Leu Val Phe Leu Leu Val Pro Ala Leu Ala Leu Phe
       50                  55                  60
   Leu Leu Gly Tyr Val Leu Ser Ala Arg Thr Trp Arg Leu Leu Thr Gly
   65                  70                  75                  80
   Cys Cys Ser Ser Ala Arg Ala Ser Cys Gly Ser Ala Leu Arg Gly Ser
                   85                  90                  95
   Leu Val Cys Ala Gln Ile Ser Ala Ala Ala Ala Leu Ala Pro Leu Thr
               100                 105                 110
   Trp Val Ala Val Ala Leu Leu Gly Gly Ala Phe Tyr Glu Cys Ala Ala
           115                 120                 125
   Thr Gly Ser Ala Ala Phe Ala Gln Arg Leu Cys Leu Gly Arg Asn Arg
       130                 135                 140
   Ser Cys Ala Ala Glu Leu Pro Leu Val Pro Cys Asn Gln Ala Lys Ala
   145                 150                 155                 160
   Ser Asp Val Gln Asp Leu Leu Lys Asp Leu Lys Ala Gln Ser Gln Val
                   165                 170                 175
   Leu Gly Trp Ile Leu Ile Ala Val Val Ile Ile Ile Leu Leu Ile Phe
               180                 185                 190
   Thr Ser Val Thr Arg Cys Leu Ser Pro Val Ser Phe Leu Gln Leu Lys
           195                 200                 205
   Phe Trp Lys Ile Tyr Leu Glu Gln Glu Gln Gln Ile Leu Lys Ser Lys
       210                 215                 220
   Ala Thr Glu His Ala Thr Glu Leu Ala Lys Glu Asn Ile Lys Cys Phe
   225                 230                 235                 240
   Phe Glu Gly Ser His Pro Lys Glu Tyr Asn Thr Pro Ser Met Lys Glu
                   245                 250                 255
   Trp Gln Gln Ile Ser Ser Leu Tyr Thr Phe Asn Pro Lys Gly Gln Tyr
               260                 265                 270
   Tyr Ser Met Leu His Lys Tyr Val Asn Arg Lys Glu Lys Thr His Ser
           275                 280                 285
   Ile Arg Ser Thr Glu Gly Asp Thr Val Ile Pro Val Leu Gly Phe Val
       290                 295                 300
   Asp Ser Ser Gly Ile Asn Ser Thr Pro Glu Leu Asp Tyr Lys Asp Asp
   305                 310                 315                 320
   Asp Asp Lys
<210> 176
   <211> 20
   <212> DNA
   <213> Artificial
<220>
   <223> Synthetic polynucleotide
<400> 176
   tcgtctctgc cgttctcagg 20
<210> 177
   <211> 24
   <212> DNA
   <213> Artificial
<220>
   <223> Synthetic polynucleotide
<400> 177
   gggatggtat ggagacattt attg 24
<210> 178
   <211> 60
   <212> DNA
   <213> Artificial
<220>
   <223> Synthetic polynucleotide
<400> 178
   ctagctagcc accatggact acaaagacga tgacgacaag agcggcgcct gcacgagcta 60
<210> 179
   <211> 30
   <212> DNA
   <213> Artificial
<220>
   <223> Synthetic polynucleotide
<400> 179
   cggaattctt atgggactgg tccacatagg 30
<210> 180
   <211> 37
   <212> DNA
   <213> Artificial
<220>
   <223> Synthetic polynucleotide
<400> 180
   ctagctagcc accatgagcg gcgcctgcac gagctac 37
<210> 181
   <211> 57
   <212> DNA
   <213> Artificial
<220>
   <223> Synthetic polynucleotide
<400> 181
   ccggaattct tacttgtcgt catcgtcttt gtagtctggg actggtccac ataggtc 57
<210> 182
   <211> 597
   <212> DNA
   <213> Artificial
<220>
   <223> Synthetic polynucleotide
<400> 182
   atggactaca aagacgatga cgacaagagc ggcgcctgca cgagctacgt gagcgcagag     60
   caggaggtgg tgcgcggctt cagctgcccg cggccggggg gcgaggcggc cgctgtcttc    120
   tgctgcggct tccgcgacca caagtactgc tgcgacgacc cgcacagctt cttcccctac    180
   gagcacagct acatgtggtg gctcagcatt ggcgctctca taggcctgtc cgtagcagca    240
   gtggttcttc tcgccttcat tgttaccgcc tgtgtgctct gctacctgtt catcagctct    300
   aagccccaca caaagttgga cctgggcttg agcttacaga cagcaggccc tgaggaggtt    360
   tctcctgact gccaaggtgt gaacacaggc atggcggcag aagtgccaaa agtgagccct    420
   ctccagcaga gttactcctg cttgaacccg cagctggaga gcaatgaggg gcaggctgtg    480
   aactccaaac gcctcctcca tcattgcttc atggccacag tgaccaccag tgacattcca    540
   ggcagccctg aggaagcctc tgtacccaac cctgacctat gtggaccagt cccataa       597
<210> 183
   <211> 597
   <212> DNA
   <213> Artificial
<220>
   <223> Synthetic polynucleotide
<400> 183
   atgagcggcg cctgcacgag ctacgtgagc gcagagcagg aggtggtgcg cggcttcagc     60
   tgcccgcggc cggggggcga ggcggccgct gtcttctgct gcggcttccg cgaccacaag    120
   tactgctgcg acgacccgca cagcttcttc ccctacgagc acagctacat gtggtggctc    180
   agcattggcg ctctcatagg cctgtccgta gcagcagtgg ttcttctcgc cttcattgtt    240
   accgcctgtg tgctctgcta cctgttcatc agctctaagc cccacacaaa gttggacctg    300
   ggcttgagct tacagacagc aggccctgag gaggtttctc ctgactgcca aggtgtgaac    360
   acaggcatgg cggcagaagt gccaaaagtg agccctctcc agcagagtta ctcctgcttg    420
   aacccgcagc tggagagcaa tgaggggcag gctgtgaact ccaaacgcct cctccatcat    480
   tgcttcatgg ccacagtgac caccagtgac attccaggca gccctgagga agcctctgta    540
   cccaaccctg acctatgtgg accagtccca gactacaaag acgatgacga caagtaa       597
<210> 184
   <211> 198
   <212> PRT
   <213> Artificial
<220>
   <223> Synthetic polynucleotide
<400> 184
   Met Asp Tyr Lys Asp Asp Asp Asp Lys Ser Gly Ala Cys Thr Ser Tyr
   1               5                   10                  15
   Val Ser Ala Glu Gln Glu Val Val Arg Gly Phe Ser Cys Pro Arg Pro
               20                  25                  30
   Gly Gly Glu Ala Ala Ala Val Phe Cys Cys Gly Phe Arg Asp His Lys
           35                  40                  45
   Tyr Cys Cys Asp Asp Pro His Ser Phe Phe Pro Tyr Glu His Ser Tyr
       50                  55                  60
   Met Trp Trp Leu Ser Ile Gly Ala Leu Ile Gly Leu Ser Val Ala Ala
   65                  70                  75                  80
   Val Val Leu Leu Ala Phe Ile Val Thr Ala Cys Val Leu Cys Tyr Leu
                   85                  90                  95
   Phe Ile Ser Ser Lys Pro His Thr Lys Leu Asp Leu Gly Leu Ser Leu
               100                 105                 110
   Gln Thr Ala Gly Pro Glu Glu Val Ser Pro Asp Cys Gln Gly Val Asn
           115                 120                 125
   Thr Gly Met Ala Ala Glu Val Pro Lys Val Ser Pro Leu Gln Gln Ser
       130                 135                 140
   Tyr Ser Cys Leu Asn Pro Gln Leu Glu Ser Asn Glu Gly Gln Ala Val
   145                 150                 155                 160
   Asn Ser Lys Arg Leu Leu His His Cys Phe Met Ala Thr Val Thr Thr
                   165                 170                 175
   Ser Asp Ile Pro Gly Ser Pro Glu Glu Ala Ser Val Pro Asn Pro Asp
               180                 185                 190
   Leu Cys Gly Pro Val Pro
           195
<210> 185
   <211> 198
   <212> PRT
   <213> Artificial
<220>
   <223> Synthetic polypeptide
<400> 185
   Met Ser Gly Ala Cys Thr Ser Tyr Val Ser Ala Glu Gln Glu Val Val
   1               5                   10                  15
   Arg Gly Phe Ser Cys Pro Arg Pro Gly Gly Glu Ala Ala Ala Val Phe
               20                  25                  30
   Cys Cys Gly Phe Arg Asp His Lys Tyr Cys Cys Asp Asp Pro His Ser
           35                  40                  45
   Phe Phe Pro Tyr Glu His Ser Tyr Met Trp Trp Leu Ser Ile Gly Ala
       50                  55                  60
   Leu Ile Gly Leu Ser Val Ala Ala Val Val Leu Leu Ala Phe Ile Val
   65                  70                  75                  80
   Thr Ala Cys Val Leu Cys Tyr Leu Phe Ile Ser Ser Lys Pro His Thr
                   85                  90                  95
   Lys Leu Asp Leu Gly Leu Ser Leu Gln Thr Ala Gly Pro Glu Glu Val
               100                 105                 110
   Ser Pro Asp Cys Gln Gly Val Asn Thr Gly Met Ala Ala Glu Val Pro
           115                 120                 125
   Lys Val Ser Pro Leu Gln Gln Ser Tyr Ser Cys Leu Asn Pro Gln Leu
       130                 135                 140
   Glu Ser Asn Glu Gly Gln Ala Val Asn Ser Lys Arg Leu Leu His His
   145                 150                 155                 160
   Cys Phe Met Ala Thr Val Thr Thr Ser Asp Ile Pro Gly Ser Pro Glu
                   165                 170                 175
   Glu Ala Ser Val Pro Asn Pro Asp Leu Cys Gly Pro Val Pro Asp Tyr
               180                 185                 190
   Lys Asp Asp Asp Asp Lys
           195
<210> 186
   <211> 18
   <212> PRT
   <213> Artificial
<220>
   <223> Synthetic polynucleotide
<400> 186
   Met His Gln Ser Leu Thr Gln Gln Arg Ser Ser Asp Met Ser Leu Pro
   1               5                   10                  15
   Asp Ser
<210> 187
   <211> 38
   <212> DNA
   <213> Artificial
<220>
   <223> Synthetic polynucleotide
<400> 187
   ctagctagcc accatgcagg ctccccgcgc agccctag 38
<210> 188
   <211> 53
   <212> DNA
   <213> Artificial
<220>
   <223> synthetic polynucleotide
<400> 188
   ttacttgtcg tcatcgtctt tgtagtcgga ttcactgtca cttgggttgt gat 53
<210> 189
   <211> 576
   <212> DNA
   <213> Artificial
<220>
   <223> Synthetic polypeptide
<400> 189
   atgcaggctc cccgcgcagc cctagtcttc gccctggtga tcgcgctcgt tcccgtcggc     60
   cggggtaatt atgaggaatt agaaaactca ggagatacaa ctgtggaatc tgaaagacca    120
   aataaagtga ctattccaag cacatttgct gcagtgacca tcaaagaaac attaaatgca    180
   aatataaatt ctaccaactt tgctccggat gaaaatcagt tagagtttat actgatggtg    240
   ttaatcccat tgattttatt ggtcctctta cttttatccg tggtattcct tgcaacatac    300
   tataaaagaa aaagaactaa acaagaacct tctagccaag gatctcagag tgctttacag    360
   acatatgaac tgggaagtga aaacgtgaaa gtccctattt ttgaggaaga tacaccctct    420
   gttatggaaa ttgaaatgga agagcttgat aaatggatga acagcatgaa tagaaatgcc    480
   gactttgaat gtttacctac cttgaaggaa gagaaggaat caaatcacaa cccaagtgac    540
   agtgaatccg actacaaaga cgatgacgac aagtaa                              576
<210> 190
   <211> 191
   <212> PRT
   <213> Artificial
<220>
   <223> Synthetic polypeptide
<400> 190
   Met Gln Ala Pro Arg Ala Ala Leu Val Phe Ala Leu Val Ile Ala Leu
   1 5 10 15
   Val Pro Val Gly Arg Gly Asn Tyr Glu Glu Leu Glu Asn Ser Gly Asp
   20 25 30
   Thr Thr Val Glu Ser Glu Arg Pro Asn Lys Val Thr Ile Pro Ser Thr
   35 40 45
   Phe Ala Ala Val Thr Ile Lys Glu Thr Leu Asn Ala Asn Ile Asn Ser
   50 55 60
   Thr Asn Phe Ala Pro Asp Glu Asn Gln Leu Glu Phe Ile Leu Met Val
   65 70 75 80
   Leu Ile Pro Leu Ile Leu Leu Val Leu Leu Leu Leu Ser Val Val Phe
   85 90 95
   Leu Ala Thr Tyr Tyr Lys Arg Lys Arg Thr Lys Gln Glu Pro Ser Ser
   100 105 110
   Gln Gly Ser Gln Ser Ala Leu Gln Thr Tyr Glu Leu Gly Ser Glu Asn
   115 120 125
   Val Lys Val Pro Ile Phe Glu Glu Asp Thr Pro Ser Val Met Glu Ile
   130 135 140
   Glu Met Glu Glu Leu Asp Lys Trp Met Asn Ser Met Asn Arg Asn Ala
   145 150 155 160
   Asp Phe Glu Cys Leu Pro Thr Leu Lys Glu Glu Lys Glu Ser Asn His
   165 170 175
   Asn Pro Ser Asp Ser Glu Ser Asp Tyr Lys Asp Asp Asp Asp Lys
   180 185 190
<210> 191
   <211> 19
   <212> PRT
   <213> Artificial
<220>
   <223> Synthetic polypeptide
<400> 191
   Arg Gly Asn Tyr Glu Glu Leu Glu Asn Ser Gly Asp Thr Thr Val Glu
   1 5 10 15
   Ser Glu Arg
<210> 192
   <211> 17
   <212> PRT
   <213> Artificial
<220>
   <223> Synthetic polypeptide
<400> 192
   Tyr Lys Arg Lys Arg Thr Lys Gln Glu Pro Ser Ser Gln Gly Ser Gln
   1 5 10 15
   Ser
<211> 78
   <212> DNA
   <213> Artificial
<220>
   <223> Synthetic polynucleotide
<400> 193
<210> 194
   <211> 48
   <212> DNA
   <213> Artificial
<220>
   <223> synthetic polynucleotide
<400> 194
   gtaaggaagg caaacaggaa gggttcattc cacagagact ggctgtgt 48
<210> 195
   <211> 20
   <212> DNA
   <213> Artificial
<220>
   <223> Synthetic polynucleotide
<400> 195
   ccctccactg gatacagcct 20
<210> 196
   <211> 20
   <212> PRT
   <213> Artificial
<220>
   <223> synthetic polypeptide
<400> 196
   Pro Lys Thr Ser Gln Lys Glu Ala Glu Glu Asn Pro Thr Leu Pro Ala
   1 5 10 15
   Leu Asn Cys Ser
   20
<210> 197
   <211> 20
   <212> DNA
   <213> Artificial
<220>
   <223> Synthetic polynucleotide
<400> 197
   agctgaggag aacccaactc 20
<210> 198
   <211> 20
   <212> DNA
   <213> Artificial
<220>
   <223> synthetic polynucleotide
<400> 198
   cggagttatt ctggtggctt 20
<210> 199
   <211> 20
   <212> PRT
   <213> Artificial
<220>
   <223> synthetic polypeptide
<400> 199
   Thr Val Gly Gly Tyr Tyr Pro Gly Val Ile Leu Val Ala Ser Ile Val
   1               5                   10                  15
   Phe Ile Ser Phe
               20
<210> 200
   <211> 35
   <212> PRT
   <213> Artificial
<220>
   <223> synthetic polypeptide
<400> 200
   Leu Cys Ser Leu Ser Ser Leu Thr Pro Pro Cys Gly Arg Arg Leu Val
   1               5                   10                  15
   Pro Cys Leu Ser Ala Pro Ala Pro Asn Ala Pro Arg Leu Pro Ala Pro
               20                  25                  30
   Ala Arg Cys
           35
<210> 201
   <211> 108
   <212> DNA
   <213> Artificial
<220>
   <223> synthetic polynucleotide
<400> 201
   gttgtgttca ctctcctcac tgaccccgcc gtgtggtcgc cggctggtgc cttgtctgtc     60
   tgccccagcc ccgaatgccc cgagactccc ggcgccggct cggtgcag                 108

## Description

### FIELD OF THE INVENTION

This invention relates to TMEM154 related polypeptides and polynucleotides that are differentially expressed in some cancers and specific blood cells, and therefore are suitable targets for development of therapeutics and diagnostics, particularly for cancer therapy and treatment of immune related disorders.

### BACKGROUND OF THE INVENTION

Tumor antigens are ideally positioned as biomarkers and drug targets, and they play a critical role in the development of novel strategies for active and passive immunotherapy agents, to be used as stand-alone therapies or in conjunction with conventional therapies for cancer. Tumor antigens can be classified as either tumor-specific antigens (TSAs) where the antigens are expressed only in tumor cells and not in normal tissues, or tumor-associated antigens (TAAs) where the antigens are overexpressed in tumor cells but nonetheless also present at low levels in normal tissues.

TAAs and TSAs are validated as targets for passive (antibody) therapy as well as active immunotherapy using strategies to break immune tolerance and stimulate the immune system. The antigenic epitopes that are targeted by these therapeutic approaches are present at the cell surface, overexpressed in tumor cells compared to non-tumor cells, and are targeted by antibodies that block functional activity, inhibit cell proliferation, or induce cell death.

There are a growing number of tumor-associated antigens against which monoclonal antibodies have been tested or are in use as treatment for cancer. The identification and molecular characterization of novel tumor antigens expressed by human malignancies is an active field in tumor immunology. Several approaches have been used to identify tumor-associated antigens as target candidates for immunotherapy, including high throughput bioinformatic approaches, based on genomics and proteomics. The identification of novel TAAs or TSAs expands the spectrum of tumor antigen targets available for immune recognition and provides new target molecules for the development of therapeutic agents for passive immunotherapy, including monoclonal antibodies, whether unmodified or armed. Such novel antigens may also point the way to more effective therapeutic vaccines for active or adoptive immunotherapy.

Cancer vaccination involves the administration of tumor antigens and is used to break immune tolerance and induce an active T-cell response to the tumor. Vaccine therapy includes the use of naked DNA, peptides, recombinant protein, and whole cell therapy, where the patient's own tumor cells are used as the source of the vaccine. With the identification of specific tumor antigens, vaccinations are more often carried out by dendritic cell therapy, whereby dendritic cells are loaded with the relevant protein or peptide, or transfected with vector DNA or RNA.

The major applications of anti-TAA antibodies for treatment of cancer are therapy with naked antibody, therapy with a drug-conjugated antibody, and fusion therapy with cellular immunity. Ever since their discovery, antibodies were envisioned as "magic bullets" that would deliver toxic agents, such as drugs, toxins, enzymes and radioisotopes, specifically to the diseased site and leaving the non-target normal tissues unaffected. Indeed, antibodies, and in particular antibody fragments, can function as carriers of cytotoxic substances such as radioisotopes, drugs and toxins. Immunotherapy with such immunoconjugates is more effective than with the naked antibody.

In contrast to the overwhelming success of naked (such as Rituxan and Campath) and conjugated antibodies (such as Bexxar and Zevalin) in treating hematological malignancies, only modest success has been achieved in the immunotherapy of solid tumors. One of the major limitations in successful application of immunotherapy to solid tumors is the large molecular size of the intact immunoglobulin that results in prolonged serum half-life but in poor tumor penetration and uptake. Indeed, only a very small amount of administered antibody (as low as 0.01%) reaches the tumor. In addition to their size, antibodies encounter other impediments before reaching their target antigens expressed on the cell surface of solid tumors. Some of the barriers include poor blood flow in large tumors, permeability of vascular endothelium, elevated interstitial fluid pressure of tumor stroma, and heterogenous antigen expression.

With the advent of antibody engineering, small molecular weight antibody fragments exhibiting improved tumor penetration have been generated. Such antibody fragments are often conjugated to specific cytotoxic molecules and are designed to selectively deliver them to cancer cells. Still, solid tumors remain a formidable challenge for therapy, even with immunoconjugated antibody fragments.

The new wave of optimization strategies involves the use of biological modifiers to modulate the impediments posed by solid tumors. Thus, in combination to antibodies or their conjugated antibody fragments, various agents are being used to improve the tumor blood flow, enhance vascular permeability, lower tumor interstitial fluid pressure by modulating stromal cells and extracellular matrix components, upregulate expression of target antigens and improve penetration and retention of the therapeutic agent.

Immunotherapy with antibodies represents an exciting opportunity for combining with standard modalities, such as chemotherapy, as well as combinations with diverse biological agents to obtain a synergistic activity. Indeed, unconjugated mAbs are more effective when used in combination with other therapeutic agents, including other antibodies.

Passive tumor immunotherapy uses the exquisite specificity and lytic capability of the immune system to target tumor specific antigens and treat malignant disease with a minimum of damage to normal tissue. Several approaches have been used to identify tumor-associated antigens as target candidates for immunotherapy. The identification of novel tumor specific antigens expands the spectrum of tumor antigen targets available for immune recognition and provides new target molecules for the development of therapeutic agents for passive immunotherapy, including monoclonal antibodies, whether unmodified or armed. Such novel antigens may also point the way to more effective therapeutic vaccines for active or adoptive immunotherapy.

Despite recent progress in the understanding of cancer biology and cancer treatment, as well as better understanding of the molecules involved in immune responses, the success rate for cancer therapy and for the treatment of immune related disorders remains low. Therefore, there is an unmet need for new therapies which can successfully treat both cancer and immune related disorders.

Document WO2004/110369 discloses monoclonal antibodies that specifically bind a full-length variant of TMEM154, referred to as FLJ32028, which is identical to present SEQ ID NO: 42 (see Example 3, pages 88-93; see also pages 67-82).

### BRIEF SUMMARY OF THE INVENTION

In at least some embodiments, the subject disclosure provides novel amino acid and nucleic acid sequences, which are variants of the corresponding amino acid sequences and nucleic acid sequences for known or "WT" (wild type") TMEM154. According to at least some embodiments the TMEM154 proteins are differentially expressed by some cancers and specific blood cells, and therefore are suitable targets for cancer therapy, treatment of immune related conditions, and drug development. As described in greater detail below, the terms "polypeptides" and "proteins" are used to describe specific variants, the known proteins themselves or derived amino acid sequences related to TMEM154, or fragments or portions of any of the above.

In at least some embodiments, the subject disclosure provides discrete portions of TMEM154 proteins, variants, and nucleic acid sequences encoding same or fragments thereof.

In at least some embodiments, the subject disclosure provides therapeutic and diagnostic antibodies, antibody fragments and compositions comprising same, and therapies and diagnostic methods using said antibodies and antibody fragments that specifically bind to TMEM154 proteins, or variants, or a soluble or extracellular portion thereof, especially the ectodomain.

According to at least some embodiments of the present disclosure, the TMEM154 proteins and/or variants polypeptides are used as novel targets for development of drugs which specifically bind to the TMEM154 proteins and/or new variants, and/or drugs which agonize or antagonize the binding of other moieties to the TMEM154 proteins and/or new variants.

In at least some embodiments, the present disclosure provides proteins and discrete portions of a known hypothetical protein LOC201799 (SEQ ID NO:42) (SwissProt accession identifier NP_689893; TMEM154) or polynucleotides encoding same, which can be used as diagnostic markers and/or as targets for cancer therapy, treatment of immune related conditions, and drug development, and therapeutic agents which agonize or antagonize the binding of other moieties to the TMEM154 proteins and/or which agonize or antagonize at least one TMEM154 related biological activity.

According to at least some embodiments, the subject disclosure provides polypeptides comprising a sequence of amino acid residues corresponding to discrete portions of the TMEM154 proteins, including different portions of the extracellular domain corresponding to residues 23-75 of the sequence W38346_P3 (SEQ ID NO:42), corresponding to amino acid sequence depicted in SEQ ID NO:63, or fragments thereof.

According to at least some embodiments, the subject disclosure provides TMEM154 amino acid sequences selected from any of the isolated polypeptides, used for rabbit immunization and specific antibodies production, having an amino acid sequence as set forth in SEQ ID NOs:191.

According to other embodiments of the present disclosure, there is provided monoclonal or polyclonal antibodies and antibody fragments and conjugates containing such, that specifically bind TMEM154 proteins, optionally and preferably by specifically binding a sequence of SEQ ID NO: 42 or a fragment thereof or a peptide of SEQ ID NO: 191. These antibodies are potentially useful as therapeutics and/or diagnostic agents (both in vitro and in vivo diagnostic methods).

According to at least some embodiments of the disclosure these antibodies are useful for generating and selecting for anti-idiotypic antibodies specific thereto which also are potentially useful as therapeutics and/or diagnostic agents (both in vitro and in vivo diagnostic methods).

According to at least some embodiments of the disclosure, the antibodies and fragments modulate the activity elicited by TMEM154 polypeptides, and/or are immune activating or immune suppressing such as antibodies or fragments that target cells via ADCC (antibody dependent cellular cytotoxicity) or CDC (complement dependent cytotoxicity) activities.

In another embodiment the disclosure includes any of the foregoing antibodies or fragments thereof, wherein said antibody blocks or inhibits the interaction of TMEM154 polypeptides with a corresponding counterpart or cell component or tissue structure promoting an opposite activity or function.

In another embodiment the disclosure includes any of the foregoing antibodies or fragments wherein said antibody replaces or augments the interaction of any one of TMEM154 polypeptides with a corresponding counterpart or cell component or tissue structure promoting an opposite function or activity.

According to at least some embodiments of the present disclosure, there is provided pharmaceutical and diagnostic compositions that comprise a therapeutically or diagnostically effective form of any of the foregoing antibody or antibody fragment.

According to at least some embodiments of the present disclosure, there is provided pharmaceutical compositions that comprise a therapeutically effective form of any of the foregoing antibody or antibody fragment and further comprising a pharmaceutically acceptable dilulent or carrier.

According to at least some embodiments of the present disclosure, there is provided any of the foregoing therapeutically effective polyclonal or monoclonal antibodies or fragments, or anti-idiotypic antibodies specific to any of the foregoing, or a pharmaceutical composition comprising same, for treating or preventing conditions wherein TMEM154 polypeptides, or its secreted or soluble form or ECD and/or fragments or variants or homologs thereof are differentially expressed, including cancer and immune related conditions.

According to at least some embodiments of the present disclosure, there is provided any of the foregoing therapeutically effective polyclonal or monoclonal antibodies or fragments, or anti-idiotypic antibodies, or a pharmaceutical composition comprising same, specific to any of one of TMEM154 proteins, selected from a group consisting of any of SEQ ID NOs: 42, 63, 191 and/or fragments or variants or homologs thereof, for treating cancer and/or immune related conditions or disorders. According to at least some embodiments of the present disclosure, there is provided any of the foregoing therapeutically effective polyclonal or monoclonal antibodies or fragments, or anti-idiotypic antibodies, or a pharmaceutical composition comprising same, specific to any of one of TMEM154 proteins for treating lymphoma, especially Non-Hodgkin's Lymphoma, , anti CD20 (i.e. Rituximab) resistant lymphoma, Multiple Myeloma, kidney cancer, and/or pancreatic cancer, and/or immune related conditions or disorders, especially SLE (systemic lupus erythematosus).

According to still other embodiments there is provided a use of any of the foregoing specific antibodies and antibody fragments, and conjugates thereof, and pharmaceutical composition comprising same, in modulating (enhancing or inhibiting) immunity.

According to still other embodiments of the present invention there are provided antibodies and antibody fragments specific to polypeptides comprising discrete portions of TMEM154 proteins including different portions of the extracellular domain corresponding to residues 23-75 of the sequence W38346_P3 (SEQ ID NO:42), corresponding to amino acid sequence depicted in SEQ ID NO:63.

According to still other embodiments of the present disclosure there is provided a method to produce or select for anti-idiotypic antibodies specific to any of the foregoing.

According to still other embodiments there is provided a method to use any of the foregoing therapeutically effective polyclonal or monoclonal antibodies or fragments, or anti-idiotypic antibodies, or a pharmaceutical composition comprising same, for treatment or prevention of cancer, and/or immune related conditions.

According to at least some embodiments of the present disclosure there are provided methods for treating, or preventing cancer, and/or immune related conditions, comprising administering to a patient an effective amount of a foregoing antibody or fragment or a variant or a conjugate thereof, or a pharmaceutical composition comprising same.

According to at least some embodiments of the present disclosure there is provided any of the foregoing methods for treating, or preventing cancer, using any of the forgoing antibodies or fragments or a variant or a conjugate thereof, or a pharmaceutical composition comprising same.

According to at least some embodiments of the present disclosure there is provided any of the foregoing methods for treating, or preventing cancer, using any of the forgoing antibodies or fragments or a variant or a conjugate thereof, or a pharmaceutical composition comprising same, specific to any of one of the TMEM154 proteins, or its secreted or soluble form or ECD and/or portions or variants thereof, wherein the cancer is including but not limited to lymphoma, especially Non-Hodgkin's Lymphoma, anti CD20 (i.e. Rituximab) resistant lymphoma, Multiple Myeloma, kidney cancer, and/or pancreatic cancer, and/or immune related conditions or disorders, especially SLE.

In another embodiment the disclosure includes a method of inducing or enhancing an immune response, comprising administering to a patient in need thereof any of the foregoing antibodies or fragments and detecting induction or enhancement of said immune response.

In another embodiment the disclosure includes a method for potentiating a secondary immune response to an antigen in a patient, which method comprises administering effective amounts any of the foregoing antibodies or fragments. In another embodiment the invention includes the foregoing method, wherein the antigen is preferably a cancer antigen, a viral antigen or a bacterial antigen, and the patient has optionally received treatment with an anticancer vaccine or a viral vaccine.

In another embodiment the disclosure includes an antibody specific to any one of the TMEM154 proteins, or a fragment or variant or a homolog thereof that elicits apoptosis or lysis of cancer cells that express said protein.

In another embodiment the disclosure includes any of the foregoing antibodies or fragments, wherein said apoptosis or lysis activity involves CDC or ADCC activity of the antibody.

According to at least some embodiments of the present disclosure there is provided a method for inhibiting the growth of cells that express any one of the TMEM154 proteins in a subject, comprising: administering to the subject any of the corresponding foregoing antibody or a fragment or a variant conjugate thereof, or a pharmaceutical composition comprising same.

According to at least some embodiments the present disclosure provides the foregoing antibodies and fragments, wherein the antibody is a chimeric, humanized, primatized, or fully human antibody.

In another embodiment the disclosure includes any of the foregoing antibodies or fragments, wherein the antigen binding site contains from about 3-7 contiguous or non-contiguous amino acids, more typically at least 5 contiguous or non-contiguous amino acids. These binding sites include conformational and non-conformational epitopes.

According to other embodiments of the present disclosure there is provided antibody fragments and conjugates thereof including but not limited to Fab, F(ab')2, Fv or scFv fragment.

It is also an embodiment of the disclosure to directly or indirectly attach the subject antibodies and fragments to markers and other effector moieties such as a detectable marker, or to an effector moiety.

In another embodiment the disclosure includes any of the foregoing antibodies or fragments, wherein the effector moiety is selected from a drug, an enzyme (antibody-directed enzyme prodrug therapy (ADEPT)), a toxin, a radionuclide, a fluorophore, a therapeutic agent, or a chemotherapeutic agent.

In another embodiment the disclosure includes any of the foregoing antibodies or fragments, wherein the detectable marker is a radioisotope, a metal chelator, an enzyme, a fluorescent compound, a bioluminescent compound or a chemiluminescent compound.

According to at least some embodiments of the present invention there are provided compounds, including drugs which modulate (agonize or antagonize) at least one of the TMEM154-related biological activity. Such drugs include by way of example antibodies and fragments that bind any of the polypeptides selected from SEQ ID NOs: 42 191. These molecules may directly bind or modulate an activity elicited by any of the TMEM154 proteins or may indirectly modulate a at least one of the TMEM154-related activity or binding of molecules to TMEM154, and portions and variants thereof such as modulating the binding of any of the TMEM154 to its corresponding counter receptor or endogenous ligand, and can be useful for treatment or prevention of cancer, immune related conditions, including but not limited to inflammatory and autoimmune diseases, transplant rejection and graft versus host disease, and/or for blocking or enhancing immune costimulation mediated by the TMEM154 polypeptide.

According to the present disclosure, each one of the following: specific antibodies and fragments that bind the or TMEM154 polypeptides, or pharmaceutical composition comprising same, which are useful for treatment or prevention of cancer and/or immune related conditions, optionally may be used in combination therapy with other treatment methods known in the art selected from the group consisting of radiation therapy, antibody therapy, chemotherapy, surgery, or in combination therapy with other biological agents, conventional drugs, anti-cancer agents, immunosuppressants, cytotoxic drugs, chemotherapeutic agents, or in combination with therapeutic agents targeting other complement regulatory proteins (CRPs).

According to at least some embodiments of the present disclosure there is provided a use of any of the foregoing TMEM154 antibodies, for diagnosis of a disease, wherein the disease is selected from cancer and/or immune related conditions.

As used herein, the term "diagnosis of a disease" encompasses screening for a disease, diagnosing a diseases, detecting the presence or a severity of a disease, prognosis of a diseases, monitoring of disease progression and/or treatment efficacy and/or relapse of a disease, disorder or condition, as well as selecting a therapy and/or a treatment for a disease, optimization of a given therapy for a disease, monitoring the treatment of a disease, and/or predicting the suitability of a therapy for specific patients or subpopulations or determining the appropriate dosing of a therapeutic product in patients or subpopulations.

In at least some embodiments of the present disclosure, there is a use of any of the foregoing TMEM154 antibodies, for diagnosis of a cancer.

In at least some embodiments of the present disclosure, there is a use of any of the foregoing TMEM154 polypeptides, and/or polynucleotides, and/or antibodies, for diagnosis of a cancer, selected from but not limited to lymphoma, especially Non-Hodgkin's Lymphoma, anti CD20 (i.e. Rituximab) resistant lymphoma, Multiple Myeloma, kidney cancer, and/or pancreatic cancer, as well as for diagnosis of immune related conditions, especially SLE.

In at least some embodiments the present disclosure provides diagnostic methods for diagnosis of any of the foregoing diseases, disorders or conditions, comprising the detection of a polypeptide according at least some embodiments the present invention. According to at least some embodiments the present disclosure the expression, the level or relative changes in the expression or the level of the polypeptide herald the onset, severity, or prognosis of an individual with regard to a particular disease, disorder or condition. The detection may comprise detection of the expression or level of a specific polypeptide according at least some embodiments the present disclosure, via any means known in the art, and as described herein.

According to one embodiment of this disclosure, detecting the presence of the polypeptide is indicative of the presence of the disease and/or its severity and/or its progress. According to another embodiment, a change in the expression and/or the level of the polypeptide compared to its expression and/or level in a healthy subject or a sample obtained therefrom is indicative of the presence of the disease and/or its severity and/or its progress. According to a further embodiment, a change in the expression and/or level of the polypeptide compared to its level and/or expression in said subject or in a sample obtained therefrom at earlier stage is indicative of the progress of the disease. According to still further embodiment, detecting the presence and/or relative change in the expression and/or level of the polypeptide is useful for selecting a treatment and/or monitoring a treatment of the disease. According to still further embodiment, detecting the presence and/or relative change in the expression and/or level of the polypeptide is useful for prediction of the suitability of a therapeutic product for specific patients or subpopulations or for determining the appropriate dosing of a therapeutic product in patients or subpopulations. According to still further embodiment, the method comprising quantitatively and/or qualitatively determining or assessing expression of the polypeptides, whereby differences in expression from an index sample, or a sample taken from a subject prior to the initiation of the therapy, or during the course of therapy, is indicative of the efficacy, or optimal activity of the therapy.

In at least some embodiments of the present disclosure, the methods are conducted on a whole body.

In at least some embodiments of the present disclosure, the methods are conducted with a sample isolated from a subject having, predisposed to, or suspected of having the disease, disorder or condition. In at least some embodiments of the present disclosure, the sample is a cell or tissue or a body fluid sample.

In at least some embodiments, the subject disclosure therefore also relates to diagnostic methods and or assays for diagnosis a disease optionally in a biological sample taken from a subject (patient), which is optionally some type of body fluid or secretion including but not limited to seminal plasma, blood, serum, urine, prostatic fluid, seminal fluid, semen, the external secretions of the skin, respiratory, intestinal, and genitourinary tracts, tears, cerebrospinal fluid, sputum, saliva, milk, peritoneal fluid, pleural fluid, cyst fluid, broncho alveolar lavage, lavage of the reproductive system and/or lavage of any other part of the body or system in the body, and stool or a tissue sample. The term may also optionally encompass samples of in vivo cell culture constituents. The sample can optionally be diluted with a suitable eluant before contacting the sample to an antibody and/or performing any other diagnostic assay.

In at least some embodiments the present disclosure provides a method for diagnosis of a disease in a subject, comprising detecting in the subject or in a sample obtained from said subject at least one polypeptide of SEQ ID NO: 42 or a homologue or a fragment thereof.

According to at least some embodiments of the present disclosure there are provided diagnostic methods that include the use of any of the foregoing antibodies according to at least some embodiments of the present invention, by way of example in immunohistochemical assay, radioimaging assays, in-vivo imaging, positron emission tomography (PET), single photon emission computer tomography (SPECT), magnetic resonance imaging (MRI), Ultra Sound, Optical Imaging, Computer Tomography, radioimmunoassay (RIA), ELISA (enzyme-linked immunosorbent assay), slot blot, competitive binding assays, fluorimetric imaging assays, Western blot, FACS, and the like. According to at least some embodiments, the present invention includes diagnostic methods and or assays which use any of the foregoing antibodies or fragments that specifically bind any polypeptide having an amino acid sequence as set forth in any one of SEQ ID NOs: 42 191, or a fragment or a homolog thereof.

According to some embodiments of the present disclosure there is provided diagnostic methods and/or assays for detecting the presence of at least one of the polypeptides selected from a group consisting of SEQ ID NO: 42, 191, or a fragment or a variant or a homolog thereof, in vitro or in vivo in a biological sample or subject, comprising contacting the sample or the subject with an antibody having specificity for at least one of polypeptides having an amino acid sequence selected from the group consisting of SEQ ID NOs: 42, 191, or a fragment or a variant or a homologue thereof, or a combination thereof, and detecting the binding of any of the forgoing polypeptides, in the sample or in a subject to said antibody.

According to some embodiments of the present disclosure there are provided methods for diagnosis of a disease, comprising detecting the expression and or level in a subject or in a sample obtained from the subject, of at least one of TMEM154 polypeptides.

In at least some embodiments the present disclosure provides a diagnostic kit for diagnosis of a disease, comprising markers and reagents for detecting qualitative and/or quantitative changes in the expression of a polypeptide according to at least some embodiments of the present invention.

In at least some embodiments of the present disclosure, the kit comprises an antibody capable of recognizing or interacting with a polypeptide or protein according to at least some embodiments of the present invention.

The kit further comprises at least one reagent for performing an immunohistochemical assay, radioimaging assays, in-vivo imaging, positron emission tomography (PET), single photon emission computer tomography (SPECT), magnetic resonance imaging (MRI), Ultra Sound, Optical Imaging, Computer Tomography, radioimmunoassay (RIA), ELISA, slot blot, competitive binding assays, fluorimetric imaging assays, Western blot, FACS, and the like.

All nucleic acid sequences and/or amino acid sequences, according to at least some embodiments of the disclosure, relate to their isolated form.

It should be noted that oligonucleotide and polynucleotide, or peptide, polypeptide and protein, may optionally be used interchangeably in the present disclosure.

### BRIEF DESCRIPTION OF THE FIGURES

Figures 1-41 : deleted
Figure 42 shows a scatter plot, demonstrating the expression of TMEM154 transcripts that encode the TMEM154 proteins, on a virtual panel of all tissues and conditions using MED discovery engine, demonstrating overexpression of TMEM154 transcripts in kidney cancer samples compared to normal kidney samples.
Figures 43A and 43B demonstrate the expression of TMEM154 transcripts that encode the TMEM154 proteins, on a virtual panel of all tissues and conditions using MED discovery engine. Figure 43 shows a scatter plot, demonstrating overexpression of TMEM154 transcripts in pancreas cancer samples compared to normal pancreas samples. Figure 43B presents Kaplan-Meier survival curves of Rituximab treated DLBCL in corelation to TMEM154 expression. In Figure 43B the time scale is shown in years; solid line represents high TMEM154 expression; fragmented line represents low TMEM154 expression.
Figures 44A and 44B show a histogram showing the expression of hypothetical protein FLJ32028, TMEM154 W38346 transcripts which are detectable by amplicon as depicted in sequence name W38346_seg6-20F1R1 (SEQ ID NO: 106) in different normal tissues (Figure 44B is a continuation of Figure 44A).
Figures 45A and 45B show a histogram showing the expression of hypothetical protein FLJ32028, TMEM154 W38346 transcripts which are detectable by amplicon as depicted in sequence name W38346_seg6-20F1R1 (SEQ NO: 106) in blood specific panel (Figure 54B is a continuation of Figure 45A).
Figure 46 presents the DNA sequence of the TMEM154_T0_FLAG (SEQ ID NO:189); FLAG sequence is in underlined.
Figure 47 presents the amino acid sequence of TMEM154_P3_FLAG (SEQ NO:190); FLAG sequence is in underlined.
Figures 48A and 48B present the localization results for TMEM154_P3.
Figures 49A and 49B present the specific cell staining localized to the cell membrane, observed using purified TM21 antibodies on TMEM154 transfected cells. Figure 49A and 49B present the results obtained using TM21 antibodies purified from rabbit #6285 and rabbit #6286, respectively.
Figures 50A and 50B present the specific cell staining localized to the cell membrane, observed using purified TM101 antibodies on TMEM154 transfected cells. Figures 50A and 50B present the results obtained using TM101 antibodies purified from rabbit #6248 and rabbit #6249, respectively.
Figures 51A-51C present the results of cell staining observed using purified TM21 and TM101 antibodies on the negative control pIRESpuro3 HEK-293T transfected cells. Figures 51A and 51B present the results obtained using TM21 antibodies purified from rabbit #6285 and rabbit #6286, respectively. Figure 51C presents the results obtained using TM101 antibodies purified from rabbit #6249.

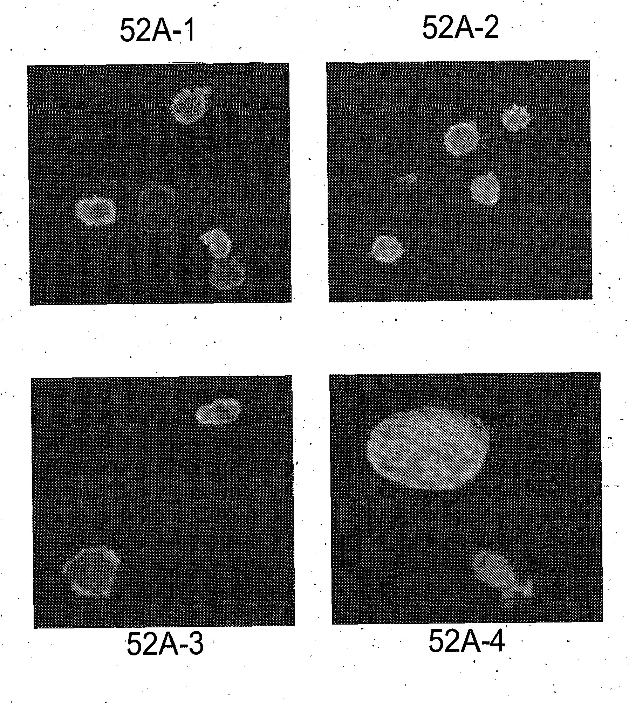

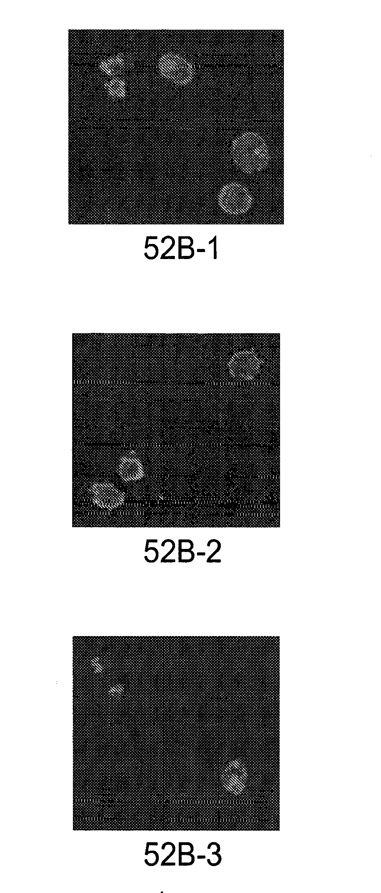

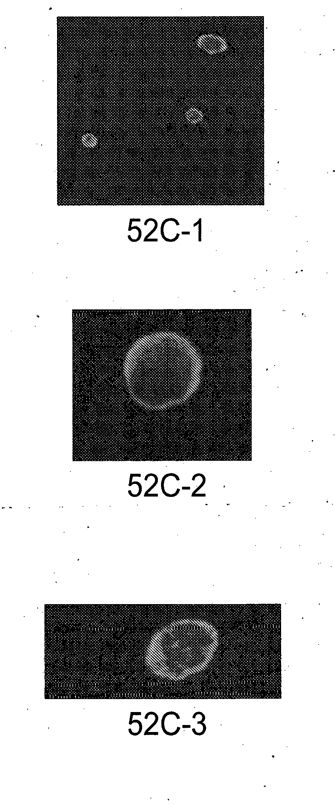

Figures 52A-52C present specific cell staining localized to the cell membrane, observed using purified TM21 and TM101 antibodies on three different cell lines: Figures 52A-1 -- 52A-4 present the results on CESS (ATCC cat no TIB-190) calls; Figures 52B-1-52B-3 present the results on Ramos (ATCC cat no CRL-1923) cells; and Figures 52C-1-52C-3 present the results on Daudi (ATCC cat no CCL-213) cells. Figures 52A-1 and 52A-2 present the results obtained using TM21 antibodies purified from rabbit #6285 and rabbit #6286, respectively. Figures 52A-3 and 52A-4 present the results obtained using TM101 antibodies purified from rabbit #6248 and rabbit #6249, respectively. Figures 52B-1 and 52B-2 present the results obtained using TM21 antibodies.purified from rabbit #6285 and rabbit #6286, respectively. Figure 52B-3 presents the results obtained using TM101 antibodies purified from rabbit #6248. Figures 52C-1 and 52C-2 present the results obtained using TM21 antibodies purified from rabbit #6285 and rabbit #6286, respectively. Figure 52C-3 presents the results obtained using TM101 antibodies purified from rabbit #6248.

### DETAILED DESCRIPTION OF THE INVENTION

The present invention is defined in the appended claims. All other references to "the invention" and its embodiments in this specification are to be read as references to "the disclosure", unless they describe embodiments actually covered by the claims.

The present invention, in some embodiments, relates to any one of the polypeptides referred to as TMEM154 polypeptides, and its corresponding nucleic acid sequence, and fragments and variants and homologs thereof, and the use thereof as a therapeutic and/or diagnostic target. According to at least some embodiments, there are provided uses of these polypeptides and discrete portions thereof as a drug target for therapeutic antibodies. According to at least some embodiments, the invention relates to diagnostic and therapeutic polyclonal and monoclonal antibodies and fragments thereof that specifically bind TMEM154 polypeptides and portions and variants thereof, especially those that target the extracellular domains or portions or variants thereof. According to at least some embodiments, the invention provides human or chimeric monoclonal antibodies and fragments thereof and anti-idiotypic antibodies, that bind specifically to any of the amino acid sequences as set forth in SEQ NOs: 42, 191, and variants and fragments and homologs thereof.

According to at least some embodiments of the invention, the antibodies are derived from particular heavy and light chain germline sequences and/or comprise particular structural features such as CDR regions comprising particular amino acid sequences. The invention provides isolated antibodies, methods of making such antibodies, immunoconjugates and bispecific molecules comprising such antibodies and pharmaceutical and diagnostic compositions containing the antibodies, immunoconjugates or bispecific molecules.

According to at least some embodiments of the invention, the specific antibodies may be used for the treatment and/or diagnosis of cancer and/or immune related conditions, as described herein.

In order that the present invention may be more readily understood, certain terms are first defined. Additional definitions are set forth throughout the detailed description.

The term "TMEM154 protein", as used herein, includes any protein encoded by a TMEM154 gene product, including the known or "wild type" protein, any splice variants thereof, any other variants thereof, or any fragments thereof (including but not limited to any extracellular portions thereof).

The term "TMEM154 polypeptide", as used herein, refers to a polypeptide encoded by any one of the nucleic acid sequences set forth in any one of SEQ ID NOs:23, 38-41, 106, and fragments and homologous thereof, especially those possessing at least 80, 85, 90, 95, 96, 97, 98, 99% sequence identity therewith. These nucleic acid sequences are referred to herein as " TMEM154 polynucleotide". The term also refers to any one of the polypeptides set forth in any one of SEQ ID NOs:42-46; extracellular portions thereof, set forth in any one of SEQ ID NOs:63, 64; unique bridge, edge portion, tail or head portion thereof, set forth in any one of SEQ ID NOs: 161, 162; protein fragments selected from any of the isolated polypeptides, used for rabbit immunization and specific antibodies production, set forth in any one of SEQ ID NOs:191, 192; and fragments and homologous thereof, especially those possessing at least 80, 85, 90, 95, 96, 97, 98, 99% sequence identity therewith. The term "TMEM154 polynucleotide" or "TMEM154 polypeptide", as used herein, further refers to any one of the foregoing polynucleotides and polypeptides, respectively, that are differentially expressed e.g., in cancer, including but not limited to kidney cancer, pancreatic cancer, multiple myeloma, lymphomas, especially non-Hodgkins lymphoma, wherein the cancer may be non-metastatic, invasive or metastatic as well as non-malignant disorders such as immune related conditions or disorders including but not limited to inflammatory or autoimmune diseases, transplant rejection and graft versus host disease, specifically SLE.

The term the "soluble ectodomain (ECD)" or "ectodomain" or "extracellular ectodomain" of a polypeptide TMEM154 refers to the polypeptide sequences below or the corresponding nucleic acid sequences (which do not comprise the signal peptide and the TM (transmembrane portion) of the TMEM154 protein):
One ECD region of the polypeptide W38346_P3 (SEQ ID NO:42):
   W38346_P3_23-75 (SEQ ID NO:63) - sequence: EELENSGDTTVESERPNKVTIPSTFAAVTIKETLNANINSTNFAPDENQLE (and optionally bridging amino acids of any of one, two, three, four, five, six, seven, eight, nine or 10 amino acids on either side, starting anywhere from residue 13 and ending anywhere up to residue 85; and also non-linear epitopes incorporating this sequence or a portion thereof, as well as any of one, two, three, four, five, six, seven, eight, nine or 10 non-contiguous amino acids of the sequence);
One ECD region of the polypeptide W38346_P4 (SEQ ID NO:45):
   W38346_P4_20-105 (SEQ ID NO:64) - sequence: ATYYKRKRTKQEPSSQGSQSALQTYELGSENVKVPIFEEDTPSVMEIEMEELDKWM NSMNRNADFECLPTLKEEKESNHNPSDSES (and optionally bridging amino acids of any of one, two, three, four, five, six, seven, eight, nine or 10 amino acids on either side, starting anywhere from residue 10 and ending anywhere up to residue 115; and also non-linear epitopes incorporating this sequence or a portion thereof, as well as any of one, two, three, four, five, six, seven, eight, nine or 10 non-contiguous amino acids of the sequence);
One ECD region of the polypeptide W38346_P7 (SEQ NO:46):
   W38346_P7_23-75 (SEQ ID NO:63) - sequence: EELENSGDTTVESERPNKVTIPSTFAAVTIKETLNANINSTNFAPDENQLE (and optionally bridging amino acids of any of one, two, three, four, five, six, seven, eight, nine or 10 amino acids on either side, starting anywhere from residue 13 and ending anywhere up to residue 85; and also non-linear epitopes incorporating this sequence or a portion thereof, as well as any of one, two, three, four, five, six, seven, eight, nine or 10 non-contiguous amino acids of the sequence); and fragments and variants and homologs thereof possessing at least 80%, at least 85%, at least 90%, at least 95, at least 96, at least 97, at least 98 or at least 99% sequence identity therewith.

The term "immune response" refers to the action of, for example, lymphocytes, antigen presenting cells, phagocytic cells, granulocytes, and soluble macromolecules produced by the above cells or cells produced by the liver or spleen (including antibodies, cytokines, and complement) that results in selective damage to, destruction of, or elimination from the human body of invading pathogens, cells or tissues infected with pathogens, cancerous cells, or, in cases of autoimmunity or pathological inflammation, normal human cells or tissues.

The term "antibody" as referred to herein includes whole polyclonal and monoclonal antibodies and any antigen binding fragment (i.e., "antigen-binding portion") or single chains thereof. An "antibody" refers to a glycoprotein comprising at least two heavy (H) chains and two light (L) chains inter-connected by disulfide bonds, or an antigen binding portion thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "antigen-binding portion" of an antibody (or simply "antibody portion"), as used herein, refers to one or more fragments of an antibody that retain the ability to specifically bind to an antigen (e.g., TMEM154 polypeptides and proteins). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the V Light, V Heavy, Constant light (CL) and CH1 domains; (ii) a F(ab').2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

An "isolated antibody", as used herein, is intended to refer to an antibody that is substantially free of other antibodies having different antigenic specificities (e.g., an isolated antibody that specifically binds TMEM154 proteins or TMEM154 polypeptides is substantially free of antibodies that specifically bind antigens other than TMEM154 proteins or polypeptides, respectively. An isolated antibody that specifically binds TMEM154 proteins or polypeptides may, however, have cross-reactivity to other antigens, such as TMEM154 proteins or TMEM154 polypeptides from other species, respectively. Moreover, an isolated antibody may be substantially free of other cellular material and/or chemicals.

The terms "monoclonal antibody" or "monoclonal antibody composition" as used herein refer to a preparation of antibody molecules of single molecular composition. A monoclonal antibody composition displays a single binding specificity and affinity for a particular epitope.

The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies according to at least some embodiments of the invention may include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human germline immunoglobulin sequences. In one embodiment, the human monoclonal antibodies are produced by a hybridoma which includes a B cell obtained from a transgenic nonhuman animal, e.g., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo.

As used herein, "isotype" refers to the antibody class (e.g., IgM or IgG1) that is encoded by the heavy chain constant region genes.

The phrases "an antibody recognizing an antigen" and "an antibody specific for an antigen" are used interchangeably herein with the term "an antibody which binds specifically to an antigen."

As used herein, an antibody that "specifically binds" to human TMEM154 proteins or polypeptides is intended to refer to an antibody that binds to human TMEM154 proteins or polypeptides optionally one with a KD of 5X10 -8 M or less, 3X10 - 8 M or less, or 1X.10 -9 M or less.

The term "K-assoc" or "Ka", as used herein, is intended to refer to the association rate of a particular antibody-antigen interaction, whereas the term "Kdiss" or "Kd," as used herein, is intended to refer to the dissociation rate of a particular antibody-antigen interaction. The term "KD", as used herein, is intended to refer to the dissociation constant, which is obtained from the ratio of Kd to Ka (i.e., Kd/Ka) and is expressed as a molar concentration (M). KD values for antibodies can be determined using methods well established in the art. A preferred method for determining the KD of an antibody is by using surface Plasmon resonance, optionally using a biosensor system such as a Biacore® system.

As used herein, the term "high affinity" for an IgG antibody refers to an antibody having a KD of 10⁻⁸ M or less, 10⁻⁹ M or less or 10⁻¹⁰ M or less for a target antigen. However, "high affinity" binding can vary for other antibody isotypes. For example, "high affinity" binding for an IgM isotype refers to an antibody having a KD of 10⁻⁷ M or less, or 10⁻⁸ M or less.

As used herein, the term "tail" refers to a peptide sequence at the end of an amino acid sequence that is unique to a splice variant according to the present invention. Therefore, a splice variant having such a tail may optionally be considered as a chimera, in that at least a first portion of the splice variant is typically highly homologous (often 100% identical) to a portion of the corresponding known protein, while at least a second portion of the variant comprises the tail.

As used herein, the term "head" refers to a peptide sequence at the beginning of an amino acid sequence that is unique to a splice variant according to the present invention. Therefore, a splice variant having such a head may optionally be considered as a chimera, in that at least a first portion of the splice variant comprises the head, while at least a second portion is typically highly homologous (often 100% identical) to a portion of the corresponding known protein.

As used herein, the term "an edge portion" refers to a connection between two portions of a splice variant according to the present invention that were not joined in the wild type or known protein. An edge may optionally arise due to a join between the above "known protein" portion of a variant and the tail, for example, and/or may occur if an internal portion of the wild type sequence is no longer present, such that two portions of the sequence are now contiguous in the splice variant that were not contiguous in the known protein. A "bridge" may optionally be an edge portion as described above, but may also include a join between a head and a "known protein" portion of a variant, or a join between a tail and a "known protein" portion of a variant, or a join between an insertion and a "known protein" portion of a variant.

In some embodiments, a bridge between a tail or a head or a unique insertion, and a "known protein" portion of a variant, comprises at least about 10 amino acids, or in some embodiments at least about 20 amino acids, or in some embodiments at least about 30 amino acids, or in some embodiments at least about 40 amino acids, in which at least one amino acid is from the tail/head/insertion and at least one amino acid is from the "known protein" portion of a variant. In some embodiments, the bridge may comprise any number of amino acids from about 10 to about 40 amino acids (for example, 10, 11, 12, 13...37, 38, 39, 40 amino acids in length, or any number in between).

It should be noted that a bridge cannot be extended beyond the length of the sequence in either direction, and it should be assumed that every bridge description is to be read in such manner that the bridge length does not extend beyond the sequence itself.

Furthermore, bridges are described with regard to a sliding window in certain contexts below. For example, certain descriptions of the bridges feature the following format: a bridge between two edges (in which a portion of the known protein is not present in the variant) may optionally be described as follows: a bridge portion of CONTIG-NAME_P1 (representing the name of the protein), comprising a polypeptide having a length "n", wherein n is at least about 10 amino acids in length, optionally at least about 20 amino acids, at least about 30 amino acids, at least about 40 amino acids, or at least about 50 amino acids in length, wherein at least two amino acids comprise XX (2 amino acids in the center of the bridge, one from each end of the edge), having a structure as follows (numbering according to the sequence of CONTIG-NAME_P1): a sequence starting from any of amino acid numbers 49-x to 49 (for example); and ending at any of amino acid numbers 50 + ((n-2) - x) (for example), in which x varies from 0 to n-2. In this example, it should also be read as including bridges in which n is any number of amino acids between 10-50 amino acids in length. Furthermore, the bridge polypeptide cannot extend beyond the sequence, so it should be read such that 49-x (for example) is not less than 1, nor 50 + ((n-2) - x) (for example) greater than the total sequence length.

The term "*cancer*" as used herein should be understood to encompass any neoplastic disease (whether invasive or metastatic) which is characterized by abnormal and uncontrolled cell division causing malignant growth or tumor. Non-limiting examples of cancer which may be treated with a composition according to at least some embodiments of the present invention are solid tumors, sarcomas, hematological malignancies, including but not limited to breast cancer (e.g. breast carcinoma), cervical cancer, ovary cancer (ovary carcinoma), endometrial cancer, melanoma, bladder cancer (bladder carcinoma), lung cancer (e.g. adenocarcinoma and non-small cell lung cancer), pancreatic cancer (e.g. pancreatic carcinoma such as exocrine pancreatic carcinoma), colon cancer (e.g. colorectal carcinoma, such ascolon adenocarcinoma and colon adenoma), prostate cancer including the advanced disease, hematopoietic tumors of lymphoid lineage (e.g. leukemia, acute lymphocytic leukemia, chronic lymphocytic leukemia, B-cell lymphoma, Burkitt's lymphoma, multiple myeloma, Hodgkin's lymphoma, Non-Hodgkin's lymphoma, anti CD20 (i.e. Rituximab) resistant lymphoma), myeloid leukemia (for example, acute myelogenous leukemia (AML), chronic myelogenous leukemia), thyroid cancer, thyroid follicular cancer, myelodysplastic syndrome (MDS), tumors of mesenchymal origin (e.g. fibrosarcomas and rhabdomyosarcomas), melanoma, uveal melanoma, teratocarcinoma, neuroblastoma, glioma, glioblastoma, benign tumor of the skin (e.g. keratoacanthomas), renal cancer, anaplastic large-cell lymphoma, esophageal squamous cells carcinoma, hepatocellular carcinoma, follicular dendritic cell carcinoma, intestinal cancer, muscle-invasive cancer, seminal vesicle tumor, epidermal carcinoma, spleen cancer, bladder cancer, head and neck cancer, stomach cancer, liver cancer, bone cancer, brain cancer, cancer of the retina, biliary cancer, small bowel cancer, salivary gland cancer, cancer of uterus, cancer of testicles, cancer of connective tissue, prostatic hypertrophy, myelodysplasia, Waldenstrom's macroglobinaemia, nasopharyngeal, neuroendocrine cancer, myelodysplastic syndrome, mesothelioma, angiosarcoma, Kaposi's sarcoma, carcinoid, oesophagogastric, fallopian tube cancer, peritoneal cancer, papillary serous mullerian cancer, malignant ascites, gastrointestinal stromal tumor (GIST), and a hereditary cancer syndrome such as Li-Fraumeni syndrome and Von Hippel-Lindau syndrome (VHL).

With regard to ovarian cancer, the disease is selected from the group including but not limited to primary and metastatic cancer of the ovary, including epithelial ovarian cancer such as serous, mucinous, endometroid, clear cell, mixed epithelial, undifferentiated carcinomas and Brenner tumor, as well as other non-epithelial neoplasms of the ovary, including germ cell malignancies.

With regard to breast cancer, the disease is selected from the group including but not limited to primary and metastatic cancer of the breast, including mammary carcinomas such as Infiltrating Ductal carcinoma, Ductal carcinoma in-situ, Infiltrating Lobular carcinoma, Lobular carcinoma in-situ, Inflammatory breast cancer, Paget's disease of the breast, and other non-epithelial neoplasms of the breast, including fibrosarcomas, leiomyosarcomas, rhapdomyosarcomas, angiosarcomas, cystosarcoma phyllodes.

With regard to lung cancer, the disease is selected from the group consisting of but not limited to squamous cell lung carcinoma, lung adenocarcinoma, carcinoid, small cell lung cancer or non-small cell lung cancer.

With regard to liver cancer, the disease is selected from the group consisting of but not limited to primary and metastatic cancers of the liver and intrahepatic bile duct, including hepatocellular carcinoma, cholangiocarcinoma, hepatic angiosarcoma and hepatoblastoma.

With regard to renal cancer, the disease is selected from the group consisting of but not limited to primary and metastatic cancer of the kidney, including renal cell carcinoma (i.e. renal adenocarcinoma), as well as other non-epithelial neoplasms of the ovary, including nephroblastoma (i.e. Wilm's tumor), transitional cell neoplasms of the renal pelvis, and various sarcomas of renal origin.

With regard to colon cancer, the disease is selected from the group consisting of but not limited to primary and metastatic cancer of the colon, including adenocarcinoma, mucinous carcinoma (including signet ring cell-type and medullary), adenosquamous carcinoma, carcinoid, small cell carcinoma, squamous cell carcinoma, undifferentiated carcinoma, as well as other non-epithelial neoplasms of the colon, including lymphoma, melanoma and sarcoma.

With regard to pancreatic cancer, the disease is selected from the group consisting of but not limited to primary and metastatic cancers of the exocrine pancreas, including adenocarcinoma, serous and mucinous cystadenocarcinomas, acinar cell carcinoma, undifferentiated carcinoma, pancreatoblastoma and neuroendocrine tumors such as insulinoma.

With regard to melanoma, the disease is selected from the group consisting of but not limited to primary and metastatic malignant melanoma, including cutaneous melanoma such as superficial spreading melanoma, nodular melanoma, acral lentiginous melanoma and lentigo maligna melanoma, as well as mucosal melanoma, intraocular melanoma, desmoplastic/neurotropic melanoma and melanoma of soft parts (clear cell sarcoma).

With regard to prostate cancer, the disease is selected from the group consisting of but not limited to primary and metastatic cancer of the prostate, including prostatic intraepithelial neoplasia, atypical adenomatous hyperplasia, prostate adenocarcinoma, mucinous or signet ring tumor, adenoid cystic carcinoma, prostatic duct carcinoma, carcinoid and small-cell undifferentiated cancer.

As used herein the term *"hematological malignancies"* refers to acute lymphocytic leukemia, chronic lymphocytic leukemia (CLL), acute lymphoblastic leukemia, acute myelogenous leukemia, chronic myelogenous leukemia, multiple myeloma, B-cell lymphoma, such as Hodgkin's lymphoma, non-Hodgkin's lymphoma (NHL), anti CD20 (i.e. Rituximab) resistant lymphoma, low grade/follicular NHL, small cell lymphocytic (SL) NHL, small cell NHL, grade I small cell follicular NHL, grade II mixed small and large cell follicular NHL, grade III large cell follicular NHL, large cell NHL, Diffuse Large B-Cell NHL, intermediate grade diffuse NHL, high grade immunoblastic NHL, high grade lymphoblastic NHL, high grade small non- cleaved cell NHL, bulky disease NHL, mantle cell lymphoma, Mucosa-Associated Lymphatic Tissue lymphoma (MALT), Burkitt lymphoma, Mediastinal large B cell lymphoma, Nodal marginal zone B cell lymphoma (NMZL), Splenic marginal zone lymphoma (SMZL), Extranodal marginal zone B cell lymphoma, Intravascular large B cell lymphoma, Primary effusion lymphoma, Lymphomatoid granulomatosis, B-cell prolymphocytic leukemia, Precursor B lymphoblastic leukemia, Hairy cell leukemia, AIDS-related lymphoma and Waldenstrom's Macroglobulinernia.

The term "immune related condition" as used herein will encompass any disease disorder or condition selected from the group including but not limited to multiple sclerosis; psoriasis; rheumatoid arthritis; psoriatic arthritis, systemic lupus erythematosus (SLE); ulcerative colitis; Crohn's disease; benign lymphocytic angiitis, thrombocytopenic purpura, idiopathic thrombocytopenia, idiopathic autoimmune hemolytic anemia, pure red cell aplasia, Sjogren's syndrome, rheumatic disease, connective tissue disease, inflammatory rheumatism, degenerative rheumatism, extra-articular rheumatism, juvenile rheumatoid arthritis, arthritis uratica, muscular rheumatism, chronic polyarthritis, cryoglobulinemic vasculitis, ANCA-associated vasculitis, antiphospholipid syndrome, myasthenia gravis, autoimmune haemolytic anaemia, Guillian-Barre syndrome, chronic immune polyneuropathy, autoimmune thyroiditis, insulin dependent diabetes mellitus, type I diabetes, Addison's disease, membranous glomerulonephropathy, Goodpasture's disease, autoimmune gastritis, pernicious anaemia, pemphigus vulgarus, cirrhosis, primary biliary cirrhosis, dermatomyositis, polymyositis, fibromyositis, myogelosis, celiac disease, immunoglobulin A nephropathy, Henoch-Schonlein purpura, Evans syndrome, atopic dermatitis, psoriasis, psoriasis arthropathica, Graves' disease, Graves' ophthalmopathy, scleroderma, systemic scleroderma, asthma, allergy, primary biliary cirrhosis, Hashimoto's thyroiditis, primary myxedema, sympathetic ophthalmia, autoimmune uveitis, hepatitis, chronic action hepatitis, collagen diseases, ankylosing spondylitis, periarthritis humeroscapularis, panarteritis nodosa, chondrocalcinosis, Wegener's granulomatosis, microscopic polyangiitis, chronic urticaria, bullous skin disorders, pemphigoid, atopic eczema, Devic's disease, childhood autoimmune hemolytic anemia, Refractory or chronic Autoimmune Cytopenias, Prevention of development of Autoimmune Anti-Factor VIII Antibodies in Acquired Hemophilia A, Cold Agglutinin Disease, Neuromyelitis Optica, Stiff Person Syndrome, gingivitis, periodontitis, pancreatitis, myocarditis, vasculitis, gastritis, gout, gouty arthritis, and inflammatory skin disorders, selected from the group consisting of psoriasis, atopic dermatitis, eczema, rosacea, urticaria, and acne, normocomplementemic urticarial vasculitis, pericarditis, myositis, anti-synthetase syndrome, scleritis, macrophage activation syndrome, Bechet's Syndrome, PAPA Syndrome, Blau's Syndrome, gout, adult and juvenile Still's disease, cryropyrinopathy, Muckle-Wells syndrome, familial cold-induced auto-inflammatory syndrome, neonatal onset multisystemic inflammatory disease, familial Mediterranean fever, chronic infantile neurologic, cutaneous and articular syndrome, systemic juvenile idiopathic arthritis, Hyper IgD syndrome, Schnitzler's syndrome, and TNF receptor-associated periodic syndrome (TRAPS), inflammatory bowel disease, Good pasture's syndrome, pernicious anemia, autoimmune atrophic gastritis, ulceratis colitis, mixed connective tissue disease, panarteriitis nodosa, progressive systemic scleroderma, peptic ulcers, ulcers, chronic bronchitis, acute lung injury, pulmonary inflammation, airway hyper-responsiveness, septic shock, inflammatory skin disorders, myogelosis, chondrocalcinosis, thyroditis, allergic oedema, granulomas, immune disorders associated with graft transplantation rejection, such as acute and chronic rejection of organ transplantation, allogenic stem cell transplantation, autologous stem cell transplantation, bone marrow tranplantation, and graft versus host disease.

As used herein the term "***treatment***" refers to care provided to relieve illness and refers to both a therapeutic treatment or prophylactic/preventative measures, wherein the objective is to prevent or slow down (lessen) the targeted pathologic condition or disorder. Those in need of treatment include those already with the disorder as well as those prone to have the disorder or those in whom the disorder is to be prevented. The term treatment as used herein refers also to "maintenance therapy", which is a treatment that is given to keep a pathologic condition or disorder from coming back after it has disappeared following the initial therapy.

The term "therapeutically effective amount" refers to an amount of agent according to the present invention that is effective to treat a disease or disorder in a mammal.

As used herein the term "***diagnosis***" refers to the process of identifying a medical condition or disease by its signs, symptoms, and in particular from the results of various diagnostic procedures, including e.g. detecting the expression of the nucleic acids or polypeptides according to at least some embodiments of the invention in a biological sample (e.g. in cells, tissue or serum, as defined below) obtained from an individual. Furthermore, as used herein the term "diagnosis" encompasses screening for a disease, detecting a presence or a severity of a disease, distinguishing a disease from other diseases including those diseases that may feature one or more similar or identical symptoms, providing prognosis of a disease, monitoring disease progression or relapse, as well as assessment of treatment efficacy and/or relapse of a disease, disorder or condition, as well as selecting a therapy and/or a treatment for a disease, optimization of a given therapy for a disease, monitoring the treatment of a disease, and/or predicting the suitability of a therapy for specific patients or subpopulations or determining the appropriate dosing of a therapeutic product in patients or subpopulations. The diagnostic procedure can be performed in vivo or in vitro. It should be noted that a "biological sample obtained from the subject" may also optionally comprise a sample that has not been physically removed from the subject.

As used herein the term ***"combination therapy**"* refers to the simultaneous or consecutive administration of two or more medications or types of therapy to treat a single disease. In particular, the term refers to the use of any of the polypeptides, polynucleotides, antibodies or pharmaceutical compositions according to at least some embodiments of the invention in combination with at least one additional medication or therapy. Thus, treatment of a disease using the agents according to at least some embodiments of the present invention may be combined with therapies well known in the art that include, but are not limited to, radiation therapy, antibody therapy, chemotherapy or surgery or in combination therapy with other biological agents, conventional drugs, anti-cancer agents, immunosuppressants, cytotoxic drugs for cancer, chemotherapeutic agents. According to at least some embodiments, treatment of Multiple Myeloma using the agents according to at least some embodiments of the present invention may be combined with an agent including but not limited to Melphalan, prednisone, thalidomide (MPT), or combination Bortezomib (Velcade), melphalan, prednisone (VMP) or a combination of Lenalidomide plus low-dose dexamethasone.

According to at least some embodiments, treatment of ovarian cancer using the agents according to at least some embodiments of the present invention may be combined with an agent including but not limited to paclitaxol and cisplatin.

According to at least some embodiments, treatment of rheumatoid arthritis disorder using the agents according to at least some embodiments of the present invention may be combined with but not limited to a first-line combination treatment with a drug such as aspirin and cortisone (corticosteroids), which are used to reduce pain and inflammation, and one or more second-line combination drugs, such as gold, methotrexate, and hydroxychloroquine (Plaquenil), promote disease remission and prevent progressive joint destruction. According to at least some embodiments, treatment of rheumatoid arthritis disorder may optionally feature an agent according to the present invention combined with an agent including but not limited to methotrexate and rituximab.

As used herein, the term "subject" includes any human or nonhuman animal. The term "nonhuman animal" includes all vertebrates, e.g., mammals and non-mammals, such as nonhuman primates, sheep, dogs, cats, horses, cows, chickens, amphibians, reptiles, etc.

Various aspects of the invention are described in further detail in the following subsections.

### NUCLEIC ACIDS

A "nucleic acid fragment" or an "oligonucleotide" or a "polynucleotide" are used herein interchangeably to refer to a polymer of nucleic acid residues. A polynucleotide sequence according to at least some embodiments of the present invention refers to a single or double stranded nucleic acid sequences which is isolated and provided in the form of an RNA sequence, a complementary polynucleotide sequence (cDNA), a genomic polynucleotide sequence and/or a composite polynucleotide sequences (e.g., a combination of the above).

Thus, the present invention encompasses nucleic acid sequences described hereinabove; fragments thereof, sequences hybridizable therewith, sequences homologous thereto [e.g., at least 90%, at least 95, 96, 97, 98 or 99 % or more identical to the nucleic acid sequences set forth herein], sequences encoding similar polypeptides with different codon usage, altered sequences characterized by mutations, such as deletion, insertion or substitution of one or more nucleotides, either naturally occurring or man induced, either randomly or in a targeted fashion. The present invention also encompasses homologous nucleic acid sequences (i.e., which form a part of a polynucleotide sequence according to at least some embodiments of the present invention), which include sequence regions unique to the polynucleotides according to at least some embodiments of the present invention.

In cases where the polynucleotide sequences according to at least some embodiments of the present invention encode previously unidentified polypeptides, the present invention also encompasses novel polypeptides or portions thereof, which are encoded by the isolated polynucleotide and respective nucleic acid fragments thereof described hereinabove.

Thus, the present invention also encompasses polypeptides encoded by the polynucleotide sequences according to at least some embodiments of the present invention. The present invention also encompasses homologues of these polypeptides, such homologues can be at least 90 %, at least 95, 96, 97, 98 or 99 % or more homologous to the amino acid sequences set forth below, as can be determined using BlastP software of the National Center of Biotechnology Information (NCBI) using default parameters. Finally, the present invention also encompasses fragments of the above described polypeptides and polypeptides having mutations, such as deletions, insertions or substitutions of one or more amino acids, either naturally occurring or man induced, either randomly or in a targeted fashion.

Oligonucleotides designed for carrying out the methods according to at least some embodiments of the present invention for any of the sequences provided herein (designed as described above) can be generated according to any oligonucleotide synthesis method known in the art such as enzymatic synthesis or solid phase synthesis. Equipment and reagents for executing solid-phase synthesis are commercially available from, for example, Applied Biosystems. Any other means for such synthesis may also be employed; the actual synthesis of the oligonucleotides is well within the capabilities of one skilled in the art.

Oligonucleotides used according to this aspect according to at least some embodiments of the present invention are those having a length selected from a range of about 10 to about 200 bases, optionally about 15 to about 150 bases, about 20 to about 100 bases, or about 20 to about 50 bases.

The oligonucleotides according to at least some embodiments of the present invention may comprise heterocyclic nucleosides consisting of purines and the pyrimidines bases, bonded in a 3' to 5' phosphodiester linkage.

### PEPTIDES

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide" and "protein" include glycoproteins, as well as non-glycoproteins.

Polypeptide products can be biochemically synthesized such as by employing standard solid phase techniques. Such methods include exclusive solid phase synthesis, partial solid phase synthesis methods, fragment condensation, classical solution synthesis. These methods are optionally used when the peptide is relatively short (i.e., 10 kDa) and/or when it cannot be produced by recombinant techniques (i.e., not encoded by a nucleic acid sequence) and therefore involves different chemistry.

Solid phase polypeptide synthesis procedures are well known in the art and further described by John Morrow Stewart and Janis Dillaha Young, Solid Phase Peptide Syntheses (2nd Ed., Pierce Chemical Company, 1984).

Synthetic polypeptides can be purified by preparative high performance liquid chromatography [Creighton T. (1983) Proteins, structures and molecular principles. WH Freeman and Co. N.Y.] and the composition of which can be confirmed via amino acid sequencing.

In cases where large amounts of a polypeptide are desired, it can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

It will be appreciated that peptides according to at least some embodiments of the present invention may be degradation products, synthetic peptides or recombinant peptides as well as peptidomimetics, typically, synthetic peptides and peptoids and semipeptoids which are peptide analogs, which may have, for example, modifications rendering the peptides more stable while in a body or more capable of penetrating into cells. Such modifications include, but are not limited to N terminus modification, C terminus modification, peptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992). Further details in this respect are provided hereinunder.

Peptide bonds (-CO-NH-) within the peptide may be substituted, for example, by N-methylated bonds (-N(CH3)-CO-), ester bonds (-C(R)H-C-O-O-C(R)-N-), ketomethylen bonds (-CO-CH2-), α-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefinic double bonds (-CH=CH-), retro amide bonds (-NH-CO-), peptide derivatives (-N(R)-CH2-CO-), wherein R is the "normal" side chain, naturally presented on the carbon atom.

These modifications can occur at any of the bonds along the peptide chain and even at several (2-3) at the same time.

Natural aromatic amino acids, Trp, Tyr and Phe, may be substituted by synthetic non-natural acid such as Phenylglycine, TIC, naphthylelanine (Nol), ring-methylated derivatives of Phe, halogenated derivatives of Phe or o-methyl-Tyr.

In addition to the above, the peptides according to at least some embodiments of the present invention may also include one or more modified amino acids or one or more non-amino acid monomers (e.g. fatty acids, complex carbohydrates etc).

As used herein in the specification and in the claims section below the term "amino acid" or "amino acids" is understood to include the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

The peptides according to at least some embodiments of the present invention might include one or more non-natural or natural polar amino acids, including but not limited to serine and threonine which are capable of increasing peptide solubility due to their hydroxyl-containing side chain.

The peptides according to at least some embodiments of the present invention can be biochemically synthesized such as by using standard solid phase techniques. These methods include exclusive solid phase synthesis, partial solid phase synthesis methods, fragment condensation, classical solution synthesis. These methods are optionally used when the peptide is relatively short (i.e., 10 kDa) and/or when it cannot be produced by recombinant techniques (i.e., not encoded by a nucleic acid sequence) and therefore involves different chemistry.

Solid phase peptide synthesis procedures are well known in the art and further described by John Morrow Stewart and Janis Dillaha Young, Solid Phase Peptide Syntheses (2nd Ed., Pierce Chemical Company, 1984).

Synthetic peptides can be purified by preparative high performance liquid chromatography [Creighton T. (1983) Proteins, structures and molecular principles. WH Freeman and Co. N.Y.] and the composition of which can be confirmed via amino acid sequencing.

In cases where large amounts of the peptides according to at least some embodiments of the present invention are desired, the peptides can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

### RECOMBINANT EXPRESSION OF POLYPEPTIDES

Methods for introduction of heterologous polynucleotides into mammalian cells are well known in the art and include dextran-mediated transfection, calcium phosphate precipitation, polybrene-mediated transfection, protoplast fusion, electroporation, encapsulation of the polynucleotide(s) in liposomes, biolistic injection and direct microinjection of the DNA into nuclei. In addition, nucleic acid molecules may be introduced into mammalian cells by viral vectors. Methods of transforming cells are well known in the art. See, e.g., U.S. Patent Nos. 4,399,216, 4,912,040, 4,740,461, and 4,959,455 (which patents are hereby incorporated herein by reference). Methods of transforming plant cells are well known in the art, including, e.g., Agrobacterium-mediated transformation, biolistic transformation, direct injection, electroporation and viral transformation. Methods of transforming bacterial and yeast cells are also well known in the art.

Mammalian cell lines available as hosts for expression are well known in the art and include many immortalized cell lines available from e.g. the American Type Culture Collection (ATCC). These include, inter alia, Chinese hamster ovary (CHO) cells, NS0, SP2 cells, HEK-293T cells, NIH-3T3 cells, HeLa cells, baby hamster kidney (BHK) cells, monkey kidney cells (COS), human hepatocellular carcinoma cells (e.g., Hep G2), A549 cells, 3T3 cells, and a number of other cell lines. Mammalian host cells include human, mouse, rat, dog, monkey, pig, goat, bovine, horse and hamster cells. Cell lines of particular preference are selected through determining which cell lines have high expression levels. Other cell lines that may be used are insect cell lines, such as Sf9 cells, amphibian cells, bacterial cells, plant cells and fungal cells. When recombinant expression vectors encoding the polypeptides according to at least some embodiments of the invention or fragments thereof are introduced into mammalian host cells, the polypeptides are produced by culturing the host cells for a period of time sufficient to allow for expression of the polypeptide in the host cells or, more preferably, secretion of the polypeptide into the culture medium in which the host cells are grown. Polypeptides can be recovered from the culture medium using standard protein purification methods. Plant host cells include, e.g., Nicotiana, Arabidopsis, duckweed, corn, wheat, potato, etc. Bacterial host cells include E. coli and Streptomyces species. Yeast host cells include Schizosaccharomyces pombe, Saccharomyces cerevisiae and Pichia pastoris.

Further, expression of the polypeptides according to at least some embodiments of the invention (or other moieties derived therefrom) from production cell lines can be enhanced using a number of known techniques. For example, the glutamine synthetase gene expression system (the GS system) is a common approach for enhancing expression under certain conditions. The GS system is discussed in whole or part in connection with European Patent Nos. 0 216 846, 0 256 055, 0 338 841 and 0 323 997.

It is likely that polypeptides expressed by different cell lines or in transgenic animals will have different glycosylation patterns. However, all polypeptides encoded by the nucleic acid molecules provided herein, or comprising the amino acid sequences provided herein are part of the instant invention, regardless of their glycosylation pattern.

### VECTORS

To express the polypeptides according to at least some embodiments of the invention, or fragments thereof, DNAs encoding partial or full-length polypeptides, obtained as described above, are inserted into expression vectors such that the genes are operatively linked to transcriptional and translational control sequences. Expression vectors include plasmids, retroviruses, adenoviruses, adeno-associated viruses (AAV), plant viruses such as cauliflower mosaic virus, tobacco mosaic virus, cosmids, YACs, EBV derived episomes, and the like. The gene is ligated into a vector such that transcriptional and translational control sequences within the vector serve their intended function of regulating the transcription and translation of the gene. The expression vector and expression control sequences are chosen to be compatible with the expression host cell used. The gene is inserted into the expression vector by standard methods (e.g., ligation of complementary restriction sites on the gene fragment and vector, or blunt end ligation if no restriction sites are present).

A convenient vector is one that encodes a functionally complete sequence, with appropriate restriction sites engineered so that any sequence can be easily inserted and expressed, as described above. Polyadenylation and transcription termination occur at native chromosomal sites downstream of the coding regions. The recombinant expression vector can also encode a signal peptide that facilitates secretion of the polypeptide from a host cell. The gene may be cloned into the vector such that the signal peptide is linked in-frame to the amino terminus of the gene.

In addition to the nucleic acid according to at least some embodiments of the invention, the recombinant expression vectors carry regulatory sequences that control the expression of the gene in a host cell. It will be appreciated by those skilled in the art that the design of the expression vector, including the selection of regulatory sequences may depend on such factors as the choice of the host cell to be transformed, the level of expression of protein desired, etc. Preferred regulatory sequences for mammalian host cell expression include viral elements that direct high levels of protein expression in mammalian cells, such as promoters and/or enhancers derived from retroviral LTRs, cytomegalovirus (CMV) (such as the CMV promoter/enhancer), Simian Virus 40 (SV40) (such as the SV40 promoter/enhancer), adenovirus, (e.g., the adenovirus major late promoter (AdMLP)), polyoma and strong mammalian promoters such as native immunoglobulin and actin promoters. For further description of viral regulatory elements, and sequences thereof, see e.g., U.S. Pat. Nos. 5,168,062, 4,510,245, and 4,968,615. Methods of expressing polypeptides in bacterial cells or fungal cells, e.g., yeast cells, are also well known in the art.

In addition to the nucleic acids according to at least some embodiments of the invention and regulatory sequences, the recombinant expression vectors according to at least some embodiments of the invention may carry additional sequences, such as sequences that regulate replication of the vector in host cells (e.g., origins of replication) and selectable marker genes. The selectable marker gene facilitates selection of host cells into which the vector has been introduced (see, e.g., U.S. Pat. Nos. 4,399,216, 4,634,665 and 5,179,017). For example, typically the selectable marker gene confers resistance to drugs, such as G418, hygromycin or methotrexate, on a host cell into which the vector has been introduced. Preferred selectable marker genes include the dihydrofolate reductase (DHFR) gene, the neo gene (for G418 selection), and the glutamate synthetase gene.

### PROTEIN MODIFICATIONS

### FUSION PROTEINS

According to at least some embodiments, a fusion protein may be prepared from a protein according to at least some embodiments of the invention by fusion with a portion of an immunoglobulin comprising a constant region of an immunoglobulin. Optionally, the portion of the immunoglobulin comprises a heavy chain constant region which is optionally and more preferably a human heavy chain constant region. The heavy chain constant region is optionally an IgG heavy chain constant region, and optionally an Fc chain, or an IgG Fc fragment that comprises CH2 and CH3 domains. Although any IgG subtype may optionally be used, the IgG1 subtype is preferred. The Fc chain may optionally be a known or "wild type" Fc chain, or alternatively may be mutated. Non-limiting, illustrative, exemplary types of mutations are described in US Patent Application No. 20060034852, published on February 16, 2006. The term "Fc chain" also optionally comprises any type of Fc fragment.

Several of the specific amino acid residues that are important for antibody constant region-mediated activity in the IgG subclass have been identified. Inclusion, substitution or exclusion of these specific amino acids therefore allows for inclusion or exclusion of specific immunoglobulin constant region-mediated activity. Furthermore, specific changes may result in aglycosylation for example and/or other desired changes to the Fc chain. At least some changes may optionally be made to block a function of Fc which is considered to be undesirable, such as an undesirable immune system effect, as described in greater detail below.

Non-limiting, illustrative examples of mutations to Fc which may be made to modulate the activity of the fusion protein include the following changes (given with regard to the Fc sequence nomenclature as given by Kabat, from Kabat EA et al: Sequences of Proteins of Immunological Interest. US Department of Health and Human Services, NIH, 1991): 220C - > S; 233-238 ELLGGP - > EAEGAP; 265D - > A, preferably in combination with 434N -> A; 297N - > A (for example to block N-glycosylation); 318-322 EYKCK - > AYACA; 330-331AP - > SS; or a combination thereof (see for example M. Clark, "Chemical Immunol and Antibody Engineering", pp 1-31 for a description of these mutations and their effect). The construct for the Fc chain which features the above changes optionally and preferably comprises a combination of the hinge region with the CH2 and CH3 domains.

The above mutations may optionally be implemented to enhance desired properties or alternatively to block non-desired properties. For example, aglycosylation of antibodies was shown to maintain the desired binding functionality while blocking depletion of T-cells or triggering cytokine release, which may optionally be undesired functions (see M. Clark, "Chemical Immunol and Antibody Engineering", pp 1-31). Substitution of 331 proline for serine may block the ability to activate complement, which may optionally be considered an undesired function (see M. Clark, "Chemical Immunol and Antibody Engineering", pp 1-31). Changing 330alanine to serine in combination with this change may also enhance the desired effect of blocking the ability to activate complement.

Residues 235 and 237 were shown to be involved in antibody-dependent cell-mediated cytotoxicity (ADCC), such that changing the block of residues from 233-238 as described may also block such activity if ADCC is considered to be an undesirable function.

Residue 220 is normally a cysteine for Fc from IgG1, which is the site at which the heavy chain forms a covalent linkage with the light chain. Optionally, this residue may be changed to a serine, to avoid any type of covalent linkage (see M. Clark, "Chemical Immunol and Antibody Engineering", pp 1-31).

The above changes to residues 265 and 434 may optionally be implemented to reduce or block binding to the Fc receptor, which may optionally block undesired functionality of Fc related to its immune system functions (see "Binding site on Human IgG1 for Fc Receptors", Shields et al, Vol 276, pp 6591-6604, 2001).

The above changes are intended as illustrations only of optional changes and are not meant to be limiting in any way. Furthermore, the above explanation is provided for descriptive purposes only, without wishing to be bound by a single hypothesis.

### ADDITION OF GROUPS

If a protein according to the present invention is a linear molecule, it is possible to place various functional groups at various points on the linear molecule which are susceptible to or suitable for chemical modification. Functional groups can be added to the termini of linear forms of the protein according to at least some embodiments of the invention. In some embodiments, the functional groups improve the activity of the protein with regard to one or more characteristics, including but not limited to, improvement in stability, penetration (through cellular membranes and/or tissue barriers), tissue localization, efficacy, decreased clearance, decreased toxicity, improved selectivity, improved resistance to expulsion by cellular pumps, and the like. For convenience sake and without wishing to be limiting, the free N-terminus of one of the sequences contained in the compositions according to at least some embodiments of the invention will be termed as the N-terminus of the composition, and the free C-terminal of the sequence will be considered as the C-terminus of the composition. Either the C-terminus or the N-terminus of the sequences, or both, can be linked to a carboxylic acid functional groups or an amine functional group, respectively.

Non-limiting examples of suitable functional groups are described in Green and Wuts, "Protecting Groups in Organic Synthesis", John Wiley and Sons, Chapters 5 and 7, 1991, the teachings of which are incorporated herein by reference. Preferred protecting groups are those that facilitate transport of the active ingredient attached thereto into a cell, for example, by reducing the hydrophilicity and increasing the lipophilicity of the active ingredient, these being an example for "a moiety for transport across cellular membranes".

These moieties can optionally be cleaved in vivo, either by hydrolysis or enzymatically, inside the cell. (Ditter et al., J. Pharm. Sci. 57:783 (1968); Ditter et al., J. Pharm. Sci. 57:828 (1968); Ditter et al., J. Pharm. Sci. 58:557 (1969); King et al., Biochemistry 26:2294 (1987); Lindberg et al., Drug Metabolism and Disposition 17:311 (1989); and Tunek et al., Biochem. Pharm. 37:3867 (1988), Anderson et al., Arch. Biochem. Biophys. 239:538 (1985) and Singhal et al., FASEB J. 1:220 (1987)). Hydroxyl protecting groups include esters, carbonates and carbamate protecting groups. Amine protecting groups include alkoxy and aryloxy carbonyl groups, as described above for N-terminal protecting groups. Carboxylic acid protecting groups include aliphatic, benzylic and aryl esters, as described above for C-terminal protecting groups. In one embodiment, the carboxylic acid group in the side chain of one or more glutamic acid or aspartic acid residue in a composition according to at least some embodiments of the present invention is protected, optionally with a methyl, ethyl, benzyl or substituted benzyl ester.

Non-limiting, illustrative examples of N-terminal protecting groups include acyl groups (-CO-R1) and alkoxy carbonyl or aryloxy carbonyl groups (-CO-O-R1), wherein R1 is an aliphatic, substituted aliphatic, benzyl, substituted benzyl, aromatic or a substituted aromatic group. Specific examples of acyl groups include but are not limited to acetyl, (ethyl)-CO-, n-propyl-CO-, iso-propyl-CO-, n-butyl-CO-, sec-butyl-CO-, t-butyl-CO-, hexyl, lauroyl, palmitoyl, myristoyl, stearyl, oleoyl phenyl-CO-, substituted phenyl-CO-, benzyl-CO- and (substituted benzyl)-CO-. Examples of alkoxy carbonyl and aryloxy carbonyl groups include CH3-O-CO-, (ethyl)-O-CO-, n-propyl-O-CO-, iso-propyl-O-CO-, n-butyl-O-CO-, sec-butyl-O-CO-, t-butyl-O-CO-, phenyl-O- CO-, substituted phenyl-O-CO- and benzyl-O-CO-, (substituted benzyl)- O-CO-, Adamantan, naphtalen, myristoleyl, toluen, biphenyl, cinnamoyl, nitrobenzoy, toluoyl, furoyl, benzoyl, cyclohexane, norbornane, or Z-caproic. In order to facilitate the N-acylation, one to four glycine residues can be present in the N-terminus of the molecule.

The carboxyl group at the C-terminus of the compound can be protected, for example, by a group including but not limited to an amide (i.e., the hydroxyl group at the C-terminus is replaced with -NH ₂, -NHR₂ and -NR₂R₃) or ester (i.e. the hydroxyl group at the C-terminus is replaced with -OR₂). R₂ and R₃ are optionally independently an aliphatic, substituted aliphatic, benzyl, substituted benzyl, aryl or a substituted aryl group. In addition, taken together with the nitrogen atom, R₂ and R₃ can optionally form a C4 to C8 heterocyclic ring with from about 0-2 additional heteroatoms such as nitrogen, oxygen or sulfur. Non-limiting suitable examples of suitable heterocyclic rings include piperidinyl, pyrrolidinyl, morpholino, thiomorpholino or piperazinyl. Examples of C-terminal protecting groups include but are not limited to -NH₂, -NHCH₃, -N(CH₃)₂, -NH(ethyl), -N(ethyl)₂, -N(methyl) (ethyl), -NH(benzyl), -N(C1-C4 alkyl)(benzyl), -NH(phenyl), -N(C1-C4 alkyl) (phenyl), -OCH₃, -O-(ethyl), -O-(n-propyl), -O-(n-butyl), -O-(iso-propyl), -O-(sec- butyl), -O-(t-butyl), -O-benzyl and -O-phenyl.

### SUBSTITUTION BY PEPTIDOMIMETIC MOIETIES

A "peptidomimetic organic moiety" can optionally be substituted for amino acid residues in the composition of this invention both as conservative and as non-conservative substitutions. These moieties are also termed "non-natural amino acids" and may optionally replace amino acid residues, amino acids or act as spacer groups within the peptides in lieu of deleted amino acids. The peptidomimetic organic moieties optionally have steric, electronic or configurational properties similar to the replaced amino acid and such peptidomimetics are used to replace amino acids in the essential positions, and are considered conservative substitutions. However such similarities are not necessarily required. According to at least some embodiments of the present invention, one or more peptidomimetics are selected such that the composition at least substantially retains its physiological activity as compared to the native protein according to the present invention.

Peptidomimetics may optionally be used to inhibit degradation of the peptides by enzymatic or other degradative processes. The peptidomimetics can optionally be produced by organic synthetic techniques. Non-limiting examples of suitable peptidomimetics include D amino acids of the corresponding L amino acids, tetrazol (Zabrocki et al., J. Am. Chem. Soc. 110:5875-5880 (1988)); isosteres of amide bonds (Jones et al., Tetrahedron Lett. 29: 3853-3856 (1988)); LL-3-amino-2-propenidone-6-carboxylic acid (LL-Acp) (Kemp et al., J. Org. Chem. 50:5834-5838 (1985)). Similar analogs are shown in Kemp et al., Tetrahedron Lett. 29:5081-5082 (1988) as well as Kemp et al., Tetrahedron Lett. 29:5057-5060 (1988), Kemp et al., Tetrahedron Lett. 29:4935-4938 (1988) and Kemp et al., J. Org. Chem. 54:109-115 (1987). Other suitable but exemplary peptidomimetics are shown in Nagai and Sato, Tetrahedron Lett. 26:647-650 (1985); Di Maio et al., J. Chem. Soc. Perkin Trans., 1687 (1985); Kahn et al., Tetrahedron Lett. 30:2317 (1989); Olson et al., J. Am. Chem. Soc. 112:323-333 (1990); Garvey et al., J. Org. Chem. 56:436 (1990). Further suitable exemplary peptidomimetics include hydroxy-1,2,3,4-tetrahydroisoquinoline- 3-carboxylate (Miyake et al., J. Takeda Res. Labs 43:53-76 (1989)); 1,2,3,4-tetrahydro- isoquinoline-3-carboxylate (Kazmierski et al., J. Am. Chem. Soc. 133:2275-2283 (1991)); histidine isoquinolone carboxylic acid (HIC) (Zechel et al., Int. J. Pep. Protein Res. 43 (1991)); (2S, 3S)-methyl-phenylalanine, (2S, 3R)-methyl-phenylalanine, (2R, 3S)-methyl- phenylalanine and (2R, 3R)-methyl-phenylalanine (Kazmierski and Hruby, Tetrahedron Lett. (1991)).

Exemplary, illustrative but non-limiting non-natural amino acids include beta-amino acids (beta3 and beta2), homo-amino acids, cyclic amino acids, aromatic amino acids, Pro and Pyr derivatives, 3-substituted Alanine derivatives, Glycine derivatives, ring-substituted Phe and Tyr Derivatives, linear core amino acids or diamino acids. They are available from a variety of suppliers, such as Sigma-Aldrich (USA) for example.

### CHEMICAL MODIFICATIONS

In the present invention any part of a protein according to at least some embodiments of the invention may optionally be chemically modified, i.e. changed by addition of functional groups. For example the side amino acid residues appearing in the native sequence may optionally be modified, although as described below alternatively other parts of the protein may optionally be modified, in addition to or in place of the side amino acid residues. The modification may optionally be performed during synthesis of the molecule if a chemical synthetic process is followed, for example by adding a chemically modified amino acid. However, chemical modification of an amino acid when it is already present in the molecule ("in situ" modification) is also possible.

The amino acid of any of the sequence regions of the molecule can optionally be modified according to any one of the following exemplary types of modification (in the peptide conceptually viewed as "chemically modified"). Non-limiting exemplary types of modification include carboxymethylation, acylation, phosphorylation, glycosylation or fatty acylation. Ether bonds can optionally be used to join the serine or threonine hydroxyl to the hydroxyl of a sugar. Amide bonds can optionally be used to join the glutamate or aspartate carboxyl groups to an amino group on a sugar (Garg and Jeanloz, Advances in Carbohydrate Chemistry and Biochemistry, Vol. 43, Academic Press (1985); Kunz, Ang. Chem. Int. Ed. English 26:294-308 (1987)). Acetal and ketal bonds can also optionally be formed between amino acids and carbohydrates. Fatty acid acyl derivatives can optionally be made, for example, by acylation of a free amino group (e.g., lysine) (Toth et al., Peptides: Chemistry, Structure and Biology, Rivier and Marshal, eds., ESCOM Publ., Leiden, 1078-1079 (1990)).

As used herein the term "chemical modification", when referring to a protein or peptide according to the present invention, refers to a protein or peptide where at least one of its amino acid residues is modified either by natural processes, such as processing or other post-translational modifications, or by chemical modification techniques which are well known in the art. Examples of the numerous known modifications typically include, but are not limited to: acetylation, acylation, amidation, ADP-ribosylation, glycosylation, GPI anchor formation, covalent attachment of a lipid or lipid derivative, methylation, myristylation, pegylation, prenylation, phosphorylation, ubiquitination, or any similar process.

Other types of modifications optionally include the addition of a cycloalkane moiety to a biological molecule, such as a protein, as described in PCT Application No. WO 2006/050262. These moieties are designed for use with biomolecules and may optionally be used to impart various properties to proteins.

Furthermore, optionally any point on a protein may be modified. For example, pegylation of a glycosylation moiety on a protein may optionally be performed, as described in PCT Application No. WO 2006/050247. One or more polyethylene glycol (PEG) groups may optionally be added to O-linked and/or N-linked glycosylation. The PEG group may optionally be branched or linear. Optionally any type of water-soluble polymer may be attached to a glycosylation site on a protein through a glycosyl linker.

### ALTERED GLYCOSYLATION

Proteins according to at least some embodiments of the invention may be modified to have an altered glycosylation pattern (i.e., altered from the original or native glycosylation pattern). As used herein, "altered" means having one or more carbohydrate moieties deleted, and/or having at least one glycosylation site added to the original protein.

Glycosylation of proteins is typically either N-linked or O-linked. N-linked refers to the attachment of the carbohydrate moiety to the side chain of an asparagine residue. The tripeptide sequences, asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be used.

Addition of glycosylation sites to proteins according to at least some embodiments of the invention is conveniently accomplished by altering the amino acid sequence of the protein such that it contains one or more of the above-described tripeptide sequences (for N-linked glycosylation sites). The alteration may also be made by the addition of, or substitution by, one or more serine or threonine residues in the sequence of the original protein (for O-linked glycosylation sites). The protein's amino acid sequence may also be altered by introducing changes at the DNA level.

Another means of increasing the number of carbohydrate moieties on proteins is by chemical or enzymatic coupling of glycosides to the amino acid residues of the protein. Depending on the coupling mode used, the sugars may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, and in Aplin and Wriston, CRC Crit. Rev. Biochem., 22: 259-306 (1981).

Removal of any carbohydrate moieties present on proteins according to at least some embodiments of the invention may be accomplished chemically or enzymatically. Chemical deglycosylation requires exposure of the protein to trifluoromethanesulfonic acid, or an equivalent compound. This treatment results in the cleavage of most or all sugars except the linking sugar (N-acetylglucosamine or N-acetylgalactosamine), leaving the amino acid sequence intact.

Chemical deglycosylation is described by Hakimuddin et al., Arch. Biochem. Biophys., 259: 52 (1987); and Edge et al., Anal. Biochem., 118: 131 (1981). Enzymatic cleavage of carbohydrate moieties on proteins can be achieved by the use of a variety of endo- and exo-glycosidases as described by Thotakura et al., Meth. Enzymol., 138: 350 (1987).

### ANTI-TMEM154 ANTIBODIES

The antibodies according to at least some embodiments of the invention including those having the particular germline sequences, homologous antibodies, antibodies with conservative modifications, engineered and modified antibodies are characterized by particular functional features or properties of the antibodies. For example, the antibodies bind specifically to human TMEM154 polypeptides. Optionally, an antibody according to at least some embodiments of the invention binds to corresponding TMEM154 polypeptides with high affinity, for example with a KD of 10 -8 M or less or 10 -9 M or less or even 10 -10 M or less. The Anti-TMEM154 antibodies according to at least some embodiments of the invention optionally exhibit one or more of the following characteristics:
(i) binds to one of the corresponding human TMEM154 polypeptides with a KD of 5.X10 -8 M or less;
(ii) binds to one of the TMEM154 antigen expressed by cancer cells including for example ovarian cancer, lung cancer, colon cancer, breast cancer, kidney cancer, liver cancer, pancreatic cancer, prostate cancer, melanoma and hematological malignancies such as Multiple Myeloma, lymphoma, Non-Hodgkin's lymphoma, anti CD20 (i.e. Rituximab) resistant lymphoma, leukemia, T cell leukemia, but does not substantially bind to normal cells. In addition, optionally these antibodies and conjugates thereof will be effective in eliciting selective killing of such cancer cells and for modulating immune responses involved in autoimmunity and cancer.

Optionally, the antibody binds to one of the corresponding human TMEM154 antigens with a KD of 3X10 -8 M or less, or with a KD of 1X10 -9 M or less, or with a KD of 0.1.X10 -9 M or less, or with a KD Of 0.05.X10 -9 M or less or with a KD of between 1X10 -9 and 1X10 -11 M.

Standard assays to evaluate the binding ability of the antibodies toward TMEM154 polypeptides are known in the art, including for example, ELISAs, Western blots and RIAs. Suitable assays are described in detail in the Examples. The binding kinetics (e.g., binding affinity) of the antibodies also can be assessed by standard assays known in the art, such as by Biacore analysis.

Upon production of Anti-TMEM154 antibody sequences from antibodies can bind to TMEM154 the VH and VL sequences can be "mixed and matched" to create other anti-TMEM154 binding molecules according to at least some embodiments of the invention. TMEM154 binding of such "mixed and matched" antibodies can be tested using the binding assays described above. e.g., ELISAs). Optionally, when VH and VL chains are mixed and matched, a VH sequence from a particular VH/VL pairing is replaced with a structurally similar VH sequence. Likewise, optionally a VL sequence from a particular VH/VL pairing is replaced with a structurally similar VL sequence. For example, the VH and VL sequences of homologous antibodies are particularly amenable for mixing and matching.

### ANTIBODIES HAVING PARTICULAR GERMLINE SEQUENCES

In certain embodiments, an antibody according to at least some embodiments of the invention comprises a heavy chain variable region from a particular germline heavy chain immunoglobulin gene and/or a light chain variable region from a particular germline light chain immunoglobulin gene.

As used herein, a human antibody comprises heavy or light chain variable regions that is "the product of" or "derived from" a particular germline sequence if the variable regions of the antibody are obtained from a system that uses human germline immunoglobulin genes. Such systems include immunizing a transgenic mouse carrying human immunoglobulin genes with the antigen of interest or screening a human immunoglobulin gene library displayed on phage with the antigen of interest. A human antibody that is "the product of" or "derived from" a human germline immunoglobulin sequence can be identified as such by comparing the amino acid sequence of the human antibody to the amino acid sequences of human germline immunoglobulins and selecting the human germline immunoglobulin sequence that is closest in sequence (i.e., greatest % identity) to the sequence of the human antibody.

A human antibody that is "the product of" or "derived from" a particular human germline immunoglobulin sequence may contain amino acid differences as compared to the germline sequence, due to, for example, naturally-occurring somatic mutations or intentional introduction of site-directed mutation. However, a selected human antibody typically is at least 90% identical in amino acids sequence to an amino acid sequence encoded by a human germline immunoglobulin gene and contains amino acid residues that identify the human antibody as being human when compared to the germline immunoglobulin amino acid sequences of other species (e.g., murine germline sequences). In certain cases, a human antibody may be at least 95, 96, 97, 98 or 99%, or even at least 96%, 97%, 98%, or 99% identical in amino acid sequence to the amino acid sequence encoded by the germline immunoglobulin gene. Typically, a human antibody derived from a particular human germline sequence will display no more than 10 amino acid differences from the amino acid sequence encoded by the human germline immunoglobulin gene. In certain cases, the human antibody may display no more than 5, or even no more than 4, 3, 2, or 1 amino acid difference from the amino acid sequence encoded by the germline immunoglobulin gene.

### HOMOLOGOUS ANTIBODIES

In yet another embodiment, an antibody according to at least some embodiments of the invention comprises heavy and light chain variable regions comprising amino acid sequences that are homologous to isolated Anti-TMEM154 amino acid sequences of preferred Anti-TMEM154 antibodies, respectively, wherein the antibodies retain the desired functional properties of the parent Anti-TMEM154 antibodies.

As used herein, the percent homology between two amino acid sequences is equivalent to the percent identity between the two sequences. The percent identity between the two sequences is a function of the number of identical positions shared by the sequences (i.e., % homology=# of identical positions/total # of positions X 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences according to at least some embodiments of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used.

### Antibodies with Conservative Modifications

In certain embodiments, an antibody according to at least some embodiments of the invention comprises a heavy chain variable region comprising CDR1, CDR2 and CDR3 sequences and a light chain variable region comprising CDR1, CDR2 and CDR3 sequences, wherein one or more of these CDR sequences comprise specified amino acid sequences based on preferred Anti-TMEM154 antibodies isolated and produced using methods herein, or conservative modifications thereof, and wherein the antibodies retain the desired functional properties of the Anti-TMEM154 antibodies according to at least some embodiments of the invention, respectively.

In various embodiments, Anti-TMEM154 antibody can be, for example, human antibodies, humanized antibodies or chimeric antibodies.

As used herein, the term "conservative sequence modifications" is intended to refer to amino acid modifications that do not significantly affect or alter the binding characteristics of the antibody containing the amino acid sequence. Such conservative modifications include amino acid substitutions, additions and deletions. Modifications can be introduced into an antibody according to at least some embodiments of the invention by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within the CDR regions of an antibody according to at least some embodiments of the invention can be replaced with other amino acid residues from the same side chain family and the altered antibody can be tested for retained function (i.e., the functions set forth in (c) through (j) above) using the functional assays described herein.

### ENGINEERED AND MODIFIED ANTIBODIES

An antibody according to at least some embodiments of the invention can be prepared using an antibody having one or more of the VH and/or VL sequences derived from an Anti-TMEM154antibody starting material to engineer a modified antibody, which modified antibody may have altered properties from the starting antibody. An antibody can be engineered.by modifying one or more residues within one or both variable regions (i.e., VH and/or VL), for example within one or more CDR regions and/or within one or more framework regions. Additionally or alternatively, an antibody can be engineered by modifying residues within the constant regions, for example to alter the effector functions of the antibody.

One type of variable region engineering that can be performed is CDR grafting. Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al. (1998) Nature 332:323-327; Jones, P. et al. (1986) Nature 321:522-525; Queen, C. et al. (1989) Proc. Natl. Acad. See. U.S.A. 86:10029-10033; U.S. Pat. No. 5,225,539 to Winter, and U.S. Pat. Nos. 5,530,101; 5,585,089; 5,693,762 and 6,180,370 to Queen et al.)

Suitable framework sequences can be obtained from public DNA databases or published references that include germline antibody gene sequences. For example, germline DNA sequences for human heavy and light chain variable region genes can be found in the "VBase" human germline sequence database (available on the Internet), as well as in Kabat, E. A., et al. (1991) Sequences of Proteins of Immunological Interest, Fifth Edition, U.S. Department of Health and Human Services, NIH Publication No. 91-3242; Tomlinson, I. M., et al. (1992) "The Repertoire of Human Germline VH Sequences Reveals about Fifty Groups of VH Segments with Different Hypervariable Loops" J. Mol. Biol. 227:776-798; and Cox, J. P. L. et al. (1994) "A Directory of Human Germ-line VH Segments Reveals a Strong Bias in their Usage" Eur. J Immunol. 24:827-836; the contents of each of which are expressly incorporated herein by reference.

Another type of variable region modification is to mutate amino acid residues within the VH and/or VL CDR 1, CDR2 and/or CDR3 regions to thereby improve one or more binding properties (e.g., affinity) of the antibody of interest. Site-directed mutagenesis or PCR-mediated mutagenesis can be performed to introduce the mutations and the effect on antibody binding, or other functional property of interest, can be evaluated in appropriate in vitro or in vivo assays. Optionally conservative modifications (as discussed above) are introduced. The mutations may be amino acid substitutions, additions or deletions, but are preferably substitutions. Moreover, typically no more than one, two, three, four or five residues within a CDR region are altered.

Engineered antibodies according to at least some embodiments of the invention include those in which modifications have been made to framework residues within VH and/or VL, e.g. to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived.

In addition or alternative to modifications made within the framework or CDR regions, antibodies according to at least some embodiments of the invention may be engineered to include modifications within the Fc region, typically to alter one or more functional properties of the antibody, such as serum half-life, complement fixation, Fc receptor binding, and/or antigen-dependent cellular cytotoxicity. Furthermore, an antibody according to at least some embodiments of the invention may be chemically modified (e.g., one or more chemical moieties can be attached to the antibody) or be modified to alter its glycosylation, again to alter one or more functional properties of the antibody. Such embodiments are described further below. The numbering of residues in the Fc region is that of the EU index of Kabat.

In one embodiment, the hinge region of CH1 is modified such that the number of cysteine residues in the hinge region is altered, e.g., increased or decreased. This approach is described further in U.S. Pat. No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Pat. No. 6,165,745 by Ward et al.

In another embodiment, the antibody is modified to increase its biological half life. Various approaches are possible. For example, one or more of the following mutations can be introduced: T252L, T254S, T256F, as described in U.S. Pat. No. 6,277,375 to Ward. Alternatively, to increase the biological half life, the antibody can be altered within the CH1 or CL region to contain a salvage receptor binding epitope taken from two loops of a CH2 domain of an Fc region of an IgG, as described in U.S. Pat. Nos. 5,869,046 and 6,121,022 by Presta et al.

In yet other embodiments, the Fc region is altered by replacing at least one amino acid residue with a different amino acid residue to alter the effector functions of the antibody. For example, one or more amino acids selected from amino acid residues 234, 235, 236, 237, 297, 318, 320 and 322 can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Pat. Nos. 5,624,821 and 5,648,260, both by Winter et al.

In another example, one or more amino acids selected from amino acid residues 329, 331 and 322 can be replaced with a different amino acid residue such that the antibody has altered C1q binding and/or reduced or abolished complement dependent cytotoxicity (CDC). This approach is described in further detail in U.S. Pat. Nos. 6,194,551 by Idusogie et al.

In another example, one or more amino acid residues within amino acid positions 231 and 239 are altered to thereby alter the ability of the antibody to fix complement. This approach is described further in PCT Publication WO 94/29351 by Bodmer et al.

In yet another example, the Fc region is modified to increase the ability of the antibody to mediate antibody dependent cellular cytotoxicity (ADCC) and/or to increase the affinity of the antibody for an Fcy receptor by modifying one or more amino acids at the following positions: 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439. This approach is described further in PCT Publication WO 00/42072 by Presta. Moreover, the binding sites on human IgG1 for Fc grammar, Fc gamma RII, Fc gammaRIII and FcRn have been mapped and variants with improved binding have been described (see Shields, R. L. et al. (2001) J. Biol. Chem. 276:6591-6604). Specific mutations at positions 256, 290, 298, 333, 334 and 339 are shown to improve binding to FcyRIII. Additionally, the following combination mutants are shown to improve Fcgamma.RIII binding: T256A/S298A, S298A/E333A, S298A/K224A and S298A/E333A/K334A.

In still another embodiment, the glycosylation of an antibody is modified. For example, an aglycoslated antibody can be made (i.e., the antibody lacks glycosylation). Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Pat. Nos. 5,714,350 and 6,350,861 by Co et al.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies according to at least some embodiments of the invention to thereby produce an antibody with altered glycosylation. For example, the cell lines Ms704, Ms705, and Ms709 lack the fucosyltransferase gene, FUT8 (alpha (1,6) fucosyltransferase), such that antibodies expressed in the Ms704, Ms705, and Ms709 cell lines lack fucose on their carbohydrates. The Ms704, Ms705, and Ms709 FUT8.-/- cell lines are created by the targeted disruption of the FUT8 gene in CHO/DG44 cells using two replacement vectors (see U.S. Patent Publication No. 20040110704 by Yamane et al. and Yamane-Ohnuki et al. (2004) Biotechnol Bioeng 87:614-22). As another example, EP 1,176,195 by Hanai et al. describes a cell line with a functionally disrupted FUT8 gene, which encodes a fucosyl transferase, such that antibodies expressed in such a cell line exhibit hypofucosylation by reducing or eliminating the alpha 1,6 bond-related enzyme. Hanai et al. also describe cell lines which have a low enzyme activity for adding fucose to the N-acetylglucosamine that binds to the Fc region of the antibody or does not have the enzyme activity, for example the rat myeloma cell line YB2/0 (ATCC CRL 1662). PCT Publication WO 03/035835 by Presta describes a variant CHO cell line, Lec13 cells, with reduced ability to attach fucose to Asn(297)-linked carbohydrates, also resulting in hypofucosylation of antibodies expressed in that host cell (see also Shields, R. L. et al. (2002) J. Biol. Chem. 277:26733-26740). PCT Publication WO 99/54342 by Umana et al. describes cell lines engineered to express glycoprotein-modifying glycosyl transferases (e.g., beta(1,4)-N-acetylglucosaminyltransferase III (GnTIII)) such that antibodies expressed in the engineered cell lines exhibit increased bisecting GlcNac structures which results in increased ADCC activity of the antibodies (see also Umana et al. (1999) Nat. Biotech. 17:176-180). Alternatively, the fucose residues of the antibody may be cleaved off using a fucosidase enzyme. For example, the fucosidase alpha-L-fucosidase removes fucosyl residues from antibodies (Tarentino, A. L. et al. (1975) Biochem. 14:5516-23).

Another modification of the antibodies herein that is contemplated by the invention is pegylation. An antibody can be pegylated to, for example, increase the biological (e.g., serum) half life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. Optionally, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies according to at least some embodiments of the invention. See for example, EP 0 154 316 by Nishimura et al. and EP 0 401 384 by Ishikawa et al.

### METHODS OF ENGINEERING ANTIBODIES

As discussed above, the Anti-TMEM154 antibodies having VH and VK sequences disclosed herein can be used to create new Anti-TMEM154antibodies, respectively, by modifying the VH and/or VL sequences, or the constant regions attached thereto. Thus, according to at least some embodiments of the invention, the structural features of an Anti-TMEM154 antibody according to at least some embodiments of the invention, are used to create structurally related Anti-TMEM154 antibodies that retain at least one functional property of the antibodies according to at least some embodiments of the invention, such as binding to human TMEM154. For example, one or more CDR regions of one TMEM154 antibody or mutations thereof, can be combined recombinantly with known framework regions and/or other CDRs to create additional, recombinantly-engineered, Anti-TMEM154 antibodies according to at least some embodiments of the invention, as discussed above. Other types of modifications include those described in the previous section. The starting material for the engineering method is one or more of the VH and/or VK sequences provided herein, or one or more CDR regions thereof. To create the engineered antibody, it is not necessary to actually prepare (i.e., express as a protein) an antibody having one or more of the VH and/or VK sequences provided herein, or one or more CDR regions thereof. Rather, the information contained in the sequences is used as the starting material to create a "second generation" sequences derived from the original sequences and then the "second generation" sequences is prepared and expressed as a protein.

Standard molecular biology techniques can be used to prepare and express altered antibody sequence.

OPtionally, the antibody encoded by the altered antibody sequences is one that retains one, some or all of the functional properties of the Anti-TMEM154 antibodies, respectively, produced by methods and with sequences provided herein, which functional properties include binding to TMEM154 antigen with a specific KD level or less and/or selectively binding to desired target cells such as ovarian cancer, lung cancer, breast cancer, colon cancer, kidney cancer, liver cancer, pancreatic cancer, prostate cancer, melanoma and hematological malignancies such as Multiple Myeloma, lymphoma, Non-Hodgkin's lymphoma, anti CD20 (i.e. Rituximab) resistant lymphoma, leukemia, T cell leukemia, that express TMEM154 antigen.

The functional properties of the altered antibodies can be assessed using standard assays available in the art and/or described herein.

In certain embodiments of the methods of engineering antibodies according to at least some embodiments of the invention, mutations can be introduced randomly or selectively along all or part of an Anti-TMEM154 antibody coding sequence and the resulting modified Anti-TMEM154 antibodies can be screened for binding activity and/or other desired functional properties.

Mutational methods have been described in the art. For example, PCT Publication WO 02/092780 by Short describes methods for creating and screening antibody mutations using saturation mutagenesis, synthetic ligation assembly, or a combination thereof. Alternatively, PCT Publication WO 03/074679 by Lazar et al. describes methods of using computational screening methods to optimize physiochemical properties of antibodies.

### NUCLEIC ACID MOLECULES ENCODING ANTIBODIES

According to at least some embodiments of the inventionpertains to nucleic acid molecules that encode the antibodies according to at least some embodiments of the invention. The nucleic acids may be present in whole cells, in a cell lysate, or in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art. See, F. Ausubel, et al., ed. (1987) Current Protocols in Molecular Biology, Greene Publishing and Wiley Interscience, New York. A nucleic acid according to at least some embodiments of the invention can be, for example, DNA or RNA and may or may not contain intronic sequences. In a preferred embodiment, the nucleic acid is a cDNA molecule.

Nucleic acids according to at least some embodiments of the invention can be obtained using standard molecular biology techniques. For antibodies expressed by hybridomas (e.g., hybridomas prepared from transgenic mice carrying human immunoglobulin genes as described further below), cDNAs encoding the light and heavy chains of the antibody made by the hybridoma can be obtained by standard PCR amplification or cDNA cloning techniques. For antibodies obtained from an immunoglobulin gene library (e.g., using phage display techniques), nucleic acid encoding the antibody can be recovered from the library.

Once DNA fragments encoding VH and VL segments are obtained, these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker.

The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see e.g., Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see e.g., Kabat, E. A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region.

To create a scFv gene, the VH- and VL-encoding DNA fragments are operatively linked to another fragment encoding a flexible linker, e.g., encoding the amino acid sequence (Gly4-Ser)3, such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., (1990) Nature 348:552-554).

### Production Of Anti-TMEM154 Monoclonal Antibodies

Monoclonal antibodies (mAbs) according to at least some embodiments of the present invention can be produced by a variety of techniques, including conventional monoclonal antibody methodology e.g., the standard somatic cell hybridization technique of Kohler and Milstein (1975) Nature 256:495. Although somatic cell hybridization procedures are preferred, in principle, other techniques for producing monoclonal antibody can be employed e.g., viral or oncogenic transformation of B lymphocytes.

A preferred animal system for preparing hybridomas is the murine system. Hybridoma production in the mouse is a very well-established procedure. Immunization protocols and techniques for isolation of immunized splenocytes for fusion are known in the art. Fusion partners (e.g., murine myeloma cells) and fusion procedures are also known.

Chimeric or humanized antibodies according to at least some embodiments of the present invention can be prepared based on the sequence of a murine monoclonal antibody prepared as described above. DNA encoding the heavy and light chain immunoglobulins can be obtained from the murine hybridoma of interest and engineered to contain non-murine (e.g.,. human) immunoglobulin sequences using standard molecular biology techniques. For example, to create a chimeric antibody, the murine variable regions can be linked to human constant regions using methods known in the art (see e.g., U.S. Pat. No. 4,816,567 to Cabilly et al.). To create a humanized antibody, the murine CDR regions can be inserted into a human framework using methods known in the art (see e.g., U.S. Pat. No. 5,225,539 to Winter, and U.S. Pat. Nos. 5,530,101; 5,585,089; 5,693,762 and 6,180,370 to Queen et al.).

In a preferred embodiment, the antibodies according to at least some embodiments of the invention are human monoclonal antibodies. Such human monoclonal antibodies directed against TMEM154 can be generated using transgenic or transchromosomic mice carrying parts of the human immune system rather than the mouse system. These transgenic and transchromosomic mice include mice referred to herein as the HuMAb Mouse RTM and KM Mouse. RTM. respectively, and are collectively referred to herein as "human Ig mice." The HuMAb Mouse TM. (Medarex. Inc.) contains human immunoglobulin gene miniloci that encode unrearranged human heavy (.mu. and.gamma.) and.kappa. light chain immunoglobulin sequences, together with targeted mutations that inactivate the endogenous.mu. and.kappa. chain loci (see e.g., Lonberg, et al. (1994) Nature 368(6474): 856-859). Accordingly, the mice exhibit reduced expression of mouse IgM or.kappa., and in response to immunization, the introduced human heavy and light chain transgenes undergo class switching and somatic mutation to generate high affinity human IgGkappa. monoclonal (Lonberg, N. et al. (1994), supra; reviewed in Lonberg, N. (1994) Handbook of Experimental Pharmacology 113:49-101; Lonberg, N. and Huszar, D. (1995) Intern. Rev. Immunol. 13: 65-93, and Harding, F. and Lonberg, N. (1995) Ann. N.Y. Acad. Sci. 764:536-546). The preparation and use of the HuMab Mouse RTM., and the genomic modifications carried by such mice, is further described in Taylor, L. et al. (1992) Nucleic Acids Research 20:6287-6295; Chen, J. et al. (1993) International Immunology 5:647-656; Tuaillon et al. (1993) Proc. Natl. Acad. Sci. USA 90:3720-3724; Choi et al. (1993) Nature Genetics 4:117-123; Chen, J. et al. (1993) EMBO J. 12: 821-830; Tuaillon et al. (1994) J. Immunol. 152:2912-2920; Taylor, L. et al. (1994) International Immunology 6:579-591; and Fishwild, D. et al. (1996) Nature Biotechnology 14: 845-851. See further, U.S. Pat. Nos. 5,545,806; 5,569,825; 5,625,126; 5,633,425; 5,789,650; 5,877,397; 5,661,016; 5,814,318; 5,874,299; and 5,770,429; all to Lonberg and Kay; U.S. Pat. No. 5,545,807 to Surani et al.; PCT Publication Nos. WO 92/03918, WO 93/12227, WO 94/25585, WO 97/13852, WO 98/24884 and WO 99/45962, all to Lonberg and Kay; and PCT Publication No. WO 01/14424 to Korman et al.

In another embodiment, human antibodies according to at least some embodiments of the invention can be raised using a mouse that carries human immunoglobulin sequences on transgenes and transchomosomes, such as a mouse that carries a human heavy chain transgene and a human light chain transchromosome. Such mice, referred to herein as "KM mice TM.", are described in detail in PCT Publication WO 02/43478 to Ishida et al.

Still further, alternative transgenic animal systems expressing human immunoglobulin genes are available in the art and can be used to raise anti-TMEM154 antibodies according to at least some embodiments of the invention. For example, an alternative transgenic system referred to as the Xenomouse (Abgenix, Inc.) can be used; such mice are described in, for example, U.S. Pat. Nos. 5,939,598; 6,075,181; 6,114,598; 6, 150,584 and 6,162,963 to Kucherlapati et al.

Moreover, alternative transchromosomic animal systems expressing human immunoglobulin genes are available in the art and can be used to raise Anti-TMEM154 antibodies according to at least some embodiments of the invention. For example, mice carrying both a human heavy chain transchromosome and a human light chain transchromosome, referred to as "TC mice" can be used; such mice are described in Tomizuka et al. (2000) Proc. Natl. Acad Sci. USA 97:722-727. Furthermore, cows carrying human heavy and light chain transchromosomes have been described in the art (Kuroiwa et al. (2002) Nature Biotechnology 20:889-894) and can be used to raise Anti-TMEM154 antibodies according to at least some embodiments of the invention.

Human monoclonal antibodies according to at least some embodiments of the invention can also be prepared using phage display methods for screening libraries of human immunoglobulin genes. Such phage display methods for isolating human antibodies are established in the art. See for example: U.S. Pat. Nos. 5,223,409; 5,403,484; and 5,571,698 to Ladner et al.; U.S. Pat. Nos. 5,427,908 and 5,580,717 to Dower et al.; U.S. Pat. Nos. 5,969,108 and 6,172,197 to McCafferty et al.; and U.S. Pat. Nos. 5,885,793; 6,521,404; 6,544,73 1; 6,555,313; 6,582,915 and 6,593,081 to Griffiths et al.

Human monoclonal antibodies according to at least some embodiments of the invention can also be prepared using SCID mice into which human immune cells have been reconstituted such that a human antibody response can be generated upon immunization. Such mice are described in, for example, U.S. Pat. Nos. 5,476,996 and 5,698,767 to Wilson et al.

### IMMUNIZATION OF HUMAN IG MICE

When human Ig mice are used to raise human antibodies according to at least some embodiments of the invention, such mice can be immunized with a purified or enriched preparation of TMEM154 antigen and/or recombinant TMEM154, or an TMEM154 fusion protein, as described by Lonberg, N. et al. (1994) Nature 368(6474): 856-859; Fishwild, D. et al. (1996) Nature Biotechnology 14: 845-851; and PCT Publication WO 98/24884 and WO 01/14424. Preferably, the mice will be 6-16 weeks of age upon the first infusion. For example, a purified or recombinant preparation (5-50.mu.g) of TMEM154 antigen can be used to immunize the human Ig mice intraperitoneally.

Prior experience with various antigens by others has shown that the transgenic mice respond when initially immunized intraperitoneally (IP) with antigen in complete Freund's adjuvant, followed by every other week IP immunizations (up to a total of 6) with antigen in incomplete Freund's adjuvant. However, adjuvants other than Freund's are also found to be effective. In addition, whole cells in the absence of adjuvant are found to be highly immunogenic. The immune response can be monitored over the course of the immunization protocol with plasma samples being obtained by retroorbital bleeds. The plasma can be screened by ELISA (as described below), and mice with sufficient titers of anti-TMEM154 human immunoglobulin can be used for fusions. Mice can be boosted intravenously with antigen 3 days before sacrifice and removal of the spleen. It is expected that 2-3 fusions for each immunization may need to be performed. Between 6 and 24 mice are typically immunized for each antigen. Usually both HCo7 and HCo12 strains are used. In addition, both HCo7 and HCo12 transgene can be bred together into a single mouse having two different human heavy chain transgenes (HCo7/HCo 12). Alternatively or additionally, the KM Mouse. RTM. strain can be used.

### GENERATION OF HYBRIDOMAS PRODUCING HUMAN MONOCLONAL ANTIBODIES

To generate hybridomas producing human monoclonal antibodies according to at least some embodiments of the invention, splenocytes and/or lymph node cells from immunized mice can be isolated and fused to an appropriate immortalized cell line, such as a mouse myeloma cell line. The resulting hybridomas can be screened for the production of antigen-specific antibodies. For example, single cell suspensions of splenic lymphocytes from immunized mice can be fused to one-sixth the number of P3X63-Ag8.653 nonsecreting mouse myeloma cells (ATCC, CRL 1580) with 50% PEG. Cells are plated at approximately 2 X 10 -5 in flat bottom microtiter plate, followed by a two week incubation in selective medium containing 20% fetal Clone Serum, 18% "653" conditioned media, 5% origen (IGEN), 4 mM L-glutamine, 1 mM sodium pyruvate, 5 mM HEPES, 0.055 mM 2-mercaptoethanol, 50 units/ml penicillin, 50 mg/ml streptomycin, 50 mg/ml gentamycin and 1X HAT (Sigma; the HAT is added 24 hours after the fusion). After approximately two weeks, cells can be cultured in medium in which the HAT is replaced with HT. Individual wells can then be screened by ELISA for human monoclonal IgM and IgG antibodies. Once extensive hybridoma growth occurs, medium can be observed usually after 10-14 days. The antibody secreting hybridomas can be replated, screened again, and if still positive for human IgG, the monoclonal antibodies can be subcloned at least twice by limiting dilution. The stable subclones can then be cultured in vitro to generate small amounts of antibody in tissue culture medium for characterization.

To purify human monoclonal antibodies, selected hybridomas can be grown in two-liter spinner-flasks for monoclonal antibody purification. Supernatants can be filtered and concentrated before affinity chromatography with protein A-Sepharose (Pharmacia, Piscataway, N.J.). Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80 degrees C.

### GENERATION OF TRANSFECTOMAS PRODUCING MONOCLONAL ANTIBODIES

Antibodies according to at least some embodiments of the invention also can be produced in a host cell transfectoma using, for example, a combination of recombinant DNA techniques and gene transfection methods as is well known in the art (e.g., Morrison, S. (1985) Science 229:1202).

For example, to express the antibodies, or antibody fragments thereof, DNAs encoding partial or full-length light and heavy chains, can be obtained by standard molecular biology techniques (e.g., PCR amplification or cDNA cloning using a hybridoma that expresses the antibody of interest) and the DNAs can be inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present). The light and heavy chain variable regions of the antibodies described herein can be used to create full-length antibody genes of any antibody isotype by inserting them into expression vectors already encoding heavy chain constant and light chain constant regions of the desired isotype such that the VH segment is operatively linked to the CH segments within the vector and the VK segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to the antibody chain genes, the recombinant expression vectors according to at least some embodiments of the invention carry regulatory sequences that control the expression of the antibody chain genes in a host cell. The term "regulatory sequence" is intended to include promoters, enhancers and other expression control elements (e.g., polyadenylation signals) that control the transcription or translation of the antibody chain genes. Such regulatory sequences are described, for example, in Goeddel (Gene Expression Technology. Methods in Enzymology 185, Academic Press, San Diego, Calif. (1990)). It will be appreciated by those skilled in the art that the design of the expression vector, including the selection of regulatory sequences, may depend on such factors as the choice of the host cell to be transformed, the level of expression of protein desired, etc. Preferred regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (e.g., the adenovirus major late promoter (AdMLP) and polyoma. Alternatively, nonviral regulatory sequences may be used, such as the ubiquitin promoter or.beta.-globin promoter. Still further, regulatory elements composed of sequences from different sources, such as the SR alpha. promoter system, which contains sequences from the SV40 early promoter and the long terminal repeat of human T cell leukemia virus type 1 (Takebe, Y. et al. (1988) Mol. Cell. Biol. 8:466-472).

In addition to the antibody chain genes and regulatory sequences, the recombinant expression vectors according to at least some embodiments of the invention may carry additional sequences, such as sequences that regulate replication of the vector in host cells (e.g., origins of replication) and selectable marker genes. The selectable marker gene facilitates selection of host cells into which the vector has been introduced (see, e.g., U.S. Pat. Nos. 4,399,216, 4,634,665 and 5,179,017, all by Axel et al.). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr-host cells with methotrexate selection/amplification) and the neo gene (for G418 selection).

For expression of the light and heavy chains, the expression vectors encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibodies according to at least some embodiments of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M. A. and Wood, C. R. (1985) Immunology Today 6:12-13).

Preferred mammalian host cells for expressing the recombinant antibodies according to at least some embodiments of the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, e.g., as described in R. J. Kaufman and P. A. Sharp (1982) Mol. Biol. 159:601-621), NSO myeloma cells, COS cells and SP2 cells. In particular, for use with NSO myeloma cells, another preferred expression system is the GS gene expression system disclosed in WO 87/04462, WO 89/01036 and EP 338,841. When recombinant expression vectors encoding antibody genes are introduced into mammalian host cells, the antibodies are produced by culturing the host cells for a period of time sufficient to allow for expression of the antibody in the host cells or, preferably, secretion of the antibody into the culture medium in which the host cells are grown. Antibodies can be recovered from the culture medium using standard protein purification methods.

### CHARACTERIZATION OF ANTIBODY BINDING TO ANTIGEN

Antibodies according to at least some embodiments of the invention can be tested for binding to TMEM154 by, for example, standard ELISA. Briefly, microtiter plates are coated with purified TMEM154 at 0.25.mu.g/ml in PBS, and then blocked with 5% bovine serum albumin in PBS. Dilutions of antibody (e.g., dilutions of plasma from TMEM154-immunized mice) are added to each well and incubated for 1-2 hours at 37 degrees C. The plates are washed with PBS/Tween and then incubated with secondary reagent (e.g., for human antibodies, a goat-anti-human IgG Fc-specific polyclonal reagent) conjugated to alkaline phosphatase for 1 hour at 37 degrees C. After washing, the plates are developed with pNPP substrate (1 mg/ml), and analyzed at OD of 405-650. Preferably, mice which develop the highest titers will be used for fusions.

An ELISA assay as described above can also be used to screen for hybridomas that show positive reactivity with TMEM154 immunogen. Hybridomas that bind with high avidity to TMEM154 are subcloned and further characterized. One clone from each hybridoma, which retains the reactivity of the parent cells (by ELISA), can be chosen for making a 5-10 vial cell bank stored at -140 degrees C., and for antibody purification.

To purify anti-TMEM154 antibodies, selected hybridomas can be grown in two-liter spinner-flasks for monoclonal antibody purification. Supernatants can be filtered and concentrated before affinity chromatography with protein A-sepharose (Pharmacia, Piscataway, N.J.). Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80 degrees C.

To determine if the selected anti-TMEM154 monoclonal antibodies bind to unique epitopes, each antibody can be biotinylated using commercially available reagents (Pierce, Rockford, Ill.). Competition studies using unlabeled monoclonal antibodies and biotinylated monoclonal antibodies can be performed using TMEM154 coated-ELISA plates as described above. Biotinylated mAb binding can be detected with a strep-avidin-alkaline phosphatase probe.

To determine the isotype of purified antibodies, isotype ELISAs can be performed using reagents specific for antibodies of a particular isotype. For example, to determine the isotype of a human monoclonal antibody, wells of microtiter plates can be coated with 1.mu.g/ml of anti-human immunoglobulin overnight at 4 degrees C. After blocking with 1% BSA, the plates are reacted with 1mug /ml or less of test monoclonal antibodies or purified isotype controls, at ambient temperature for one to two hours. The wells can then be reacted with either human IgG1 or human IgM-specific alkaline phosphatase-conjugated probes. Plates are developed and analyzed as described above.

anti-TMEM154 human IgGs can be further tested for reactivity with TMEM154 antigen, by Western blotting. Briefly, TMEM154 antigen can be prepared and subjected to sodium dodecyl sulfate polyacrylamide gel electrophoresis. After electrophoresis, the separated antigens are transferred to nitrocellulose membranes, blocked with 10% fetal calf serum, and probed with the monoclonal antibodies to be tested. Human IgG binding can be detected using anti-human IgG alkaline phosphatase and developed with BCIP/NBT substrate tablets (Sigma Chem. Co., St. Louis, Mo.).

### CONJUGATES OR IMMUNOCONJUGATES

According to at least some embodiments, the present invention features immunoconjugates comprising an anti-TMEM154 antibody, or a fragment thereof, conjugated to a therapeutic moiety, such as a cytotoxin, a drug (e.g., an immunosuppressant) or a radiotoxin. Such conjugates are referred to herein as "immunoconjugates". Immunoconjugates that include one or more cytotoxins are referred to as "immunotoxins." A cytotoxin or cytotoxic agent includes any agent that is detrimental to (e.g., kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

Other preferred examples of therapeutic cytotoxins that can be conjugated to an antibody according to at least some embodiments of the invention include duocarmycins, calicheamicins, maytansines and auristatins, and derivatives thereof. An example of a calicheamicin antibody conjugate is commercially available (Mylotarg.TM.; Wyeth).

Cytotoxins can be conjugated to antibodies according to at least some embodiments of the invention using linker technology available in the art. Examples of linker types that have been used to conjugate a cytotoxin to an antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (e.g., cathepsins B, C, D).

For further discussion of types of cytotoxins, linkers and methods for conjugating therapeutic agents to antibodies, see also Saito, G. et al. (2003) Adv. Drug Deliv. Rev. 55:199-215; Trail, P. A. et al. (2003) Cancer Immunol. Immunother. 52:328-337; Payne, G. (2003) Cancer Cell 3:207-212; Allen, T. M. (2002) Nat. Rev. Cancer 2:750-763; Pastan, I. and Kreitman, R. J. (2002) Curr. Opin. Investig. Drugs 3:1089-1091; Senter, P. D. and Springer, C. J. (2001) Adv. Drug Deliv. Rev. 53:247-264.

Antibodies according to at least some embodiments of the present invention also can be conjugated to a radioactive isotope to generate cytotoxic radiopharmaceuticals, also referred to as radioimmunoconjugates. Examples of radioactive isotopes that can be conjugated to antibodies for use diagnostically or therapeutically include, but are not limited to, iodine 131, indium 111, yttrium 90 and lutetium 177. Method for preparing radioimmunconjugates are established in the art. Examples of radioimmunoconjugates are commercially available, including Zevalin.TM. (IDEC Pharmaceuticals) and Bexxar.TM. (Corixa Pharmaceuticals), and similar methods can be used to prepare radioimmunoconjugates using the antibodies according to at least some embodiments of the invention.

The antibody conjugates according to at least some embodiments of the invention can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-.gamma.; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

### BISPECIFIC MOLECULES

In another aspect, the present invention features bispecific molecules comprising an anti-TMEM154 antibody, or a fragment thereof, according to at least some embodiments of the invention. An antibody according to at least some embodiments of the invention, or antigen-binding portions thereof, can be derivatized or linked to another functional molecule, e.g., another peptide or protein (e.g., another antibody or ligand for a receptor) to generate a bispecific molecule that binds to at least two different binding sites or target molecules. The antibody according to at least some embodiments of the invention may in fact be derivatized or linked to more than one other functional molecule to generate multispecific molecules that bind to more than two different binding sites and/or target molecules; such multispecific molecules are also intended to be encompassed by the term "bispecific molecule" as used herein. To create a bispecific molecule according to at least some embodiments of the invention, an antibody according to at least some embodiments of the invention can be functionally linked (e.g., by chemical coupling, genetic fusion, noncovalent association or otherwise) to one or more other binding molecules, such as another antibody, antibody fragment, peptide or binding mimetic, such that a bispecific molecule results.

Accordingly, the present invention includes bispecific molecules comprising at least one first binding specificity for a TMEM154 polypeptide and a second binding specificity for a second target epitope. In a particular embodiment according to at least some embodiments of the invention, the second target epitope is an Fc receptor, e.g., human Fc gamma RI (CD64) or a human Fc alpha receptor (CD89). Therefore, the invention includes bispecific molecules capable of binding both to Fc gamma. R, Fc alpha R or Fc epsilon R expressing effector cells (e.g., monocytes, macrophages or polymorphonuclear cells (PMNs)), and to target cells expressing a TMEM154 polypeptide. These bispecific molecules target TMEM154 polypeptide expressing cells to effector cell and trigger Fc receptor-mediated effector cell activities, such as phagocytosis of TMEM154 polypeptide expressing cells, antibody dependent cell-mediated cytotoxicity (ADCC), cytokine release, or generation of superoxide anion.

In an embodiment according to at least some embodiments of the invention in which the bispecific molecule is multispecific, the molecule can further include a third binding specificity, in addition to an anti-Fc binding specificity and an anti-6f binding specificity. In one embodiment, the third binding specificity is an anti-enhancement factor (EF) portion, e.g., a molecule which binds to a surface protein involved in cytotoxic activity and thereby increases the immune response against the target cell.

The "anti-enhancement factor portion" can be an antibody, functional antibody fragment or a ligand that binds to a given molecule, e.g., an antigen or a receptor, and thereby results in an enhancement of the effect of the binding determinants for the Fc receptor or target cell antigen. The "anti-enhancement factor portion" can bind an Fc receptor or a target cell antigen. Alternatively, the anti-enhancement factor portion can bind to an entity that is different from the entity to which the first and second binding specificities bind. For example, the anti-enhancement factor portion can bind a cytotoxic T-cell (e.g., via CD2, CD3, CD8, CD28, CD4, CD40, ICAM-1 or other immune cell that results in an increased immune response against the target cell).

In one embodiment, the bispecific molecules according to at least some embodiments of the invention comprise as a binding specificity at least one antibody, or an antibody fragment thereof, including, e.g., an Fab, Fab', F(ab')₂, Fv, or a single chain Fv. The antibody may also be a light chain or heavy chain dimer, or any minimal fragment thereof such as a Fv or a single chain construct as described in Ladner et al. U.S. Pat. No. 4,946,778.

The production and characterization of certain preferred anti-Fc gamma. monoclonal antibodies are described by Fanger et al. in PCT Publication WO 88/00052 and in U.S. Pat. No. 4,954,617. These antibodies bind to an epitope of Fc R1, FcyRII or FcyRIII at a site which is distinct from the Fc binding site of the receptor and, thus, their binding is not blocked substantially by physiological levels of IgG. Specific anti-Fc RI antibodies useful in this invention are mAb 22, mAb 32, mAb 44, mAb 62 and mAb 197. The hybridoma producing mAb 32 is available from the American Type Culture Collection, ATCC Accession No. HB9469. In other embodiments, the anti-Fcy receptor antibody is a humanized form of monoclonal antibody 22 (H22). The production and characterization of the H22 antibody is described in Graziano, R.F. et al. (1995) J. Immunol. 155 (10): 4996-5002 and PCT Publication WO 94/10332. The H22 antibody producing cell line is deposited at the American Type Culture Collection under the designation HAO22CLI and has the accession no. CRL 11177.

In still other preferred embodiments, the binding specificity for an Fc receptor is provided by an antibody that binds to a human IgA receptor, e.g., an Fc-alpha receptor (Fc alpha RI(CD89)), the binding of which is preferably not blocked by human immunoglobulin A (IgA). The term "IgA receptor" is intended to include the gene product of one alpha.-gene (Fc alpha RI) located on chromosome 19. This gene is known to encode several alternatively spliced transmembrane isoforms of 55 to 10 kDa.

Fc alpha RI (CD89) is constitutively expressed on monocytes/macrophages, eosinophilic and neutrophilic granulocytes, but not on non-effector cell populations. Fc alpha RI has medium affinity (Approximately 5X10-7 M-1) for both IgA1 and IgA2, which is increased upon exposure to cytokines such as G-CSF or GM-CSF (Morton, H. C. et al. (1996) Critical Reviews in Immunology 16:423-440). Four Fca RI-specific monoclonal antibodies, identified as A3, A59, A62 and A77, which bind Fc alpha RI outside the IgA ligand binding domain, have been described (Monteiro, R. C. et al. (1992) J. Immunol. 148:1764).

Fc alpha RI and Fc gamma RI are preferred trigger receptors for use in the bispecific molecules according to at least some embodiments of the invention because they are (1) expressed primarily on immune effector cells, e.g., monocytes, PMNs, macrophages and dendritic cells; (2) expressed at high levels (e.g., 5,000-100,000 per cell); (3) mediators of cytotoxic activities (e.g., ADCC, phagocytosis); (4) mediate enhanced antigen presentation of antigens, including self-antigens, targeted to them.

While human monoclonal antibodies are preferred, other antibodies which can be employed in the bispecific molecules according to at least some embodiments of the invention are murine, chimeric and humanized monoclonal antibodies.

The bispecific molecules according to at least some embodiments of the present invention can be prepared by conjugating the constituent binding specificities, e.g., the anti-FcR and anti-TMEM154 polypeptide binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-S-acetyl-thioacetate (SATA), 5,5'-dithiobis(2-nitrobenzoic acid) (DTNB), o-phenylenedimaleimide (oPDM), N-succinimidyl-3-(2-pyridyld- ithio)propionate (SPDP), and sulfosuccinimidyl 4-(N-maleimidomethyl) cyclohaxane-1-carboxylate (sulfo-SMCC) (see e.g., Karpovsky et al. (1984) J. Exp. Med. 160:1686; Liu, M A et al. (1985) Proc. Natl. Acad. Sci. USA 82:8648). Other methods include those described in Paulus (1985) Behring Ins. Mitt. No. 78, 118-132; Brennan et al. (1985) Science 229:81-83), and Glennie et al. (1987) J. Immunol. 139: 2367-2375). Preferred conjugating agents are SATA and sulfo-SMCC, both available from Pierce Chemical Co. (Rockford, Ill.).

When the binding specificities are antibodies, they can be conjugated via sulfhydryl bonding of the C-terminus hinge regions of the two heavy chains. In a particularly preferred embodiment, the hinge region is modified to contain an odd number of sulfhydryl residues, preferably one, prior to conjugation.

Alternatively, both binding specificities can be encoded in the same vector and expressed and assembled in the same host cell. This method is particularly useful where the bispecific molecule is a mAbXmAb, mAbXFab, FabXF(ab')2 or ligandXFab fusion protein. A bispecific molecule according to at least some embodiments of the invention can be a single chain molecule comprising one single chain antibody and a binding determinant, or a single chain bispecific molecule comprising two binding determinants. Bispecific molecules may comprise at least two single chain molecules. Methods for preparing bispecific molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the bispecific molecules to their specific targets can be confirmed by, for example, enzyme-linked immunosorbent assay (ELISA), radioimmunoassay (RIA), FACS analysis, bioassay (e.g., growth inhibition), or Western Blot assay. Each of these assays generally detects the presence of protein-antibody complexes of particular interest by employing a labeled reagent (e.g., an antibody) specific for the complex of interest. For example, the FcR-antibody complexes can be detected using e.g., an enzyme-linked antibody or antibody fragment which recognizes and specifically binds to the antibody-FcR complexes. Alternatively, the complexes can be detected using any of a variety of other immunoassays. For example, the antibody can be radioactively labeled and used in a radioimmunoassay (RIA) (see, for example, Weintraub, B., Principles of Radioimmunoassays, Seventh Training Course on Radioligand Assay Techniques, The Endocrine Society, March, 1986). The radioactive isotope can be detected by such means as the use of a gamma. counter or a scintillation counter or by autoradiography.

### PHARMACEUTICAL COMPOSITIONS

In another aspect, the present invention provides a composition, e.g., a pharmaceutical composition, containing one or a combination of monoclonal antibodies, or antigen-binding portions thereof, according to at least some embodiments of the present invention, formulated together with a pharmaceutically acceptable carrier. Such compositions may include one or a combination of (e.g., two or more different) antibodies, or immunoconjugates or bispecific molecules according to at least some embodiments of the invention. For example, a pharmaceutical composition according to at least some embodiments of the invention can comprise a combination of antibodies (or immunoconjugates or bispecifics) that bind to different epitopes on the target antigen or that have complementary activities.

Thus, the present invention features a pharmaceutical composition comprising a therapeutically effective amount of a therapeutic agent according to the present invention.

The pharmaceutical composition according to the present invention is further optionally used for the treatment of cancer and/or immune related conditions or disorders.

The therapeutic agents according to at least some embodiments of the present invention can be provided to the subject alone, or as part of a pharmaceutical composition where they are mixed with a pharmaceutically acceptable carrier.

Pharmaceutical compositions according to at least some embodiments of the invention also can be administered in combination therapy, i.e., combined with other agents. For example, the combination therapy can include an anti-TMEM154 antibody, combined with at least one other therapeutic or immune modulatory agent.

A composition according to at least some embodiments of the present invention can be administered via one or more routes of administration using one or more of a variety of methods known in the art. As will be appreciated by the skilled artisan, the route and/or mode of administration will vary depending upon the desired results. Preferred routes of administration for antibodies according to at least some embodiments of the invention include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion). Depending on the route of administration, the active compound, i.e., antibody, immunoconjugate, or bispecific molecule, may be coated in a material to protect the compound from the action of acids and other natural conditions that may inactivate the compound.

A pharmaceutical composition according to at least some embodiments of the invention also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like. Examples of suitable aqueous and nonaqueous carriers that may be employed in the pharmaceutical compositions according to at least some embodiments of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions according to at least some embodiments of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin. Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0.01 per cent to about ninety-nine percent of active ingredient, optionally from about 0.1 per cent to about 70 per cent, optionally from about 1 per cent to about 30 per cent of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms according to at least some embodiments of the invention are dictated by and directly dependent on (a) the unique characteristics of the active compound and the particular therapeutic effect to be achieved, and (b) the limitations inherent in the art of compounding such an active compound for the treatment of sensitivity in individuals.

For administration of the antibody, the dosage ranges from about 0.0001 to 100 mg/kg, and more usually 0.01 to 5 mg/kg, of the host body weight. For example dosages can be 0.3 mg/kg body weight, 1 mg/kg body weight, 3 mg/kg body weight, 5 mg/kg body weight or 10 mg/kg body weight or within the range of 1-10 mg/kg. An exemplary treatment regime entails administration once per week, once every two weeks, once every three weeks, once every four weeks, once a month, once every 3 months or once every three to 6 months.

Alternatively, antibody can be administered as a sustained release formulation, in which case less frequent administration is required. Dosage and frequency vary depending on the half-life of the antibody in the patient. In general, human antibodies show the longest half life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions according to at least some embodiments of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions according to at least some embodiments of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective dosage" of an anti-TMEM154 antibody according to at least some embodiments of the invention preferably results in a decrease in severity of disease symptoms, an increase in frequency and duration of disease symptom-free periods, an increase in lifespan, disease remission, or a prevention of impairment or disability due to the disease affliction. For example, for the treatment of TMEM154 polypeptide positive tumors, e.g., ovarian tumors, lung tumors, breast tumors, colon tumors, kidney tumors, liver tumors, pancreatic tumors, prostate cancer, melanoma and hematological malignancies such as Multiple Myeloma, lymphoma, Non-Hodgkin's lymphoma, anti CD20 (i.e. Rituximab) resistant lymphoma, leukemia, T cell leukemia, a "therapeutically effective dosage" optionally inhibits cell growth or tumor growth by at least about 20%, 40%, 60%, 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition in vitro by assays known to the skilled practitioner.

Alternatively or additionally, a "therapeutically effective dosage" preferably results in at least stable disease, preferably partial response, more preferably complete response, as assessed by the WHO or RECIST criteria for tumor response (Natl Cancer Inst 1999;91:523-8 and Cancer 1981;47:207-14).

A therapeutically effective amount of a therapeutic compound can decrease tumor size, or otherwise ameliorate symptoms in a subject, or otherwise support partial or complete stable disease and/or partial or complete response as determined above. One of ordinary skill in the art would be able to determine such amounts based on such factors as the subject's size, the severity of the subject's symptoms, and the particular composition or route of administration selected.

Therapeutic compositions can be administered with medical devices known in the art. For example, in a preferred embodiment, a therapeutic composition according to at least some embodiments of the invention can be administered with a needles hypodermic injection device, such as the devices disclosed in U.S. Pat. Nos. 5,399,163; 5,383,851; 5,312,335; 5,064,413; 4,941,880; 4,790,824; or 4,596,556. Examples of well-known implants and modules useful in the present invention include: U.S. Pat. No. 4,487,603, which discloses an implantable micro-infusion pump for dispensing medication at a controlled rate; U.S. Pat. No. 4,486,194, which discloses a therapeutic device for administering medicaments through the skin; U.S. Pat. No. 4,447,233, which discloses a medication infusion pump for delivering medication at a precise infusion rate; U.S. Pat. No. 4,447,224, which discloses a variable flow implantable infusion apparatus for continuous drug delivery; U.S. Pat. No. 4,439,196, which discloses an osmotic drug delivery system having multi-chamber compartments; and U.S. Pat. No. 4,475,196, which discloses an osmotic drug delivery system. These patents are incorporated herein by reference. Many other such implants, delivery systems, and modules are known to those skilled in the art.

In certain embodiments, the antibodies or other TMEM154 related drugs according to at least some embodiments of the invention can be formulated to ensure proper distribution in vivo. For example, the blood-brain barrier (BBB) excludes many highly hydrophilic compounds. To ensure that the therapeutic compounds according to at least some embodiments of the invention cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., V. V. Ranade (1989) J. Clin. Pharmacol. 29:685). Exemplary targeting moieties include folate or biotin (see, e.g., U.S. Pat. No. 5,416,016 to Low et al.); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180); surfactant protein A receptor (Briscoe et al. (1995) Am. J Physiol. 1233:134); p120 (Schreier et al. (1994) J. Biol. Chem. 269:9090); see also K. Keinanen; M. L. Laukkanen (1994) FEBS Lett. 346:123; J. J. Killion; I. J. Fidler (1994) Immunomethods 4:273.

Given the specific binding of the antibodies according to at least some embodiments of the invention for TMEM154 polypeptides, the antibodies can be used to specifically detect TMEM154 expression on the surface of cells and, moreover, can be used to purify TMEM154 antigen via immunoaffinity purification.

Furthermore, given the expression of TMEM154 polypeptides on various tumor cells, the human antibodies, antibody compositions and methods according to at least some embodiments of the present invention can be used to treat a subject with a tumorigenic disorder, e.g., a disorder characterized by the presence of tumor cells expressing TMEM154 antigen such as ovarian cancer, colon cancer, lung cancer, breast cancer, kidney cancer, liver cancer, pancreatic cancer, prostate cancer, melanoma and hematological malignancies such as Multiple Myeloma, lymphoma, Non-Hodgkin's lymphoma, anti CD20 (i.e. Rituximab) resistant lymphoma, leukemia, T cell leukemia, as mentioned.

In one embodiment, the antibodies (e.g., human monoclonal antibodies, multispecific and bispecific molecules and compositions) according to at least some embodiments of the invention can be used to detect levels of a TMEM154 polypeptide or levels of cells which contain a TMEM154 polypeptide, respectively, on their membrane surface, which levels can then be linked to certain disease symptoms.

Alternatively, the antibodies can be used to inhibit or block functioning of TMEM154 polypeptides which, in turn, can be linked to the prevention or amelioration of certain disease symptoms, thereby implicating TMEM154 polypeptides, respectively, as a mediator of the disease. This can be achieved by contacting a sample and a control sample with the anti-TMEM154 antibody under conditions that allow for the formation of a complex between the corresponding antibody and TMEM154 polypeptides. Any complexes formed between the antibody and TMEM154 polypeptides are detected and compared in the sample and the control.

In another embodiment, the antibodies (e.g., human antibodies, multispecific and bispecific molecules and compositions) according to at least some embodiments of the invention can be initially tested for binding activity associated with therapeutic or diagnostic use in vitro. For example, compositions according to at least some embodiments of the invention can be tested using low cytometric assays.

As previously described, human anti-TMEM154 antibodies according to at least some embodiments of the invention can be co-administered with one or other more therapeutic agents, e.g., an cytotoxic agent, a radiotoxic agent or an immunosuppressive agent. The antibody can be linked to the agent (as an immunocomplex) or can be administered separate from the agent. In the latter case (separate administration), the antibody can be administered before, after or concurrently with the agent or can be co-administered with other known therapies, e.g., an anti-cancer therapy, e.g., radiation. Such therapeutic agents include, among others, anti-neoplastic agents such as doxorubicin (adriamycin), cisplatin bleomycin sulfate, carmustine, chlorambucil, and cyclophosphamide hydroxyurea which, by themselves, are only effective at levels which are toxic or subtoxic to a patient. Cisplatin is intravenously administered as a 100 mg/dose once every four weeks and adriamycin is intravenously administered as a 60-75 mg/ml dose once every 21 days. Co-administration of the human anti-TMEM154 antibodies, or antigen binding fragments thereof, according to at least some embodiments of the present invention with chemotherapeutic agents provides two anti-cancer agents which operate via different mechanisms which yield a cytotoxic effect to human tumor cells. Such co-administration can solve problems due to development of resistance to drugs or a change in the antigenicity of the tumor cells which would render them unreactive with the antibody.

Also within the scope according to at least some embodiments of the present invention are kits comprising the TMEM154 polypeptide or antibody compositions according to at least some embodiments of the invention (e.g., human antibodies, bispecific or multispecific molecules, or immunoconjugates) and instructions for use. The kit can further contain one ore more additional reagents, such as an immunosuppressive reagent, a cytotoxic agent or a radiotoxic agent, or one or more additional human antibodies according to at least some embodiments of the invention (e.g., a human antibody having a complementary activity which binds to an epitope in the TMEM154 antigen distinct from the first human antibody).

In other embodiments, the subject can be additionally treated with an agent that modulates, e.g., enhances or inhibits, the expression or activity of Fcy or Fcy receptors by, for example, treating the subject with a cytokine. Preferred cytokines for administration during treatment with the multispecific molecule include of granulocyte colony-stimulating factor (G-CSF), granulocyte- macrophage colony-stimulating factor (GM-CSF), interferon-.gamma. (IFN-.gamma.), and tumor necrosis factor (TNF).

The compositions (e.g., human antibodies, multispecific and bispecific molecules) according to at least some embodiments of the invention can also be used to target cells expressing Fc gamma R or TMEM154, for example for labeling such cells. For such use, the binding agent can be linked to a molecule that can be detected. Thus, the invention provides methods for localizing ex vivo or in vitro cells expressing Fc receptors, such as FcgammaR, or TMEM154 antigen. The detectable label can be, e.g., a radioisotope, a fluorescent compound, an enzyme, or an enzyme co-factor.

### DIAGNOSTIC USES OF TMEM154 POLYPEPTIDES AND ANTIBODIES

In certain embodiments the polypeptides and/or polynucleotides according to at least some embodiments of the present invention are used as markers for diagnosis of diseases wherein TMEM154 polypeptides are differentially present. According to at least some embodiments, the diseases are selected from but not limited to cancer, and immune related conditions (as defined herein).

According to further embodiments markers according to at least some embodiments of the present invention might optionally be used alone or in combination one or more other compounds described herein, and/or in combination with known markers for lung cancer, including but not limited to CEA, CA15-3, Beta-2-microglobulin, CA19-9, TPA, and/or in combination with the known proteins for the variant marker as described herein.

According to further embodiments markers according to at least some embodiments of the present invention might optionally be used alone or in combination with one or more other compounds described herein, and/or in combination known markers for ovarian cancer, including but not limited to CEA, CA125 (Mucin 16), CA72-4TAG, CA-50, CA 54-61, CA-195 and CA 19-9 in combination with CA-125, and/or in combination with the known proteins for the variant marker as described herein.

According to further embodiments markers according to at least some embodiments of the present invention might optionally be used alone or in combination with one or more other compounds described herein, and/or in combination with known markers for breast cancer, including but not limited to Calcitonin, CA15-3 (Mucin1), CA27-29, TPA, a combination of CA 15-3 and CEA, CA 27.29 (monoclonal antibody directed against MUC1), Estrogen 2 (beta), HER-2 (c-erbB2), and/or in combination with the known proteins for the variant marker as described herein.

According to further embodiments markers according to at least some embodiments of the present invention might optionally be used alone or in combination with one or more other compounds described herein, and/or in combination with known markers for renal cancer, including but not limited to urinary protein, creatinine or creatinine clearance, and/or markers used for the diagnosis or assessment of prognosis of renal cancer, specifically of renal cell carcinoma, including but not limited to vascular endothelial growth factor, interleukin-12, the soluble interleukin-2 receptor, intercellular adhesion molecule-1, human chorionic gonadotropin beta, insulin-like growth factor-1 receptor, Carbonic anhydrase 9 (CA 9), endostatin, Thymidine phosphorylase and/or in combination with the known proteins for the variant marker as described herein.

According to further embodiments markers according to at least some embodiments of the present invention might optionally be used alone or in combination with one or more other compounds described herein, and/or in combination with known markers for liver cancer, including but not limited to Alpha fetoprotein (AFP), des-gamma-carboxyprothrombin (DCP), Squamous cell carcinoma antigen (SCCA)-immunoglobulin M (IgM), AFP (L3), or fucosylated AFP, GP73 (a golgi protein marker) and its fucosylated form, (TGF)-beta1, HS-GGT, free insulin-like growth factor (IGF)-II.

According to further embodiments markers according to at least some embodiments of the present invention might optionally be used alone or in combination with one or more other compounds described herein, and/or in combination with known markers for melanoma cancer, including but not limited to S100-beta, melanoma inhibitory activity (MIA), lactate dehydrogenase (LDH), tyrosinase, 5-S-Cysteinyldopa, L-Dopa/L-tyrosine, VEGF, bFGF, IL-8, ICAM-1, MMPs, IL-6, IL-10, sIL-2R (soluble interleukin-2-receptor), sHLA-DR (soluble HLA-DR), sHLA-class-1 (soluble HLA-class I), TuM2-PK, Fas/CD95, sHLA-class-1 (soluble HLA-class I), Albumin, TuM2-PK (Tumour pyruvate kinase type M2), sFas/CD95, YKL-40, CYT-MAA (cytoplasmic melanoma-associated antigen), HMW-MAA (high-molecular-weight melanoma-associated antigen), STAT3, STAT1, gp100/HMB45, p16 INK4A, PTEN, pRb (retinoblastoma protein), EGFR, p-Akt, c-Kit, c-myc, AP-2, HDM2, bcl-6, Ki67 (detected by Mib1), Cyclin A, B, D, E, p21CIP1, Geminin, PCNA (proliferating cell nuclear antigen), bcl-2, bax, bak, APAF-1, LYVE-1 (lymphatic vascular endothelial hyaluronan receptor-1), PTN, P-Cadherin, E-Cadherin, Beta-catenin, Integrins beta1 and beta3, MMPs (matrix metalloproteinases), Dysadherin, CEACAM1 (carcinoembryonic-antigen-related cell-adhesion molecule 1), Osteonectin, TA, Melastatin, ALCAM/CD166 (Activated leukocyte cell adhesion molecule), CXCR4, Metallothionein.

According to further embodiments n markers according to at least some embodiments of the present invention might optionally be used alone or in combination with one or more other compounds described herein, and/or in combination with known markers for prostate cancer, including but not limited to PSA, PAP (prostatic acid phosphatase), CPK-BB, PSMA, PCA3, DD3, and/or in combination with the known protein(s) for the variant marker as described herein.

According to further embodiments markers according to at least some embodiments of the present invention might optionally be used alone or in combination with one or more other compounds described herein, and/or in combination with known markers for pancreatic cancer, including but not limited to CA 19-9, and/or in combination with the known protein(s) for the variant marker as described herein.

According to further embodiments markers according to at least some embodiments of the present invention might optionally be used alone or in combination with one or more other compounds described herein, and/or in combination with known markers for hematological cancer, including but not limited to soluble forms of tumor markers like P-Selectin, CD-22, interleukins, cytokines, and/or in combination with the known protein(s) for the variant marker as described herein.

According to further embodiments markers according to at least some embodiments of the present invention might optionally be used alone or in combination with one or more other compounds described herein, and/or in combination with known markers for colon cancer, including but not limited to CEA, CA19-9, CA50, and/or in combination with the known proteins for the variant marker as described herein. The diagnostic assay is performed in a subject or in a sample obtained from a subject.

According to some embodiments, the sample taken from a subject to perform a diagnostic assay according to at least some embodiments of the present invention is selected from the group consisting of a body fluid or secretion including but not limited to blood, serum, urine, plasma, prostate fluid, seminal fluid, semen, the external secretions of the skin, respiratory, intestinal, and genitourinary tracts, tears, cerebrospinal fluid, sputum, saliva, milk, peritoneal fluid, pleural fluid, cyst fluid, secretions of the breast ductal system (and/or lavage thereof), broncho alveolar lavage, lavage of the reproductive system and lavage of any other part of the body or system in the body; samples of any organ including isolated cells or tissues, wherein the cell or tissue can be obtained from an organ selected from, but not limited to lung, colon, kidney, pancreas, ovary, prostate, liver, skin, bone marrow, lymph node, breast, and/or blood tissue; stool or a tissue sample, or any combination thereof. Prior to be subjected to the diagnostic assay, the sample can optionally be diluted with a suitable diluent. In certain embodiments, cells obtained from the sample are cultured in vitro prior to performing the diagnostic assay.

Numerous well known tissue or fluid collection methods can be utilized to collect the biological sample from a subject in order to determine the level of nucleic acid and/or polypeptide of the marker of interest in the subject.

Examples include, but are not limited to, fine needle biopsy, needle biopsy, core needle biopsy and surgical biopsy (e.g., brain biopsy), and lavage. Regardless of the procedure employed, once a biopsy/sample is obtained the level of the marker can be determined and a diagnosis can thus be made.

In at least some embodiments the present invention provides variant proteins, which may optionally be used as diagnostic markers, optionally as markers for in vivo imaging. According to at least some embodiments the present invention therefore overcomes the many deficiencies of the background art with regard to the need to obtain tissue samples and subjective interpretations of results. As in vivo imaging markers, the markers according to at least some embodiments of the present invention may also provide different and/or better measurement parameters for various diseases and/or pathological conditions. Molecular imaging using these markers could be performed in conjunction with other imaging modalities as CT and MRI which capture body anatomy and overlap it with the in-vivo marker distribution.

In at least some embodiments the present invention further relates to diagnostic assays for detecting a disease, particularly in a sample taken from a subject (patient), optionally a blood sample or a body secretion sample. In at least some embodiments of the present invention, the diagnostic assays are immunoassays, including, for example, immunohistochemical assay, radioimaging assays, in-vivo imaging, positron emission tomography (PET), single photon emission computer tomography (SPECT), magnetic resonance imaging (MRI), Ultra Sound, Optical Imaging, Computer Tomography, radioimmunoassay (RIA), ELISA, slot blot, competitive binding assays, fluorimetric imaging assays, Western blot, FACS, and the like. The diagnostic assays can be qualitative or quantitative.

In some embodiments, the phrase "differentially present" refers to differences in the quantity of a marker present in a sample taken from subjects having one of the herein-described diseases or conditions as compared to a comparable sample taken from subjects who do not have one of the herein-described diseases or conditions. A polypeptide is differentially present between the two samples if the amount of the polypeptide in one sample is significantly different from the amount of the polypeptide in the other sample. It should be noted that if the marker is detectable in one sample and not detectable in the other, then such a marker can be considered to be differentially present. Optionally, a relatively low amount of up-regulation may serve as the marker, as described herein. One of ordinary skill in the art could easily determine such relative levels of the markers; further guidance is provided in the description of each individual marker below.

The term "marker" in the context of the present invention refers to a peptide, or a polypeptide, which is differentially present in a sample taken from subjects having one of the herein-described diseases or conditions, as compared to a comparable sample taken from subjects who do not have one the above-described diseases or conditions.

According to at least some embodiments of the present invention, a diagnostic assay can provide qualitative or quantitative information on the level of the markers in the sample.

In some embodiments, the phrase "qualitative" when in reference to differences in expression levels of a polypeptide as described herein, refers to the presence versus absence of expression, or in some embodiments, the temporal regulation of expression, or in some embodiments, the timing of expression, or in some embodiments, any post-translational modifications to the expressed molecule, and others, as will be appreciated by one skilled in the art. In some embodiments, the phrase "quantitative" when in reference to differences in expression levels of a polypeptide as described herein, refers to absolute differences in quantity of expression, as determined by any means, known in the art, or in other embodiments, relative differences, which may be statistically significant, or in some embodiments, when viewed as a whole or over a prolonged period of time, etc., indicate a trend in terms of differences in expression.

The term "level" refers to expression levels of polypeptides of the marker according to at least some embodiments of the present invention.

In certain embodiments, the diagnostic markers according to at least some embodiments of the invention are correlated to a condition or disease by their mere presence or absence. In other embodiments, threshold levels of the diagnostic markers can be established, and the level of the markers in a patient's sample can be compared to the threshold levels.

In some embodiments, the term "test amount" of a marker refers to an amount of a marker in a subject's sample that is consistent with a diagnosis of a particular disease or condition. A test amount can be either in absolute amount (e.g., microgram/ml) or a relative amount (e.g., relative intensity of signals).

In some embodiments, the term "control amount" of a marker can be any amount or a range of amounts to be compared against a test amount of a marker. For example, a control amount of a marker can be the amount of a marker in a patient with a particular disease or condition or a person without such a disease or condition. A control amount can be either in absolute amount (e.g., microgram/ml) or a relative amount (e.g., relative intensity of signals).

In some embodiments, the term "detect" refers to identifying the presence, absence or amount of the object to be detected.

In some embodiments, the term "label" includes any moiety or item detectable by spectroscopic, photo chemical, biochemical, immunochemical, or chemical means. For example, useful labels include 32P, 35S, fluorescent dyes, electron-dense reagents, enzymes (e.g., as commonly used in an ELISA), biotin-streptavadin, dioxigenin, haptens and proteins for which antisera or monoclonal antibodies are available. The label often generates a measurable signal, such as a radioactive, chromogenic, or fluorescent signal, that can be used to quantify the amount of bound label in a sample. The label can be incorporated in or attached to a probe either covalently, or through ionic, van der Waals or hydrogen bonds. The label may be directly or indirectly detectable. Indirect detection can involve the binding of a second label to the first label, directly or indirectly. For example, the label can be the ligand of a binding partner, such as biotin, which is a binding partner for streptavadin. The binding partner may itself be directly detectable, for example, an antibody may be itself labeled with a fluorescent molecule. The binding partner also may be indirectly detectable. Quantitation of the signal is achieved by, e.g., scintillation counting, densitometry, or flow cytometry.

Exemplary detectable labels, optionally for use with immunoassays, include but are not limited to magnetic beads, fluorescent dyes, radiolabels, enzymes (e.g., horse radish peroxide, alkaline phosphatase and others commonly used in an ELISA), and calorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

The phrase "specifically (or selectively) binds" to an antibody or "specifically (or selectively) immunoreactive with," or "specifically interacts or binds" when referring to a protein or peptide (or other epitope), refers, in some embodiments, to a binding reaction that is determinative of the presence of the protein in a heterogeneous population of proteins and other biologics. Thus, under designated immunoassay conditions, the specified antibodies bind to a particular protein at least two times greater than the background (non-specific signal) and do not substantially bind in a significant amount to other proteins present in the sample. Specific binding to an antibody under such conditions may require an antibody that is selected for its specificity for a particular protein. For example, polyclonal antibodies raised to seminal basic protein from specific species such as rat, mouse, or human can be selected to obtain only those polyclonal antibodies that are specifically immunoreactive with seminal basic protein and not with other proteins, except for polymorphic variants and alleles of seminal basic protein. This selection may be achieved by subtracting out antibodies that cross-react with seminal basic protein molecules from other species. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity). Typically a specific or selective reaction will be at least twice background signal or noise and more typically more than 10 to 100 times background.Diagnostic assays according to at least some embodiments of the present invention include, but are not limited to immunoassays and nucleic acid based assays. "Immunoassay" is an assay that uses an antibody to specifically bind an antigen. The immunoassay is characterized by the use of specific binding properties of a particular antibody to isolate, target, and/or quantify the antigen.

According to at least some embodiments, the present invention provides a method for detecting the polypeptides according to at least some embodiments of the invention in a biological sample, comprising: contacting a biological sample with an antibody specifically recognizing a polypeptide according to at least some embodiments of the present invention and detecting said interaction; wherein the presence of an interaction correlates with the presence of a polypeptide in the biological sample.

Non-limiting examples of methods or assays are described below.

The present invention also relates to kits based upon such diagnostic methods or assays.

### IMMUNOASSAYS

Immunological binding assays include, for example, an enzyme immune assay (EIA) such as enzyme-linked immunosorbent assay (ELISA), a radioimmune assay (RIA), a Western blot assay, or a slot blot assay (see, e.g., U.S. Pat. No 4,366,241; 4,376,110; 4,517,288; and 4,837,168). Generally, a subject or a sample obtained from a subject is contacted with an antibody that specifically binds a polypeptide according to at least some embodiments of the invention, or a fragment thereof. Optionally, the antibody can be fixed to a solid support prior to contacting the antibody with a sample. Examples of solid supports include but are not limited to glass or plastic in the form of, e.g., a microtiter plate, a stick, a bead, or a microbead. After incubating the sample with antibodies, the mixture is washed and the antibody-marker complex formed can be detected. This can be accomplished by incubating the washed mixture with a detection reagent. Alternatively, the marker in the sample can be detected using an indirect assay, wherein, for example, a second, labeled antibody is used to detect bound marker-specific antibody. Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume, concentrations and the like. Usually the assays will be carried out at ambient temperature, although they can be conducted over a range of temperatures, such as 10 °C to 40 °C.

The amount of an antibody-marker complex can optionally be determined by comparing to a standard or to a control amount and/or signal.

Radio-immunoassay (RIA): According to one embodiment, this method involves contacting the biological sample with a specific antibody followed by a radiolabeled secondary antibody or antibody binding protein (e.g., protein A labeled with I125) immobilized on a precipitable carrier such as agarose beads. The number of counts in the precipitated pellet is proportional to the amount of the marker polypeptide in the sample.

Enzyme linked immunosorbent assay (ELISA): This method involves fixation of a sample containing the target polypeptide to a surface such as a well of a microtiter plate. A substrate specific antibody coupled to an enzyme is applied and allowed to bind to the target polypeptide. Presence of the antibody is then detected and quantitated by a colorimetric reaction employing the enzyme coupled to the antibody. Enzymes commonly employed in this method include horseradish peroxidase and alkaline phosphatase. The amount of substrate present in the sample is proportional to the amount of color produced. A substrate standard is generally employed to improve quantitative accuracy.

Western blot: This method involves separation of a solution containing the target polypeptide by means of an acrylamide gel followed by transfer of the polypeptides to a membrane (e.g., nylon or PVDF). Presence of the target polypeptide is then detected by specific antibodies, which are in turn detected by antibody binding reagents. Antibody binding reagents may be, for example, protein A, or secondary antibodies. Antibody binding reagents may be radiolabeled or enzyme linked as described hereinabove. Detection may be by autoradiography, colorimetric reaction or chemiluminescence. This method allows both quantitative analysis of the amount of target polypeptide and determination of its identity by a relative position on the membrane which is indicative of a migration distance in the acrylamide gel during electrophoresis.

Immunohistochemical analysis: This method involves detection of a substrate in situ in fixed cells by specific antibodies. The antibodies may be enzyme linked or linked to fluorophores. Detection is by microscopy and subjective evaluation. If enzyme linked antibodies are employed, a colorimetric reaction may be required.

Fluorescence activated cell sorting (FACS): This method involves detection of a target polypeptide in situ in cells by specific antibodies. The antibodies are linked to fluorophores. Detection is by means of a cell sorting machine which reads the wavelength of light emitted from each cell as it passes through a light beam. This method may employ two or more antibodies simultaneously.

### RADIO-IMAGING METHODS

These methods include but are not limited to, positron emission tomography (PET) and single photon emission computed tomography (SPECT). Both of these techniques are non-invasive, and can be used to detect and/or measure a wide variety of tissue events and/or functions, such as detecting cancerous cells for example. Unlike PET, SPECT can optionally be used with two labels simultaneously. SPECT has some other advantages as well, for example with regard to cost and the types of labels that can be used. For example, US Patent No. 6,696,686 describes the use of SPECT for detection of breast cancer.

According to at least some embodiments the present invention also relates to kits based upon such diagnostic methods or assays.

### THERANOSTICS:

According to at least some embodiments the present invention also relates to the ude of markers and antibodies according to at least some embodiments of the invention for theranostics. The term theranostics describes the use of diagnostic testing to diagnose the disease, choose the correct treatment regime according to the results of diagnostic testing and/or monitor the patient response to therapy according to the results of diagnostic testing. Theranostic tests optionally may be used to select patients for treatments that are particularly likely to benefit them and unlikely to produce side-effects. They can also provide an early and objective indication of treatment efficacy in individual patients, so that (if necessary) the treatment can be altered with a minimum of delay. For example: DAKO and Genentech together created HercepTest and Herceptin (trastuzumab) for the treatment of breast cancer, the first theranostic test approved simultaneously with a new therapeutic drug. In addition to HercepTest (which is an immunohistochemical test), other theranostic tests are in development which use traditional clinical chemistry, immunoassay, cell-based technologies and nucleic acid tests. PPGx's recently launched TPMT (thiopurine S-methyltransferase) test, which is enabling doctors to identify patients at risk for potentially fatal adverse reactions to 6-mercaptopurine, an agent used in the treatment of leukemia. Also, Nova Molecular pioneered SNP genotyping of the apolipoprotein E gene to predict Alzheimer's disease patients' responses to cholinomimetic therapies and it is now widely used in clinical trials of new drugs for this indication. Thus, the field of theranostics represents the intersection of diagnostic testing information that predicts the response of a patient to a treatment with the selection of the appropriate treatment for that particular patient.

### SURROGATE MARKERS:

According to at least some embodiments the present invention also relates to the ude of markers and antibodies according to at least some embodiments of the invention as Surrogate markers. A surrogate marker is a marker, that is detectable in a laboratory and/or according to a physical sign or symptom on the patient, and that is used in therapeutic trials as a substitute for a clinically meaningful endpoint. The surrogate marker is a direct measure of how a patient feels, functions, or survives which is expected to predict the effect of the therapy. The need for surrogate markers mainly arises when such markers can be measured earlier, more conveniently, or more frequently than the endpoints of interest in terms of the effect of a treatment on a patient, which are referred to as the clinical endpoints. Ideally, a surrogate marker will be biologically plausible, predictive of disease progression and measurable by standardized assays (including but not limited to traditional clinical chemistry, immunoassay, cell-based technologies, nucleic acid tests and imaging modalities).

Surrogate endpoints were used first mainly in the cardiovascular area. For example, antihypertensive drugs have been approved based on their effectiveness in lowering blood pressure. Similarly, in the past, cholesterol-lowering agents have been approved based on their ability to decrease serum cholesterol, not on the direct evidence that they decrease mortality from atherosclerotic heart disease. The measurement of cholesterol levels is now an accepted surrogate marker of atherosclerosis. In addition, currently two commonly used surrogate markers in HIV studies are CD4+ T cell counts and quantitative plasma HIV RNA (viral load). In some embodiments of this invention, the polypeptide/polynucleotide expression pattern may serve as a surrogate marker for a particular disease, as will be appreciated by one skilled in the art.

The following examples are offered for illustrative purposes only, and are not intended to limit the scope of the present invention in any way.

### EXAMPLES

### EXAMPLE 1:

### METHODS USED TO ANALYZE THE EXPRESSION OF THE RNA

The targets according to at least some embodiments of the present invention were tested with regard to their expression in various cancerous and non-cancerous tissue samples and/or with regard to its expression in a wide panel of human samples which contains various types of immune cells, and hematological malignancies samples and cell lines, as well as several samples of normal tissues. A description of the samples used in the normal and cancerous tissue panels used in Example 3_2 (presented in Figure 13) is provided in Table 1. The list of the blood specific RNA samples used for the qRT-PCR analysis is provided in Table 2 below. A description of the Multiple Myeloma samples from the blood panel described in Table 2, is provided in Table 2_1. A description of the samples used in the normal tissue panels are provided in Table 3. A description of the samples used in the ovary cancer testing panel is provided in Table 4 below. The key for the table 4 is given in table 4_1. Tests were then performed as described in the "Materials and Experimental Procedures" section below.

**Table 1**

| Well | Sample Name | Ct | Quantity | Well | Sample Name | Ct | Quantity |
|---|---|---|---|---|---|---|---|
| 1 | A1 | 31.988285 | 100 | 91 | N.Testis (#276) | 32.4772 | 48.8715 |
| 2 | A2 | 28.521492 | 1000 | 94 | N.Trach ea (#314) | 31.908 | 71.3431 |
| 3 | A3 | 24.747038 | 10000 | 106 | Head/Ne ck T. (Larynx, #402) | 31.7226 | 80.7009 |
| 4 | A4 | 21.234554 | 100000 | 107 | Head/Ne ck T. (Pharyn x, #403) | 30.1814 | 224.8 |
| 5 | A5 | 17.710257 | 1000000 | 108 | Head/Ne ck T. (Tongue , #404) | 31.1225 | 120.26 |
| 6 | A6 | 31.214949 | 100 | 109 | Head/Ne ck T. (Tonsil, #405) | 33.464 | 25.3618 |
| 7 | A7 | 27.833765 | 1000 | 110 | Kidney T. (#167) | 28.9694 | 503.108 |
| 8 | A8 | 24.486034 | 10000 | 111 | Kidney T. (#168) | 30.175 | 225.761 |
| 9 | A9 | 20.853727 | 100000 | 112 | Kidney T. (#169) | 34.01 | 17.6431 |
| 10 | A10 | 17.610092 | 1000000 | 113 | Kidney T. (#170) | 30.4578 | 187.069 |
| 11 | A11 | 30.853067 | 100 | 114 | Kidney T. (#171) | 30.5402 | 177.099 |
| 12 | A12 | 27.679554 | 1000 | 115 | Liver T. (#326) | 31.9156 | 70.9841 |
| 13 | A13 | 24.258177 | 10000 | 116 | Liver T. (#327) | 31.0544 | 125.828 |
| 14 | A14 | 20.678856 | 100000 | 117 | Liver T. (#328) | 28.3689 | 749.942 |
| 15 | A15 | 17.408792 | 1000000 | 118 | Liver T. (#329) | 29.0953 | 462.736 |
| 17 | Water | Undetermined | 0 | 123 | Lung T. (NSC, #157) | 31.9388 | 69.8983 |
| 28 | N. Bone Marrow Stromal Cells (#394) | 37.212994 | 2.098594 | 124 | Lung T. (NSC, #158) | 33.0217 | 34.0301 |
| 30 | N.Brain Cerebel lum (#123) | 34.93981 | 9.509247 | 125 | Lung T. (NSC, #159) | 29.9511 | 261.985 |
| 36 | N.Brain Thalam us (#131) | 34.98086 | 9.253285 | 126 | Lung T. (NSC, #160) | 31.4862 | 94.4371 |
| 37 | N.Breast (#259) | 31.53689 | 91.30512 | 134 | Lympho ma (#287) | 28.2315 | 821.668 |
| 39 | N.Cervix (#260) | 33.32578 | 27.80238 | 135 | Lympho ma (#288) | 33.1223 | 31.8298 |
| 40 | N.Colon (#261) | 31.046032 | 126.5304 | 136 | Lympho ma (#290) | 27.9259 | 1006.72 |
| 45 | N.Esop hagus (#307) | 34.37151 | 13.87403 | 137 | Lympho ma (#289) | 29.1648 | 441.834 |
| 46 | N.Heart (#118) | 34.899815 | 9.765454 | 138 | Lympho ma (#291) | 29.3026 | 403.177 |
| 53 | N.Kidney (#264) | 32.810036 | 39.17111 | 139 | Lympho ma (#292) | 33.3113 | 28.0704 |
| 54 | N.Kidney (#265) | 31.380625 | 101.2991 | 140 | Lympho ma (#293) | 28.0062 | 954.41 |
| 55 | N.Kidney (#311) | 35.80812 | 5.3393 | 141 | Lympho ma (#294) | 28.9552 | 507.88 |
| 56 | N.Liver (#266) | 36.25151 | 3.976376 | 142 | Lympho ma (#295) | 27.6835 | 1182.74 |
| 57 | N.Liver (#267) | 34.554146 | 12.28795 | 143 | Lympho ma (#296) | 30.0861 | 239.499 |
| 67 | N.Lung (#268) | 32.817394 | 38.97998 | 144 | Lympho ma (#297) | 30.4276 | 190.865 |
| 68 | N.Lung (#313) | 32.38373 | 52.00325 | 145 | Lympho ma (#298) | 30.8015 | 148.863 |
| 70 | N.Lym ph Node (#269) | 30.1909 | 223.3843 | 146 | Lympho ma (#299) | 30.1717 | 226.256 |
| 71 | N.Lym ph Node (#315) | 28.42075 | 724.5305 | 147 | Lympho ma (#300) | 30.2177 | 219.437 |
| 73 | N.Ovar y (#270) | 33.379852 | 26.82083 | 148 | Melano ma (#162) | 32.4551 | 49.5932 |
| 74 | N.Pancr eas (#271) | 35.97763 | 4.770353 | 149 | Melano ma (#163) | 28.9535 | 508.481 |
| 76 | N.Perip heral Blood Leukoc ytes (#302) | 29.496643 | 354.3804 | 150 | Melano ma (#166) | 37.4152 | 1.83466 |
| 77 | N.Prost ate (#272) | 36.119064 | 4.34232 | 151 | Melano ma (#165) | 27.2872 | 1539.16 |
| 81 | N.Skin (#273) | 38.019768 | 1.227525 | 152 | Melano ma (#164) | 33.0435 | 33.5416 |
| 82 | N.Skin (#319) | 33.879433 | 19.24218 | 162 | Pancreas T. (#186) | 30.7932 | 149.691 |
| 83 | N.Small Intestine (#320) | 31.034052 | 127.5421 | 163 | Prostate T. (#378) | 33.5837 | 23.422 |
| 84 | N.Small Intestine: Jejunum (#321) | 32.072933 | 63.93684 | 164 | Prostate T. (#379) | 33.1311 | 31.6443 |
| 87 | N.Splee n (#274) | 33.140545 | 31.44526 | 165 | Prostate T. (#380) | 31.9457 | 69.5817 |
| 88 | N.Splee n (#322) | 26.349798 | 2870.087 | 166 | Prostate T. (#381) | 32.1304 | 61.5407 |
| 89 | N.Stom ach (#275) | Undetermined | 0 | 167 | Prostate T. (#382) | 31.5726 | 89.1632 |
| 90 | N.Stom ach (#323) | 31.639095 | 85.30818 | 168 | Prostate T. (#383) | 34.3888 | 13.7151 |

**Table 2**

| Blood panel sample | Description | Organ/ Cell type | Tumor Type |
|---|---|---|---|
| 1_PBMC2 | PBMCs | blood-derived cells | |
| 2_PBMC3 | PBMCs | blood-derived cells | |
| 3_Bcell1 | B cells | blood-derived cells | |
| 4_Bcell2 | B cells | blood-derived cells | |
| 5_J_Bcell | B cells | blood-derived cells | |
| 6_K_Bcells_act | Bcells activated | blood-derived cells | |
| 7_Tcell1 | T cells | blood-derived cells | |
| 8_Tcell2 | T cells | blood-derived cells | |
| 9_M_CD8 | CD4+ T cells | blood-derived cells | |
| 10_G_CD4_unt | CD8+ T cells | blood-derived cells | |
| 11_H_CD4_Beads | CD4+ w Activation beads | blood-derived cells | |
| 12_I_CD4_Beads_IL12 | CD4 w act. Beads+IL12 | blood-derived cells | |
| 13_95_CD4+CD25- | CD4+CD25- | blood-derived cells | |
| 15_NK | NK cells | blood-derived cells | |
| 16_CD34+_1548 | CD34+(PCBM1548) | blood-derived cells | |
| 17_CD34+_1028 | CD34+(PCBM1028) | blood-derived cells | |
| 18_PMN | PMNs | blood-derived cells | |
| 19_A_Mono | Monocytes | blood-derived cells | |
| 20_B_Macro_imma | Macrophages immature | blood-derived cells | |
| 21_C_Macro_mat | Macrophages mature | blood-derived cells | |
| 22_D_DCs_immat | DCs immature | blood-derived cells | |
| 23_E_DCs_mat_LPS | DCs mature LPS | blood-derived cells | |
| 24_F_DCs_mat_CK | DCs mature CK | blood-derived cells | |
| 25_L_DCs+T | DCs +T cells | blood-derived cells | |
| 26_Lym1 | 13987A1 | Lymph Node | Lymphoma |
| 27_Lym2 | 43594B1 | Muscle | lymphoma |
| 28_Lym3 | 65493A1 | Testis | Lymphoma |
| 29_MalLym3 | 75894A1 | Brain | Lymphoma |
| 30_NonHod_SCLym | 83325A1 | Lymph Node | NHL Small Cell |
| 31_NonHod_FolLym | 76943A1(5 tubes) | Lymph Node | NHL Follicular |
| 32_Lym_Fol_GI | CN_4_ASRBNA35 | | NHL Follicular Grade I (Small Cell) |
| 33_Lym_Fol_GII | CN_1_113GHA8J | | NHL Follicular Grade II (mixed Small & Large Cell) |
| 34_Lym_Fol_GIII | CN_8_VXML6AXI | | NHL Follicular Grade III (Large Cell) |
| 35_MalLym1 | 7621.8B1 | Testis | NHL Large Cell |
| 36_MalLym2 | 76102A1 | Lymph Node | NHL Large Cell |
| 37_Lym_DifBCell1 | CN_2_4HDLNA2R | | NHL Diffuse Large B-Cell |
| 38_Lym_DifBCell2 | CN_3_4M4S7AAM | | NHL Diffuse Large B-Cell |
| 39_Lym_DifBCell3 | CN_5_HEODOAR2 | | NHL Diffuse Large B-Cell |
| 40_NonHod_Lym1 | 77332A1(5 tubes) | Colon | NHL Diffuse Large B-Cell |
| 41_MalLym4 | 76161A1 | Spleen | NHL Diffuse Large B-Cell |
| 42_Lym_MantleCell1 | CN_6_MAE47AOY | | NHL Mantle Cell |
| 43_Lym_MantleCell2 | CN_7_VJU9OAO9 | | NHL Mantle Cell |
| 44_NonHod_Lym2 | 95377A1(5 tubes) | Spleen | NHL |
| 45_THP_1 | THP-1 | monocytes | AML cell line |
| 77-MM_Patient1 | Multiple Myeloma Patient | See Table 2_1 | |
| 78-MM_Patient2 | Multiple Myeloma Patient | See Table 2_1 | |
| 79-MM_Patient3 | Multiple Myeloma Patient | See Table 2_1 | |
| 80-MM_Patient4 | Multiple Myeloma Patient | See Table 2_1 | |
| 81-MM_Patient5 | Multiple Myeloma Patient | See Table 2_1 | |
| 82-MM_Patient6 | Multiple Myeloma Patient | See Table 2_1 | |
| 83-MM_Patient7 | Multiple Myeloma Patient | See Table 2_1 | |
| 84-MM_Tumor1 | Multiple Myeloma Tumor | See Table 2_1 | |
| 85-MM_Tumor2 | Multiple Myeloma Tumor | See Table 2_1 | |
| 87-MM_Tumor4 | Multiple Myeloma Tumor | See Table 2_1 | |
| 59_NCI_H929 | NCI-H929 | B lymphoblasts | Multiple Myeloma cell line |
| 60_MC/CAR | MC/CAR | B lymphoblasts | Multiple Myeloma cell line |
| 61_U266 | U266 | B lymphoblasts | Multiple Myeloma cell line |
| 62_RPMI8226 | RPMI8226 | B lymphoblasts | Multiple Myeloma cell line |
| 63_IM_9 | IM-9 | B lymphoblasts | Multiple Myeloma cell line |
| 64_cereN | cerebellum normal | cerebellum normal | |
| 65_kidneyN1 | kidney normal | kidney normal | |
| 66_kidneyN2 | kidney normal | kidney normal | |
| 67_KidneyN3 | kidney normal | kidney normal | |
| 68_colonN1 | colon normal | colon normal | |
| 69_colonN2 | colon normal | colon normal | |
| 70_stomN | stomach normal | stomach normal | |
| 71_liverN | liver normal | liver normal | |
| 72_lungN1 | lung normal | lung normal | |
| 73_lungN2 | lung normal | lung normal | |
| 74_small intestineN | small intestine | small intestine | |
| 75_brainN | brain normal mix | brain normal mix | |
| 76_heartN | heart normal mix | heart normal mix | |

**Table 2_1: Multiple Myeloma samples details**

| | patient ID | tumor ID | Diagnosis | Bone disease | Heavy chain isotype | Light chain isotype | ISS | Sex |
|---|---|---|---|---|---|---|---|---|
| 77-MM_Patient1 | 1289 | | Amyloidosis | UD | IgG | kappa | | |
| 78-MM_Pat ient2 | 1441 | | myeloma | No | IgG | - | satge 2 | |
| 79-MM_Pat ient3 | 1647 | | myeloma | UD | IgG | kappa | stage 3 | |
| 80-MM_Pat ient4 | 1434 | | myeloma | UD | IgG | kappa | satge 3 | |
| 81-MM_Pat ient5 | 1650 | | Undetermained (myeloma vs.MGUS) | UD | IgG | lambda | stage 2 | |
| 82-MM_Pat ient6 | 1661 | | myeloma | Yes | urine secretor | lambda | stage 1 | |
| 83-MM_Pat ient7 | 1058 | | myeloma | Yes | IgG | lambda | | |
| 84-MM_Tu mor1 | 1016 | LAP CLκ-1 | Plasma cell leukemia | UD | IgG | kappa | | - |
| 85-MM_Tu mor2 | 1065 | LAG κ-1A | myeloma | Yes | IgG | kappa | | F |
| 87-MM_Tu mor4 | 1178 | LAG κ-2 | myeloma | Yes | IgG | kappa | | M |

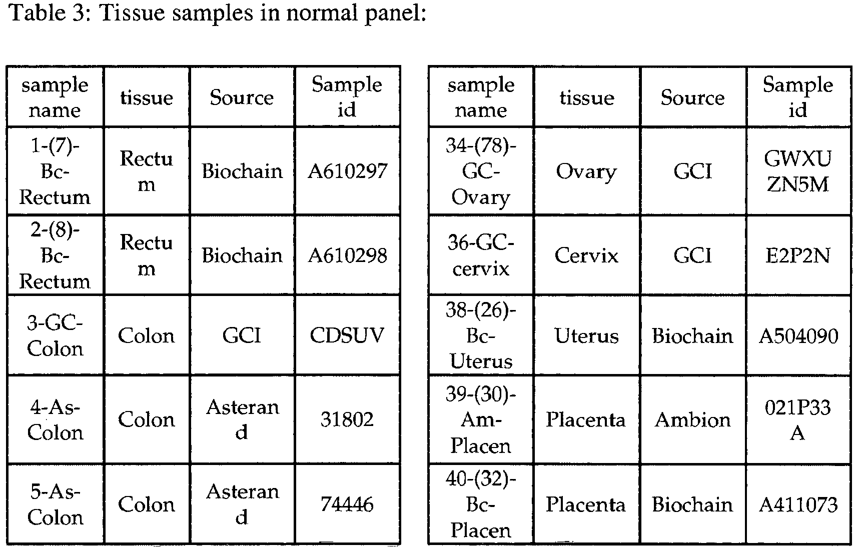

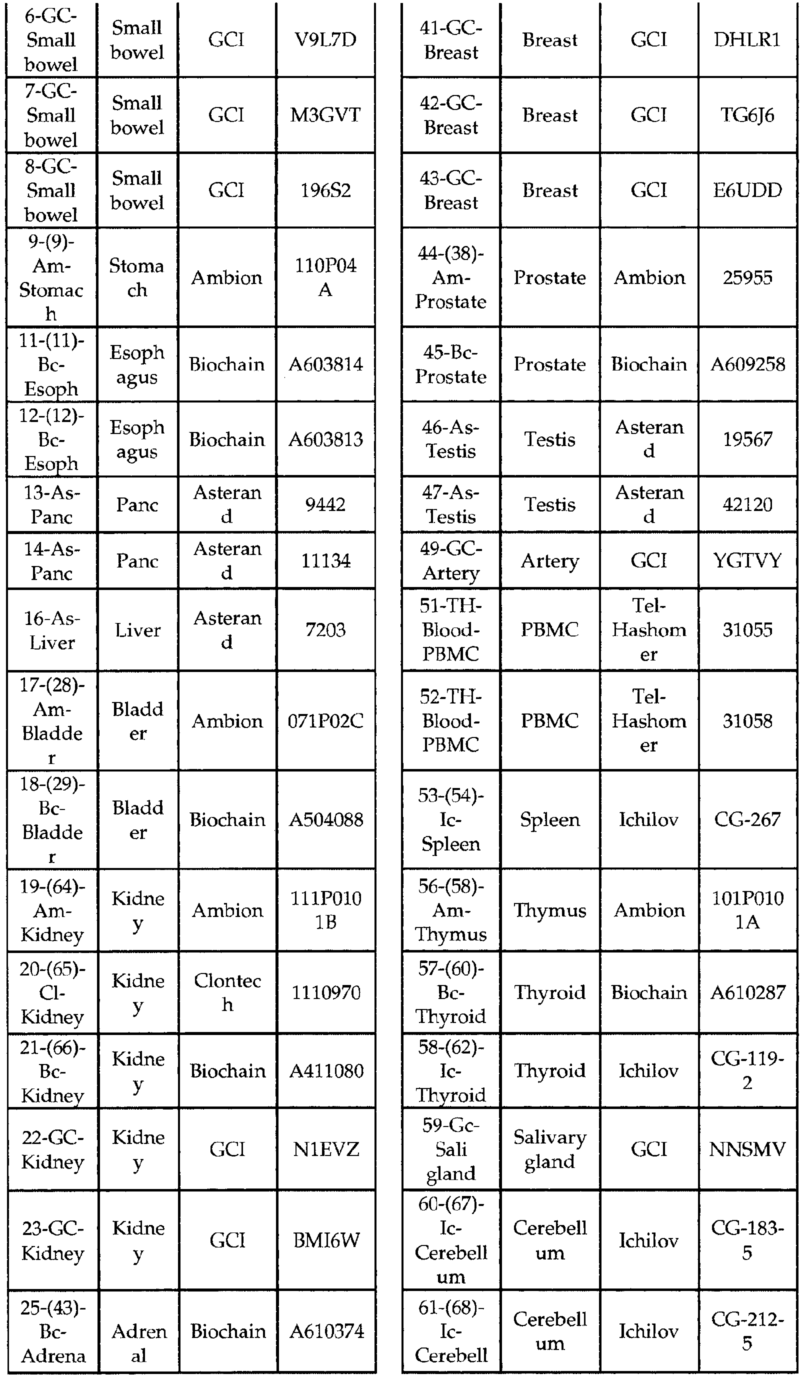

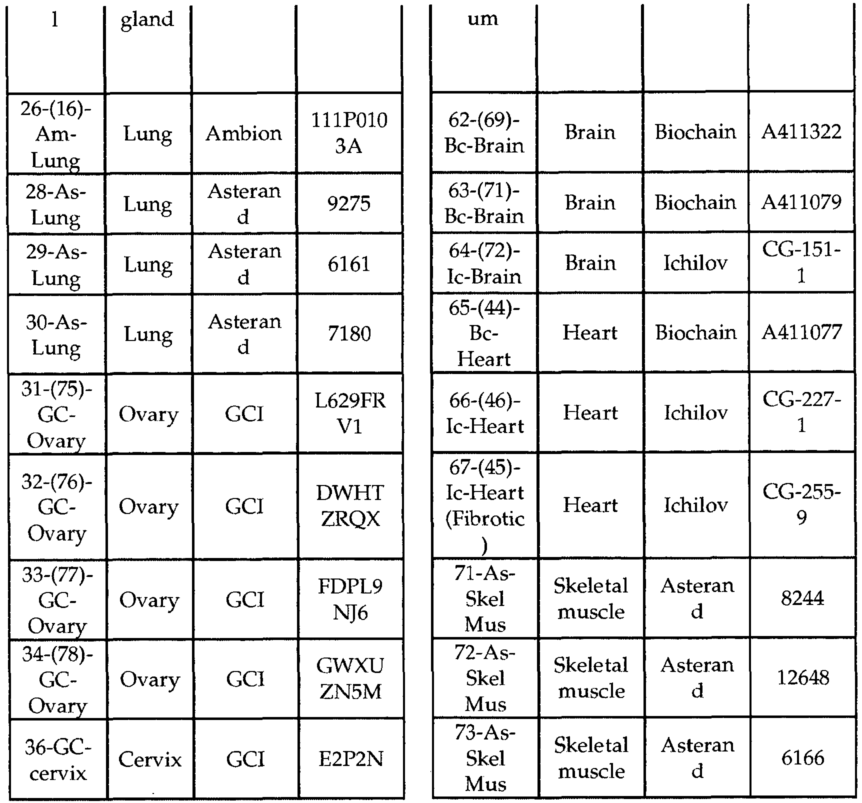

**Table 4: Tissue samples in ovary panel**

| Source | sample name | Ovary tissue type | Stage | Sample id |
|---|---|---|---|---|
| Asterand | 1-As-Ser SI | Serous carcinoma | I | 71900A2 |
| Asterand | 2-As-Ser SI | Serous carcinoma | I | 70270A1 |
| Asterand | 3-As-Ser SIB | Serous carcinoma | IB | 40771B1 |
| Asterand | 4-As-Ser SIB | Serous carcinoma | IB | 32667B1 |
| Asterand | 5-As-Ser SIC | Serous carcinoma | IC | 22996A1 |
| Asterand | 6-As-Ser SIIA | Serous carcinoma | IIA | 40773C1 |
| GCI | 7-GC-Ser SIIB | Serous carcinoma | IIB | 20370 |
| GCI | 8-GC-Ser SIIB | Serous carcinoma | IIB | 7B3DP |
| Asterand | 9-As-Ser SIIIC | Serous carcinoma | IIIC | 19832A1 |
| GCI | 10-GC-Ser SIIIC | Serous carcinoma | IIIC | 3NTIS |
| GCI | 11-GC-Ser SIIIC | Serous carcinoma | IIIC | CEJUS |
| GCI | 12-GC-Ser SIIIC | Serous carcinoma | IIIC | 5NCLK |
| GCI | 13-GC-Ser SIIIC | Serous carcinoma | IIIC | 1HI5H |
| GCI | 14-GC-Ser SIIIC | Serous carcinoma | IIIC | 7RMHZ |
| GCI | 15-GC-Ser SIIIC | Serous carcinoma | IIIC | 4WAAB |
| GCI | 16-GC-Ser SIIIC | Serous carcinoma | IIIC | 79Z67 |
| GCI | 17-GC-Ser SIIIC | Serous carcinoma | IIIC | DDSNL |
| GCI | 18-GC-Ser SIV | Serous carcinoma | IV | DH8PH |
| GCI | 19-GC-Endo SIA | Endometrioid Carcinoma | IA | E2WKF |
| GCI | 20-GC-Endo SIA | Endometrioid Carcinoma | IA | 5895C |
| GCI | 21-GC-Endo SIA | Endometrioid Carcinoma | IA | 533DX |
| GCI | 22-GC-Endo SIA | Endometrioid Carcinoma | IA | HZ2EY |
| GCI | 23-GC-Endo SIA | Endometrioid Carcinoma | IA | RWOIV |
| GCI | 24-GC-Endo SIIA | Endometrioid Carcinoma | IIA | 1U52X |
| GCI | 25-GC-Endo SIIB | Endometrioid Carcinoma | IIB | A17WS |
| GCI | 26-GC-Endo SIIIC | Endometrioid Carcinoma | IIIC | 1VT3I |
| GCI | 27-GC-Endo SIIIC | Endometrioid Carcinoma | IIIC | PZQXH |
| GCI | 28-GC-Endo SIV | Endometrioid Carcinoma | IV | I8VHZ |
| GOG | 29-(21)-GO-Muc SIA | Mucinous Carcinoma | IA | 95-10-G020 |
| GCI | 30-GC-Muc SIC | Mucinous Carcinoma | IC | IMDA1 |
| Asterand | 31-As-Muc SIC | Mucinous Carcinoma | IC | 18920A1 |
| ABS | 32-(22)-AB-Muc SIC | Mucinous Carcinoma | IC | A0139 |
| GCI | 33-GC-Muc SIIA | Mucinous Carcinoma | IIA | NJM4U |
| ABS | 34-(20)- AB-Muc SIIIA | Mucinous Carcinoma | IIIA | USA-00273 |
| GCI | 35-GC-Muc SIIIA | Mucinous Carcinoma | IIIA | RAFCW |
| Asterand | 36-As-Muc SIIIC | Mucinous Carcinoma | IIIC | 72888A1 |
| Asterand | 37-As-Muc SIIIC | Mucinous Carcinoma | IIIC | 29374B1 |
| GCI | 38-GC-Muc Border SIA | Mucinous borderline tumor | IA | SC656 |
| GCI | 39-GC-Muc Border SIA | Mucinous borderline tumor | IA | 3D5FO |
| GCI | 40-GC-Muc Border SIA | Mucinous borderline tumor | IA | 7JP3F |
| GOG | 41-(62)-Go-Ben Muc | Benign mucinous | | 99-10-G442 |
| GCI | 43-GC-Ben Muc | Benign mucinous | | QLIKY |
| Asterand | 44-As-Ben Muc | Benign mucinous | | 30534A1 |
| GOG | 45-(56)-GO-Ben Muc | Benign mucinous | | 99-01-G407 |
| GCI | 46-GC-Ben Muc | Benign mucinous | | 943EC |
| GCI | 47-GC-Ben Muc | Benign mucinous | | JO8W7 |
| Asterand | 48-As-Ben Ser | Benign seruos | | 30645B1 |
| GOG | 49-(64)-GO-Ben Ser | Benign seruos | | 99-06-G039 |
| GCI | 50-GC-Ben Ser | Benign seruos | | DQQ2F |
| Asterand | 51-As-Ben Ser | Benign seruos | | 8275A1 |
| Asterand | 52-As-N BM | Normal | | 23054A1 |
| Asterand | 53-As-N BM | Normal | | 30488A1 |
| Asterand | 54-As-N BM | Normal | | 30496B1 |
| Asterand | 55-As-N BM | Normal | | 30499C1 |
| GCI | 56-GC-N PS | Normal | | WPU1U |
| GCI | 58-GC-N PS | Normal | | 76VM9 |
| GCI | 59-GC-N PS | Normal | | DWHTZ |
| GCI | 60-GC-N PS | Normal | | SJ2R2 |
| GCI | 61-GC-N PS | Normal | | 9RQMN |
| GCI | 62-GC-N PS | Normal | | TOAE5 |
| GCI | 63-GC-N PS | Normal | | TW9PM |
| GCI | 64-GC-N PS | Normal | | 2VND2 |
| GCI | 65-GC-N PS | Normal | | L629F |
| GCI | 66-GC-N PS | Normal | | XLB23 |
| GCI | 67-GC-N PS | Normal | | IDUVY |
| GCI | 68-GC-N PS | Normal | | ZCXAD |
| GCI | 69-GC-N PS | Normal | | PEQ6C |
| GCI | 70-GC-N PS | Normal | | DD73B |
| GCI | 71-GC-N PS | Normal | | E2UF7 |
| GCI | 74-GC-N PS | Normal | | FDPL9 |
| BioChain | 76-(46)-Bc-N PM | Normal | | A504086 |
| Ichilov | 77-(71)-Ic-N PM | Normal | | CG-188-7 |
| BioChain | 78-(48)-Bc-N PM | Normal | | A504087 |

### Materials and Experimental Procedures Used to Obtain Expression Data

### RNA preparation -

RNA was obtained from ABS (Wilmington, DE 19801, USA, http://www.absbioreagents.com), BioChain Inst. Inc. (Hayward, CA 94545 USA www.biochain.com), GOG for ovary samples- Pediatic Cooperative Human Tissue Network, Gynecologic Oncology Group Tissue Bank, Children Hospital of Columbus (Columbus OH 43205 USA), Clontech (Franklin Lakes, NJ USA 07417, www.clontech.com), Ambion (Austin, TX 78744 USA, http://www.ambion.com), Asternad (Detroit, MI 48202-3420, USA, www.asterand.com), AllCells, LLC. (Emeryville, CA 94608 USA, www,allcells.co,), IMBCR- Institute for Myeloma and Bone cancer research (West Hollywood, CA 90069, USA, www.imbcr.org) and from Genomics Collaborative Inc.a Division of Seracare (Cambridge, MA 02139, USA, www.genomicsinc.com). Alternatively, RNA was generated from blood cells, cell lines or tissue samples using TRI-Reagent (Molecular Research Center), according to Manufacturer's instructions. Tissue and RNA samples were obtained from patients or from postmortem. Most total RNA samples were treated with DNaseI (Ambion).

RT PCR - Purified RNA (2-10 µg) was mixed with 300-1500 ng Random Hexamer primers (Invitrogen) and 500 µM dNTP in a total volume of 31.2 to 156 µl. The mixture was incubated for 5 min at 65 °C and then quickly chilled on ice. Thereafter, 10-50 µl of 5X SuperscriptII first strand buffer (Invitrogen), 4.8 to 24 µl 0.1M DTT and 80-400 units RNasin (Promega) were added, and the mixture was incubated for 10 min at 25 °C, followed by further incubation at 42 °C for 2 min. Then, 2-10 µl (400-2000 units) of SuperscriptII (Invitrogen) was added and the reaction (final volume of 50-250µl) was incubated for 50 min at 42 °C and then inactivated at 70 °C for 15min. The resulting cDNA was diluted 1:20 in TE buffer (10 mM Tris pH=8, 1 mM EDTA pH=8).

Real-Time RT-PCR analysis carried out as described below- cDNA (5µl), prepared as described above, was used as a template in Real-Time PCR reactions (final volume of 20 µl) using the SYBR Green I assay (PE Applied Biosystem) with specific primers and UNG Enzyme (Eurogentech or ABI or Roche). The amplification was effected as follows: 50 °C for 2 min, 95 °C for 10 min, and then 40 cycles of 95 °C for 15 sec, followed by 60 °C for 1 min, following by dissociation step. Detection was performed by using the PE Applied Biosystem SDS 7000. The cycle in which the reactions achieved a threshold level of fluorescence (Ct= Threshold Cycle, described in detail below) was registered and was used to calculate the relative transcript quantity in the RT reactions. The relative quantity was calculated using the equation Q=efficiency^-Ct. The efficiency of the PCR reaction was calculated from a standard curve, created by using different dilutions of several reverse transcription (RT) reactions. To minimize inherent differences in the RT reaction, the resulting relative quantities were normalized using a normalization factor calculated in the following way:

The expression of several housekeeping (HSKP) genes was checked on every panel. The relative quantity (Q) of each housekeeping gene in each sample, calculated as described above, was divided by the median quantity of this gene in all panel samples to obtain the "relative Q rel to MED". Then, for each sample the median of the "relative Q rel to MED" of the selected housekeeping genes was calculated and served as normalization factor of this sample for further calculations. It should be noted that this type of analysis provides relative quantification.

For each RT sample, the expression of the specific amplicon was normalized to the normalization factor calculated from the expression of different house keeping genes as described in section above.

These house keeping genes are different for each panel.

The sequences for primers and amplicons of the housekeeping genes measured in all the ovary cancer examples are HPRT1, SDHA and G6PD.

SDHA (GenBank Accession No. NM_004168 (SEQ ID NO:136); amplicon - SDHA-amplicon (SEQ ID NO:85)), SDHA Forward primer (SEQ ID NO:83); SDHA Reverse primer (SEQ ID NO:84);

HPRT1 (GenBank Accession No. NM_000194 (SEQ ID NO:137); amplicon - HPRT1-amplicon (SEQ ID NO:88)); HPRT1 Forward primer (SEQ ID NO:86)), HPRT1 Reverse primer (SEQ ID NO:87);

G6PD (GenBank Accession No. NM_000402 (SEQ ID NO:138); G6PD amplicon (SEQ ID NO: 91)), G6PD Forward primer (SEQ ID NO:89), G6PD Reverse primer (SEQ ID NO:90).

The sequences of the housekeeping genes measured in all the examples on normal tissue samples panel were as follows:

SDHA (GenBank Accession No. NM_004168 (SEQ ID NO:136); amplicon - SDHA-amplicon (SEQ ID NO:85)), Forward primer (SEQ ID NO:83), SDHA Reverse primer (SEQ ID NO:84).

Ubiquitin (GenBank Accession No. BC000449 (SEQ ID NO:139); amplicon - Ubiquitin-amplicon (SEQ ID NO: 82)), Ubiquitn Forward primer (SEQ ID NO:80), Ubiquitin Reverse primer (SEQ ID NO:81).

TATA box (GenBank Accession No. NM_003194 (SEQ ID NO:140); TATA amplicon (SEQ ID NO: 79)), TATA box Forward primer (SEQ ID NO:77), TATA box Reverse primer (SEQ ID NO:78).

The sequences of the housekeeping genes measured in all the examples of blood panel were as follows:

HSB1L_HUMAN (Accession No. Q9Y450 (SEQ ID NO:141)), T05337_seg30-34F1-Forward primer (SEQ ID NO:68), T05337_seg30-34R1 Reverse primer (SEQ ID NO:69), T05337_seg30-34Amplicon (SEQ ID NO:70).

DHSA_HUMAN (Accession No P31040 (SEQ ID NO:142)), M78124_seg45-48F1 Forward primer (SEQ ID NO:71), M78124_seg45-48R1-Reverse primer (SEQ ID NO:72), M78124_seg45-48Amplicon (SEQ ID NO:73).

SLC25A3 (Accession No Q7Z7N7 (SEQ ID NO:144)), SSMPCPseg24-25-29F1- Forward primer (SEQ ID NO:74), SSMPCPseg24-25-29R1- Reverse primer (SEQ ID NO:75), SSMPCPseg24-25-29Amplicon (SEQ ID NO:76).

SFRS4_HUMSRP75A (Accession NO Q08170 (SEQ ID NO:143)), HUMSRP75Aseg30-33F1 Forward primer (SEQ ID NO:65), HUMSRP75Aseg30-33R1 Reverse primer (SEQ ID NO:66), HUMSRP75Aseg30-33Amplicon (SEQ ID NO:67).

HPRT1 (Accession No. NM_000194 (SEQ ID NO:137), HUMHPRTCseg5-7F1 - forward primer (SEQ ID NO:34), HUMHPRTCseg5-7R1 - reverse primer (SEQ ID NO:37), HUMHPRTCseg5-7 Amplicon (SEQ ID NO:126).

TBP -TATA Box binding protein (Accession NO P20226 (SEQ ID NO:145)), HSTFIIDXseg7-9F1- forward primer (SEQ ID NO:128), HSTFIIDXseg7-9R1 - reverse primer (SEQ ID NO:129), HSTFIIDXseg7-9 Amplicon (SEQ ID NO:130).

Another methodology used to predict the expression pattern of the proteins according to at least some embodiments of the invention was MED discovery engine:

MED is a platform for collection of public gene-expression data, normalization, annotation and performance of various queries. Expression data from the most widely used Affymetrix microarrays is downloaded from the Gene Expression Omnibus (GEO - www.ncbi.nlm.nih.gov/GEO). Data is multiplicatively normalized by setting the 95 percentile to a constant value (normalized expression=1200), and noise is filtered by setting the lower 30% to 0. Experiments are annotated, first automatically, and then manually, to identify tissue and condition, and chips are grouped according to this annotation, and cross verification of this grouping by comparing the overall expression pattern of the genes of each chip to the overall average expression pattern of the genes in this group. Each probeset in each group is assigned an expression value which is the median of the expressions of that probeset in all chips included in the group. The vector of expression of all probesets within a certain group is the virtual chip of that group, and the collection of all such virtual chips is a virtual panel. The panel (or sub-panels) can be queried to identify probesets with a required behavior (e.g. specific expression in a sub-set of tissues, or differential expression between disease and healthy tissues). These probesets are linked to LEADS contigs and to RefSeqs (http://www.ncbi.nlm.nih.gov/RefSeq/) by probe-level mapping, for further analysis.

The Affymetrix platforms that are downloaded are HG-U95A and the HG-U133 family (A,B, A2.0 and PLUS 2.0). Than three virtual panels were created: U95 and U133 Plus 2.0, based on the corresponding platforms, and U133 which uses the set of common probesets for HG-U133A, HG-U133A2.0 and HG-U133 PLUS 2.0+.

The results of the MED discovery engine are presented in scatter plots. The scatter plot is a compact representation of a given panel (collection of groups). The y-axis is the (normalized) expression and the x-axis describes the groups in the panel. For each group, the median expression is represented by a solid marker., and the expression values of the different chips in the group are represented by small dashes ("-"). The groups are ordered and marked as follows - "Other" groups (e.g. benign, non-cancer diseases, etc.) with a triangle, Treated cells with a square, Normal with a circle, Matched with a cross, and Cancer with a diamond. The number of chips in each group is also written adjacent to it's name.

### EXAMPLE 6

### EXAMPLE 6_1

### DESCRIPTION FOR CLUSTER W38346

Cluster W38346 (internal ID 70579958) features 4 transcripts of interest, the names for which are given in Table 38. The selected protein variants are given in table 39.

**Table 38 - Transcripts of interest**

| **Transcript Name** |
|---|
| W38346_T0 (SEQ ID NO:38) |
| W38346_T1 (SEQ ID NO:39) |
| W38346_T2 (SEQ ID NO:40) |
| W38346_T5 (SEQ ID NO:41) |

**Table 39 - Proteins of interest**

| **Protein Name** | **Corresponding Transcript(s)** |
|---|---|
| W38346_P3 (SEQ ID NO:42) | W38346_T0 (SEQ ID NO9:38); W38346_T1 (SEQ ID NO:39) |
| W38346_P4 (SEQ ID NO:45) | W38346_T2 (SEQ ID NO:40) |
| W38346_P7 (SEQ ID NO:46) | W38346_T5 (SEQ ID NO:41) |

These sequences are variants of the known hypothetical protein LOC201799 (SEQ ID NO:42) (SwissProt accession identifier NP_689893; synonims: TMEM154).

TMEM154 (transmembrane protein 154) was identified in 2 full length cDNA projects (Strausberg *et al.* 2002, PNAS 99(26): 16899-903; Ota et al. 2004, Nat Genet 36(1): 40-5). However no research was published about TMEM154 specifically.

Sequence corresponding to W38346_P3 (SEQ ID NO:42) has been reported in WO2004110369 patent application, which purports that sequence of FLJ32028, corresponding to W38346_P3 (SEQ ID NO:42), is associated with human chronic lymphocytic leukemia. The application further relates to monoclonal antibodies and methods for antibody screening and production and their use as diagnostic marker or therapeutic target for B-CLL. However, The WO2004110369 patent application does not provide any specific teaching or incentive that would direct a skilled artisan to use antibodies specific to the TMEM154 for the treatment or diagnosis of lymphoma, especially Non-Hodgkin's Lymphoma, anti CD20 (i.e. Rituximab) resistant lymphoma, Multiple Myeloma, kidney cancer or pancreatic cancer, and/or immune related conditions, particularly SLE. The WO2004110369 patent application does not teach also, that TMEM154 soluble ectodomain, as well as fragments thereof and conjugates and antibodies against it can be used as therapeutic or diagnostic agents for treatment of of lymphoma, especially Non-Hodgkin's Lymphoma, anti CD20 (i.e. Rituximab) resistant lymphoma, Multiple Myeloma, kidney cancer or pancreatic cancer, and/or immune related conditions, particularly SLE.

PRO92173, corresponding to W38346_P3 (SEQ ID NO:42) protein, has been reported in WO2004081199 patent application, among other genes showing altered patterns of expression in autoimmune diseases for use in diagnosis, prevention and treatment thereof. The WO2004081199 patent application does not teach however, that that sequence corresponding to PRO92173 differentially expressed in systemic lupus erythematosus (SLE), and/or in cancer, especially in lymphoma, especially Non-Hodgkin's Lymphoma, anti CD20 (i.e. Rituximab) resistant lymphoma, Multiple Myeloma, kidney cancer or pancreatic cancer. The WO2004081199 patent application does not teach also, that that sequence corresponding to PRO92173 can be used as drug target for treatment of SLE, and/or of cancer, including lymphoma, especially Non-Hodgkin's Lymphoma, anti CD20 (i.e. Rituximab) resistant lymphoma, Multiple Myeloma, kidney cancer or pancreatic cancer, and/or diagnosis thereof. The WO2004081199 patent application does not teach also, that PRO92173 soluble ectodomain, as well as fragments thereof and conjugates and antibodies against it can be used as therapeutic or diagnostic agents for treatment of cancer including lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE.

TMEM154 sequence corresponding to W38346_P3 (SEQ ID NO:42) has been also reported in WO03090694 patent application, among other genes used for diagnosing or monitoring autoimmune and chronic inflammatory diseases. The WO03090694 patent application does not teach however, that sequence corresponding to TMEM154 can be used as drug targets for treatment of immune related conditions, particularly SLE. The WO03090694 patent application does not teach also, that sequence corresponding to TMEM154 are differentially expressed in cancer, especially in lymphoma, especially Non-Hodgkin's Lymphoma, anti CD20 (i.e. Rituximab) resistant lymphoma, Multiple Myeloma, kidney cancer or pancreatic cancer. The WO03090694 patent application does not teach also, that sequences corresponding to TMEM154 can be used as drug targets for treatment of lymphoma, especially Non-Hodgkin's Lymphoma, anti CD20 (i.e. Rituximab) resistant lymphoma, Multiple Myeloma, kidney cancer or pancreatic cancer, or diagnosis thereof. The WO03090694 patent application does not teach also, that TMEM154 soluble ectodomain, as well as fragments thereof and conjugates and antibodies against it can be used as therapeutic or diagnostic agents for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE.

TMEM154 antigen corresponding to W38346_P3 (SEQ ID NO:42) has been reported in WO06020266 patent application, which purports to disclose polypeptides and antibodies derived from chronic lymphocytic leukemia cells, and uses thereof. WO06020266 does not teach however, that sequences corresponding to TMEM154 can be used as drug targets for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE. The WO06020266 patent application does not provide any specific teaching or incentive that would direct a skilled artisan to use antibodies specific to the TMEM154 for the treatment or diagnosis of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE. The WO06020266 patent application does not teach also, that TMEM154 soluble ectodomain, as well as fragments thereof and conjugates and antibodies against it can be used as therapeutic or diagnostic agents for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus).

TMEM154 antigen corresponding to W38346_P3 (SEQ ID NO:42) has been reported in WO2008112177, WO200270539 and EP1293569 patent applications. TMEM154 antigen corresponding to W38346_P4 (SEQ ID NO:45) has been reported in WO2008112177 patent application. However, none of these patent applications teaches that sequences corresponding to TMEM154 can be used as drug targets for treatment or diagnosing of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus). These applications do not teach also, that TMEM154 soluble ectodomain, as well as fragments thereof and conjugates and antibodies against it can be used as therapeutic or diagnostic agents for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus).

TMEM154 antigen corresponding to W38346_P7 (SEQ ID NO:46) has been reported by the applicants of the present application in US patent application No: 11/043,860. However, there is no teaching in US 11/043,860 application that W38346_P7 (SEQ ID NO:46) soluble ectodomain, as well as fragments thereof and specific antibodies against it can be used as therapeutic or diagnostic agents for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus).

In particular this invention uses TMEM154 antigen and discrete portions thereof as a drug target for therapeutic small molecules, peptides, antibodies, antisense RNAs, siRNAs, ribozymes, and the like. More particularly the invention relates to diagnostic and therapeutic polyclonal and monoclonal antibodies and fragments thereof that bind TMEM154, and portions and variants thereof. According to at least some embodiments of the invention there is a use antibodies and antibody fragments against TMEM154 antigen, its secreted or soluble form or ECD and/or variants, conjugates, or fragments thereof and fragments and variants thereof for treating and diagnosing cancer, especially for treatment of lymphoma, especially Non-Hodgkin's Lymphoma, anti CD20 (i.e. Rituximab) resistant lymphoma, Multiple Myeloma, kidney cancer or pancreatic cancer, and/or immune related conditions, especially SLE (systemic lupus erythematosus), wherein this antigen is differentially expressed.

As noted above, cluster W38346 features 4 transcripts, which were listed in Table 38 above. These transcripts encode for proteins which are variants of protein hypothetical protein LOC201799 (SEQ ID NO:42). A description of each variant protein according to at least some embodiments of the invention is now provided.

Variant protein W38346_P3 (SEQ ID NO:42) according to at least some embodiments of the invention has an amino acid sequence as encoded by transcriptsW38346_T0 (SEQ ID NO:38) and W38346_T1 (SEQ ID NO:39).

The localization of the variant protein was determined according to results from a number of different software programs and analyses, including analyses from SignalP and other specialized programs. The variant protein is believed to be located as follows with regard to the cell: membrane.

The coding portion of transcript W38346_T0 (SEQ ID NO:38) starts at position 233 and ends at position 781. The transcript also has the following SNPs as listed in Table 40 (given according to their position on the nucleotide sequence, with the alternative nucleic acid listed).

**Table 40 - Nucleic acid SNPs**

| **Polymorphism** | **SNP position(s) on nucleotide sequence** |
|---|---|
| C -> T | 511 |
| A -> C | 1104 |
| A -> T | 2446 |
| A -> G | 2446, 3013, 3075 |
| C -> A | 2611,3030 |
| C -> G | 2611 |

The coding portion of transcript W38346_T1 (SEQ ID NO:39) starts at position 233 and ends at position 781. The transcript also has the following SNPs as listed in Table 41 (given according to their position on the nucleotide sequence, with the alternative nucleic acid listed).

**Table 41 - Nucleic acid SNPs**

| **Polymorphism** | **SNP position(s) on nucleotide sequence** |
|---|---|
| C -> T | 511 |
| A -> C | 1104 |
| A -> T | 2446 |
| A -> G | 2446 |
| C -> A | 2611 |
| C -> G | 2611 |

Variant protein W38346_P4 (SEQ ID NO:45) according to at least some embodiments of the invention has an amino acid sequence as encoded by transcripts W38346_T2 (SEQ ID NO:40). A description of the relationship of the variant protein according to at least some embodiments of the invention to known proteins is as follows:

### 2. Comparison report between W38346_P4 and known proteins Q96MQ8_HUMAN:

A. An isolated chimeric polypeptide encoding for W38346_P4, comprising a amino acid sequence being at least 90% homologous to MVLIPLILLVLLLLSVVFLATYYKRKRTKQEPSSQGSQSALQTYELGSENVKVPIFEE DTPSVMEIEMEELDKWMNSMNRNADFECLPTLKEEKESNHNPSDSES corresponding to amino acids 79 - 183 of known proteins Q96MQ8_HUMAN, which also corresponds to amino acids 1 - 105 of W38346_P4.

The localization of the variant protein was determined according to results from a number of different software programs and analyses, including analyses from SignalP and other specialized programs. The variant protein is believed to be located as follows with regard to the cell: secreted.

The coding portion of transcript W38346_T2 (SEQ ID NO:40) starts at position 516 and ends at position 830. The transcript also has the following SNPs as listed in Table 42 (given according to their position on the nucleotide sequence, with the alternative nucleic acid listed).

**Table 42 - Nucleic acid SNPs**

| **Polymorphism** | **SNP position(s) on nucleotide sequence** |
|---|---|
| C -> T | 560 |
| A -> C | 1153 |
| A -> T | 2495 |
| A -> G | 2495, 3062, 3124 |
| C -> A | 2660,3079 |
| C -> G | 2660 |

Variant protein W38346_P7 (SEQ ID NO:46) according to at least some embodiments of the invention has an amino acid sequence as encoded by transcripts W38346_T5 (SEQ ID NO:41). A description of the relationship of the variant protein according to at least some embodiments of the invention to known proteins is as follows:
1. Comparison report between W38346_P7 (SEQ ID NO:46) and known proteins Q96MQ8_HUMAN and NP_689893:
   A. An isolated chimeric polypeptide, comprising a first amino acid sequence being at least 90% homologous to MQAPRAALVFALVIALVPVGRGNYEELENSGDTTVESERPNKVTIPSTFAAVTIKET LNANINSTNFAPDENQLEFILMVLIPLILLVLLLLSVVFLATYYKRKRTKQEPSSQGS QSALQT corresponding to amino acids 1 - 121 of known proteins Q96MQ8_HUMAN and NP_689893, which also corresponds to amino acids 1 - 121 of W38346_P7 (SEQ ID NO:46), and a second amino acid sequence being at least 70%, optionally at least 80%, preferably at least 85%, more preferably at least 90% and most preferably at least 95% homologous to a polypeptide having the sequence CKIQLSWKVIPAFCLESSHRNAL (SEQ ID NO: 162) corresponding to amino acids 122 - 144 of W38346_P7 (SEQ ID NO:46), wherein said first amino acid sequence and second amino acid sequence are contiguous and in a sequential order.
   B. An isolated polypeptide of an edge portion of W38346_P7 (SEQ ID NO:46), comprising an amino acid sequence being at least 70%, optionally at least about 80%, preferably at least about 85%, more preferably at least about 90% and most preferably at least about 95% homologous to the sequence CKIQLSWKVIPAFCLESSHRNAL (SEQ ID NO: 162) of W38346_P7 (SEQ ID NO:46).
2. Comparison report between W38346_P7 (SEQ ID NO:46) and known protein Q6P9G4_HUMAN (SEQ ID NO:44):
   A. An isolated chimeric polypeptide, comprising a first amino acid sequence being at least 90% homologous to MQAPRAALVFALVIALVPVGRGNYEELENSGDTTVESERPNKVTIPSTFAAVTIKET LNANINSTNFAPDENQLEFILMVLIPLILLVLLLL corresponding to amino acids 1 - 92 of known protein Q6P9G4_HUMAN (SEQ ID NO:44), which also corresponds to amino acids 1 - 92 of W38346_P7 (SEQ ID NO:46), a bridging amino acid S corresponding to amino acid 93 of W38346_P7 (SEQ ID NO:46), a second amino acid sequence being at least 90% homologous to VVFLATYYKRKRTKQEPSSQGSQSALQT corresponding to amino acids 94 - 121 of known protein Q6P9G4_HUMAN (SEQ ID NO:44), which also corresponds to amino acids 94 - 121 of W38346_P7 (SEQ ID NO:46), and a third amino acid sequence being at least 70%, optionally at least 80%, preferably at least 85%, more preferably at least 90% and most preferably at least 95% homologous to a polypeptide having the sequence CKIQLSWKVIPAFCLESSHRNAL (SEQ ID NO: 162) corresponding to amino acids 122 - 144 of W38346_P7 (SEQ ID NO:46), wherein said first amino acid sequence, bridging amino acid, second amino acid sequence and third amino acid sequence are contiguous and in a sequential order.
   B. An isolated polypeptide of an edge portion of W38346_P7 (SEQ ID NO:46), comprising an amino acid sequence being at least 70%, optionally at least about 80%, preferably at least about 85%, more preferably at least about 90% and most preferably at least about 95% homologous to the sequence CKIQLSWKVIPAFCLESSHRNAL (SEQ ID NO: 162) of W38346_P7 (SEQ ID NO:46).

The localization of the variant protein was determined according to results from a number of different software programs and analyses, including analyses from SignalP and other specialized programs. The variant protein is believed to be located as follows with regard to the cell: membrane.

Variant protein W38346_P7 (SEQ ID NO:46) also has the following non-silent SNPs (Single Nucleotide Polymorphisms) as listed in Table 43, (given according to their position(s) on the amino acid sequence, with the alternative amino acid(s) listed).

**Table 43 - Amino acid mutations**

| **SNP position(s) on amino acid sequence** | **Alternative amino acid(s)** |
|---|---|
| 137 | E -> D |

The coding portion of transcript W38346_T5 (SEQ ID NO:41) starts at position 233 and ends at position 664. The transcript also has the following SNPs as listed in Table 44 (given according to their position on the nucleotide sequence, with the alternative nucleic acid listed).

**Table 44 - Nucleic acid SNPs**

| **Polymorphism** | **SNP position(s) on nucleotide sequence** |
|---|---|
| C -> T | 511 |
| A -> C | 643 |
| G -> | 701 |
| A -> G | 703 |
| T -> A | 738 |

### EXAMPLE 6_2: EXPRESSION ANALYSIS OF TMEM154 TRANSCRIPTS

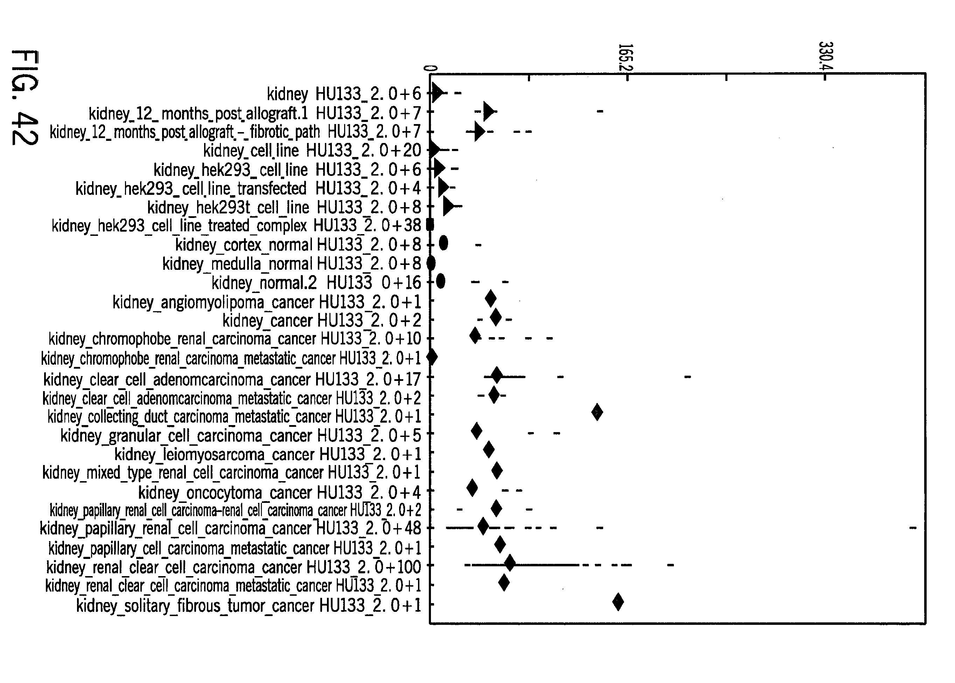

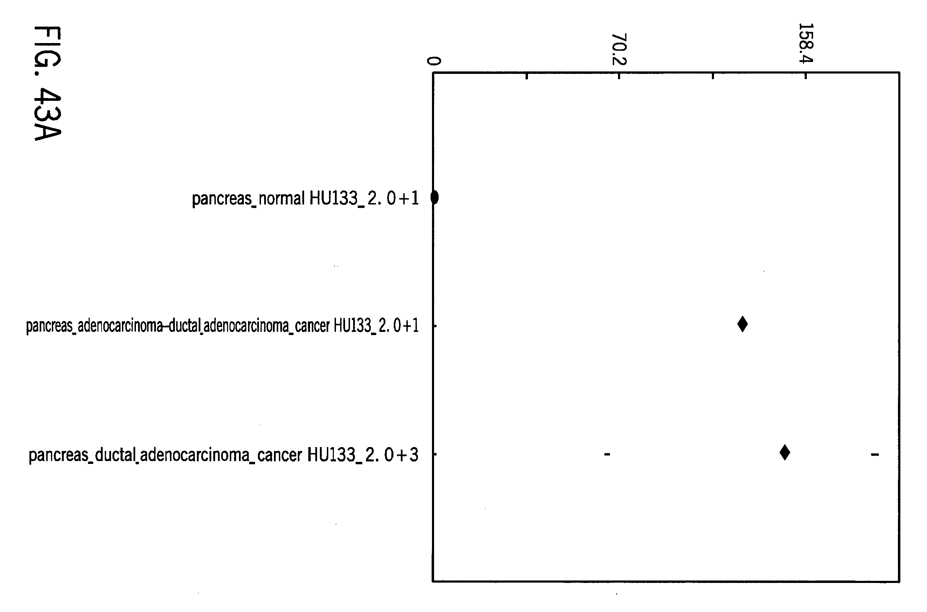

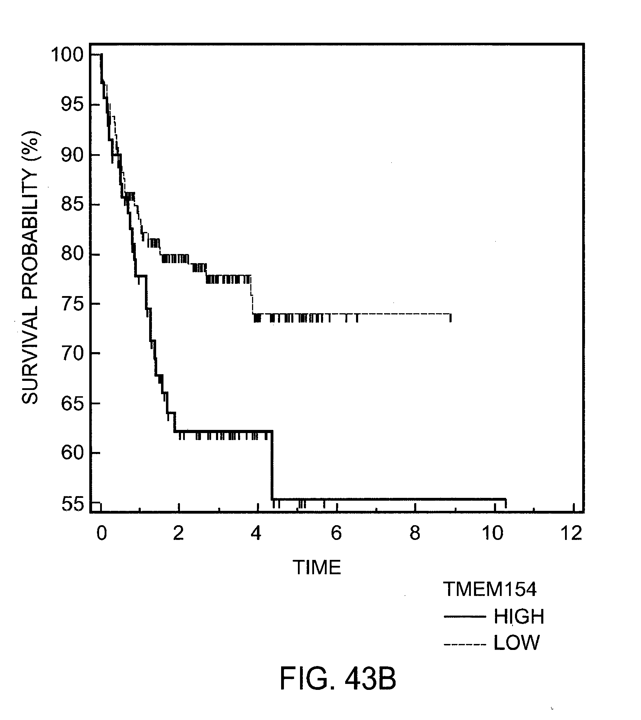

TMEM154 transcripts were also found to be over expressed in kidney cancer, as is demonstrated in Figure 42 and in pancreatic cancer, as is demonstrated in Figure 43. Figures 42-43 show expression graphs of Affymetrix probe set 238063_at. Figure 42 shows the expression of TMEM154 transcripts in microarray chips from kidney cancer and normal kidney experiments. As can be seen TMEM154 transcripts are overexpressed in kidney cancer tissues (diamond markers) relative to its expression in normal kidney (circle and triangle markers).

Figure 43A shows the expression of TMEM154 transcripts in microarray chips from pancreatic cancer and normal pancreas experiments. As can be seen TMEM154 transcripts are overexpressed in pnacreatic cancer tissues (diamond markers).

Figure 43B shows Kaplan-Meier survival curves of the high expression of the TMEM154 probe (238063_at, samples over median expression), and low expression (below median) in a samples from Rituximab treated DLBCL (Diffuse large B-cell lymphoma). It is evident that TMEM154 high expression is corelated with poor survival, and hance can serve as a potential treatment for anti CD20 resistant lymphoma paetients. In Figure 43B the time scale is shown in years; solid line represents high TMEM154 expression; fragmented line represents low TMEM154 expression.

### Expression of hypothetical protein FLJ32028, TMEM154 W38346 transcripts which are detectable by amplicon as depicted in sequence name W38346_seg6-20F1R1 (SEQ ID NO: 106) in different normal tissues and blood specific panel

Expression of hypothetical protein FLJ32028, TMEM154 transcripts detectable by or according to seg6-20F1R1 - W38346_seg6-20F1R1 (SEQ ID NO: 106) amplicon and primers W38346_seg6-20F (SEQ ID NO: 104) and W38346_seg6-20R (SEQ ID NO: 105) was measured by real time PCR in blood panel and normal panel. The samples used for blood panel are detailed in Tables 2 and 2_1. The samples used for normal panel are detailed in Table 3.

### Normal panel -

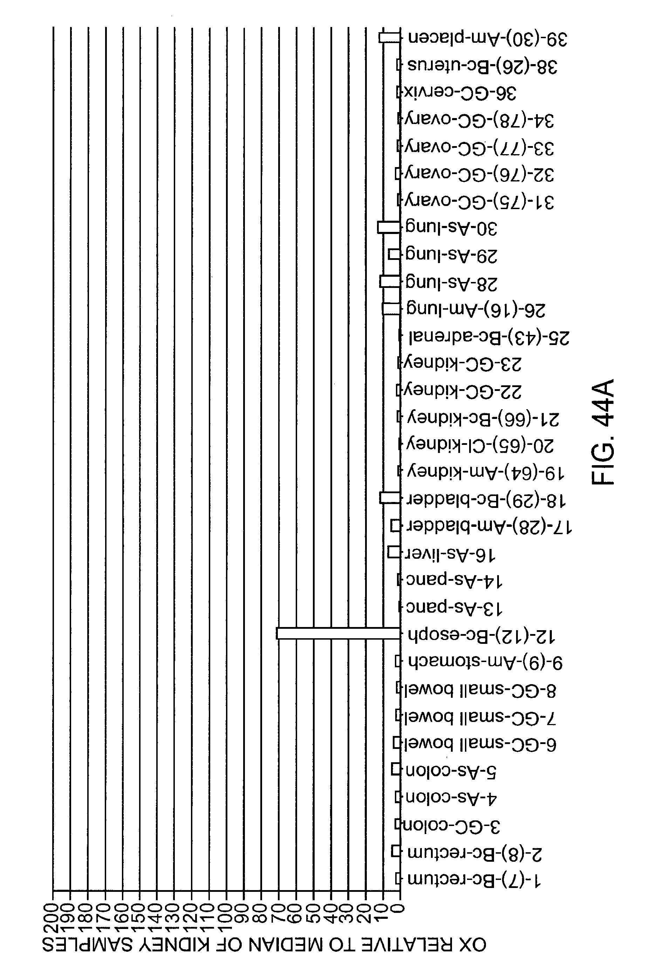

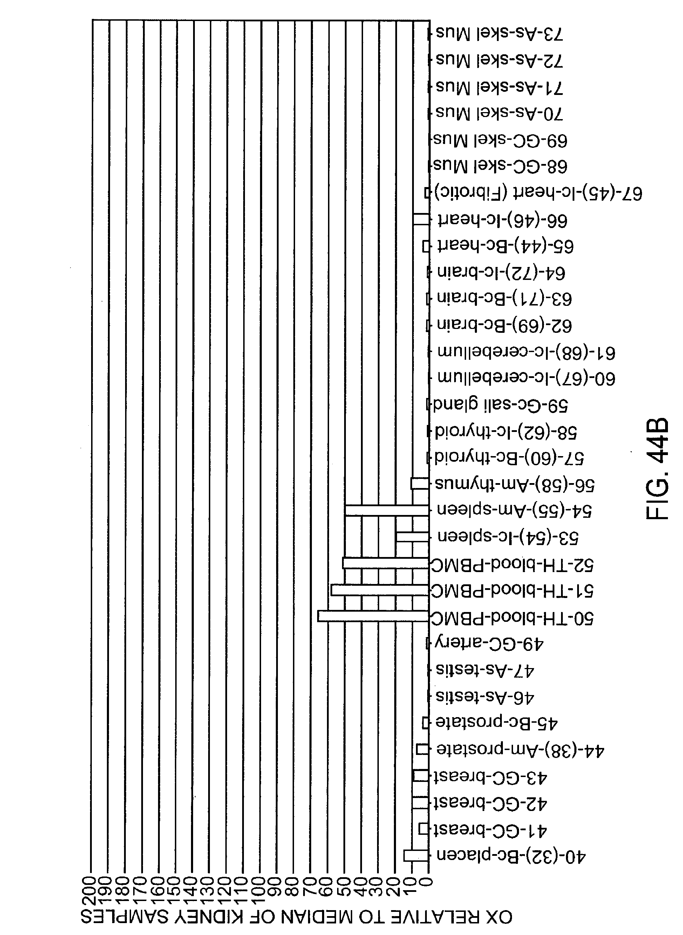

For each RT sample, the expression of the above amplicon was normalized to the normalization factor calculated from the expression of several house keeping genes as described in example 1. The normalized quantity of each RT sample was then divided by the median of the quantities of the kidney samples (sample numbers 19-23, Table 3 above), to obtain a value of relative expression of each sample relative to median of the kidney samples, as shown in Figure 44. High expression was observed in normal PBMCs, spleen and esophagus.

For blood panel - For each RT sample, the expression of the above amplicon was normalized to the normalization factor calculated from the expression of several house keeping genes as described in example 1. The normalized quantity of each RT sample was then divided by the median of the quantities of the kidney normal samples (sample numbers 65-67, Table 2 above), to obtain a value of relative expression of each sample relative to median of the kidney normal samples.

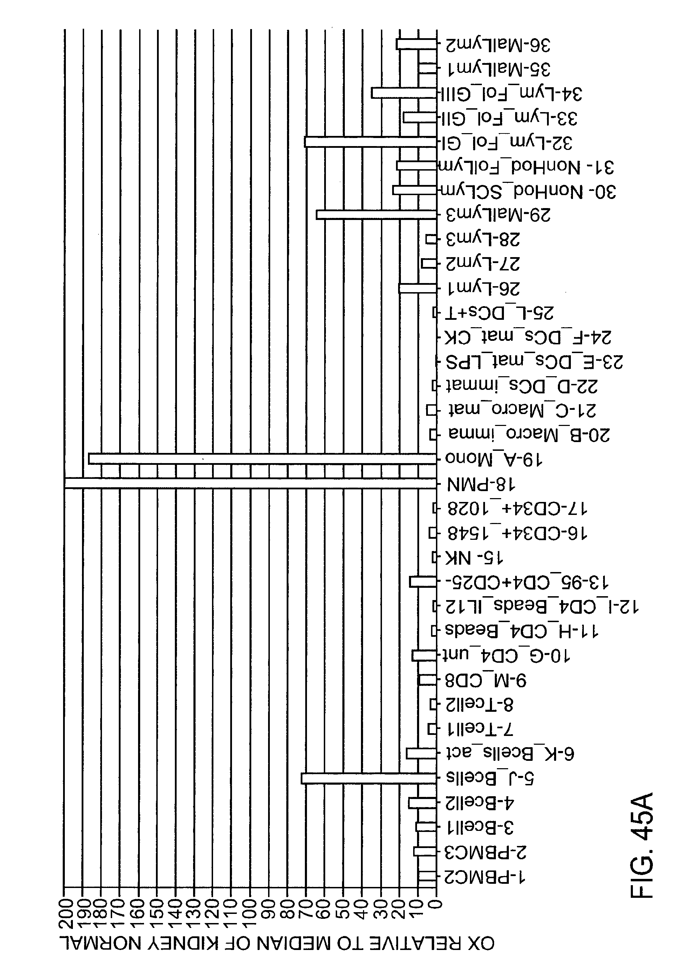

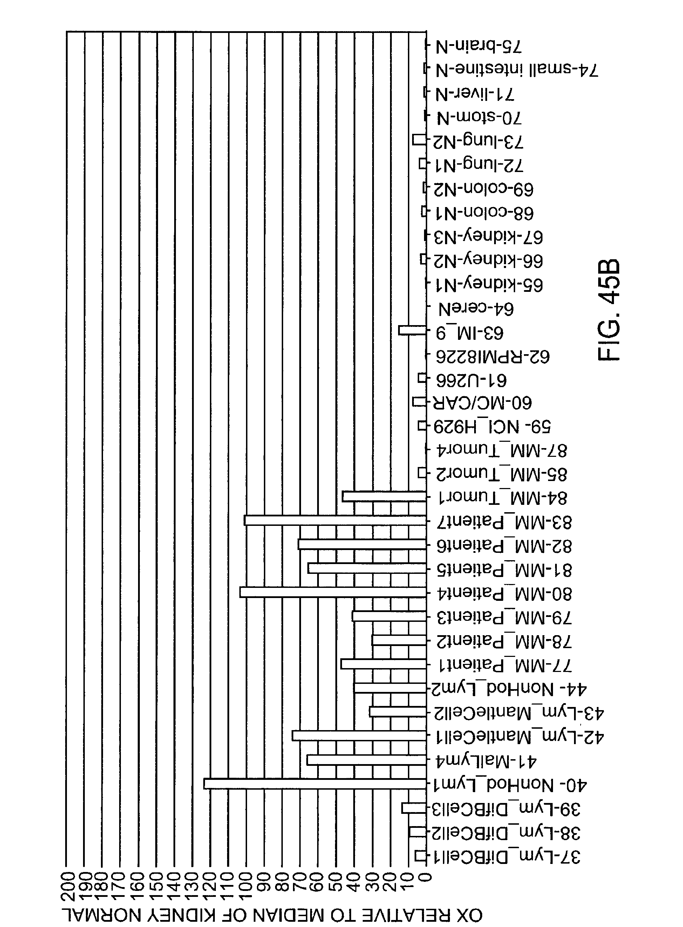

The results of this analysis are depicted in the histogram in Figure 45. Expression of the above-indicated hypothetical protein FLJ32028, TMEM154 transcripts is high in PMN, monocytes, multiple myeloma patients and several lymphomas samples.
Forward Primer (W38346_seg6-20F) (SEQ ID NO:104):
CCTTCTAGCCAAGGATCTCAGAGTG
Reverse Primer (W38346_seg6-20R) (SEQ ID NO: 105):
CTTGGGTTGTGATTTGATTCCTTCTC
Amplicon (W38346_seg6-20F1R1 (SEQ ID NO: 106)) (SEQ ID NO:106):

### EXAMPLE 6_3: CLONING OF CGEN928 TMEM154_T0P3 ORF FUSED TO FLAG TAG

Cloning of TMEM154_T0_P3 open reading frame (ORF) fused to FLAG was carried out by RT PCR as described below.

RT18 PMNs (RT-PCR product resulted from sample 18, Table 2) and RT19 monocytes (RT-PCR product resulted from sample 19, Table 2) from the blood panel were diluted 1:20 in TE buffer (10mM Tris, 1 mM EDTA pH 8) and served as a template for PCR.

PCR was done using GoTaq ReadyMix (Promega, catalog number M122) under the following conditions: 10µl - cDNA described above; 1.5 µl - H2O; and 0.5µl (10µM) - of each primer #100-952 (SEQ ID NO:187) and #100-953 (SEQ ID NO:188) in a total reaction volume of 25µl; with a reaction program of 2 minutes in 94°C; 35 cycles of: 30 seconds at 94°C, 30 seconds at 55°C, 1 minute at 72°C; then 10 minutes at 72°C. Primers which were used include gene specific sequences; restriction enzyme sites; Kozak sequence and FLAG tag.

5µl of PCR product were loaded onto a 1.2% agarose gel stained with ethidium bromide, electrophoresed in 1xTAE solution at 100V, and visualized with UV light. After verification of expected band size, PCR product was purified using QiaQuick™ PCR Purification kit (Qiagen, catalog number: 28004). The purified PCR product was digested with NheI and AgeI restriction enzymes (New England Biolabs, Beverly, MA, U.S.A.). After digestion, DNA was loaded onto a 1.2 % agarose gel as described above. The expected band size was excised and extracted from the gel using QiaQuick™ Gel Extraction kit (Qiagen, catalog number: 28707). The digested DNA was then ligated into pIRESpuro3 vector, previously digested with the above restriction enzymes, using LigaFastTM Rapid DNA Ligation System (Promega, catalog number: M8221). The resulting DNA was transformed into competent E.Coli bacteria DH5α (RBC Bioscience, Taipei, Taiwan, catalog number: RH816) according to manufacturer's instructions, then plated on LB-ampicillin agar plates for selection of recombinant plasmids, and incubated overnight at 37°C. The following day, positive colonies were screened by PCR using pIRESpuro3 vector specific primer and gene specific primer (data not shown). The PCR product was analyzed using 1.2% agarose gel as described above. After verification of expected band size, positive colonies were grown in 5 ml Terrific Broth supplemented with 100µg/ml ampicillin, with shaking overnight at 37°C. Plasmid DNA was isolated from bacterial cultures using Qiaprep™ Spin Miniprep Kit (Qiagen, catalog number: 27106). Accurate cloning was verified by sequencing the inserts (Weizmann Institute, Rehovot, Israel). Upon verification of an error-free colony (i.e. no mutations within the ORF), recombinant plasmids were processed for further analyses.

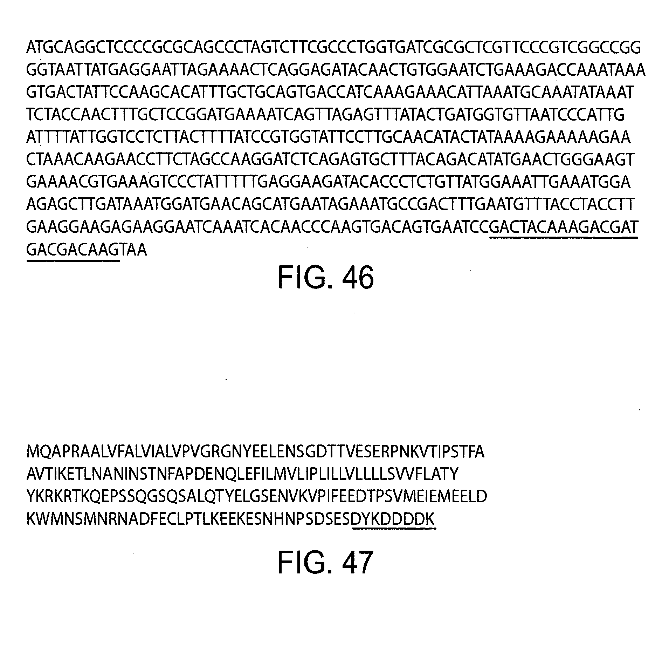

The DNA sequence of the resulting TMEM154_T0_FLAG (SEQ ID NO:189) is shown in Figure 46; FLAG sequence is in underlined.

The amino acid sequence of TMEM154_P3_FLAG (SEQ ID NO:190) is shown in Figure 47; FLAG sequence is in underlined.

### EXAMPLE 6_4: DETERMINING CELL LOCALIZATION OF TMEM154_P3

In order to determine TMEM154_P3 cellular localization, TMEM154_T0_P3 was cloned in frame to FLAG tag, as described above. Protein localization was observed upon transient transfection (Chen et al., Molecular Vision 2002; 8; 372-388) using confocal microscopy. 48 hours following transfection, the cells were stained with anti FLAG antibodies conjugated to Cy-3 flurophore and were observed for the presence of fluorescent signal.

TMEM154_T0_P3_FLAG (SEQ ID NO:189) pIRESpuro3 construct was transiently transfected into HEK-293T cells as described above. 48 hours post transient transfection, cells on coverslip were further processed for immunostaining and analysis by confocal microscopy. The cover slip was washed in phosphate buffered saline (PBS), then fixed for 15 minutes with a solution of 3.7% paraformaldehyde (PFA) (Sigma, catalog number: P-6148)/3% glucose (Sigma, catalog number: G5767) (diluted in PBS). Quenching of PFA was done by a 5 minute incubation in 3mM glycine (Sigma, catalog number: G7126) (diluted in PBS). After two 5-minute washes in PBS, cells were permeabilized with 0.1% triton-X100 (diluted in PBS) for 5 minutes. After two 5-minute washes in PBS, blocking of non-specific regions was done with 5% bovine serum albumin (BSA) (Sigma, catalog number: A4503) (diluted in PBS) for 20 minutes. The coverslip was then incubated, in a humid chamber for 1 hour, with mouse anti FLAG-Cy3 antibodies (Sigma, catalog number: A9594), diluted 1:100 in 5% BSA in PBS, followed by three 5-minute washes in PBS. The coverslip was then mounted on a slide with Gel Mount Aqueous medium (Sigma, catalog number: G0918) and cells were observed for the presence of fluorescent product using confocal microscopy.

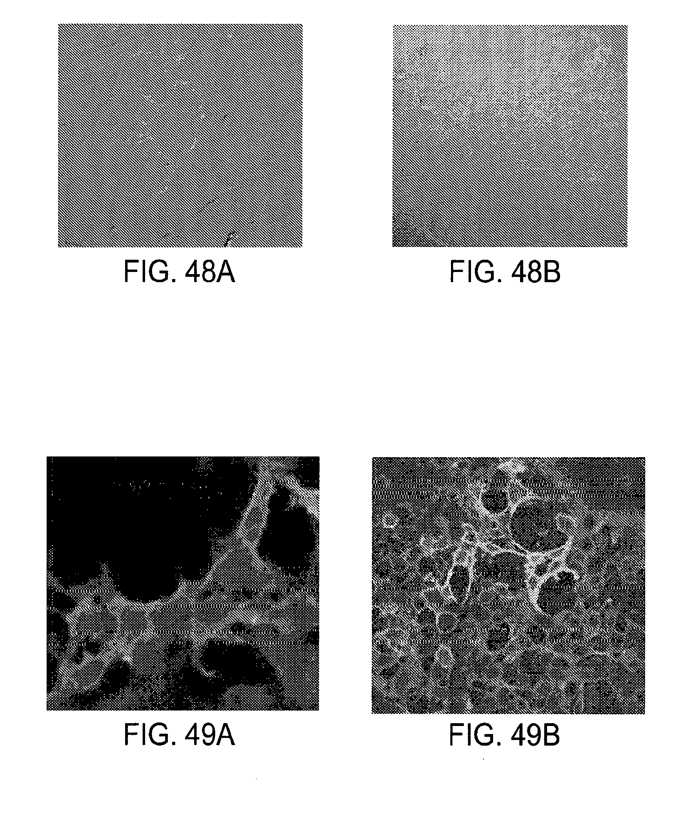

Cell localization is shown in Figure 48. TMEM154_P3 is localized to the cell membrane.

### EXAMPLE 6_5: PRODUCTION OF POLYCLONAL ANTIBODIES SPECIFIC TO TMEM154_P3 PROTEIN

All polyclonal Abs production procedure, including peptides synthesis, peptides conjugation, animal immunizations, bleeding and antibodies purification were performed at Sigma-Aldrich (Israel). Two pairs of rabbits (one pair per epitope) were injected to prepare antibodies for TMEM154_P3 (rabbit numbers 6285 and 6286, 6248 and 6249 respectively). All animal care, handling and injections were performed by Sigma (Israel).

Peptides which were used for rabbit immunization were as follows: RGNYEELENSGDTTVESER designated TM21 (SEQ ID NO:191) a sequence taken from the N' terminus corresponding to amino acids 21-39 of TMEM154_P3 protein (SEQ ID NO:42). The second peptide sequence to be used was: YKRKRTKQEPSSQGSQS designated TM101 (SEQ ID NO:192), a sequence taken from the C' terminus, corresponding to amino acids 101-117 of TMEM154_P3 protein (SEQ ID NO:42). 25mg of each peptide were synthesized with 95% purity of which 10mg were conjugated to KLH carrier. Each pair of rabbits was immunized with the corresponding conjugated peptide as follows: rabbits 6285 and 6286 were immunized with TM21 peptide (SEQ ID NO:191), and rabbits 6248 and 6249 were immunized with T101 peptide (SEQ ID NO:192). Animals were immunized every two weeks. 60ml production bleeds from each rabbit were collected and affinity purification was performed with the peptide against which the respective antibodies were raised.

### EXAMPLE 6_6: CHARACTERIZATION OF PURIFIED TMEM154_P3 ANTIBODIES BY IMMUNOSTAINING OF TMEM154 TRANSFECTED CELLS

In order to further characterize the affinity purified antibodies raised against TMEM154_P3, antibody-protein interaction was studied using immunostaining of TMEM154_P3 stable transfected HEK293T cells.

### GENERATION OF STABLE POOL EXPRESSING TMEM154_P3 PROTEIN:

Two stably transfected pool were generated, TMEM154_P3 pIRESpuro3 and the negative control empty pIRESpuro3. Both constructs were transfected into HEK-293T cells as previously described.

### IMMUNOSTAINING OF TMEM154 TRANSFECTED CELLS

500,000 cells per well of HEK-293T (ATCC, CRL-11268) stably expressing TMEM154 or the empty vector pIRES puro3, described above, were plated on sterile glass coverslips, 13mm diameter (Marienfeld, catalog number: 01 115 30), which were placed in a 6 well plate, using 2ml pre-warmed DMEM [Dulbecco's modified Eagle's Media, Biological Industries (Beit Ha'Emek, Israel), catalog number: 01-055-1A] + 10% FBS [Fetal Bovine Serum, Biological Industries (Beit Ha'Emek, Israel), catalog number: 04-001-1A] + 4mM L-Glutamine [Biological Industries (Beit Ha'Emek, Israel), catalog number: 03-020-1A].

48 hours post plating the cells on coverslips they were further processed for immunostaining and analysis by confocal microscopy. The cover slips were washed in phosphate buffered saline (PBS), then fixed for 25 minutes with a 3.7% paraformaldehyde (PFA) (Sigma, catalog number: P-6148)/3% glucose (Sigma, catalog number: G5767). After 2 5-minute washes in PBS, cells were permeabilized with 0.1% triton-X100 (diluted in PBS) for 5 minutes. After two 5-minute washes in PBS, blocking of non-specific regions was done with 5% bovine serum albumin (BSA) (Sigma, catalog number: A4503) (diluted in PBS) for 20 minutes. The coverslips were then incubated, in a humid chamber for 1 hour, with purified rabbit anti-TMEM154 antibodies described above: TM21 (Rabbit 6285, 6286 1mg/ml) was diluted 1:1000 in 5% BSA in PBS and TM101 (Rabbit 6248 6249, 1mg/ml) was diluted 1:1000 in 5% BSA. The antibodies were washed 3 times for 5-minutes in PBS. The coverslips were then incubated, in a humid chamber for 1 hour, with secondary antibody: donkey anti-rabbit conjugated to Cy-3 flurophore (Jackson ImmunoResearch, catalog number: 711-165-152), diluted 1:200 in 3% BSA in PBS. After 3 5-minute washes in PBS, the fixed coverslips were mounted on slides with Gel Mount Aqueous medium (Sigma, catalog number: G0918) and cells were observed for the presence of fluorescent product using confocal microscopy.

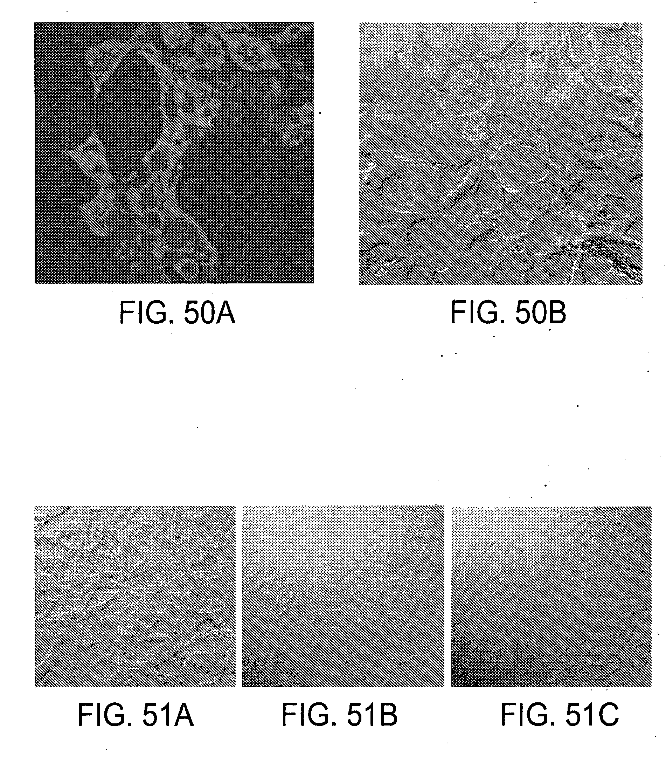

Specific cell staining localized to the cell membrane was observed using purified TM21 and TM101 antibodies on TMEM154 transfected cells (Figure 49 and Figure 50 respectively), while, no staining was observed using these antibodies on the negative control pIRESpuro3 HEK-293T transfected cells (Figure 51). The red fluorescence obtained in Figures 49 and 50 as opposed to the absence of signal in Figure 51 demonstrates the specificity of TM21 and TM101 antibodies to TMEM154_P3 (SEQ ID NO:42).

### EXAMPLE 6_7: DEMONSTRATION OF ENDOGENOUS EXPRESSION OF TMEM154_P3 BY IMMUNOSTAINING OF LYMPHOBLAST CELL LINES

In order to determine endogenous expression of TMEM154, three cell lines were selected for immustaining using specific antibodies against TMEM154_P3 protein described above.

500000 cells from each cell line: Ramos (ATCC cat no CRL-1923), CESS (ATCC cat no TIB-190), Daudi (ATCC cat no CCL-213) were fixed with 3.7% PFA containing 3% Glucose and plated on coverslips previously treated with poly-L-Lysin 0.01% (Sigma cat no P4832). Cells were further processed for immunostaining as describe above and analyzed by confocal microscopy.

Specific cell staining localized to the cell membrane was observed using purified TM21 and TM101 antibodies on all three cell lines as shown in Figure 52.

### EXAMPLE 7

### DEVELOPMENT OF FULLY HUMAN ANTI-TMEM154 ANTIBODIES

Generation Of Human Monoclonal Antibodies Against TMEM154 Antigen

Fusion proteins composed of the extracellular domain of the TMEM154 linked to an IgG2 Fc polypeptide are generated by standard recombinant methods and used as antigen for immunization.

### Transgenic HuMab Mouse.

Fully human monoclonal antibodies to TMEM154 are prepared using mice from the HCo7 strain of the transgenic HuMab Mouse®, which expresses human antibody genes. In this mouse strain, the endogenous mouse kappa light chain gene has been homozygously disrupted as described in Chen et al. (1993) EMBO J. 12:811-820 and the endogenous mouse heavy chain gene has been homozygously disrupted as described in Example 1 of PCT Publication WO 01/09187. Furthermore, this mouse strain carries a human kappa light chain transgene, KCo5, as described in Fishwild et al. (1996) Nature Biotechnology 14:845-851, and a human heavy chain transgene, HCo7, as described in U.S. Pat. Nos. 5,545,806; 5,625,825; and 5,545,807.

### HuMab Immunizations:

To generate fully human monoclonal antibodies to TMEM154 polypeptides, mice of the HCo7 HuMab Mouse® strain can be immunized with purified recombinant TMEM154 fusion protein derived from mammalian cells that are transfected with an expression vector containing the gene encoding the fusion protein. General immunization schemes for the HuMab Mouse® are described in Lonberg, N. et al (1994) Nature 368(6474): 856-859; Fishwild, D. et al. (1996) Nature Biotechnology 14: 845-851 and PCT Publication WO 98/24884. The mice are 6-16 weeks of age upon the first infusion of antigen. A purified recombinant TMEM154 antigen preparation (5-50 micro-grams, purified from transfected mammalian cells expressing TMEM154 fusion protein) is used to immunize the HuMab mice intraperitoneally.

Transgenic mice are immunized twice with antigen in complete Freund's adjuvant or Ribi adjuvant IP, followed by 3-21 days IP (up to a total of 11 immunizations) with the antigen in incomplete Freund's or Ribi adjuvant. The immune response is monitored by retroorbital bleeds. The plasma is screened by ELISA (as described below), and mice with sufficient titers of anti-TMEM154 human immunoglobulin are used for fusions. Mice are boosted intravenously with antigen 3 days before sacrifice and removal of the spleen.

### Selection of HuMab mice@ Producing Anti-TMEM154 Antibodies:

To select HuMab mice® producing antibodies that bind TMEM154 polypeptides, sera from immunized mice is tested by a modified ELISA as originally described by Fishwild, D. et al. (1996). Briefly, microtiter plates are coated with purified recombinant TMEM154 fusion protein at 1-2.mu.g/ml in PBS, 50.mu.l/wells incubated 4 degrees C. overnight then blocked with 200.mu.l/well of 5% BSA in PBS. Dilutions of plasma from TMEM154 -immunized mice are added to each well and incubated for 1-2 hours at ambient temperature. The plates are washed with PBS/Tween and then incubated with a goat-anti-human kappa light chain polyclonal antibody conjugated with alkaline phosphatase for 1 hour at room temperature. After washing, the plates are developed with pNPP substrate and analyzed by spectrophotometer at OD 415-650. Mice that developed the highest titers of anti-TMEM154 antibodies are used for fusions. Fusions are performed as described below and hybridoma supernatants are tested for anti-TMEM154 activity by ELISA.

### Generation Of Hybridomas Producing Human Monoclonal Antibodies To TMEM154 polypeptides

The mouse splenocytes, isolated from the HuMab mice, are fused with PEG to a mouse myeloma cell line based upon standard protocols. The resulting hybridomas are then screened for the production of antigen-specific antibodies. Single cell suspensions of splenic lymphocytes from immunized mice are fused to one-fourth the number of P3X63 Ag8.6.53 (ATCC CRL 1580) nonsecreting mouse myeloma cells with 50% PEG (Sigma). Cells are plated at approximately 1X10 -5 /well in flat bottom microtiter plate, followed by about two week incubation in selective medium containing 10% fetal calf serum, supplemented with origen (IGEN) in RPMI, L-glutamine, sodium pyruvate, HEPES, penicillin, streptamycin, gentamycin, 1x HAT, and beta-mercaptoethanol. After 1-2 weeks, cells are cultured in medium in which the HAT is replaced with HT. Individual wells are then screened by ELISA (described above) for human anti-TMEM154 monoclonal IgG antibodies. Once extensive hybridoma growth occurred, medium is monitored usually after 10-14 days. The antibody secreting hybridomas are replated, screened again and, if still positive for human IgG, anti-TMEM154 monoclonal antibodies are subcloned at least twice by limiting dilution. The stable subclones are then cultured in vitro to generate small amounts of antibody in tissue culture medium for further characterization.

### Hybridoma clones are selected for further analysis.

### Structural Characterization Of Desired anti-TMEM154Human Monoclonal Antibodies

The cDNA sequences encoding the heavy and light chain variable regions of the obtained anti-TMEM154 monoclonal antibodies are obtained from the resultant hybridomas, respectively, using standard PCR techniques and are sequenced using standard DNA sequencing techniques.

The nucleotide and amino acid sequences of the heavy chain variable region and of the light chain variable region are identified. These sequences may be compared to known human germline immunoglobulin light and heavy chain sequences and the CDRs of each heavy and light of the obtained anti-TMEM154 sequences identified.

Characterization Of Binding Specificity And Binding Kinetics Of anti-TMEM154 Human Monoclonal Antibodies

The binding affinity, binding kinetics, binding specificity, and cross-competition of anti-TMEM154 antibodies are examined by Biacore analysis. Also, binding specificity is examined by flow cytometry.

### Binding affinity and kinetics

anti-TMEM154 antibodies produced according to the invention are characterized for affinities and binding kinetics by Biacore analysis (Biacore AB, Uppsala, Sweden). Purified recombinant human TMEM154 fusion protein is covalently linked to a CM5 chip (carboxy methyl dextran coated chip) via primary amines, using standard amine coupling chemistry and kit provided by Biacore. Binding is measured by providing the antibodies in HBS EP buffer (provided by BIAcore AB) at a concentration of 267 nM and a flow rate of 50.mu.l/min. The antigen- antibody association kinetics are followed for 3 minutes and the dissociation kinetics are followed for 7 minutes. The association and dissociation curves are fit to a 1:1 Langmuir binding model using B1Aevaluation software (Biacore AB). To minimize the effects of avidity in the estimation of the binding constants, only the initial segments of data corresponding to association and dissociation phases are used for fitting.

### Epitope Mapping of Obtained anti-TMEM154 Antibodies

Biacore is used to determine epitope grouping of anti-TMEM154 antibodies are used to map their epitopes on the TMEM154 antigen, respectively. These different antibodies are coated on three different surfaces of the same chip to 8000 RUs each. Dilutions of each of the mAbs are made, starting at 10 mu.g/mL and is incubated with Fc fused TMEM154 (50 nM) for one hour. The incubated complex is injected over all the three surfaces (and a blank surface) at the same time for 1.5 minutes at a flow rate of 20.mu.L/min. Signal from each surface at end of 1.5 minutes, after subtraction of appropriate blanks, has been plotted against concentration of mAb in the complex. Upon analysis of the data, the anti-TMEM154 antibodies are categorized into different epitope groups depending on the epitope mapping results. The functional properties thereof are also compared.

Chinese hamster ovary (CHO) cell lines that express TMEM154 protein at the cell surface are developed and used to determine the specificity of the TMEM154 HuMAbs by flow cytometry. CHO cells are transfected with expression plasmids containing full length cDNA encoding transmembrane forms of TMEM154 antigen or a variant thereof. The transfected proteins contained an epitope tag at the N-terminus are used for detection by an antibody specific for the epitope. Binding of an anti-TMEM154 MAb is assessed by incubating the transfected cells with each of the TMEM154 antibodies at a concentration of 10 micro-grams/ml. The cells are washed and binding is detected with a FITC-labeled anti-human IgG Ab. A murine anti-epitope tag Ab, followed by labeled anti-murine IgG, is used as the positive control. Non-specific human and murine Abs are used as negative controls. The obtained data is used to assess the specificity of the HuMAbs for the TMEM154 antigen target.

These antibodies and other antibodies specific to TMEM154 polypeptides may be used in the afore-described anti-TMEM154 related therapies such as treatment of cancers wherein TMEM154 antigen is differentially expressed, such as ovarian cancer, lung cancer, breast cancer, kidney cancer, liver cancer, pancreatic cancer, prostate cancer, melanoma and hematological malignancies such as Multiple Myeloma, lymphoma, Non-Hodgkin's lymphoma, anti CD20 (i.e. Rituximab) resistant lymphoma, leukemia and T cell leukemia, involving the TMEM154 antigen, such as in the treatment of cancers and inflammatory or autoimmune diseases wherein such antibodies will e.g., prevent negative stimulation of T cell activity against desired target cancer cells or prevent the positive stimulation of T cell activity thereby eliciting a desired anti-autoimmune effect.

The invention has been described and prophetic embodiments provided relating to manufacture and selection of desired anti-TMEM154 antibodies for use as therapeutics and diagnostic methods wherein the disease or condition is associated with TMEM154 antigen.

## Claims

1. A polyclonal or monoclonal antibody or fragment that specifically binds to a TMEM154 polypeptide as represented by a part of SEQ ID NO: 42, namely SEQ ID NO: 191.

2. The antibody or fragment according to claim 1, wherein the antibody or fragment is selected from the group consisting of: a fully human antibody, a humanized or primatized antibody, a chimeric antibody, Fab, Fab', F(ab')2, F(ab'), F(ab), Fv or scFv fragment.

3. The antibody or fragment according to any one of claims 1 or 2, wherein the antibody or fragment is coupled to a detectable marker, or to an effector moiety.

4. The antibody or fragment according to claim 3, wherein the effector moiety is one or more of a radionuclide, fluorophore, an enzyme, a toxin, a therapeutic agent, a chemotherapeutic agent, a cytokine antibody, a cytokine receptor, or an immunomodulatory agent; or the detectable biomarker is one or more of a radioisotope, a metal chelator, an enzyme, a fluorescent compound, a bioluminescent compound or a chemiluminescent compound.

5. A pharmaceutical composition that comprises an antibody or a fragment according to any one of claims 1-4.

6. An antibody or a fragment according to any one of claims 1-4 for use in modulating the activity of the TMEM154 protein as represented by SEQ ID NO: 42 in the treatment of cancer or an immune related condition, wherein the immune related condition is selected from the group consisting of multiple sclerosis; psoriasis; rheumatoid arthritis; psoriatic arthritis, systemic lupus erythematosus; ulcerative colitis; Crohn's disease; benign lymphocytic angiitis, thrombocytopenic purpura, idiopathic thrombocytopenia, idiopathic autoimmune hemolytic anemia, pure red cell aplasia, Sjogren's syndrome, rheumatic disease, connective tissue disease, inflammatory rheumatism, degenerative rheumatism, extra-articular rheumatism, juvenile rheumatoid arthritis, arthritis uratica, muscular rheumatism, chronic polyarthritis, cryoglobulinemic vasculitis, ANCA-associated vasculitis, antiphospholipid syndrome, myasthenia gravis, autoimmune haemolytic anaemia, Guillian- Barre syndrome, chronic immune polyneuropathy, autoimmune thyroiditis, insulin dependent diabetes mellitus, type I diabetes, Addison's disease, membranous glomerulonephropathy, Goodpasture's disease, autoimmune gastritis, pernicious anaemia, pemphigus vulgarus, cirrhosis, primary biliary cirrhosis, dermatomyositis, polymyositis, fibromyositis, myogelosis, celiac disease, immunoglobulin A nephropathy, Henoch- Schonlein purpura, Evans syndrome, atopic dermatitis, psoriasis, psoriasis arthropathica, Graves' disease, Graves' ophthalmopathy, scleroderma, systemic scleroderma, asthma, allergy, primary biliary cirrhosis, Hashimoto's thyroiditis, primary myxedema, sympathetic ophthalmia, autoimmune uveitis, hepatitis, chronic action hepatitis, collagen diseases, ankylosing spondylitis, periarthritis humeroscapularis, panarteritis nodosa, chondrocalcinosis, Wegener's granulomatosis, microscopic polyangiitis, chronic urticaria, bullous skin disorders, pemphigoid, atopic eczema, Devic's disease, childhood autoimmune hemolytic anemia, Refractory or chronic Autoimmune Cytopenias, Acquired Hemophilia A, Cold Agglutinin Disease, Neuromyelitis Optica, Stiff Person Syndrome, gingivitis, periodontitis, pancreatitis, myocarditis, vasculitis, gastritis, gout, gouty arthritis, and inflammatory skin disorders, selected from the group consisting of psoriasis, atopic dermatitis, eczema, rosacea, urticaria, and acne, normocomplementemic urticarial vasculitis, pericarditis, myositis, antisynthetase syndrome, scleritis, macrophage activation syndrome, Bechet's Syndrome, PAPA Syndrome, Blau's Syndrome, gout, adult and juvenile Still's disease, cryropyrinopathy, Muckle- Wells syndrome, familial cold-induced auto-inflammatory syndrome, neonatal onset multisystemic inflammatory disease, familial Mediterranean fever, chronic infantile neurologic, cutaneous and articular syndrome, systemic juvenile idiopathic arthritis, Hyper IgD syndrome, Schnitzler's syndrome, and TNF receptor- associated periodic syndrome (TRAPS), immune disorders associated with graft transplantation rejection, such as acute and chronic rejection of organ transplantation, allogenic stem cell transplantation, autologous stem cell transplantation, bone marrow tranplantation, graft versus host disease, inflammatory bowel disease, Good pasture's syndrome, pernicious anemia, autoimmune atrophic gastritis, ulceratis colitis, mixed connective tissue disease, panarteritis nodosa, progressive systemic scleroderma, peptic ulcers, ulcers, chronic bronchitis, acute lung injury, pulmonary inflammation, airway hyper- responsiveness, septic shock, inflammatory skin disorders, myogelosis, chondrocalcinosis, thyroditis, allergic oedema, and granulomas.

7. The antibody of any of claims 1-4, the pharmaceutical composition according to claim 5 or the antibody for use of claim 6, for use in the treatment or prevention of cancer.

8. The antibody, pharmaceutical composition or the antibody for use of claim 7, wherein the cancer is selected from the group consisting of Non-Hodgkin's Lymphoma, Hodgkin's Lymphoma, and CD20 resistant lymphoma, Multiple Myeloma, kidney cancer and pancreatic cancer.

9. The antibody of any of claims 1-4, the pharmaceutical composition according to claim 5, or the antibody for use of claim 6 in combination with another medicament.

10. Use of an antibody or a fragment according to any one of claims 1-4 for diagnosing cancer, comprising detecting in a sample obtained from a subject the presence of a polypeptide and/or an over expressed level of said polypeptide having a sequence of the TMEM154 polypeptide, having an amino acid sequence consisting of SEQ ID NO: 42 or a fragment thereof, namely SEQ ID NO: 191; wherein said over expressed level is determined with regard to a normal level of said polypeptide in a corresponding normal tissue.

11. The use of claim 10 wherein said cancer is selected from the group consisting of Non-Hodgkin's Lymphoma, Hodgkin's Lymphoma, and CD20 resistant lymphoma, Multiple Myeloma, kidney cancer and pancreatic cancer.

12. The use of claims 10 or 11 wherein the detection is conducted by immunoassay.

13. An assay for detecting the presence of TMEM154 protein, represented by SEQ ID NO: 42, or a fragment thereof, namely SEQ ID NO: 191, in a biological sample, comprising contacting an isolated sample from a subject with an antibody or a fragment according to any of claims 1-4.

## Patentansprüche

1. Polyklonaler oder monoklonaler Antikörper oder Fragment, welcher bzw. welches spezifisch an ein TMEM154-Polypeptid wie durch einen Teil von SEQ ID Nr. 42, nämlich SEQ ID Nr. 191 dargestellt bindet.

2. Antikörper oder Fragment gemäß Anspruch 1, wobei der Antikörper oder das Fragment aus der Gruppe ausgewählt ist, die besteht aus: einem vollständig humanen Antikörper, einem humanisierten oder primatisierten Antikörper, einem chimären Antikörper, Fab-, Fab'-, F(ab')2-, F(ab')-, F(ab)-, Fv- oder scFv-Fragment.

3. Antikörper oder Fragment gemäß einem der Ansprüche 1 oder 2, wobei der Antikörper oder das Fragment an einen nachweisbaren Marker oder an eine Effektoreinheit gekuppelt ist.

4. Antikörper oder Fragment gemäß Anspruch 3, wobei die Effektoreinheit eine oder mehr von einem Radionuklid, Fluorophor, einem Enzym, einem Toxin, einem therapeutischen Mittel, einem chemotherapeutischen Mittel, einem Zytokin-Antikörper, einem Zytokin-Rezeptor oder einem immunomodulatorischen Mittel ist; oder der nachweisbare Biomarker einer oder mehr von einem Radioisotop, einem Metallchelator, einem Enzym, einer fluoreszenten Verbindung, einer biolumineszenten Verbindung oder einer chemilumineszenten Verbindung ist.

5. Pharmazeutische Zusammensetzung, welche einen Antikörper oder ein Fragment gemäß einem der Ansprüche 1-4 umfasst.

6. Antikörper oder Fragment gemäß einem der Ansprüche 1-4 zur Verwendung in der Modulierung der Aktivität des TMEM154-Proteins wie durch SEQ ID Nr. 42 dargestellt bei der Behandlung von Krebs oder einem mit dem Immunsystem in Verbindung stehenden Zustand, wobei der mit dem Immunsystem in Verbindung stehende Zustand aus der Gruppe ausgewählt ist, die aus multipler Sklerose; Psoriasis;
rheumatoider Arthritis; Arthritis psoriatica, systemischem Lupus erythematodes; Colitis ulcerosa; Morbus Crohn; benigner lymphozytärer Angiitis, thrombozytopenischer Purpura, idiopathischer Thrombozytopenie, idiopathischer autoimmunhämolytischer Anämie, aregenerativer Anämie, Sjögren-Syndrom, rheumatischer Erkrankung, Bindegewebserkrankung, rheumatischem Fieber (entzündlichem Rheumatismus), degenerativem Rheumatismus, extraartikulärem Rheumatismus, juveniler chronischer Polyarthritis (juveniler rheumatoider Arthritis), Arthritis uratica, Weichteilrheumatismus, chronischer Polyarthritis, Kryoglobulinämie-Vaskulitis, mit ANCA in Zusammenhang stehender Vaskulitis, Antiphospholipid-Syndrom, Myasthenia gravis, autoimmunhämolytischer Anämie, Guillian-Barre-Syndrom, chronischer Immunpolyneuropathie, Autoimmunthyroiditis, insulinabhängigem Diabetes mellitus, Diabetes Typ 1, Addison-Krankheit, membranöser Glomerulonephropathie, Goodpasture-Krankheit, Autoimmungastritis, perniziöser Anämie, Pemphigus vulgaris, Zirrhose, primärer biliärer Zirrhose, Dermatomyositis, Polymyositis, Fibromyositis, Myogelose, Zöliakie, Immunoglobulin A-Nephropathie, Purpura Schönlein-Henoch, Evans-Syndrom, atopischer Dermatitis, Psoriasis, Psoriasis arthropathica, Morbus Basedow, Graves-Ophthalmopathie, Skleroderm, systemischem Skleroderm, Asthma, Allergie, primärer biliärer Zirrhose, Hashimoto-Thyroiditis, primärem Myxödem, sympathischer Ophthalmie, Autoimmunuvcitis, Hepatitis, chronisch-aktiver Hepatitis, Kollagen-Erkrankungen, Morbus Bechterew, Periarthritis humeroscapularis, Panarteriitis nodosa, Chondrokalzinose, Wegener-Granulomatose, Mikropolyangiitis, chronischer Urtikaria, bullösen Hautstörungen, Pemphigoid, atopischem Ekzem, Devic-Krankheit, autoimmunhämolytischer Kindheitsanämie, refraktärer oder chronischer Autoimmunzytopenie, erworbener Hämophilie A, Kälteagglutinin-Krankheit, Neuromyelitis optica, Stiff-Person-Syndrom, Gingivitis, Periodontitis, Pankreatitis, Myokarditis, Vaskulitis, Gastritis, Gicht, Gichtarthritis besteht, und entzündlichen Hautstörungen, welche aus der Gruppe ausgewählt sind, die aus Psoriasis, atopischer Dermatitis, Ekzem, Rosazea, Urtikaria und Akne, normal komplementämischer Urtikaria-Vaskulitis, Perikarditis, Myositis, Antisynthetase-Syndrom, Skleritis, Makrophagenaktivierungssyndrom, Bechet-Syndrom, PAPA-Syndrom, Blau-Syndrom, Gicht, adulter und juveniler Still-Krankheit, Kryropyrinopathie, Muckle-Wells-Syndrom, familiärem durch Kälte hervorgerufenem Autoentzündungssyndrom, bei Neugeborenen beginnender multisystemischer Entzündungserkrankung, familiärem Mittelmeerfieher, chronischem infantilem neurologischem, kutanem und artikulärem Syndrom, systemischer juveniler idiopathischer Arthritis, Hyper IgD-Syndrom, Schnitzler-Syndrom und mit dem TNF-Rezeptor in Zusammenhang stehendem periodischem Syndrom (TRAPS), Immunstörungen in Zusammenhang mit Transplantatabstoßung, wie akute und chronische Abstoßung eines Organtransplantats, allogenen Stammzellentransplantats, autologen Stammzellentransplantats, Knochenmarktransplantats, Transplantat-Wirt-Reaktion, entzündlicher Darmerkrankung, Goodpasture-Syndrom, perniziöser Anämie, autoimmunatrophischer Gastritis, Colitis ulcerosa, gemischter Bindegewebserkrankung, Panarteriitis nodosa, progressiver systemischer Sklerodermie, Magengeschwüren, Geschwüren, chronischer Bronchitis, akuter Lungenverletzung, Lungenentzündung, Atemwegshyperreagibilität, septischem Schock, entzündlichen Hautstörungen, Myogelose, Chondrokalzinose, Thyroiditis, allergischem Ödem und Granuloma besteht.

7. Antikörper nach einem der Ansprüche 1-4, pharmazeutische Zusammensetzung gemäß Anspruch 5 oder Antikörper zur Verwendung nach Anspruch 6 zur Verwendung bei der Behandlung oder Vorbeugung von Krebs.

8. Antikörper, pharmazeutische Zusammensetzung oder Antikörper zur Verwendung nach Anspruch 7, wobei der Krebs aus der Gruppe ausgewählt ist, welche aus Non-Hodgkin-Lymphom, Hodgkin-Lymphom und CD20-resistentem Lymphom, multiplem Myelom, Nierenkrebs und Bauchspeicheldrüsenkrebs besteht.

9. Antikörper nach einem der Ansprüche 1-4, pharmazeutische Zusammensetzung gemäß Anspruch 5 oder Antikörper zur Verwendung nach Anspruch 6 in Kombination mit einem anderen Medikament.

10. Verwendung eines Antikörpers oder eines Fragments gemäß einem der Ansprüche 1-4 zur Diagnose von Krebs, umfassend Nachweisen in einer von einem Probanden erhaltenen Probe der Gegenwart eines Polypeptids und/oder einer überexprimierten Menge des Polypeptids mit einer Sequenz des TMEM154-Polypeptids mit einer Aminosäuresequenz, die aus SEQ ID Nr. 42 oder einem Fragment davon, nämlich SEQ ID Nr. 191 besteht; wobei die überexprimierte Menge bezüglich einer normalen Menge des Polypeptids in einem entsprechenden normalen Gewebe bestimmt wird.

11. Verwendung nach Anspruch 10, wobei der Krebs aus der Gruppe ausgewählt ist, welche aus Non-Hodgkin-Lymphom, Hodgkin-Lymphom und CD20-resistentem Lymphom, multiplem Myelom, Nierenkrebs und Bauchspeicheldrüsenkrebs besteht.

12. Verwendung nach den Ansprüchen 10 oder 11, wobei der Nachweis durch einen Immunassay durchgeführt wird.

13. Test zum Nachweisen der Gegenwart von TMEM154-Protein, dargestellt durch SEQ ID Nr. 42, oder ein Fragment davon, nämlich SEQ ID Nr. 191, in einer biologischen Probe, umfassend Inkontaktbringen einer isolierten Probe von einem Probanden mit einem Antikörper oder einem Fragment gemäß einem der Ansprüche 1-4.

## Revendications

1. Anticorps polyclonal ou monoclonal ou fragment qui se lie spécifiquement à un polypeptide TMEM154 tel que représenté par une partie de SEQ ID N° 42, à savoir SEQ ID N° 191.

2. Anticorps ou fragment selon la revendication 1, où l'anticorps ou le fragment est choisi dans le groupe constitué de : un anticorps totalement humain, un anticorps humanisé ou primatisé, un anticorps chimérique, un fragment Fab, Fab', F(ab')2, F(ab"), F(ab), Fv ou scFv.

3. Anticorps ou fragment selon l'une quelconque des revendications 1 ou 2, où l'anticorps ou le fragment est couplé à un marqueur détectable ou à un radical effecteur.

4. Anticorps ou fragment selon la revendication 3, où le radical effecteur est un ou plusieurs d'un radionucléide, d'un fluorophore, d'une enzyme, d'une toxine, d'un agent thérapeutique, d'un agent chimiothérapeutique, d'un anticorps de cytokine, d'un récepteur de cytokine, ou d'un agent immunomodulateur ; ou le biomarqueur détectable est un ou plusieurs d'un radio-isotope, d'un chélateur de métal, d'une enzyme, d'un composé fluorescent, d'un composé bioluminescent ou d'un composé chimioluminescent.

5. Composition pharmaceutique qui comprend un anticorps ou un fragment selon l'une quelconque des revendications 1 à 4.

6. Anticorps ou fragment selon l'une quelconque des revendications 1 à 4, pour une utilisation dans la modulation de l'activité de la protéine TMEM154 telle que représentée par SEQ TD N° 42 dans le traitement du cancer ou d'une affection liée au système immunitaire, où l'affection liée au système immunitaire est choisie dans le groupe constitué par la sclérose en plaques ; le psoriasis ; la polyarthrite rhumatoïde ; l'arthrite psoriasique ; le lupus érythémateux systémique ; la rectocolite hémorragique ; la maladie de Crohn ; l'angéite lymphocytaire bénigne, le purpura thrombocytopénique, la thrombocytopénie idiopathique, l'anémie hémolytique auto-immune idiopathique, l'érythroblastopénie, le syndrome de Sjögren, les maladies rhumatismales, les maladies du tissu conjonctif, le rhumatisme inflammatoire, le rhumatisme dégénératif, le rhumatisme extra-articulaire, la polyarthrite rhumatoïde juvénile, la goutte, le rhumatisme musculaire, la polyarthrite chronique, la vascularite cryoglobulinémique, la vascularite associée aux ANCA, le syndrome des antiphospholipides, la myasthénie grave, l'anémie hémolytique auto-immune, le syndrome de Guillain-Barré, la polyneuropathie auto-immune chronique, la thyroïdite auto-immune, le diabète insulinodépendant, le diabète de type 1, la maladie d'Addison, la glomérulonéphropathie membraneuse, la maladie de Goodpasture, la gastrite auto-immune, l'anémie pernicieuse, le pemphigus vulgaire, la cirrhose, la cirrhose biliaire primaire, la dermatomyosite, la polymyosite, la fibromyosite, la myogélose, la maladie coeliaque, la néphropathie à immunoglobulines A, le purpura de Henoch-Schonlein, le syndrome d'Evans, la dermatite atopique, le psoriasis, le psoriasis arthropathique, la maladie de Graves, l'ophtalmopathie de Graves, la sclérodermie, la sclérodermie systémique, l'asthme, l'allergie, la cirrhose biliaire primaire, la thyroïdite de Hashimoto, le myxoedème primaire, l'ophtalmie sympathique, l'uvéite auto-immune, l'hépatite, l'hépatite à action chronique, les maladies du collagène, la spondylarthrite ankylosante, la périarthrite scapulo-humérale, la périartérite noueuse, la chondrocalcinose, la granulomatose de Wegener, la polyangéite microscopique, l'urticaire chronique, les troubles cutanés bulleux, le pemphigoïde, l'eczéma atopique, la maladie de Devic, l'anémie hémolytique auto-immune de l'enfant, les cytopénies auto-immunes réfractaires ou chroniques, l'hémophilie A acquise, la maladie des agglutinines froides, la neuromyélite optique, le syndrome de Stiff Person, la gingivite, la parodontite, la pancréatite, la myocardite, la vascularite, la gastrite, la goutte, l'arthrite goutteuse, et les troubles cutanés inflammatoires, choisis dans le groupe constitué du psoriasis, la dermatite atopique, l'eczéma, la rosacée, l'urticaire, et l'acné, la vascularite urticarienne normocomplémentémique, la péricardite, la myosite, le syndrome des anti-synthétases, la sclérite, le syndrome d'activation des macrophages, le syndrome de Behçet, le syndrome PAPA, le syndrome de Blau, la goutte, la maladie de Still de l'adulte ou juvénile, la cryopyrinopathie, le syndrome de Muckle-Wells, le syndrome auto-inflammatoire familial au froid, la maladie inflammatoire multisystémique à installation néonatale, la fièvre méditerranéenne familiale, le syndrome neurologique, cutané et articulaire infantile chronique, l'arthrite idiopathique juvénile systémique, le syndrome des hyper-IgD, le syndrome de Schnitzler, et le syndrome périodique associé au récepteur du TNF (TRAPS), les troubles immunitaires associés au rejet d'une transplantation de greffe, comme le rejet aigu et chronique de transplantation d'organe, de transplantation de cellules souches allogéniques, de transplantation de cellules souches autologues, de transplantation de moelle osseuse, la maladie du greffon contre l'hôte, la maladie inflammatoire de l'intestin, le syndrome de Goodpasture, l'anémie pernicieuse, la gastrite atrophique auto-immune, la rectocolite hémorragique, la maladie mixte du tissu conjonctif, la périartérite noueuse, la sclérodermie systémique progressive, les ulcères peptiques, les ulcères, la bronchite chronique, la lésion pulmonaire aiguë, l'inflammation pulmonaire, l'hyperréactivité des voies respiratoires, le choc septique, les troubles cutanés inflammatoires, la myogélose, la chondrocalcinose, la thyroïdite, l'oedème allergique, et les granulomes.

7. Anticorps selon l'une quelconque des revendications 1 à 4, composition pharmaceutique selon la revendication 5 ou anticorps pour une utilisation selon la revendication 6, pour une utilisation dans le traitement ou la prévention du cancer.

8. Anticorps, composition pharmaceutique ou anticorps pour une utilisation selon la revendication 7, où le cancer est choisi dans le groupe constitué du lymphome non hodgkinien, le lymphome de Hodgkin, et le lymphome CD20-résistant, le myélome multiple, le cancer du rein et le cancer pancréatique.

9. Anticorps selon l'une quelconque des revendications 1 à 4, composition pharmaceutique selon la revendication 5, ou anticorps pour une utilisation selon la revendication 6 en combinaison avec un autre médicament.

10. Utilisation d'un anticorps ou d'un fragment selon l'une quelconque des revendications 1 à 4 pour le diagnostic d'un cancer, comprenant la détection dans un échantillon obtenu d'un sujet de la présence d'un polypeptide et/ou d'un taux surexprimé dudit polypeptide ayant une séquence du polypeptide TMEM154, ayant une séquence d'acides aminés constituée de SEQ ID N° 42 ou d'un fragment de celle-ci, à savoir SEQ ID N° 191 ; où ledit taux surexprimé est déterminé par rapport à un taux normal dudit polypeptide dans un tissu normal correspondant.

11. Utilisation selon la revendication 10, où ledit cancer est choisi dans le groupe constitué du lymphome non hodgkinien, le lymphome de Hodgkin, et le lymphome CD20-résistant, le myélome multiple, le cancer du rein et le cancer pancréatique.

12. Utilisation des revendications 10 ou 11, où la détection est conduite par un test immunologique.

13. Test pour la détection de la présence de la protéine TMEM154, représentée par SEQ ID N° 42, ou un fragment de celle-ci, à savoir SEQ ID N° 191, dans un échantillon biologique, comprenant la mise en contact d'un échantillon isolé d'un sujet avec un anticorps ou un fragment selon l'une quelconque des revendications 1 à 4.
